# EUROPEAN PATENT APPLICATION

(11) **EP 4 552 631 A2**
(43) Date of publication of application: **14.05.2025**
(21) Application number: 25153820.3
(22) Date of filing: 09.09.2019
(51) Int. Cl.: A61K 9/00

(54) **COMBINATION OF DASATINIB AND ADAGRASIB FOR USE IN THE TREATMENT OF NON-SMALL CELL LUNG CANCER**

(30) Priority: 10.09.2018 US 201862729169 P
(62) Divisional of application: 19858883.2
(71) Applicant: Mirati Therapeutics, Inc., Princeton, New Jersey 08543 (US)
(72) Inventor: ENGSTROM, Lars, Daniel, Carlsbad, 92009 (US); ARANDA, Ruth, Wei, San Diego, 92108 (US); OLSON, Peter, San Diego, 92122 (US); CHRISTENSEN, James, Gail, San Diego, 92130 (US)
(74) Representative: Mewburn Ellis LLP

(57) **Abstract**

The present invention relates to combination therapies for treating KRas G12C cancers. In particular, the present invention relates to methods of treating cancer in a subject in need thereof, comprising administering to the subject a therapeutically effective amount of a combination of a Src-family kinase inhibitor and a KRAS G12C inhibitor of Formula (I), Formula I-A or Formula I-B, pharmaceutical compositions comprising a therapeutically effective amounts of the inhibitors, kits comprising the compositions and methods of use therefor.

## Description

### FIELD OF THE INVENTION

The present invention relates to combination therapies useful for treating cancer. In particular, the present invention relates to therapeutically effective combinations of a Src-family kinase inhibitor and a KRas G12C inhibitor, pharmaceutical compositions comprising the inhibitors, kits comprising the compositions and methods of use therefor.

### BACKGROUND OF THE INVENTION

Kirsten Rat Sarcoma 2 Viral Oncogene Homolog ("KRas") is a small GTPase and a member of the Ras family of oncogenes. KRas serves as a molecular switch cycling between inactive (GDP-bound) and active (GTP-bound) states to transduce upstream cellular signals received from multiple tyrosine kinases to downstream effectors regulating a wide variety of processes, including cellular proliferation (e.g., see Alamgeer et al., (2013) Current Opin Pharmcol. 13:394-401).

The role of activated KRas in malignancy was observed over thirty years ago (e.g., see Santos et al., (1984) Science 223:661-664). Aberrant expression of KRas accounts for up to 20% of all cancers and oncogenic KRas mutations that stabilize GTP binding and lead to constitutive activation of KRas and downstream signaling have been reported in 25 -30% of lung adenocarcinomas. (e.g., see Samatar and Poulikakos (2014) Nat Rev Drug Disc 13(12): 928-942 doi: 10.1038/nrd428). Single nucleotide substitutions that result in missense mutations at codons 12 and 13 of the KRas primary amino acid sequence comprise approximately 40% of these KRas driver mutations in lung adenocarcinoma, with a G12C transversion being the most common activating mutation (e.g., see Dogan et al., (2012) Clin Cancer Res. 18(22):6169-6177, published online 2012 Sep 26. doi: 10.1158/1078-0432.CCR-11-3265).

The well-known role of KRas in malignancy and the discovery of these frequent mutations in KRas in various tumor types made KRas a highly attractable target of the pharmaceutical industry for cancer therapy. Notwithstanding thirty years of large-scale discovery efforts to develop inhibitors of KRas for treating cancer, no KRas inhibitor to date has demonstrated sufficient safety and/or efficacy to obtain regulatory approval (e.g., see McCormick (2015) Clin Cancer Res. 21 (8):1797-1801).

Compounds that inhibit KRas activity are highly desirable and under investigation, including those that disrupt effectors such as guanine nucleotide exchange factors (e.g., see Sun et al., (2012) Agnew Chem Int Ed Engl. 51(25):6140-6143 doi: 10.1002/anie201201358) as well as those that target KRas G12C (e.g., see Ostrem et al., (2013) Nature 503:548-551). Clearly there remains a continued interest and effort to develop inhibitors of KRas, particularly inhibitors of activating KRas mutants, including KRas G12C.

While the KRas G12C inhibitors disclosed herein are potent inhibitors of KRas G12C enzymatic activity and exhibit single agent activity inhibiting the in vitro proliferation of cell lines harboring a KRas G12C mutation, the relative potency and/or observed maximal effect of any given KRas G12C inhibitor can vary between KRAS mutant cell lines. The reason or reasons for the range of potencies and observed maximal effect is not fully understood but certain cell lines appear to possess differing intrinsic resistance. Thus, there is a need to develop alternative approaches to maximize the potency, efficacy, therapeutic index and/or clinical benefit of KRas G12C inhibitors in vitro and in vivo.

The combination therapy of the present invention, in one aspect, synergistically increases the potency of KRas G12C inhibitors resulting in improved efficacy and therapeutic index of KRas G12C inhibitors disclosed herein. The combination therapy of the present invention, in another aspect, provides improved clinical benefit to patients compared to treatment with KRas G12C inhibitors disclosed herein as a single agent.

### SUMMARY OF THE INVENTION

In one aspect of the invention, provided herein are methods of treating cancer in a subject in need thereof, comprising administering to the subject a therapeutically effective amount of a combination of a Src-family kinase inhibitor and a KRAS G12C inhibitor of formula (I): 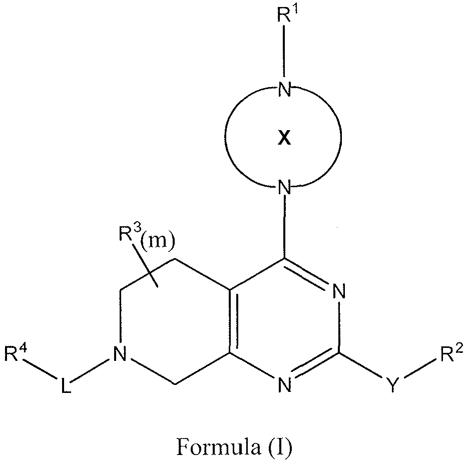 or a pharmaceutically acceptable salt thereof, wherein:
X is a 4-12 membered saturated or partially saturated monocyclic, bridged or spirocyclic ring, wherein the saturated or partially saturated monocyclic ring is optionally substituted with one or more R⁸;
Y is a bond, O, S or NR⁵;
R¹ is -C(O)C(R^{A}) 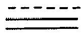 C(R^{B})ₚ or -SO₂C(R^{A}) 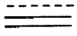 C(R^{B})p;
R² is hydrogen, alkyl, hydroxyalkyl, dihydroxyalkyl, alkylaminylalkyl, dialkylaminylalkyl, -Z-NR⁵R¹⁰, heterocyclyl, heterocyclylalkyl, aryl, heteroaryl, or heteroarylalkyl, wherein each of the Z, heterocyclyl, heterocyclylalkyl, aryl, heteroaryl, and heteroarylalkyl may be optionally substituted with one or more R⁹;
Z is C1 - C4 alkylene;
each R³ is independently C1 - C3 alkyl, oxo, or haloalkyl;
L is a bond, -C(O)-, or C1 - C3 alkylene;
R⁴ is hydrogen, cycloalkyl, heterocyclyl, aryl, aralkyl or heteroaryl, wherein each of the cycloalkyl, heterocyclyl, aryl, aralkyl and heteroaryl may be optionally substituted with one or more R⁶ or R⁷;
each R⁵ is independently hydrogen or C1 - C3 alkyl;
R⁶ is cycloalkyl, heterocyclyl, heterocyclylalkyl, aryl, or heteroaryl, wherein each of the cycloalkyl, heterocyclyl, aryl, or heteroaryl may be optionally substituted with one or more R⁷;
each R⁷ is independently halogen, hydroxyl, C1 - C6 alkyl, cycloalkyl, alkoxy, haloalkyl, amino, cyano, heteroalkyl, hydroxyalkyl or Q-haloalkyl, wherein Q is O or S;
R⁸ is oxo, C1 - C3 alkyl, C2 - C4 alkynyl, heteroalkyl, cyano, -C(O)OR⁵, -C(O)N(R⁵)₂, - N(R⁵)₂, wherein the C1 - C3 alkyl may be optionally substituted with cyano, halogen, -OR⁵, - N(R⁵)₂, or heteroaryl
each R⁹ is independently hydrogen, oxo, acyl, hydroxyl, hydroxyalkyl, cyano, halogen, C1 - C6 alkyl, aralkyl, haloalkyl, heteroalkyl, cycloalkyl, heterocyclylalkyl, alkoxy, dialkylaminyl, dialkylamidoalkyl, or dialkylaminylalkyl, wherein the C1 - C6 alkyl may be optionally substituted with cycloalkyl;
each R¹⁰ is independently hydrogen, acyl, C1 - C3 alkyl, heteroalkyl or hydroxyalkyl;
R¹¹ is haloalkyl;
R^{A} is absent, hydrogen, deuterium, cyano, halogen, C1 - C-3 alkyl, haloalkyl, heteroalkyl, -C(O)N(R⁵)₂, or hydroxyalkyl;
each R^{B} is independently hydrogen, deuterium, cyano, C1 - C3 alkyl, hydroxyalkyl, heteroalkyl, C1 - C3 alkoxy, halogen, haloalkyl, -ZNR⁵R¹¹, -C(O)N(R⁵)₂, -NHC(O)C1 - C3 alkyl, -CH₂NHC(O)C1 - C3 alkyl, heteroaryl, heteroarylalkyl, dialkylaminylalkyl, or heterocyclylalkyl wherein the heterocyclyl portion is substituted with one or more substituents independently selected from halogen, hydroxyl, alkoxy and C1 - C3 alkyl, wherein the heteroaryl or the heteroaryl portion of the heteroarylalkyl is optionally substituted with one or more R⁷;
m is zero or an integer between 1 and 2;
p is one or two; and wherein,
when is a triple bond then R^{A} is absent, R^{B} is present and p equals one,
or when 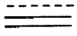 is a double bond then R^{A} is present, R^{B} is present and p equals two, or R^{A}, R^{B} and the carbon atoms to which they are attached form a 5-8 membered partially saturated cycloalkyl optionally substituted with one or more R⁷.

Also included for use in the methods provided herein are KRas G12C inhibitor compounds of Formula I having the Formula I-A: 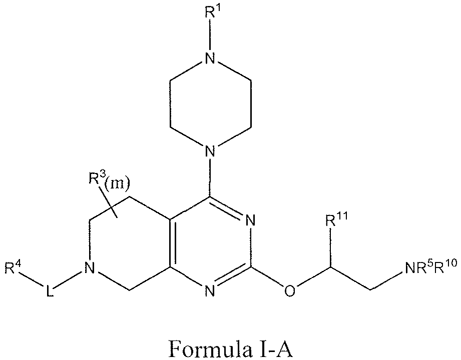 and pharmaceutically acceptable salts thereof, wherein R¹, R³, R⁴, R⁵, R¹⁰, R¹¹, L and m are as defined for Formula I, and the piperazinyl ring is optionally substituted with R⁸ wherein R⁸ is as defined for Formula I.

Also included for use in the methods provided herein are KRas G12C inhibitor compounds of Formula I having the Formula I-B: 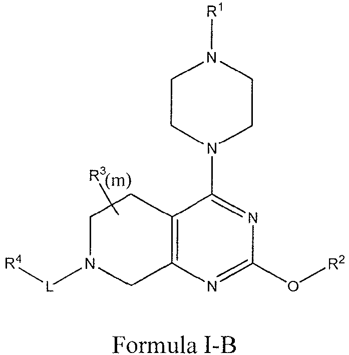 and pharmaceutically acceptable salts thereof, where R¹, R³, R⁴, L and m are as defined for Formula I, R² is heterocyclylalkyl optionally substituted with one or more R⁹ where R⁹ is as defined for Formula I, and the piperazinyl ring is optionally substituted with R⁸, where R⁸ is as defined for Formula I.

In another aspect of the invention, pharmaceutical compositions are provided for use in the methods comprising a therapeutically effective amount of a combination of a Src-family kinase inhibitor and a KRas G12C inhibitor compound Formula I, Formula I-A, or Formula 1-B, or a pharmaceutically acceptable salt thereof and a pharmaceutically acceptable excipient.

In one aspect of the invention, provided herein are methods of treating cancer in a subject in need thereof, comprising administering to the subject a therapeutically effective amount of a combination of a Src-family kinase inhibitor or a pharmaceutically acceptable salt or pharmaceutical composition thereof and a KRAS G12C inhibitor of Formula (I), Formula I-A or Formula I-B, or a pharmaceutically acceptable salt or pharmaceutical composition thereof. In one embodiment, the cancer is a KRas G12C-associated cancer. In one embodiment, the KRas G12C-associated cancer is lung cancer.

In some aspects of the invention, KRas G12C inhibitor compounds and Src-family kinase inhibitors are the only active agents in the provided combinations and methods.

Examples of Src-family kinase inhibitors suitable for the provided compositions and methods include, but are not limited to, dasatinib, ponatinib, bosutinib, vandetanib, KX-01 and saracatinib.

In yet another aspect, the invention provides for methods for increasing the sensitivity of a cancer cell to a KRas G12C inhibitor, comprising contacting the cancer cell with a therapeutically effective amount of a combination of a KRas G12C inhibitor compound of Formula (I), Formula I-A, or Formula I-B, or a pharmaceutically acceptable salt or pharmaceutical composition thereof and a Src-family kinase inhibitor or a pharmaceutically acceptable salt or pharmaceutical composition thereof, wherein the Src-family kinase inhibitor synergistically increases the sensitivity of the cancer cell to the KRas G12C inhibitor. In one embodiment, the contacting is in vitro. In one embodiment, the contacting is in vivo.

Also provided herein are methods for treating cancer in a subject in need thereof, the method comprising (a) determining that cancer is associated with a KRas G12C mutation (e.g., a KRas G12C-associated cancer) (e.g., as determined using a regulatory agency-approved, e.g., FDA-approved, assay or kit); and (b) administering to the patient a therapeutically effective amount of a combination of a Src-family kinase inhibitor or a pharmaceutically acceptable salt or pharmaceutical composition thereof and a KRas G12C inhibitor compound of Formula I, Formula I-A, Formula 1-B or a pharmaceutically acceptable salt or pharmaceutical composition thereof, wherein the Src-family kinase inhibitor synergistically increases the sensitivity of the KRas G12C-associated cancer to the KRas G12C inhibitor.

Also provided herein are kits comprising a Src-family kinase inhibitor or a pharmaceutically acceptable salt or pharmaceutical composition thereof and a KRas G12C inhibitor compound of Formula (I), Formula I-A or Formula I-B or a pharmaceutically acceptable salt or pharmaceutical composition thereof. Also provided is a kit comprising a Src-family kinase inhibitor or a pharmaceutically acceptable salt or pharmaceutical composition thereof and a KRas G12C inhibitor compound of Formula (I), Formula I-A or Formula I-B or a pharmaceutically acceptable salt or pharmaceutical composition thereof, for use in treating a KRas G12C cancer.

In a related aspect, the invention provides a kit containing a dose of a Src-family kinase inhibitor or a pharmaceutically acceptable salt or pharmaceutical composition thereof and a KRas G12C inhibitor compound of Formula (I), Formula I-A or Formula I-B or a pharmaceutically acceptable salt or pharmaceutical composition thereof in an amount effective to inhibit proliferation of cancer cells in a subject. The kit in some cases includes an insert with instructions for administration of a Src-family kinase inhibitor and a KRas G12C inhibitor compound of Formula (I), Formula I-A or Formula I-B. The insert may provide a user with one set of instructions for using the a Src-family kinase inhibitor in combination with a KRas G12C inhibitor compound of Formula (I), Formula I-A or Formula I-B or a pharmaceutically acceptable salts thereof.

In some embodiments of any of the methods described herein, before treatment with the compositions or methods of the invention, the patient was treated with one or more of a chemotherapy, a targeted anticancer agent, radiation therapy, and surgery, and optionally, the prior treatment was unsuccessful; and/or the patient has been administered surgery and optionally, the surgery was unsuccessful; and/or the patient has been treated with a platinum-based chemotherapeutic agent, and optionally, the patient has been previously determined to be non-responsive to treatment with the platinum-based chemotherapeutic agent; and/or the patient has been treated with a kinase inhibitor, and optionally, the prior treatment with the kinase inhibitor was unsuccessful; and/or the patient was treated with one or more other therapeutic agent(s).

### DETAILED DESCRIPTION OF THE INVENTION

The present invention relates to combination therapies for treating KRas G12C cancers. In particular, the present invention relates to methods of treating cancer in a subject in need thereof, comprising administering to the subject a therapeutically effective amount of a combination of a Src-family kinase inhibitor or a pharmaceutically acceptable salt or pharmaceutical composition thereof and a KRAS G12C inhibitor of Formula (I), Formula I-A or Formula I-B or a pharmaceutically acceptable salt or pharmaceutical composition thereof, pharmaceutical compositions each separately comprising a therapeutically effective amount of the inhibitors, kits comprising the compositions and methods of use therefor.

Combinations of a Src-family kinase inhibitor with a KRas G12C inhibitor compound of Formula (I), Formula I-A or Formula I-B or pharmaceutically acceptable salt thereof synergistically increase the potency of the KRas G12C inhibitor compounds of Formula (I), Formula I-A or Formula I-B or pharmaceutically acceptable salts thereof against cancer cells that express KRas G12C thereby increasing the efficacy of the KRas G12C inhibitor compounds of Formula (I), Formula I-A or Formula I-B and pharmaceutically acceptable salts thereof.

### DEFINITIONS

Unless defined otherwise, all technical and scientific terms used herein have the same meaning as is commonly understood by one of skill in the art to which this invention belongs. All patents, patent applications, and publications referred to herein are incorporated by reference.

As used herein, "KRas G12C" refers to a mutant form of a mammalian KRas protein that contains an amino acid substitution of a cysteine for a glycine at amino acid position 12. The assignment of amino acid codon and residue positions for human KRas is based on the amino acid sequence identified by UniProtKB/Swiss-Prot P01116: Variant p.Gly12Cys.

As used herein, a "KRas G12C inhibitor" refers to compounds of the present invention that are represented by Formula (I), Formula I-A and Formula I-B and pharmaceutically acceptable salts thereof as described herein. These compounds are capable of negatively modulating or inhibiting all or a portion of the enzymatic activity of KRas G12C. The KRas G12C inhibitors of the present invention interact with and irreversibly bind to KRas G12C by forming a covalent adduct with the sulfhydryl side chain of the cysteine residue at position 12 resulting in the inhibition of the enzymatic activity of KRas G12C. In one embodiment, the KRas G12C inhibitor is a compound selected from compound Nos. 1-527, or a pharmaceutically acceptable salt thereof (e.g., Example No. 234, 359, 478 or 507 or a pharmaceutically acceptable salt thereof).

A "KRas G12C-associated disease or disorder" as used herein refers to diseases or disorders associated with or mediated by or having a KRas G12C mutation. A non-limiting example of a KRas G12C-associated disease or disorder is a KRas G12C-associated cancer.

As used herein, a "Src-family kinase" refers to a member of a mammalian nonreceptor tyrosine kinase family including: Src, Yes, Fyn and Fgr (SrcA subfamily); Lck, Hck, Blk and Lyn (SrcB subfamily), and Frk subfamily.

As used herein, a "Src-family kinase inhibitor" refers to a compound that is capable of negatively modulating or inhibiting all or a portion of the enzymatic activity of one or more member of the Src-family kinases.

As used herein, the term "subject," "individual," or "patient," used interchangeably, refers to any animal, including mammals such as mice, rats, other rodents, rabbits, dogs, cats, swine, cattle, sheep, horses, primates, and humans. In some embodiments, the patient is a human. In some embodiments, the subject has experienced and/or exhibited at least one symptom of the disease or disorder to be treated and/or prevented. In some embodiments, the subject has been identified or diagnosed as having a cancer having a KRas G12C mutation (e.g., as determined using a regulatory agency-approved, e.g., FDA-approved, assay or kit). In some embodiments, the subject has a tumor that is positive for a KRas G12C mutation (e.g., as determined using a regulatory agency-approved assay or kit). The subject can be a subject with a tumor(s) that is positive for a KRas G12C mutation (e.g., identified as positive using a regulatory agency-approved, e.g., FDA-approved, assay or kit). The subject can be a subject whose tumors have a KRas G12C mutation (e.g., where the tumor is identified as such using a regulatory agency-approved, e.g., FDA-approved, kit or assay). In some embodiments, the subject is suspected of having a KRas G12C gene-associated cancer. In some embodiments, the subject has a clinical record indicating that the subject has a tumor that has a KRas G12C mutation (and optionally the clinical record indicates that the subject should be treated with any of the compositions provided herein).

The term "pediatric patient" as used herein refers to a patient under the age of 16 years at the time of diagnosis or treatment. The term "pediatric" can be further be divided into various subpopulations including: neonates (from birth through the first month of life); infants (1 month up to two years of age); children (two years of age up to 12 years of age); and adolescents (12 years of age through 21 years of age (up to, but not including, the twenty-second birthday)). Berhman RE, Kliegman R, Arvin AM, Nelson WE. Nelson Textbook of Pediatrics, 15th Ed. Philadelphia: W.B. Saunders Company, 1996; Rudolph AM, et al. Rudolph's Pediatrics, 21st Ed. New York: McGraw-Hill, 2002; and Avery MD, First LR. Pediatric Medicine, 2nd Ed. Baltimore: Williams & Wilkins; 1994.

In some embodiments of any of the methods or uses described herein, an assay is used to determine whether the patient has KRas G12C mutation using a sample (e.g., a biological sample or a biopsy sample such as a paraffin-embedded biopsy sample) from a patient (e.g., a patient suspected of having a KRas G12C-associated cancer, a patient having one or more symptoms of a KRas G12C-associated cancer, and/or a patient that has an increased risk of developing a KRas G12C-associated cancer) can include, for example, next generation sequencing, immunohistochemistry, fluorescence microscopy, break apart FISH analysis, Southern blotting, Western blotting, FACS analysis, Northern blotting, and PCR-based amplification (e.g., RT-PCR, quantitative real-time RT-PCR, allele-specific genotyping or ddPCR). As is well-known in the art, the assays are typically performed, e.g., with at least one labelled nucleic acid probe or at least one labelled antibody or antigen-binding fragment thereof.

The term "regulatory agency" is a country's agency for the approval of the medical use of pharmaceutical agents with the country. For example, a non-limiting example of a regulatory agency is the U.S. Food and Drug Administration (FDA).

The term "amino" refers to -NH₂;

The term "acyl" refers to -C(O)CH₃.

The term "alkyl" as employed herein refers to straight and branched chain aliphatic groups having from 1 to 12 carbon atoms, 1-8 carbon atoms 1-6 carbon atoms, or 1-3 carbon atoms which is optionally substituted with one, two or three substituents. Examples of alkyl groups include, without limitation, methyl, ethyl, propyl, isopropyl, butyl, isobutyl, sec-butyl, tert-butyl, pentyl, and hexyl.

The term "haloalkyl" refers to an alkyl chain in which one or more hydrogen has been replaced by a halogen. Examples of haloalkyls are trifluoromethyl, difluoromethyl and fluoromethyl.

The term "haloalkyloxy" refers to -O-haloalkyl.

An "alkylene," group is an alkyl group, as defined hereinabove, that is positioned between and serves to connect two other chemical groups. Exemplary alkylene groups include, without limitation, methylene, ethylene, propylene, and butylene.

The term "alkoxy" refers to -OC1 - C6 alkyl.

The term "cycloalkyl" as employed herein includes saturated and partially unsaturated cyclic hydrocarbon groups having 3 to 12 carbons, for example 3 to 8 carbons, and as a further example 3 to 6 carbons, wherein the cycloalkyl group additionally is optionally substituted. Examples of cycloalkyl groups include, without limitation, cyclopropyl, cyclobutyl, cyclopentyl, cyclopentenyl, cyclohexyl, cyclohexenyl, cycloheptyl, and cyclooctyl.

The term "heteroalkyl" refers to an alkyl group, as defined hereinabove, wherein one or more carbon atoms in the chain are replaced by a heteroatom selected from the group consisting of O, S, and N.

As used herein, the term "hydroxyalkyl" refers to -alkyl-OH.

The term "dihydroxyalkyl" refers to an alkyl group as defined herein wherein two carbon atoms are each substituted with a hydroxyl group.

The term "alkylaminyl" refers to -NR^{x}-alkyl, wherein R^{x} is hydrogen. In one embodiment, R^{x} is hydrogen.

The term "dialkylaminyl" refers to -N(R^{y})₂, wherein each R^{y} is C1 - C3 alkyl.

The term "alkylaminylalkyl" refers to -alkyl-NR^{x}-alkyl, wherein R^{x} is hydrogen. In one embodiment, R^{x} is hydrogen.

The term "dialkylaminylalkyl" refers to -alkyl-N(R^{y})₂, wherein each R^{y} is C1 - C4 alkyl, wherein the alkyl of the--alkyl-N(R^{y})₂ may be optionally substituted with hydroxy or hydroxyalkyl.

An "aryl" group is a C₆-C₁₄ aromatic moiety comprising one to three aromatic rings, which is optionally substituted. As one embodiment, the aryl group is a C₆-C₁₀ aryl group. Examples of aryl groups include, without limitation, phenyl, naphthyl, anthracenyl, fluorenyl, and dihydrobenzofuranyl.

An "aralkyl" or "arylalkyl" group comprises an aryl group covalently linked to an alkyl group, either of which may independently be optionally substituted or unsubstituted. An example of an aralkyl group is (C₁- C₆)alkyl(C₆-C₁₀)aryl, including, without limitation, benzyl, phenethyl, and naphthylmethyl. An example of a substituted aralkyl is wherein the alkyl group is substituted with hydroxyalkyl.

A "heterocyclyl" or "heterocyclic" group is a ring structure having from about 3 to about 12 atoms, for example 4 to 8 atoms, wherein one or more atoms are selected from the group consisting of N, O, and S, the remainder of the ring atoms being carbon. The heterocyclyl may be a monocyclic, a bicyclic, a spirocyclic or a bridged ring system. The heterocyclic group is optionally substituted with R⁷ on carbon or nitrogen at one or more positions, wherein R⁷ is as defined for Formula I. The heterocyclic group is also independently optionally substituted on nitrogen with alkyl, aryl, aralkyl, alkylcarbonyl, alkylsulfonyl, arylcarbonyl, arylsulfonyl, alkoxycarbonyl, aralkoxycarbonyl, or on sulfur with oxo or lower alkyl. Examples of heterocyclic groups include, without limitation, epoxy, azetidinyl, aziridinyl, tetrahydrofuranyl, tetrahydropyranyl, pyrrolidinyl, pyrrolidinonyl, piperidinyl, piperazinyl, imidazolidinyl, thiazolidinyl, dithianyl, trithianyl, dioxolanyl, oxazolidinyl, oxazolidinonyl, decahydroquinolinyl, piperidonyl, 4-piperidinonyl, thiomorpholinyl, thiomorpholinyl 1,1 dioxide, morpholinyl, oxazepanyl, azabicyclohexanes, azabicycloheptanes and oxa azabiocycloheptanes. Specifically excluded from the scope of this term are compounds having adjacent annular O and/or S atoms.

The term "heterocyclylalkyl" refers to a heterocyclyl group as defined herein linked to the remaining portion of the molecule via an alkyl linker, wherein the alkyl linker of the heterocyclylalkyl may be optionally substituted with hydroxy or hydroxyalkyl.

As used herein, the term "heteroaryl" refers to groups having 5 to 14 ring atoms, preferably 5, 6, 9, or 10 ring atoms; having 6, 10, or 14 π electrons shared in a cyclic array; and having, in addition to carbon atoms, from one to three heteroatoms per ring selected from the group consisting of N, O, and S. Examples of heteroaryl groups include acridinyl, azocinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, benzimidazolinyl, carbazolyl, 4aH-carbazolyl, carbolinyl, chromanyl, chromenyl, cinnolinyl, furanyl, furazanyl, imidazolinyl, imidazolyl, 1H-indazolyl, indolenyl, indolinyl, indolizinyl, indolyl, 3H-indolyl, isobenzofuranyl, isochromanyl, isoindazolyl, isoindolinyl, isoindolyl, isoquinolinyl, isothiazolyl, isoxazolyl, methylenedioxyphenyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolidinyl, pyrimidinyl, phenanthridinyl, phenanthrolinyl, phenazinyl, phenothiazinyl, phenoxathiinyl, phenoxazinyl, phthalazinyl, piperonyl, pteridinyl, purinyl, pyranyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolinyl, 2H-pyrrolyl, pyrrolyl, quinazolinyl, quinolinyl, 4H-quinolizinyl, quinoxalinyl, quinuclidinyl, tetrahydroisoquinolinyl, tetrahydroquinolinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, and xanthenyl.

A "heteroarylalkyl" group comprises a heteroaryl group covalently linked to an alkyl group, wherein the radical is on the alkyl group, either of which is independently optionally substituted or unsubstituted. Examples of heteroarylalkyl groups include a heteroaryl group having 5, 6, 9, or 10 ring atoms bonded to a C1-C6 alkyl group. Examples of heteroaralkyl groups include pyridylmethyl, pyridylethyl, pyrrolylmethyl, pyrrolylethyl, imidazolylmethyl, imidazolylethyl, thiazolylmethyl, thiazolylethyl, benzimidazolylmethyl, benzimidazolylethyl quinazolinylmethyl, quinolinylmethyl, quinolinylethyl, benzofuranylmethyl, indolinylethyl isoquinolinylmethyl, isoinodylmethyl, cinnolinylmethyl, and benzothiophenylethyl. Specifically excluded from the scope of this term are compounds having adjacent annular O and/or S atoms.

As used herein, "an effective amount" of a compound is an amount that is sufficient to negatively modulate or inhibit the activity of the desired target, i.e., a Src-family kinase or KRas G12C. Such amount may be administered as a single dosage or may be administered according to a regimen, whereby it is effective.

As used herein, a "therapeutically effective amount" of a compound is an amount that is sufficient to ameliorate, or in some manner reduce a symptom or stop or reverse progression of a condition, or negatively modulate or inhibit the activity of Src-family kinases or KRas G12C. Such amount may be administered as a single dosage or may be administered according to a regimen, whereby it is effective.

As used herein, a "therapeutically effective amount of a combination" of two compounds is an amount that together synergistically increases the activity of the combination in comparison to the therapeutically effective amount of each compound in the combination, i.e., more than merely additive. Alternatively, in vivo, the therapeutically effective amount of the combination of a Src-family kinase inhibitor and a KRas G12C inhibitor compound of Formula (I), Formula I-A, or Formula I-B, or pharmaceutically acceptable salts or pharmaceutical compositions thereof, results in an increased duration of overall survival ("OS") in subjects relative to treatment with only the KRas G12C inhibitor. In one embodiment, the therapeutically effective amount of the combination of a Src-family kinase inhibitor and a KRas G12C inhibitor compound of Formula (I), Formula I-A, or Formula I-B, or a pharmaceutically acceptable salt or pharmaceutical composition thereof, results in an increased duration of progression-free survival ("PFS") in subjects relative to treatment with only the KRas G12C inhibitor. In one embodiment, the therapeutically effective amount of the combination of a Src-family kinase inhibitor and a KRas G12C inhibitor compound of Formula (I), Formula I-A, or Formula I-B, or a pharmaceutically acceptable salt or pharmaceutical composition thereof, results in increased tumor regression in subjects relative to treatment with only the KRas G12C inhibitor. In one embodiment, the therapeutically effective amount of the combination of a Src-family kinase inhibitor and a KRas G12C inhibitor compound of Formula (I), Formula I-A, or Formula I-B, or a pharmaceutically acceptable salt or pharmaceutical composition thereof, results in increased tumor growth inhibition in subjects relative to treatment with only the KRas G12C inhibitor. In one embodiment, the therapeutically effective amount of the combination of a Src-family kinase inhibitor and a KRas G12C inhibitor compound of Formula (I), Formula I-A, or Formula I-B, or a pharmaceutically acceptable salt or pharmaceutical composition thereof, results in an improvement in the duration of stable disease in subjects compared to treatment with only the KRas G12C inhibitor. The amount of each compound in the combination may be the same or different than the therapeutically effective amount of each compound when administered alone as a monotherapy as long as the combination is synergistic. Such amounts may be administered as a single dosage or may be administered according to a regimen, whereby it is effective.

As used herein, treatment means any manner in which the symptoms or pathology of a condition, disorder or disease are ameliorated or otherwise beneficially altered. Treatment also encompasses any pharmaceutical use of the compositions herein.

As used herein, amelioration of the symptoms of a particular disorder by administration of a particular pharmaceutical composition refers to any lessening, whether permanent or temporary, lasting or transient that can be attributed to or associated with administration of the composition.

As used herein, the term "about" when used to modify a numerically defined parameter (e.g., the dose of a KRAS inhibitor or a Src inhibitor or a pharmaceutically acceptable salt thereof, or the length of treatment time with a combination therapy described herein) means that the parameter may vary by as much as 10% below or above the stated numerical value for that parameter. For example, a dose of about 5 mg/kg may vary between 4.5 mg/kg and 5.5 mg/kg. "About" when used at the beginning of a listing of parameters is meant to modify each parameter. For example, about 0.5 mg, 0.75 mg or 1.0 mg means about 0.5 mg, about 0.75 mg or about 1.0 mg. Likewise, about 5% or more, 10% or more, 15% or more, 20% or more, and 25% or more means about 5% or more, about 10% or more, about 15% or more, about 20% or more, and about 25% or more.

### INHIBITOR COMPOUNDS

In one aspect of the invention, provided herein are methods of treating cancer in a subject in need thereof, comprising administering to the subject a therapeutically effective amount of a combination of a Src-family kinase inhibitor and a KRAS G12C inhibitor of Formula (I), Formula I-A or Formula I-B or a pharmaceutically acceptable salt thereof.

### 1. Src-family Kinase Inhibitors

The Src-family of kinases is composed of nonreceptor tyrosine kinases with key roles in regulating signal transduction pathways that are involved in the regulation of cell proliferation and survival. All Src-family kinases share common functional domains including an N-terminal myristoylation sequence for membrane targeting, SH3 (Src homology domain 3), SH2, and SH1 (protein kinase) domains. The Src-family of kinases includes nine members: Src, Yes, Fyn and Fgr forming the SrcA subfamily; Lck, Hck, Blk and Lyn in the SrcB subfamily, and Frk in its own subfamily. Several Src-family kinases are ubiquitously expressed in all tissue types whereas others are expressed in only a subset of tissue types (e.g., Brown, M.T. and Cooper, J.A. (1996) Biochim. Biophys. Acta. 1287, 121-149).

Src-family kinases have been reported to be activated and/or overexpressed in various cancers (Halpern et al., Proc Natl Acad Sd USA. 1996 93(2): 824-827) and, as such, have been a promising target for anti-cancer therapies. Several inhibitors having activity against Src-family kinases have been developed and a number have received marketing approval, particularly for hematological cancers.

Exemplary inhibitors of Src-family kinases useful in the methods disclosed herein include, but are not limited to: Dasatinib (N-(2-chloro-6-methylphenyl)-2-((6-(4-(2-hydroxyethyl)piperazin-1-yl)-2-methylpyrimidin-4-yl)amino)thiazole-5-carboxamide); Ponatinib (3-(imidazo[1,2-b]pyridazin-3-ylethynyl)-4-methyl-N-(4-((4-methylpiperazin-1-yl)methyl)-3-(trifluoromethyl)phenyl)benzamide); Vandetanib (N-(4-bromo-2-fluorophenyl)-6-methoxy-7-((1-methylpiperidin-4-yl)methoxy)quinazolin-4-amine); Bosutinib (4-((2,4-dichloro-5-methoxyphenyl)amino)-6-methoxy-7-(3-(4-methylpiperazin-1-yl)propoxy)quinoline-3-carbonitrile); Saracatinib (N-(5-chlorobenzo[d][1,3]dioxol-4-yl)-7-(2-(4-methylpiperazin-1-yl)ethoxy)-5-((tetrahydro-2H-pyran-4-yl)oxy)quinazolin-4-amine); KX2-391 (N-benzyl-2-(5-(4-(2-morpholinoethoxy)phenyl)pyridin-2-yl)acetamide); SU6656 ((Z)-N,N-dimethyl-2-oxo-3-((4,5,6,7-tetrahydro-1H-indol-2-yl)methylene)indoline-5-sulfonamide); PP1 (1-(tert-butyl)-3-(p-tolyl)-1H-pyrazolo[3,4-d]pyrimidin-4-amine); WH-4-023 (2,6-dimethylphenyl (2,4-dimethoxyphenyl)(2-((4-(4-methylpiperazin-1-yl)phenyl)amino)pyrimidin-4-yl)carbamate) and KX-01 (N-benzyl-2-(5-(4-(2-morpholinoethoxy)phenyl)pyridin-2-yl)acetamide). In one embodiment, the Src inhibitor is dasatinib. In one embodiment, the Src inhibitor is saracatinib. In one embodiment, the Src inhibitor is ponatinib. In one embodiment, the Src inhibitor is vandetanib. In one embodiment, the Src inhibitor is KX-01.

Methods for manufacturing Src-family kinase inhibitors are well known to those skilled in the art and Src-family kinase inhibitors may be obtained from a wide-variety of commercial suppliers, in forms suitable for both research or human use. In addition, suitable Src-family kinase inhibitors for use in the compositions and methods disclosed herein and methods for preparing such inhibitors are disclosed in US Patent Application Publication Nos: US20160102054; US20150191467; US20150133389; US20140200239; US20110319436; US20100099710; US20090227608; US20060122199; US20050049246; US 20040014676; and US20030171389.

### 2. KRas G12C Inhibitors

In one embodiment, the KRas G12C inhibitors used in the methods are compounds of Formula (I): 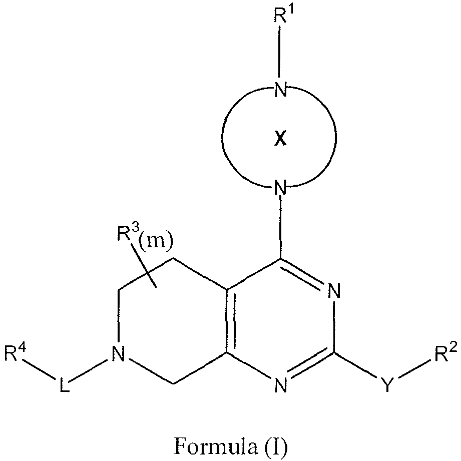 or a pharmaceutically acceptable salt thereof, wherein:
X is a 4-12 membered saturated or partially saturated monocyclic, bridged or spirocyclic ring, wherein the saturated or partially saturated monocyclic ring is optionally substituted with one or more R⁸;
Y is a bond, O, S or NR⁵;
R¹ is -C(O)C(R^{A}) 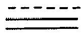 C(R^{B})ₚ or -SO₂C(R^{A}) 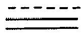 C(R^{B})ₚ;
R² is hydrogen, alkyl, hydroxyalkyl, dihydroxyalkyl, alkylaminylalkyl, dialkylaminylalkyl, -Z-NR⁵R¹⁰, heterocyclyl, heterocyclylalkyl, aryl, heteroaryl, or heteroarylalkyl, wherein each of the Z, heterocyclyl, heterocyclylalkyl, aryl, heteroaryl, and heteroarylalkyl may be optionally substituted with one or more R⁹;
Z is C1 - C4 alkylene;
each R³ is independently C1 - C3 alkyl, oxo, or haloalkyl;
L is a bond, -C(O)-, or C1 - C3 alkylene;
R⁴ is hydrogen, cycloalkyl, heterocyclyl, aryl, aralkyl or heteroaryl, wherein each of the cycloalkyl, heterocyclyl, aryl, aralkyl and heteroaryl may be optionally substituted with one or more R⁶ or R⁷;
each R⁵ is independently hydrogen or C1 - C3 alkyl;
R⁶ is cycloalkyl, heterocyclyl, heterocyclylalkyl, aryl, or heteroaryl, wherein each of the cycloalkyl, heterocyclyl, aryl, or heteroaryl may be optionally substituted with one or more R⁷;
each R⁷ is independently halogen, hydroxyl, C1 - C6 alkyl, cycloalkyl, alkoxy, haloalkyl, amino, cyano, heteroalkyl, hydroxyalkyl or Q-haloalkyl, wherein Q is O or S;
R⁸ is oxo, C1 - C3 alkyl, C2 - C4 alkynyl, heteroalkyl, cyano, -C(O)OR⁵, -C(O)N(R⁵)₂, - N(R⁵)₂, wherein the C1 - C3 alkyl may be optionally substituted with cyano, halogen, -OR⁵, - N(R⁵)₂, or heteroaryl;
each R⁹ is independently hydrogen, oxo, acyl, hydroxyl, hydroxyalkyl, cyano, halogen, Cl - C6 alkyl, aralkyl, haloalkyl, heteroalkyl, cycloalkyl, heterocyclylalkyl, alkoxy, dialkylaminyl, dialkylamidoalkyl, or dialkylaminylalkyl, wherein the C1 - C6 alkyl may be optionally substituted with cycloalkyl;
each R¹⁰ is independently hydrogen, acyl, C1 - C3 alkyl, heteroalkyl or hydroxyalkyl;
R¹¹ is haloalkyl;
R^{A} is absent, hydrogen, deuterium, cyano, halogen, C1 - C-3 alkyl, haloalkyl, heteroalkyl, -C(O)N(R⁵)₂, or hydroxyalkyl;
each R^{B} is independently hydrogen, deuterium, cyano, C1 - C3 alkyl, hydroxyalkyl, heteroalkyl, C1 - C3 alkoxy, halogen, haloalkyl, -ZNR⁵R]¹¹, -C(O)N(R⁵)₂, -NHC(O)C1 - C3 alkyl, -CH₂NHC(O)C1 - C3 alkyl, heteroaryl, heteroarylalkyl, dialkylaminylalkyl, or heterocyclylalkyl wherein the heterocyclyl portion is substituted with one or more substituents independently selected from halogen, hydroxyl, alkoxy and C1 - C3 alkyl, wherein the heteroaryl or the heteroaryl portion of the heteroarylalkyl is optionally substituted with one or more R⁷;
m is zero or an integer between 1 and 2;
p is one or two; and wherein,
when 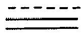 is a triple bond then R^{A} is absent, R^{B} is present and p equals one;
or when 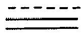 is a double bond then R^{A} is present, R^{B} is present and p equals two, or R^{A}, R^{B} and the carbon atoms to which they are attached form a 5-8 membered partially saturated cycloalkyl optionally substituted with one or more R⁷.

In one embodiment, KRas G12C inhibitors used in the methods herein includes compounds having the Formula I-A: 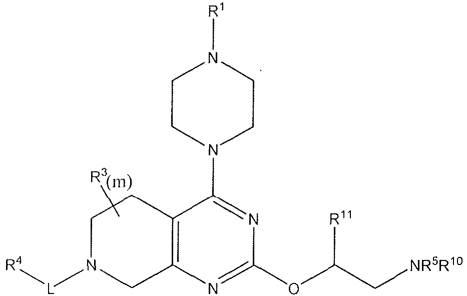 and pharmaceutically acceptable salts thereof, wherein R¹, R³, R⁴, R⁵, R¹⁰, L and m are as defined for Formula I, R¹¹ is hydrogen, methyl or hydroxyalkyl, and the piperidinyl ring is optionally substituted with R⁸ wherein R⁸ is as defined for Formula I.

In one embodiment, KRas G12C inhibitors used in the methods herein include compounds having the Formula I-B: 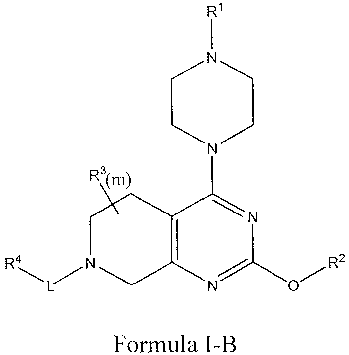 and pharmaceutically acceptable salts thereof, wherein R¹, R³, R⁴, R⁹, R¹¹, L and m are as defined for Formula I.
Nonlimiting examples of KRas G12C inhibitor compounds of Formula (I), Formula I-A and Formula I-B useful in the methods disclosed herein are selected from Example Nos. 1-527 having the following structures, respectively: 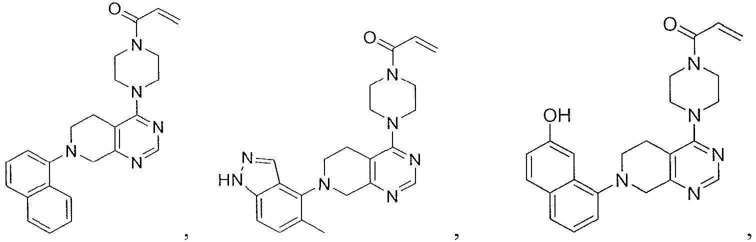 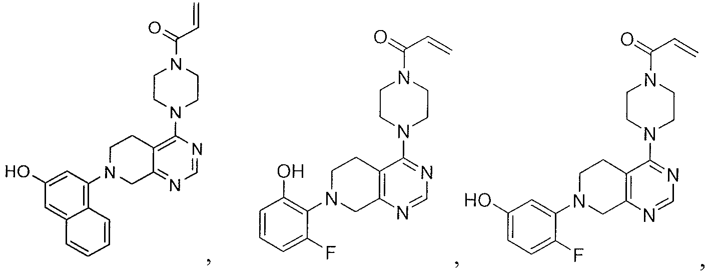 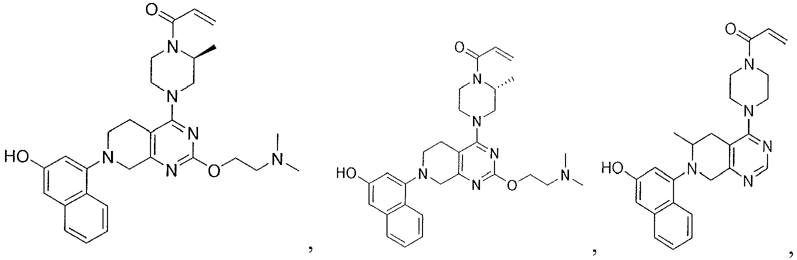 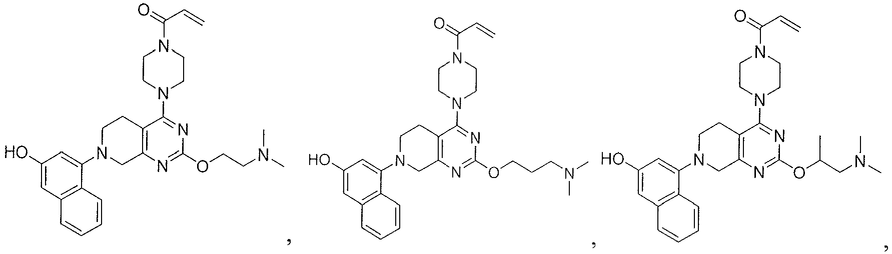 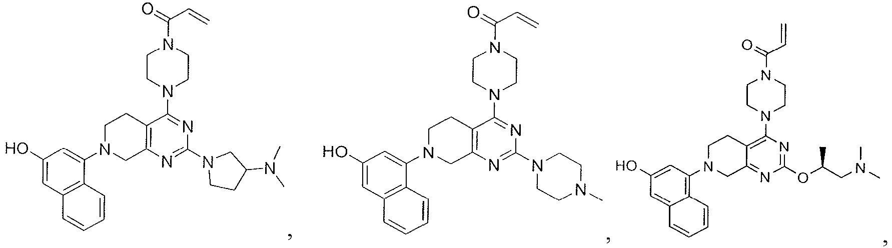 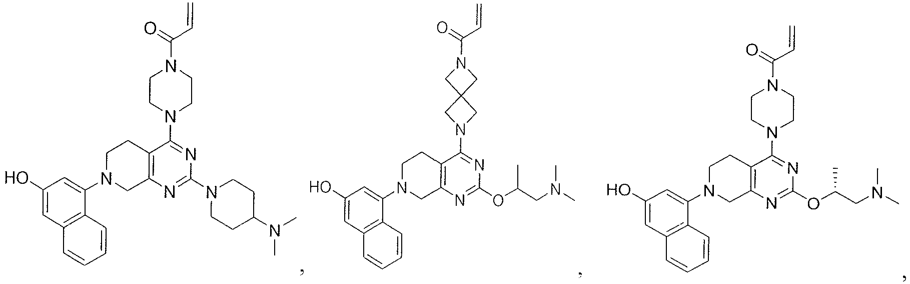 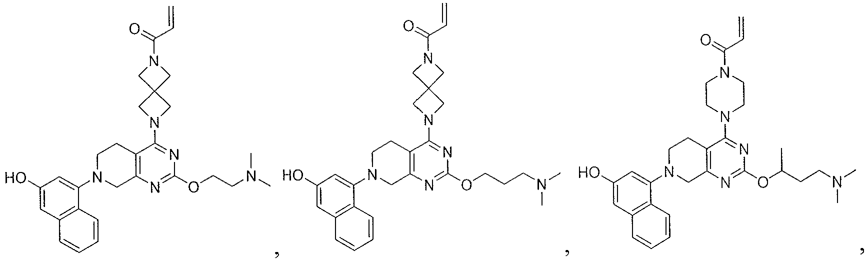 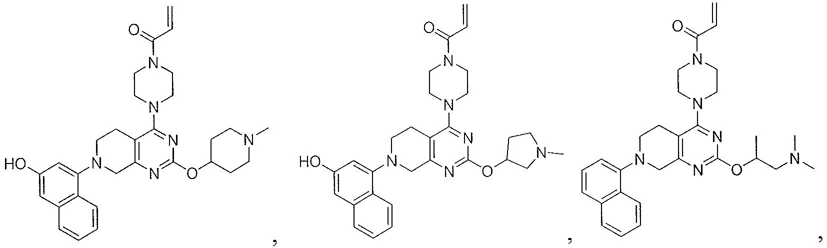 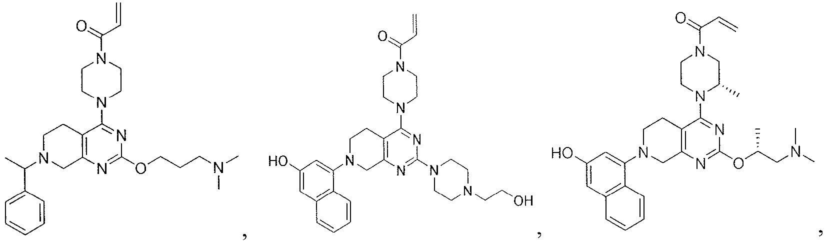 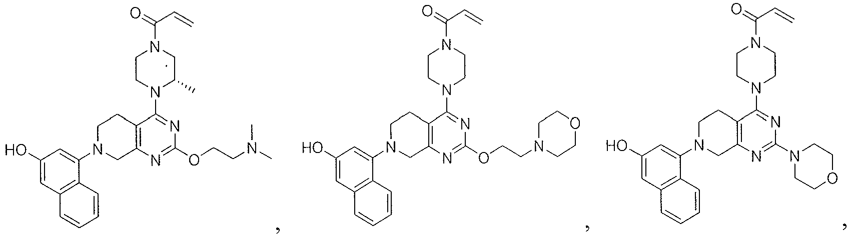 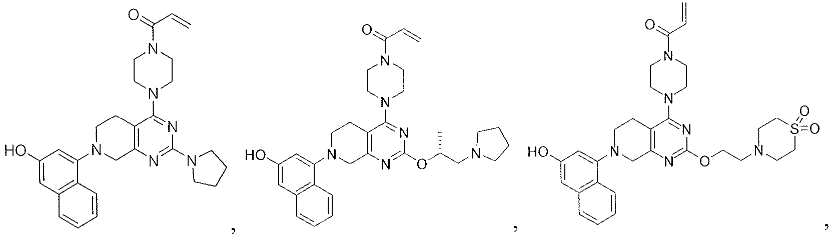 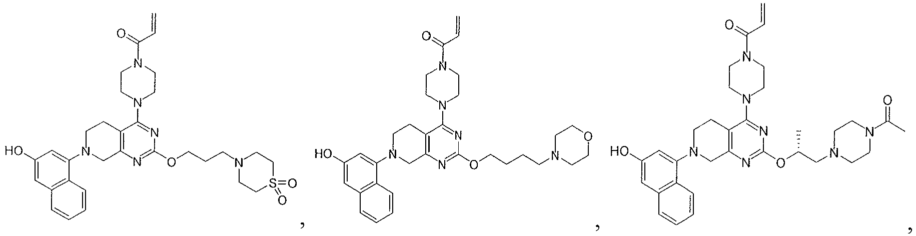 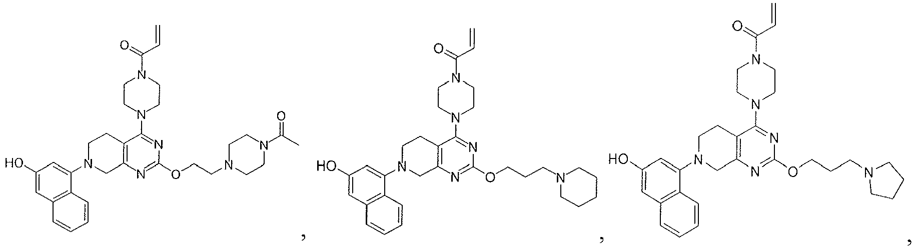 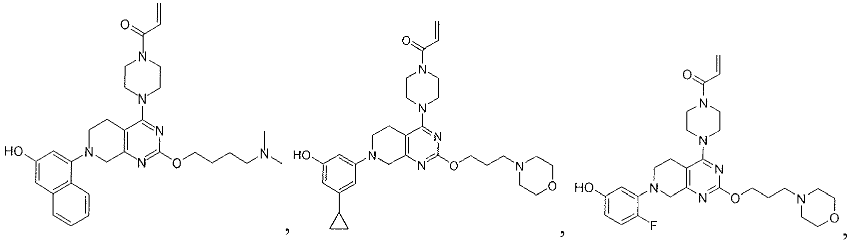 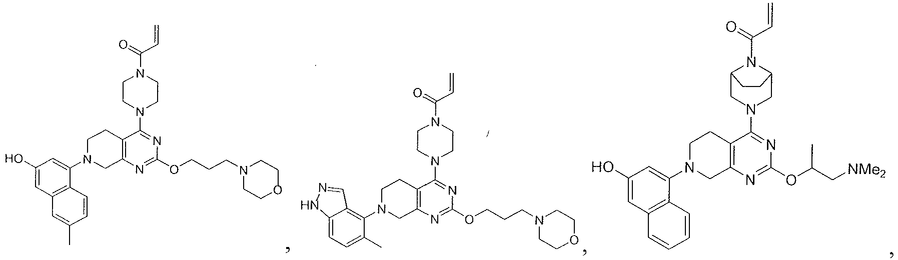 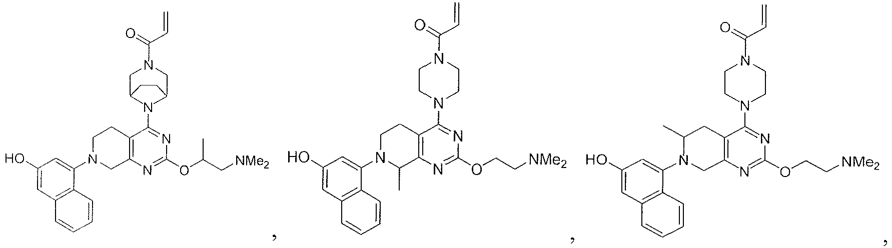 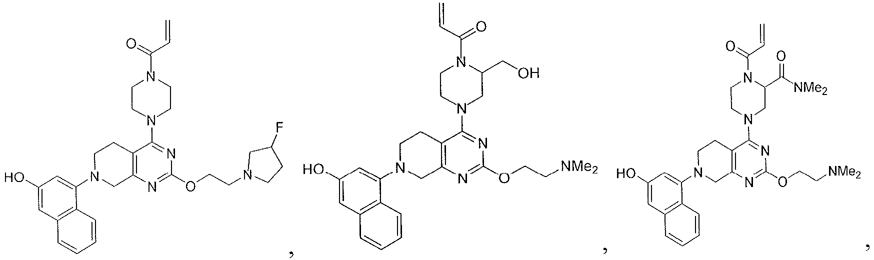 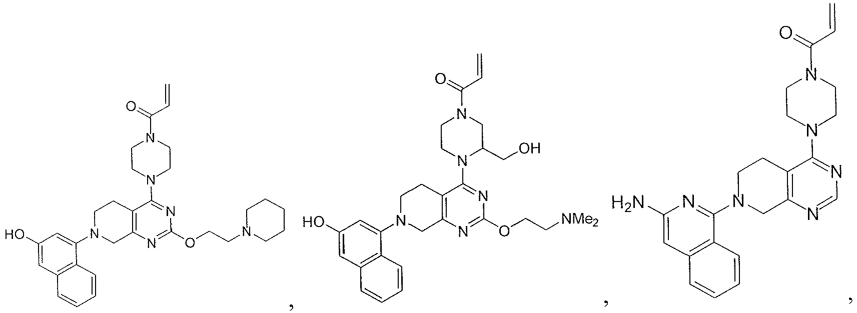 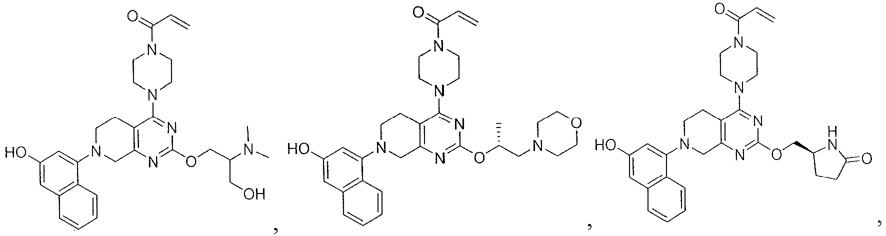 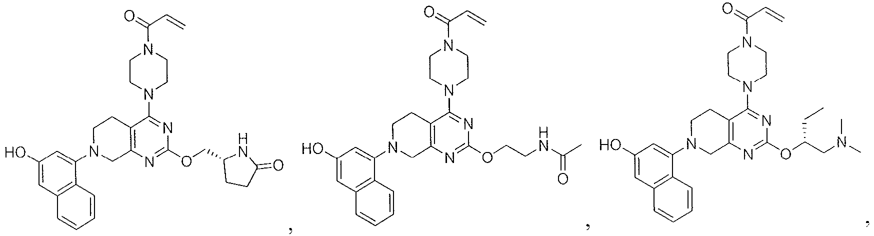 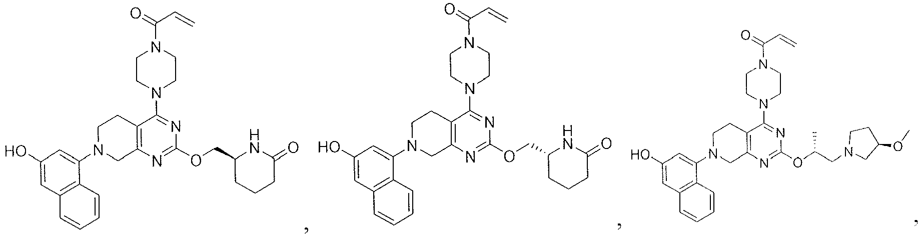 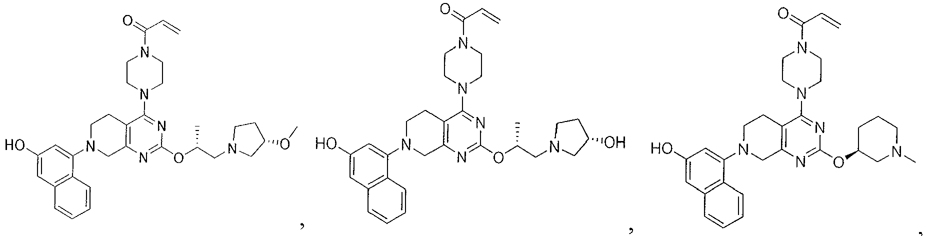 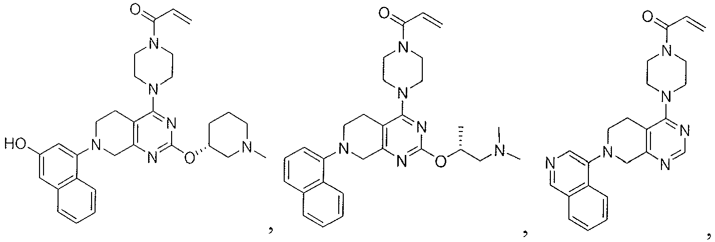 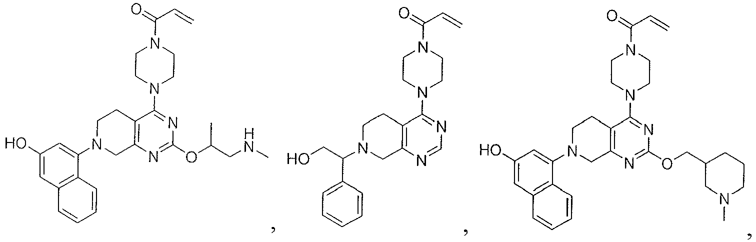 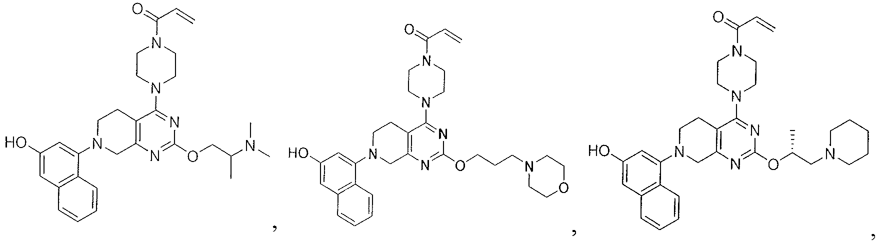 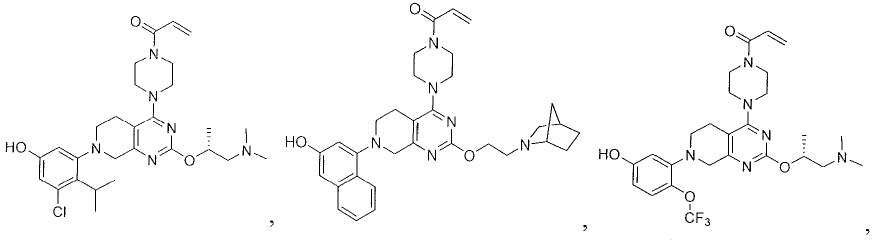 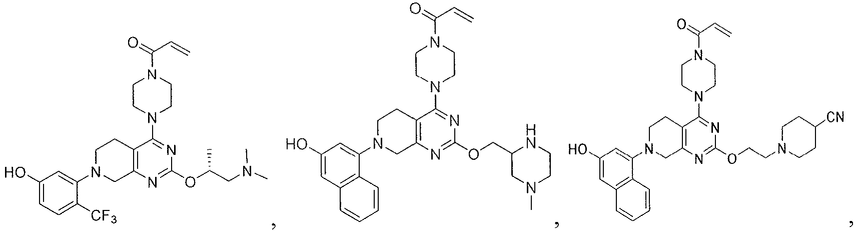 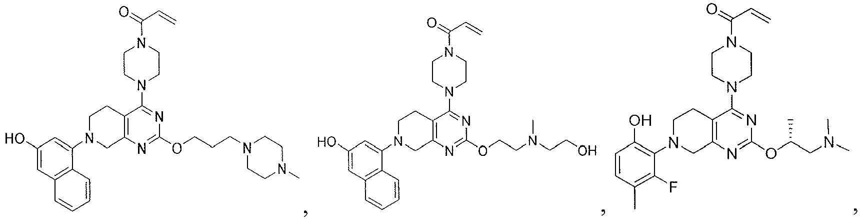 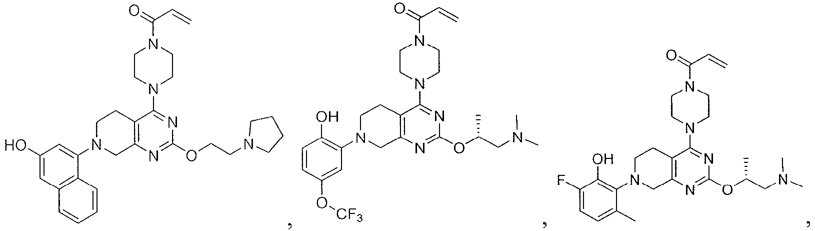 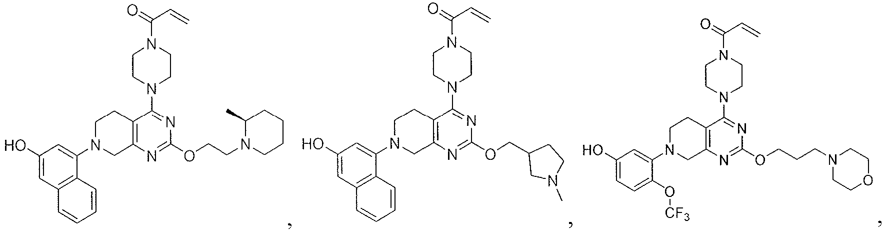 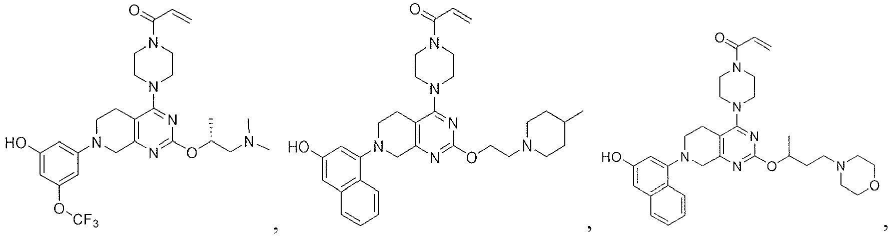 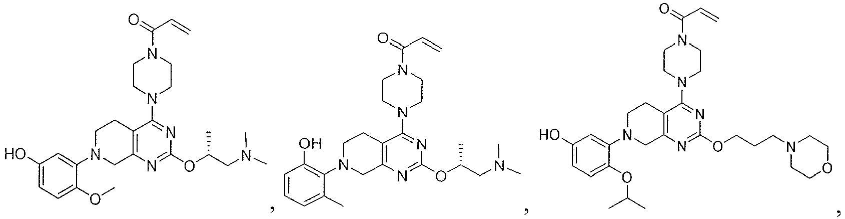 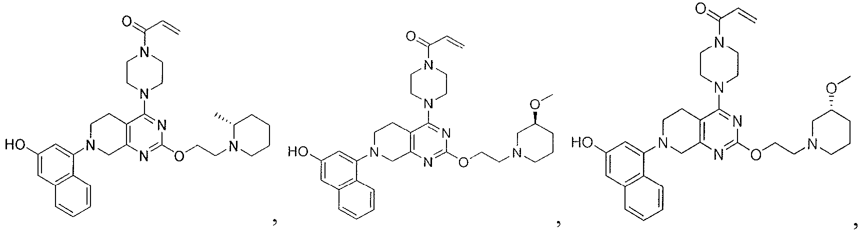 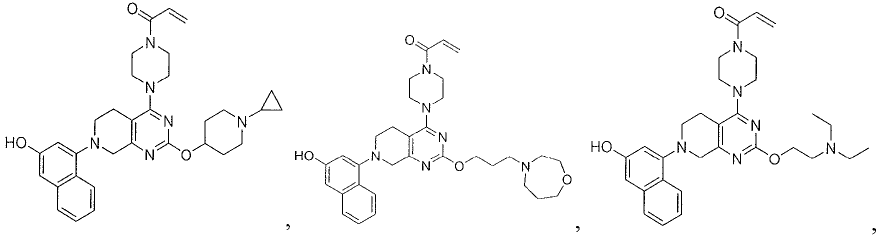 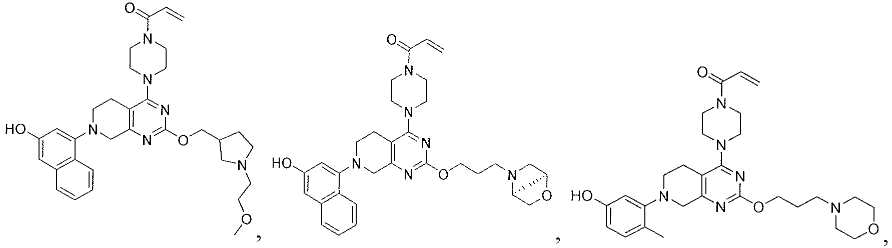 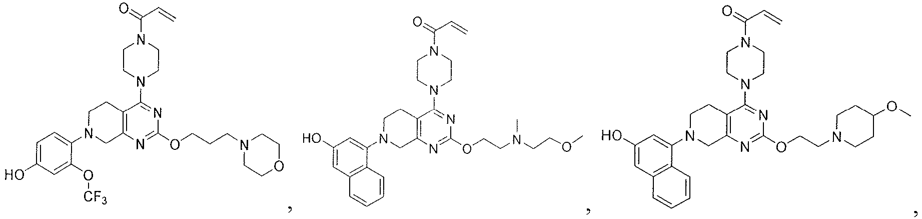 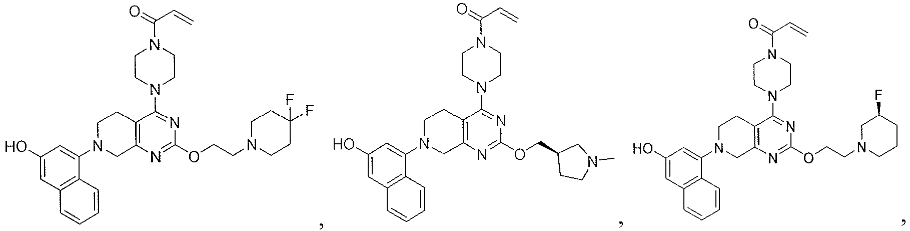 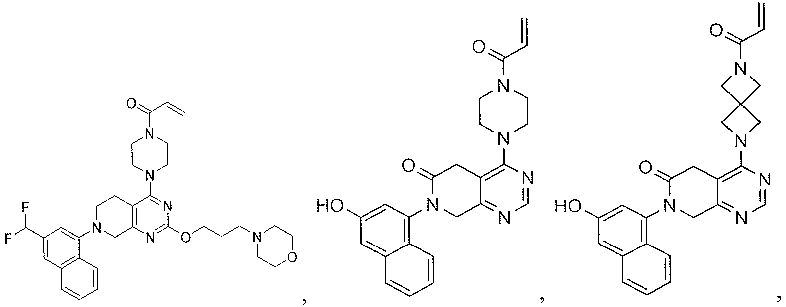 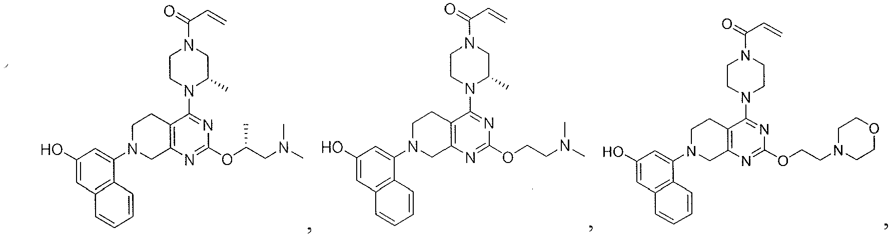 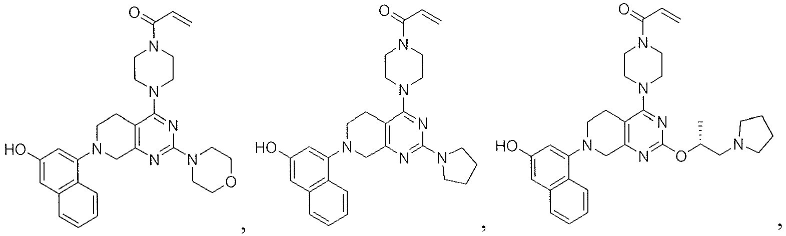 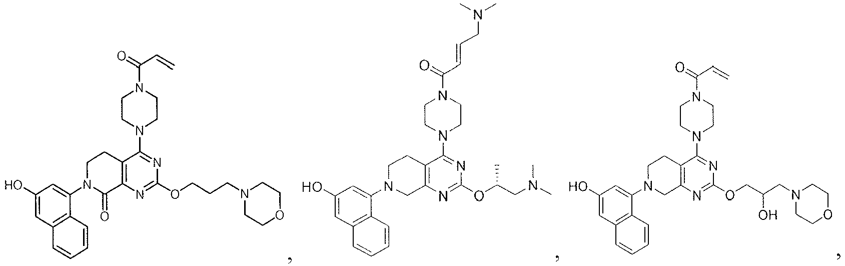 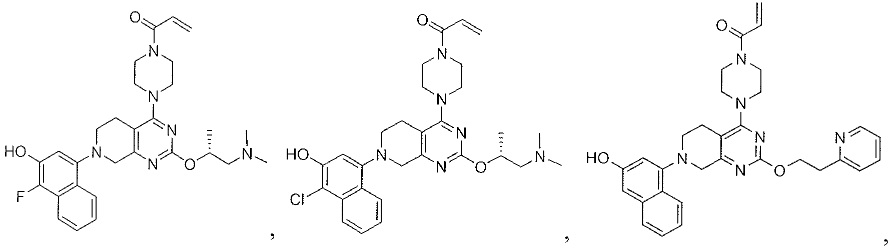 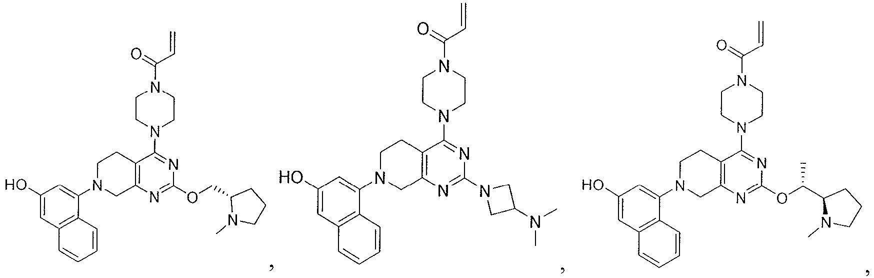 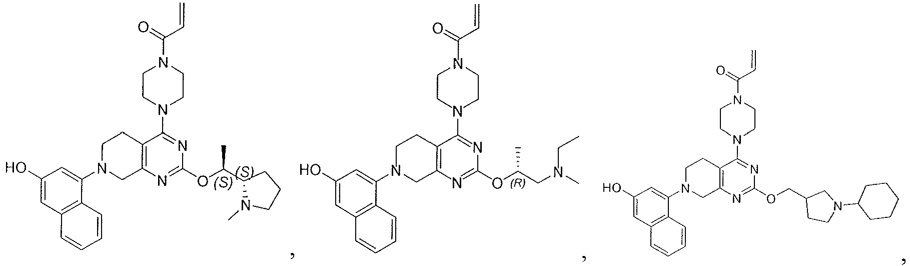 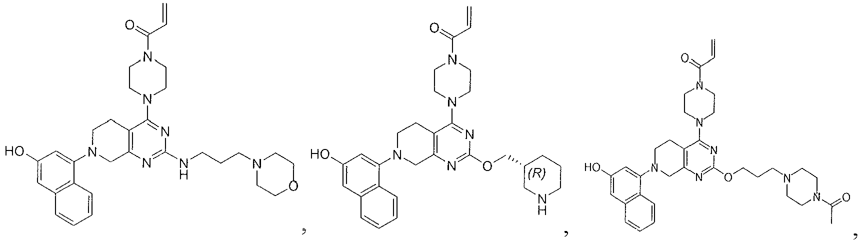 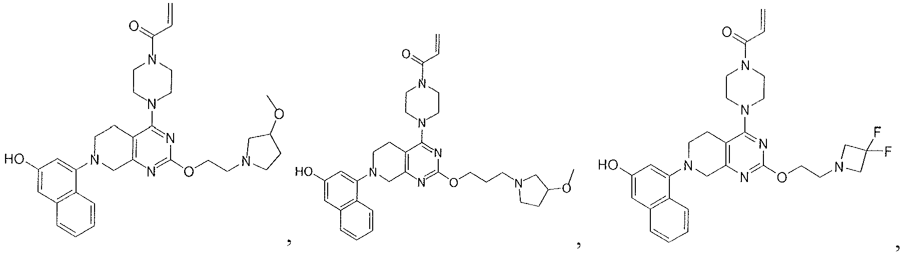 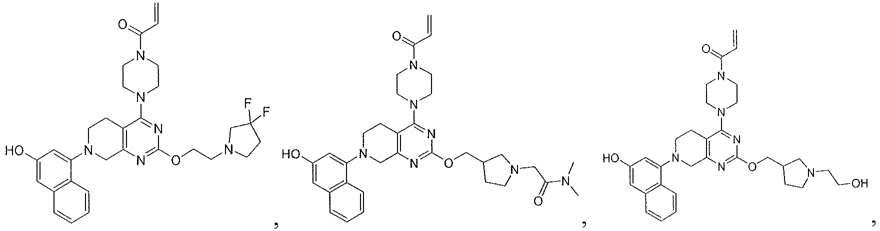 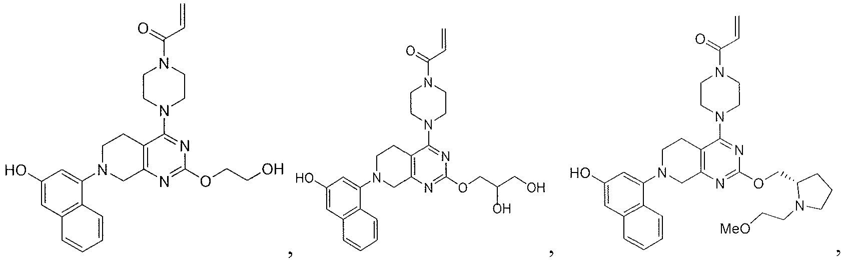 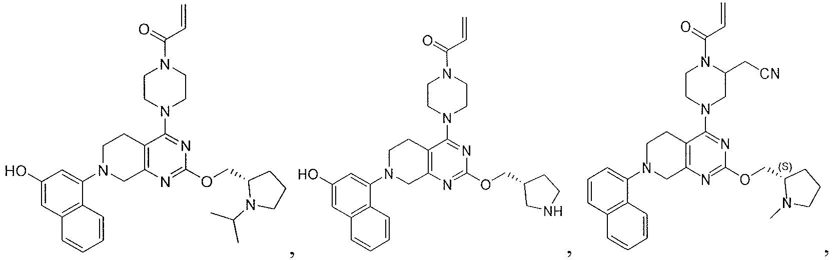 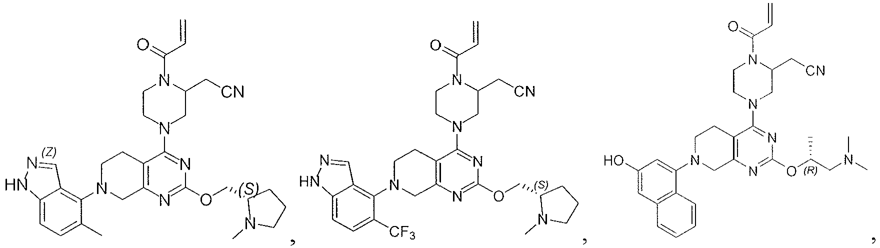 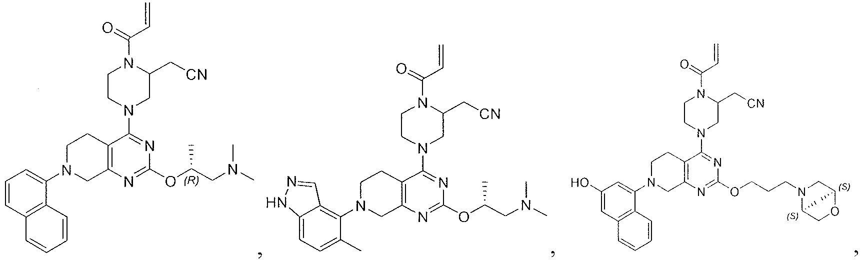 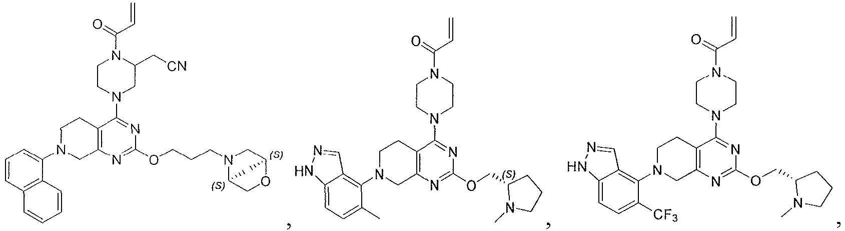 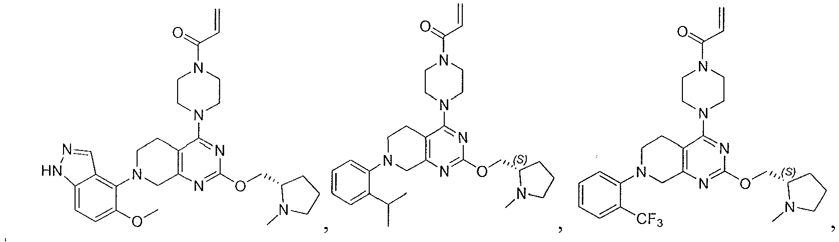 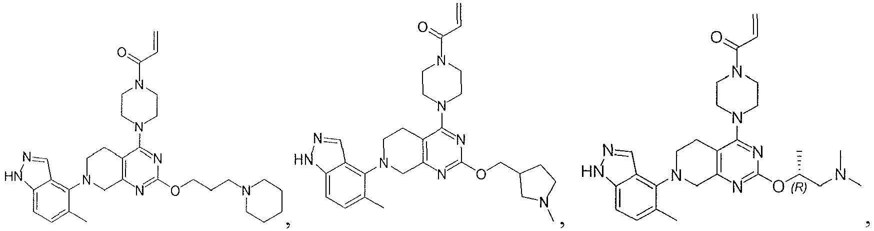 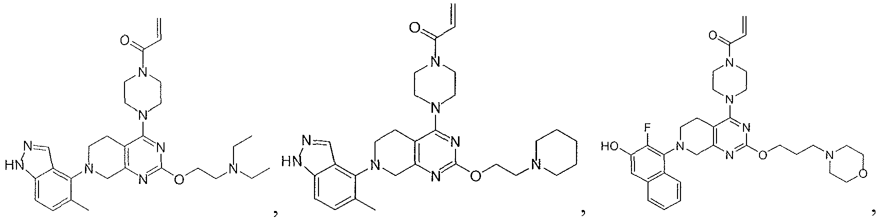 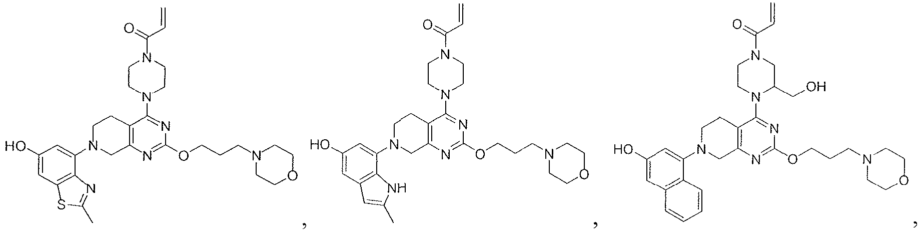 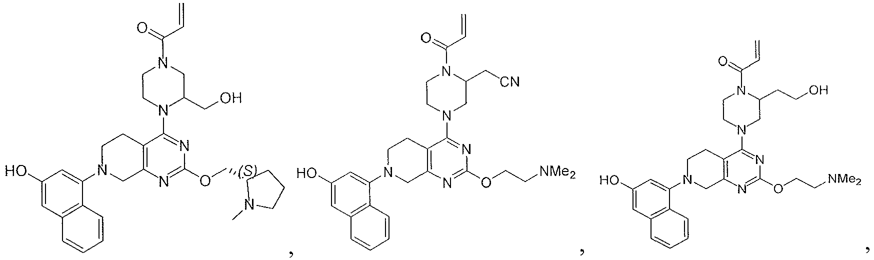 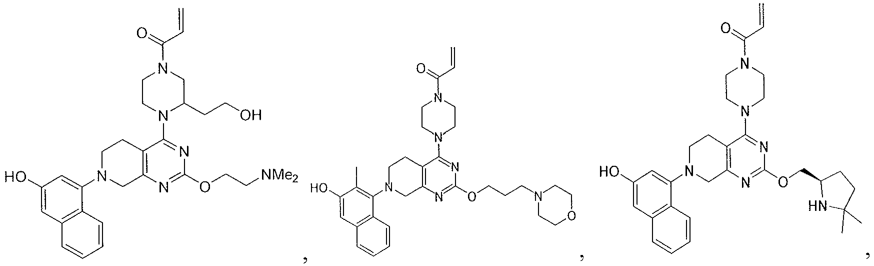 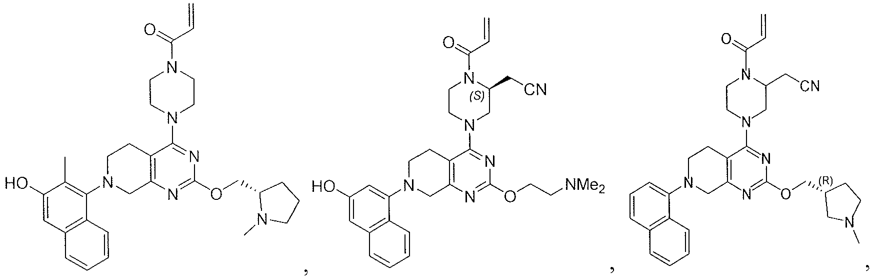 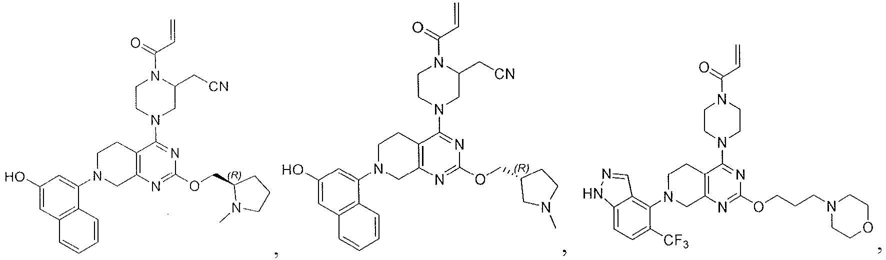 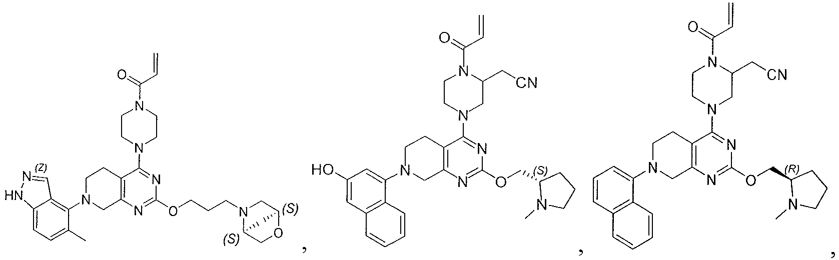 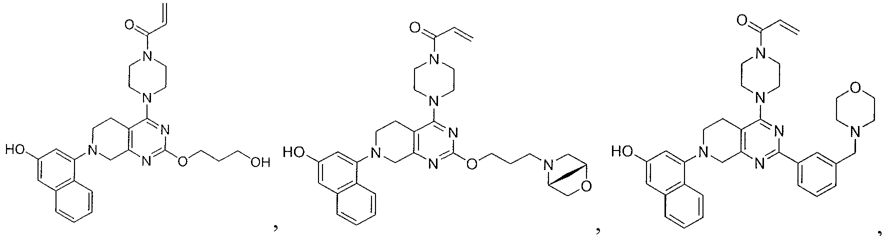 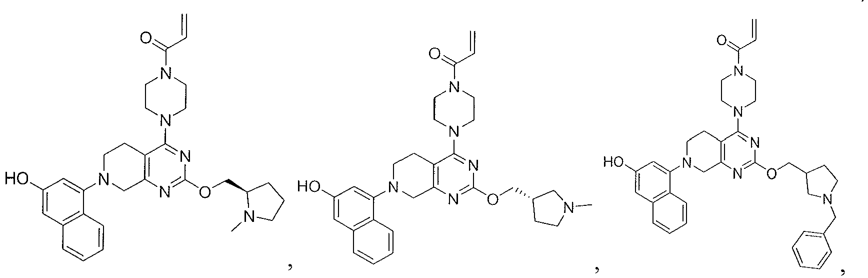 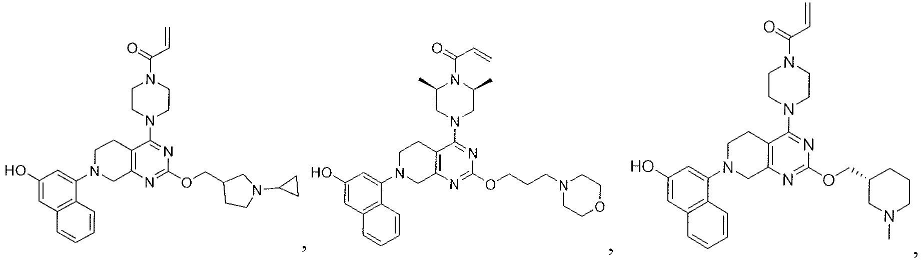 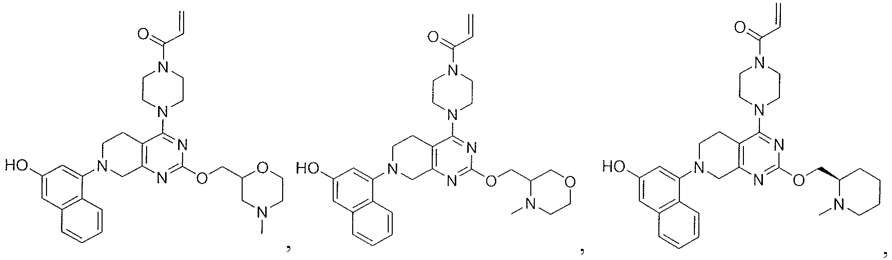 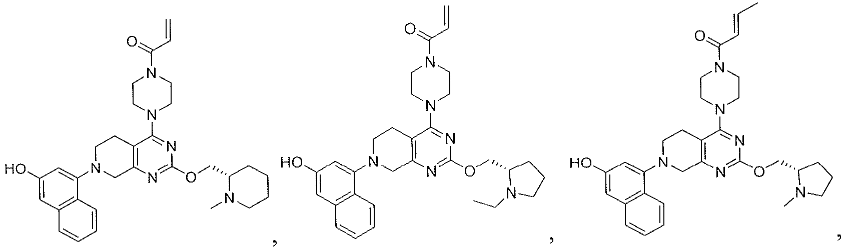 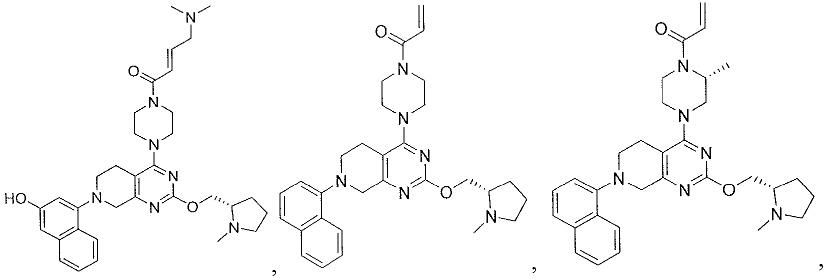 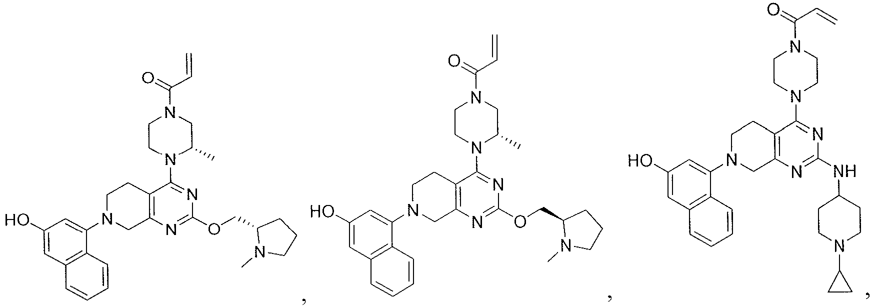 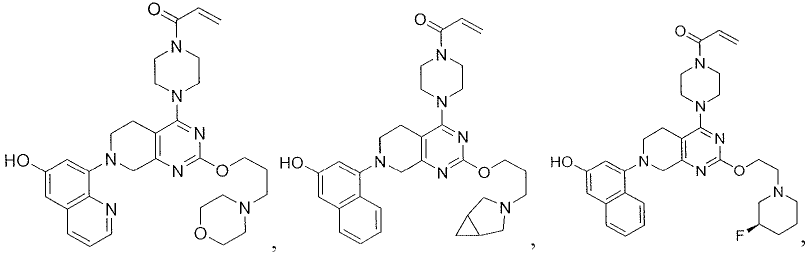 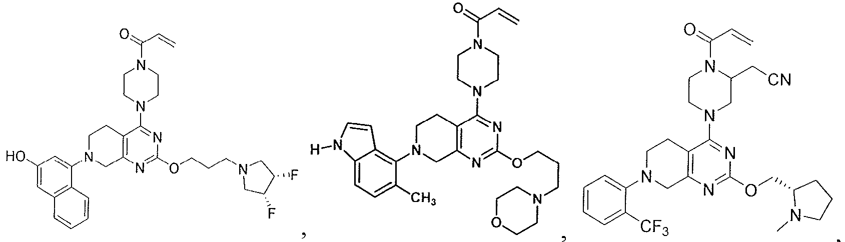 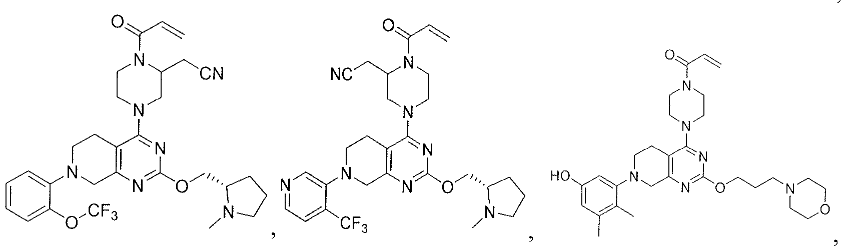 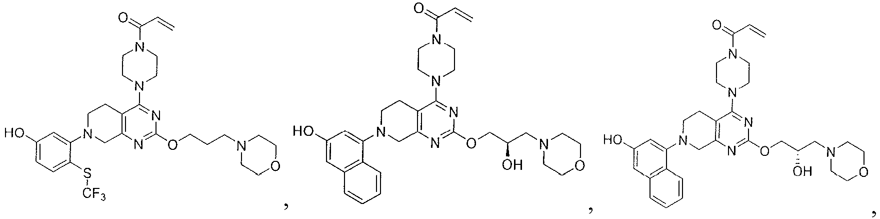 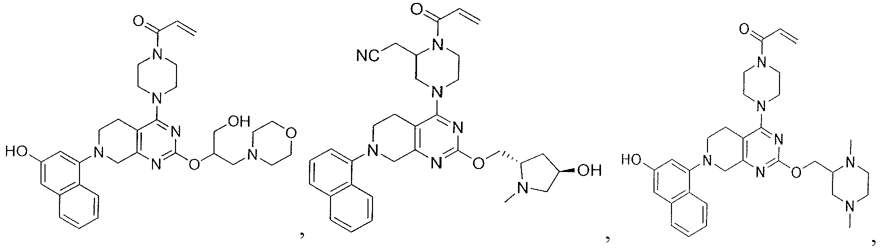 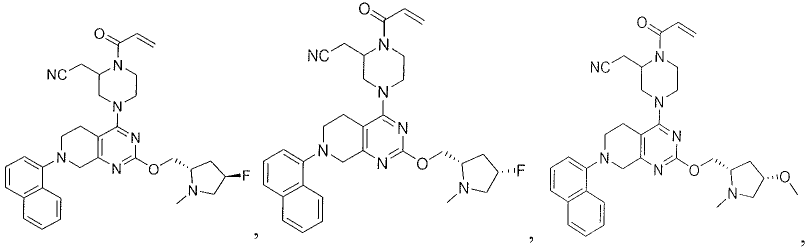 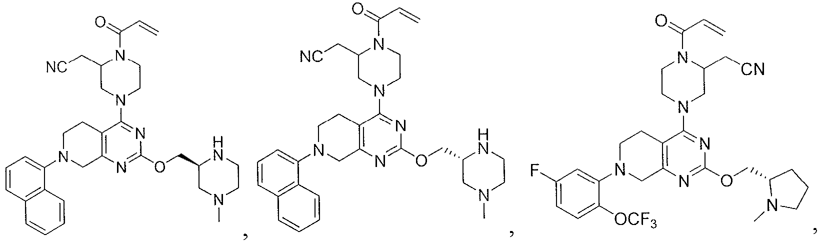 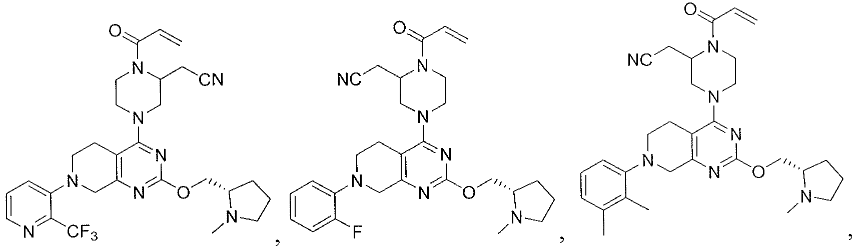 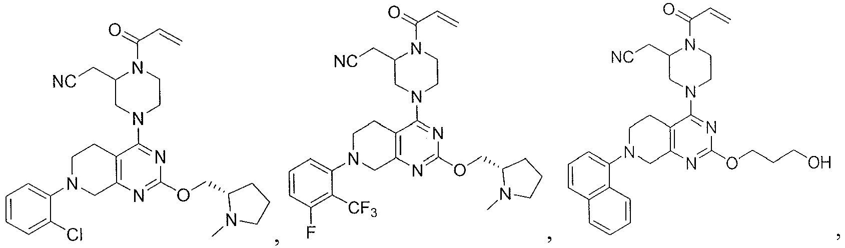 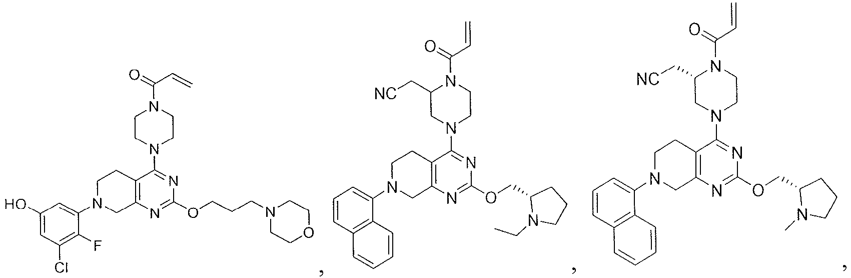 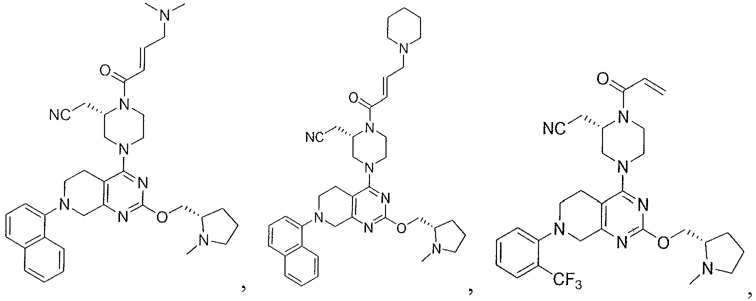 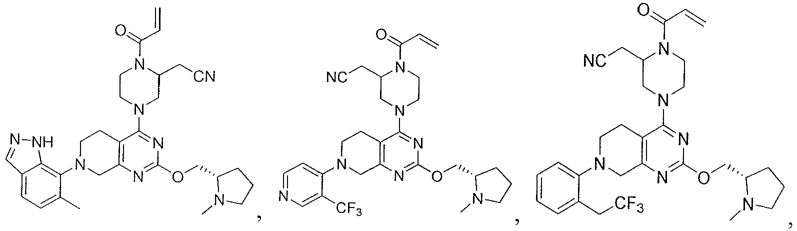 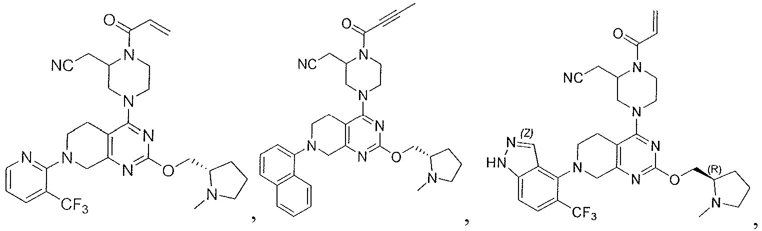 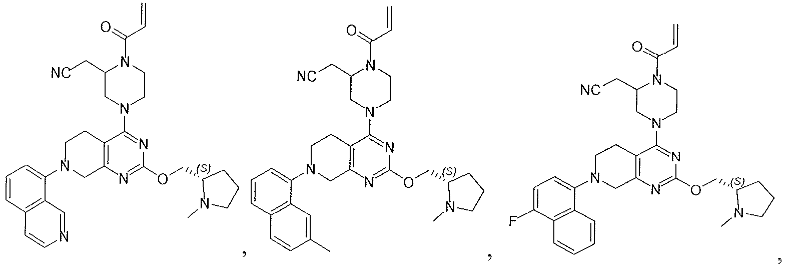 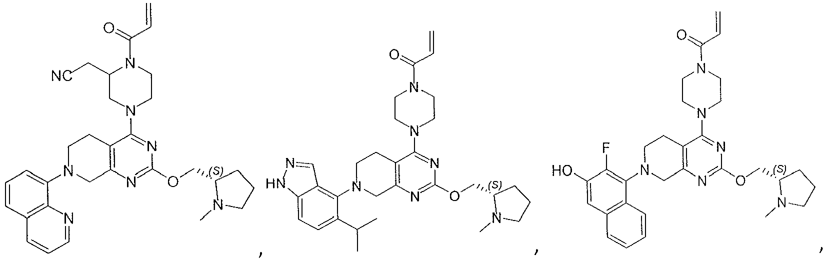 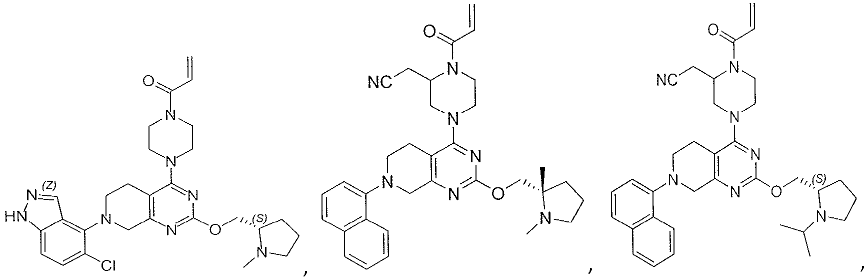 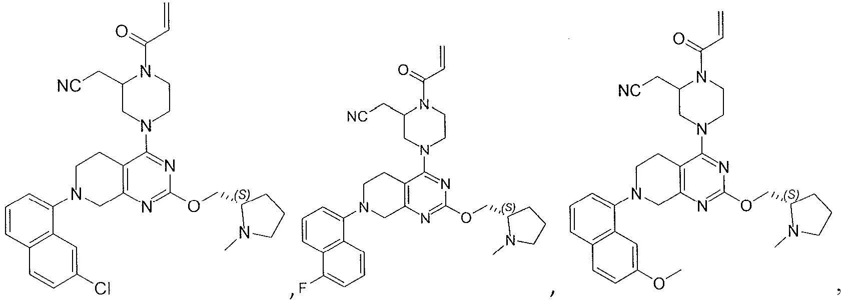 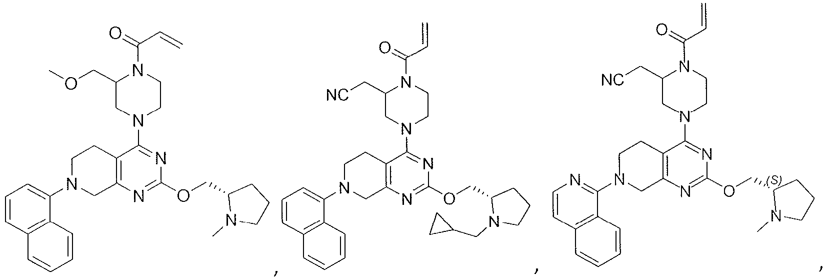 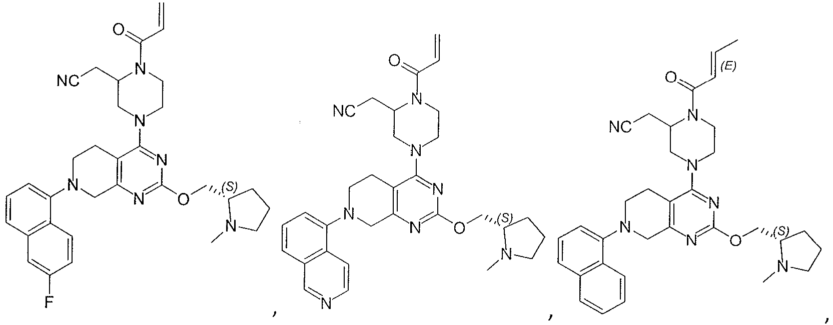 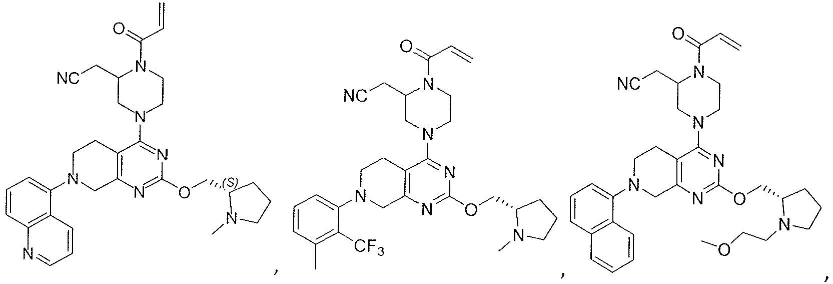 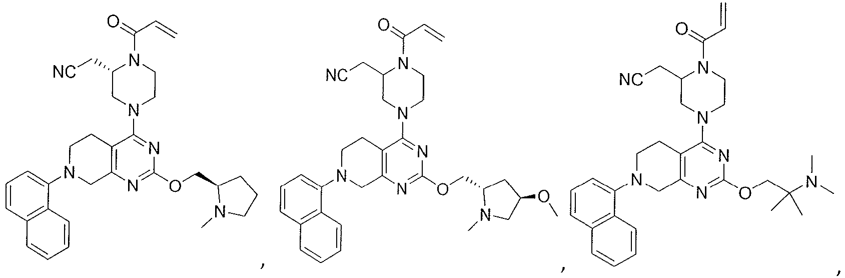 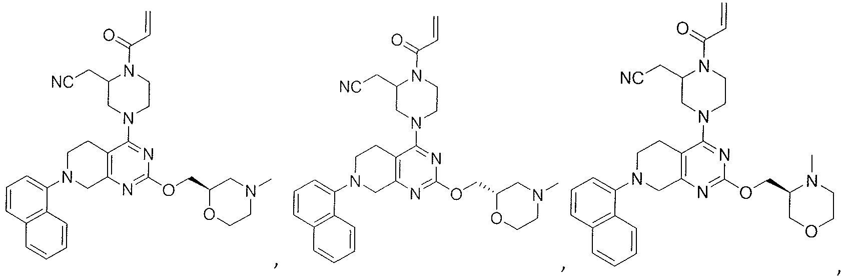 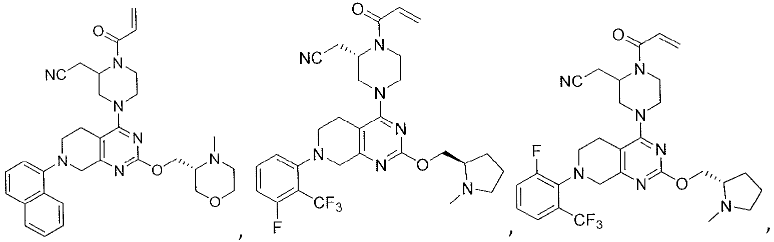 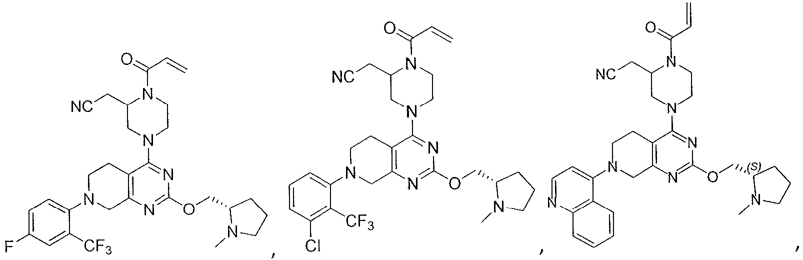 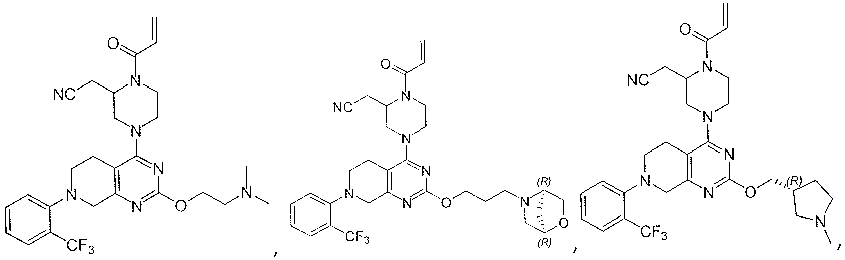 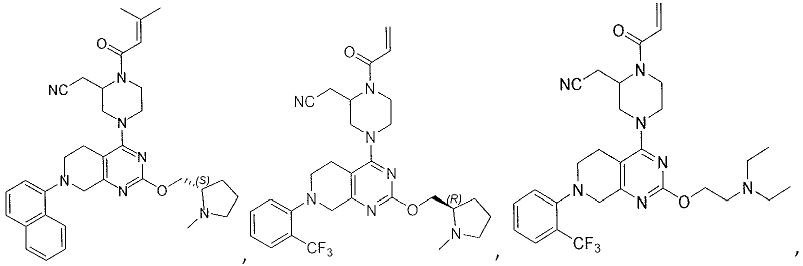 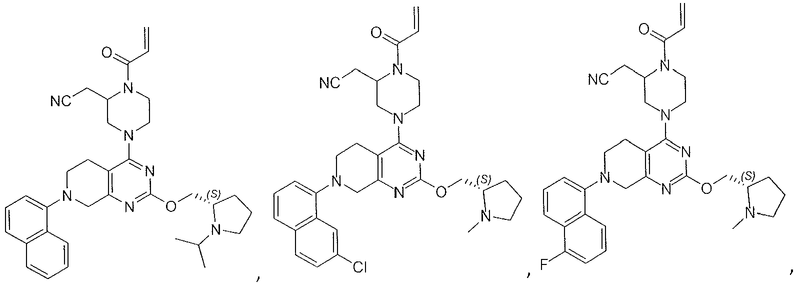 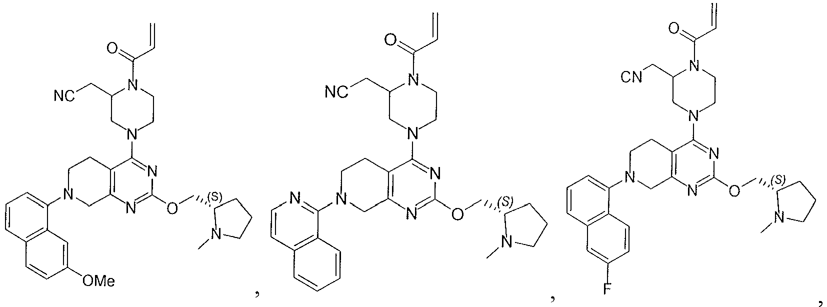 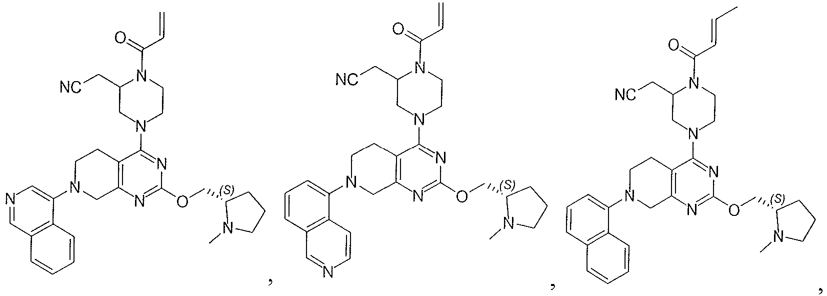 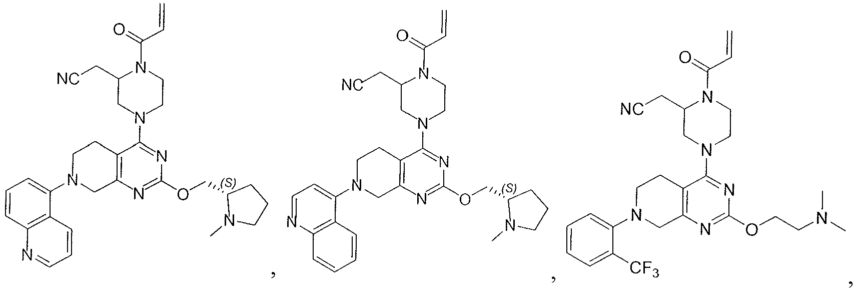 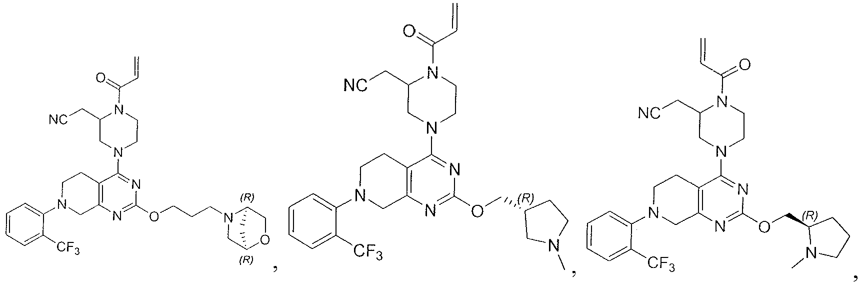 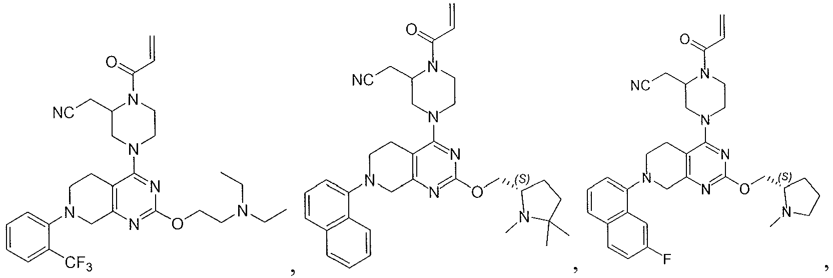 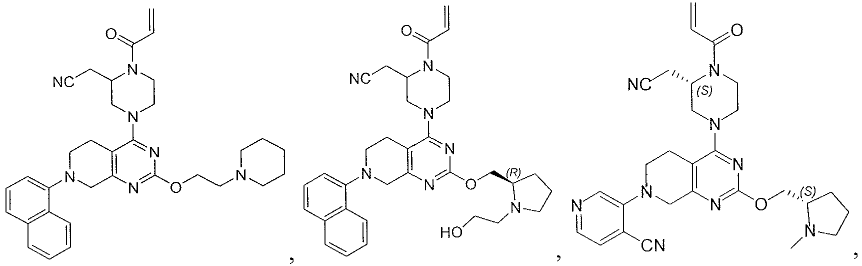 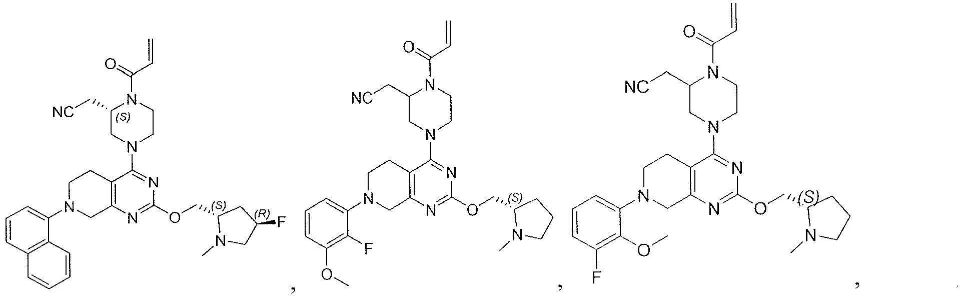 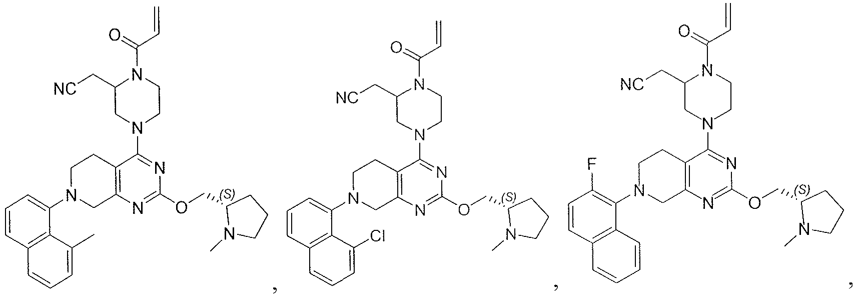 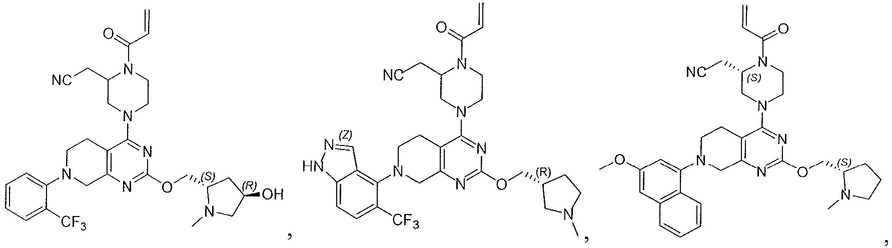 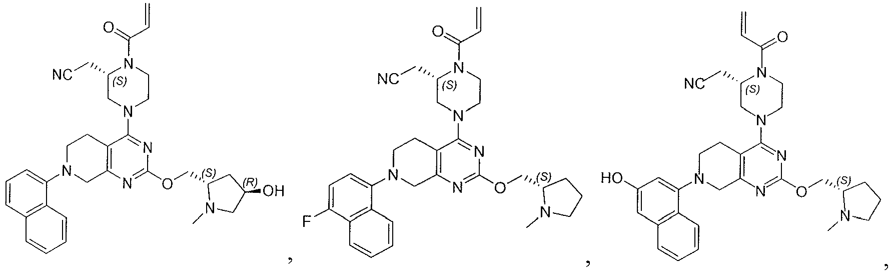 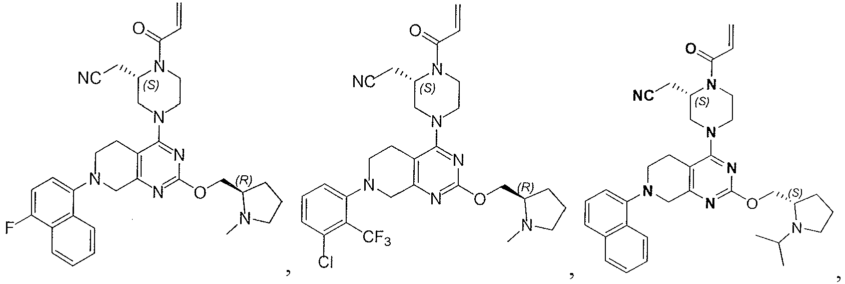 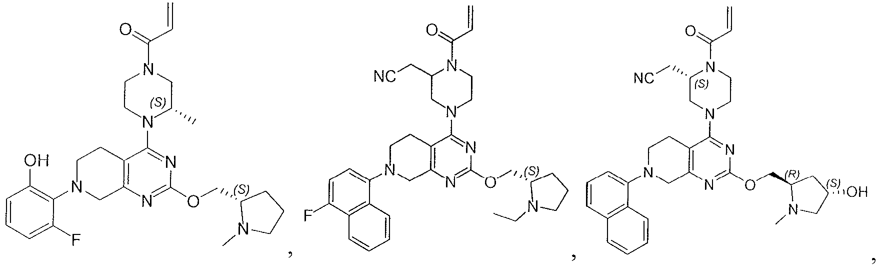 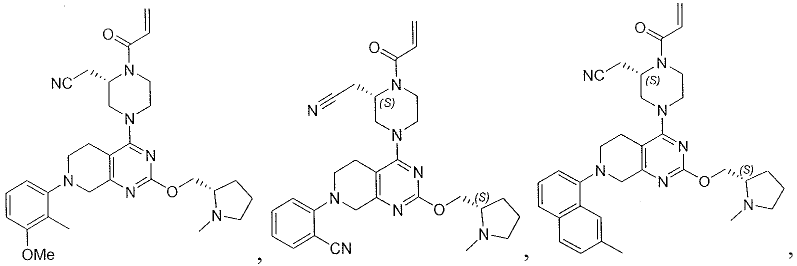 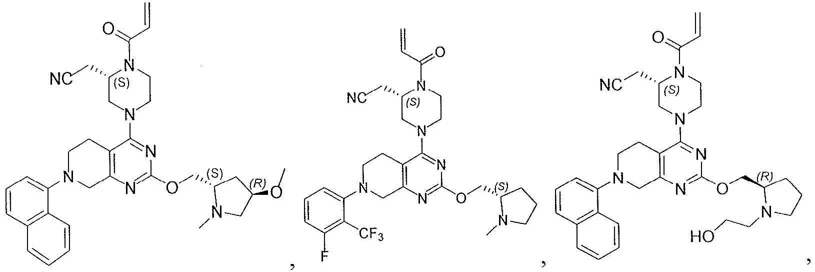 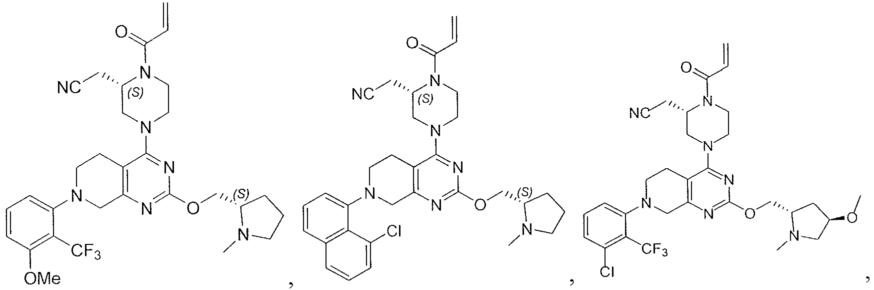 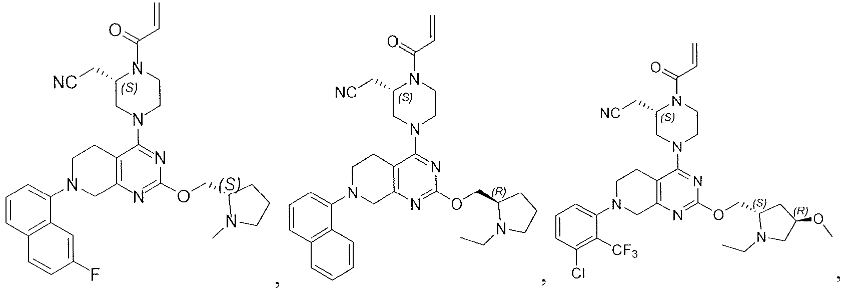 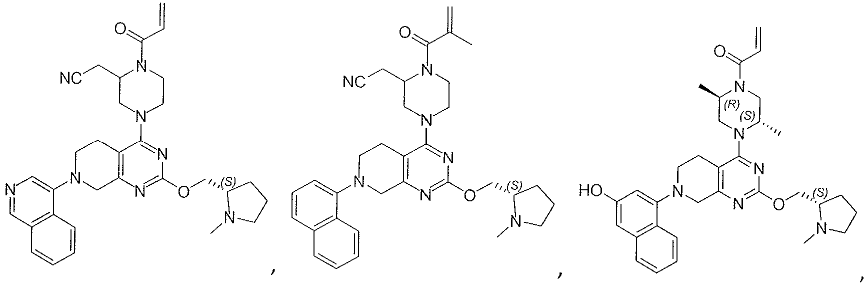 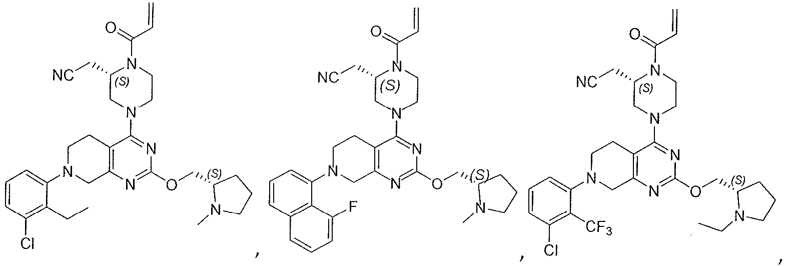 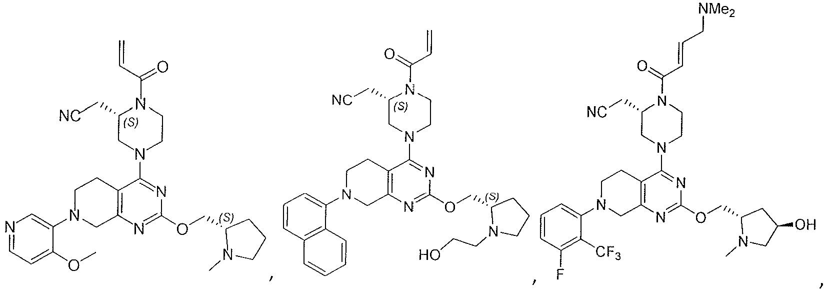 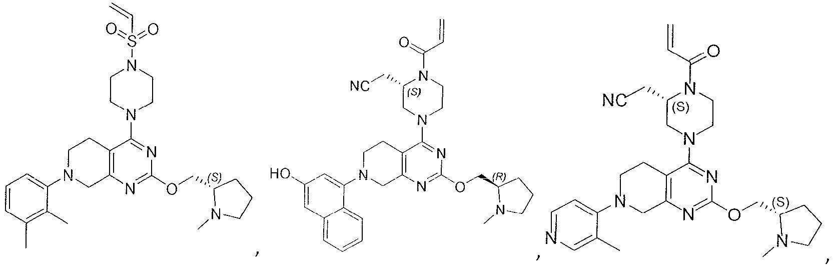 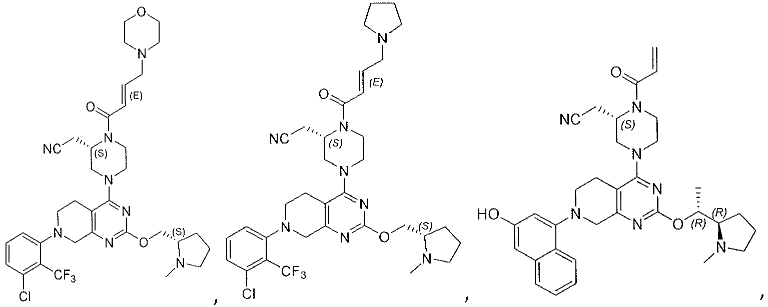 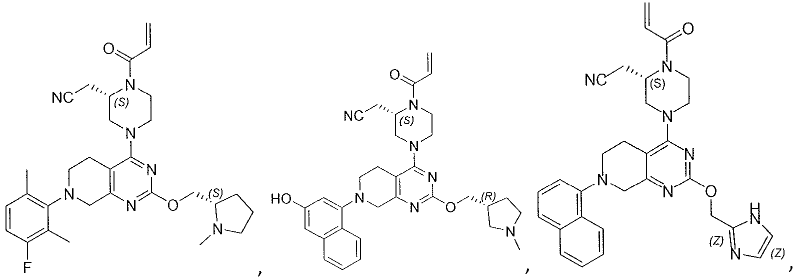 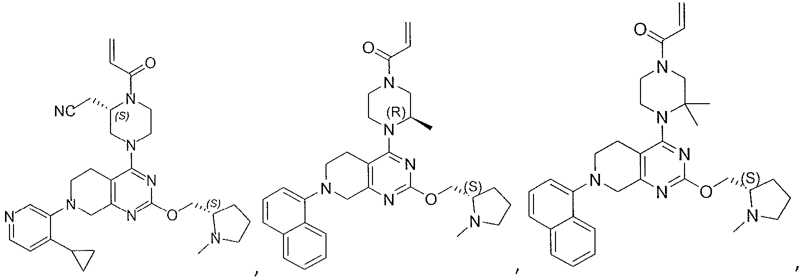 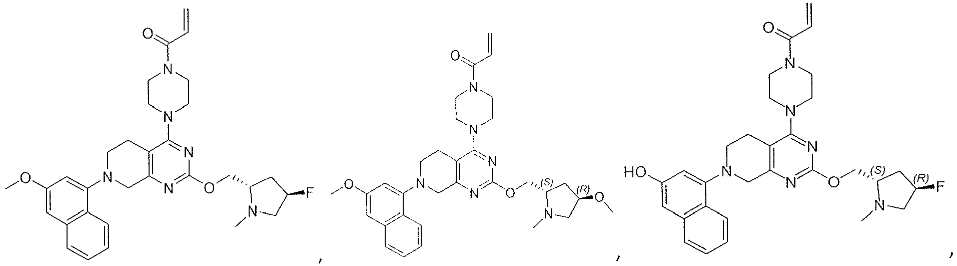 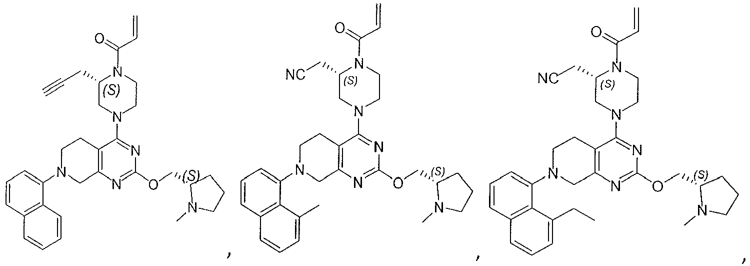 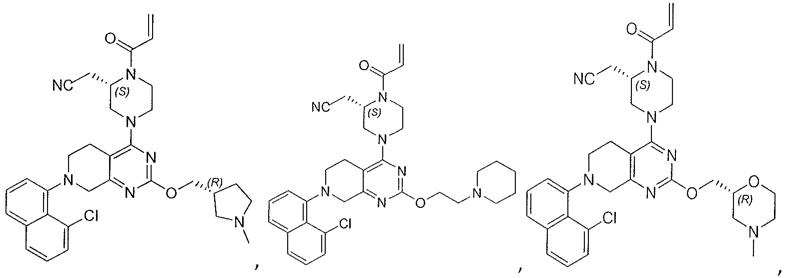 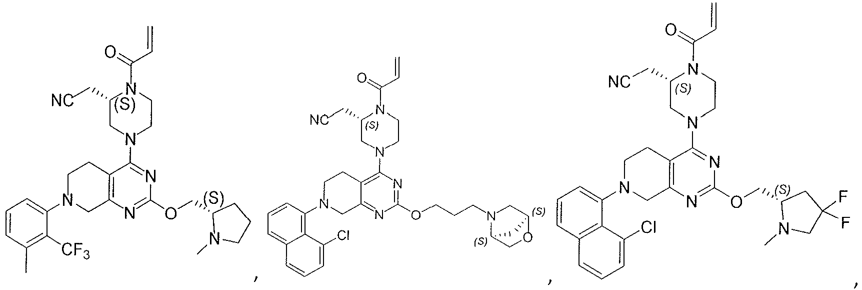 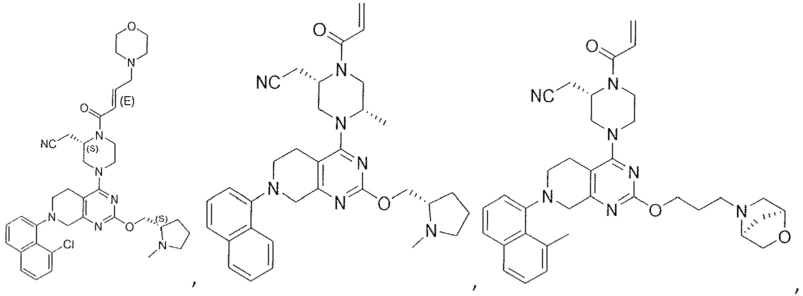 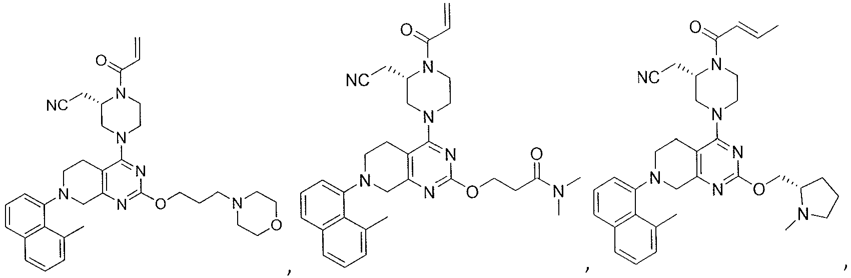 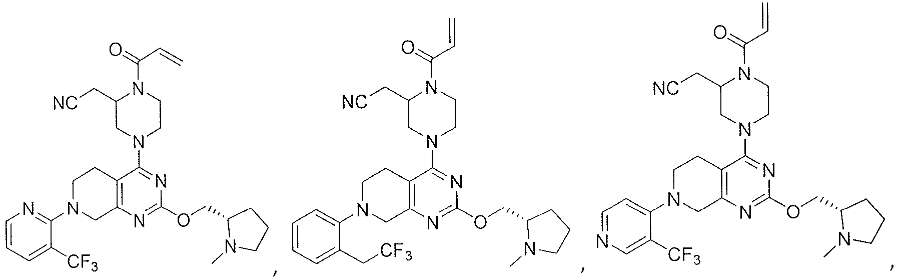 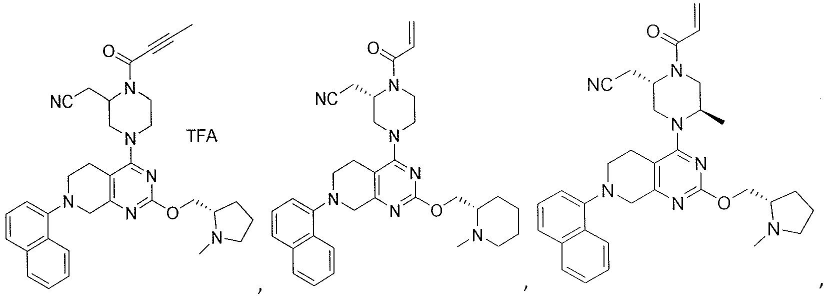 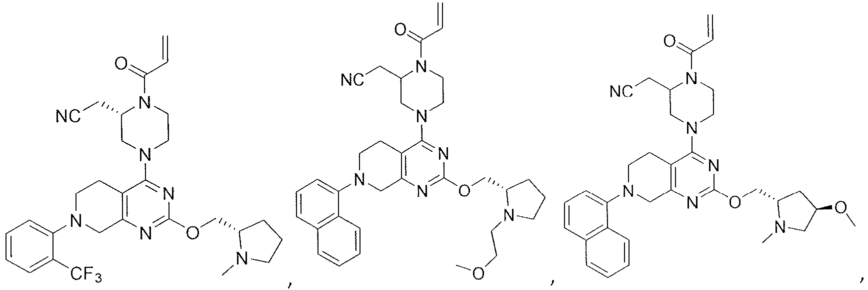 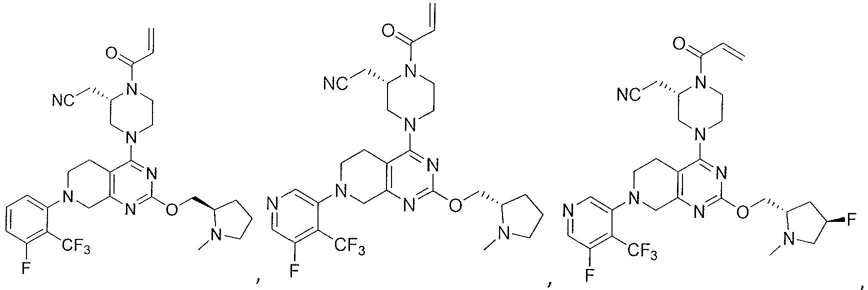 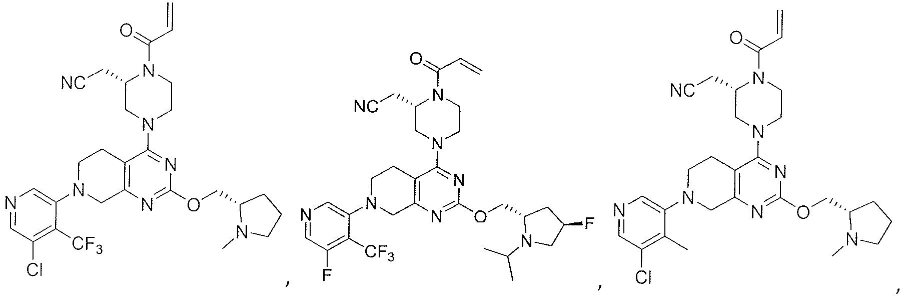 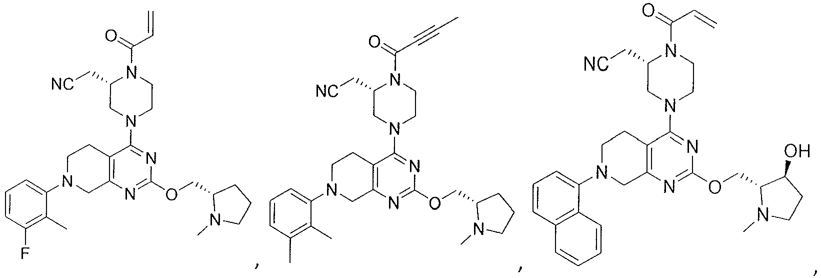 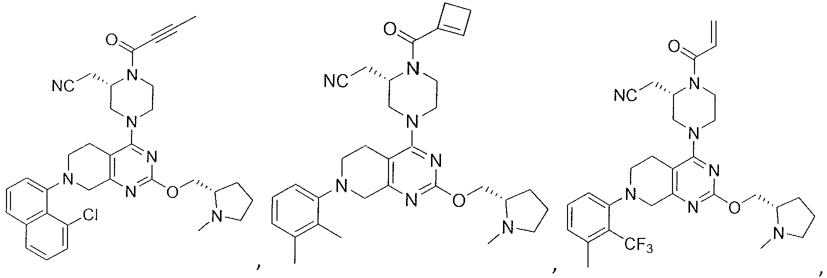 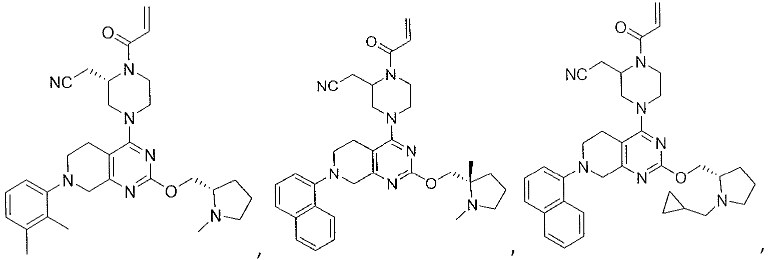 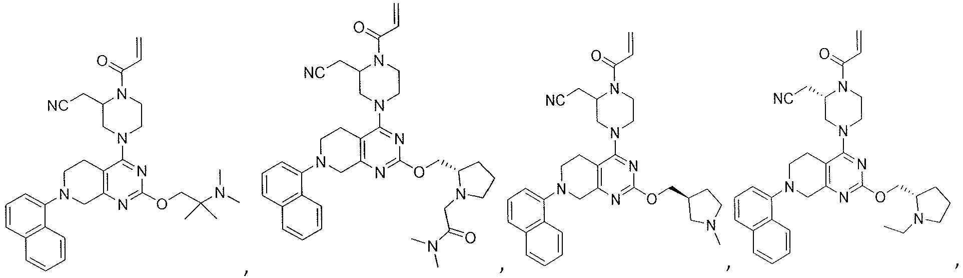 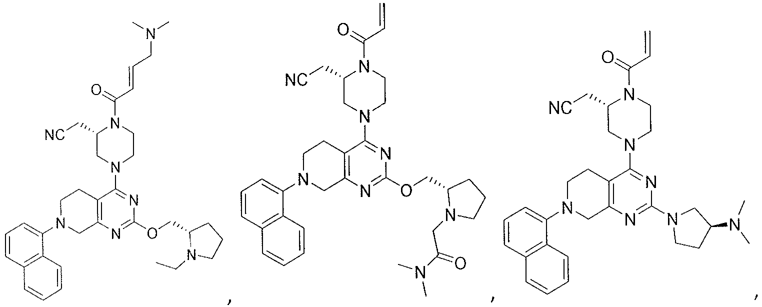 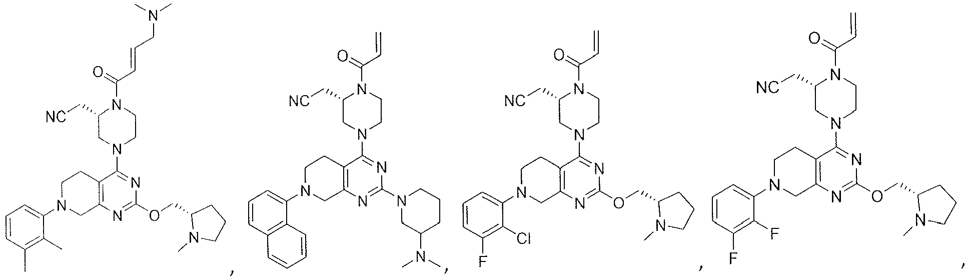 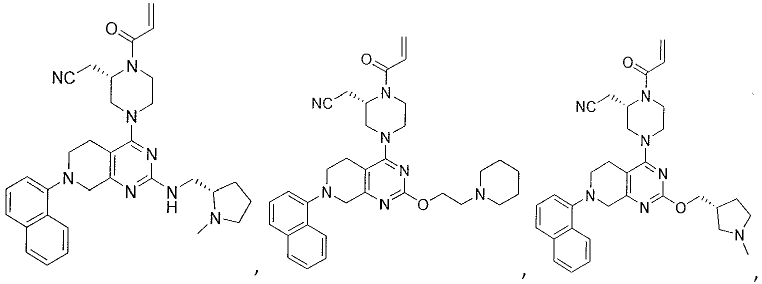 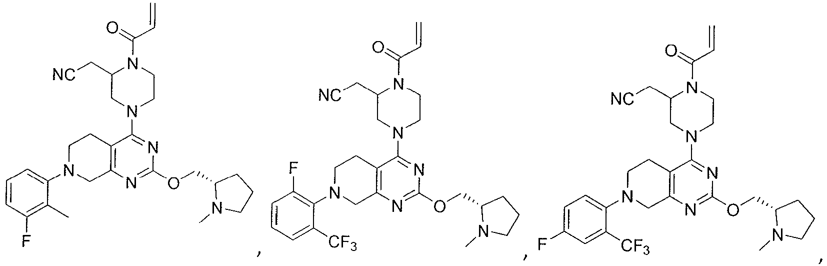 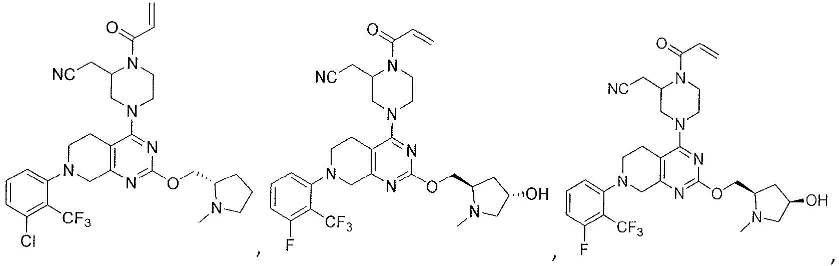 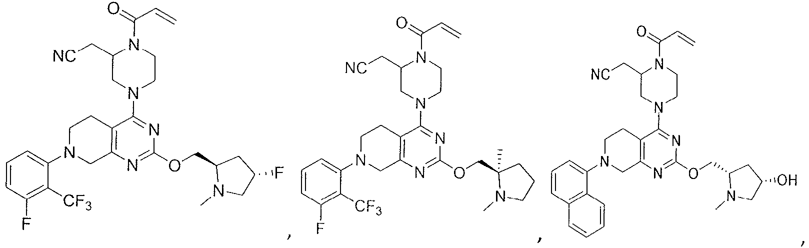 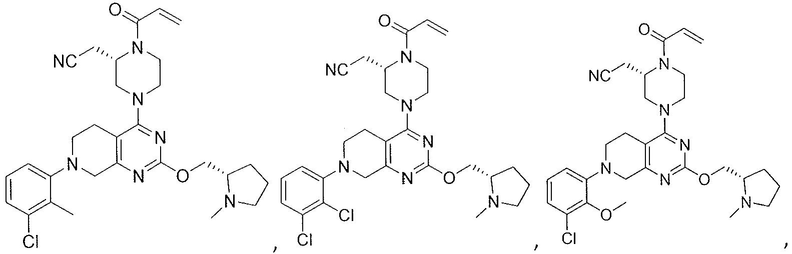 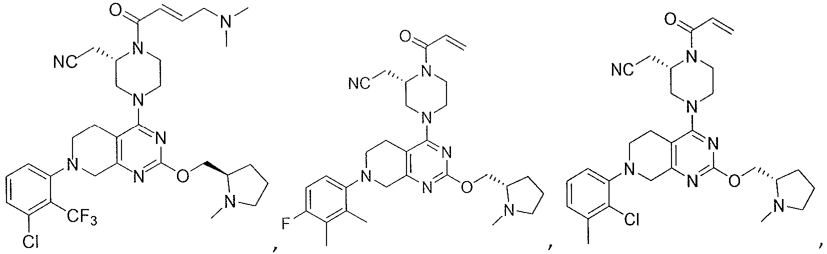 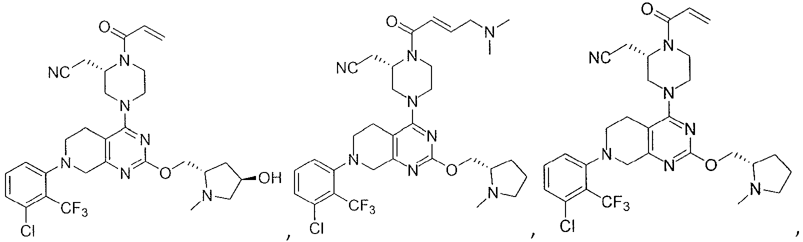 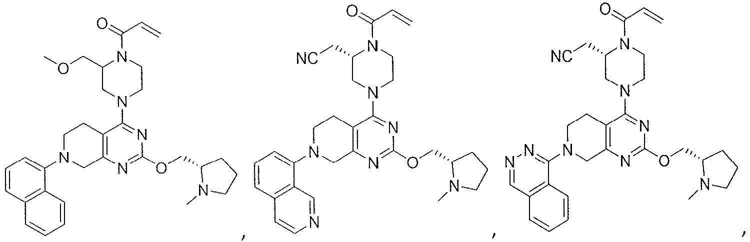 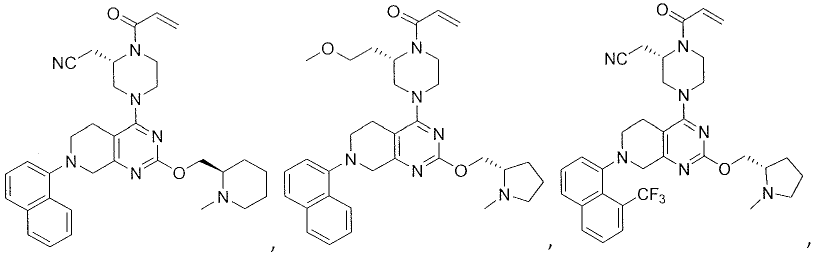 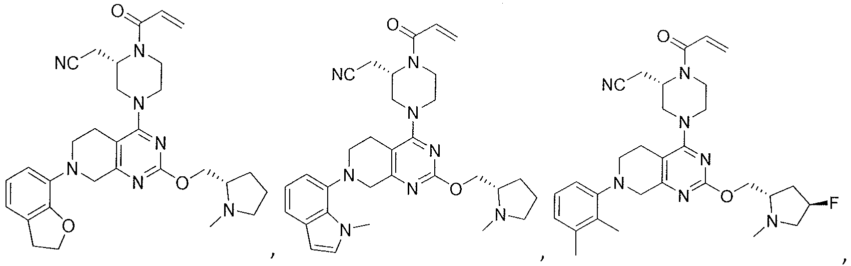 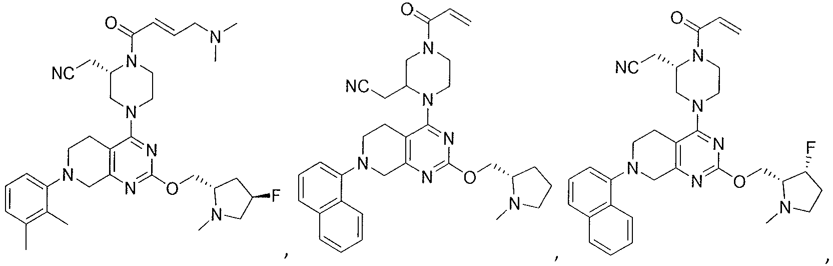 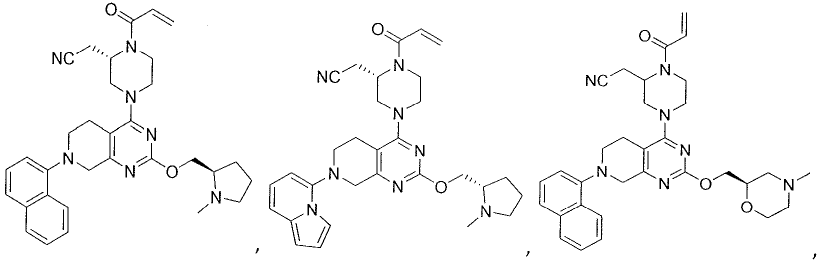 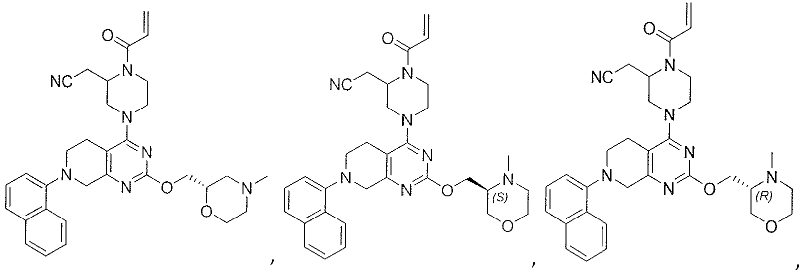 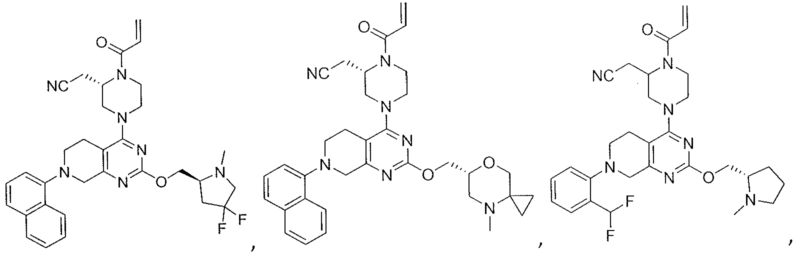 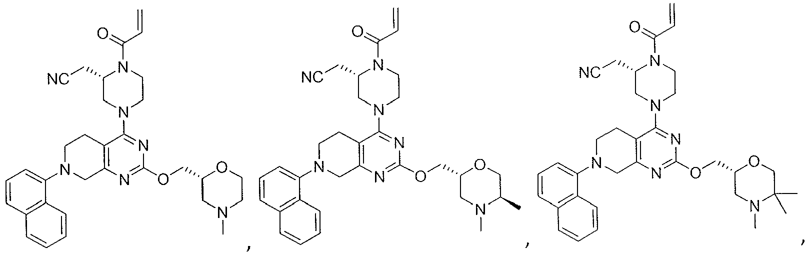 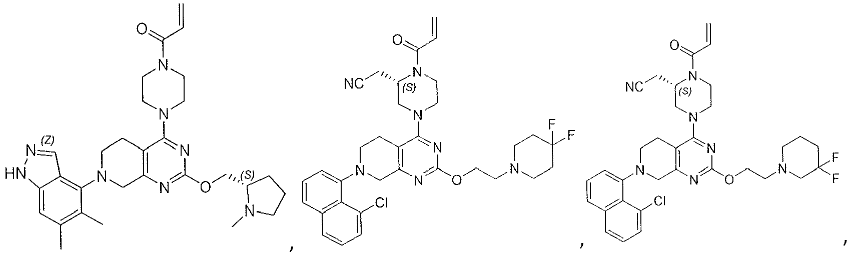 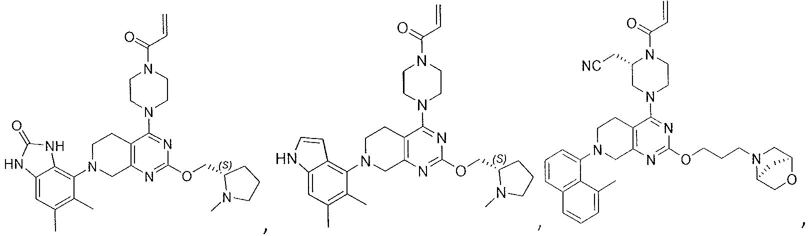 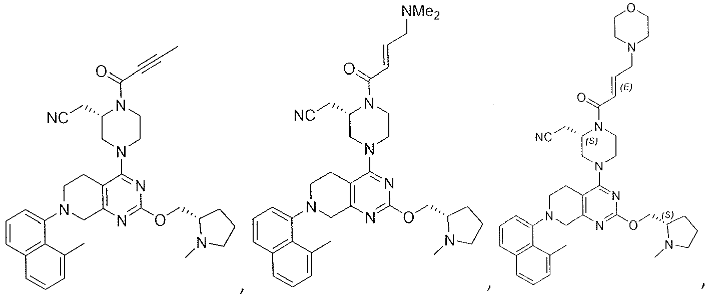 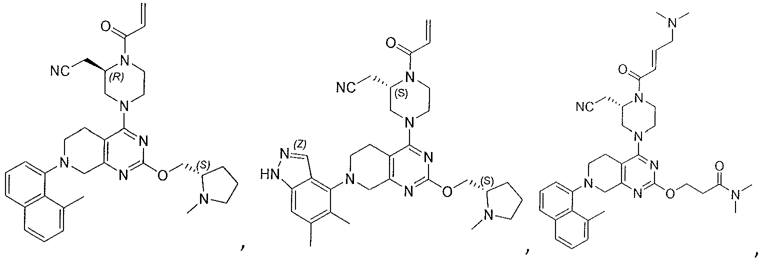 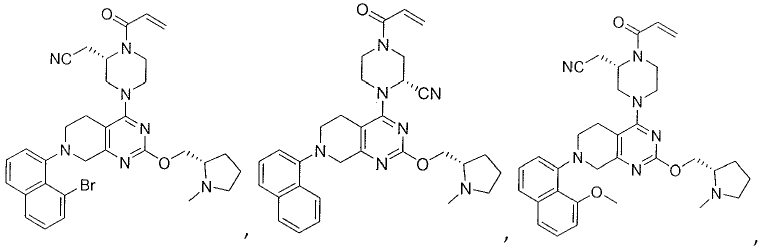 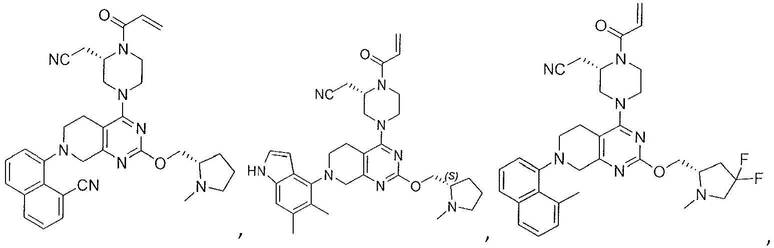 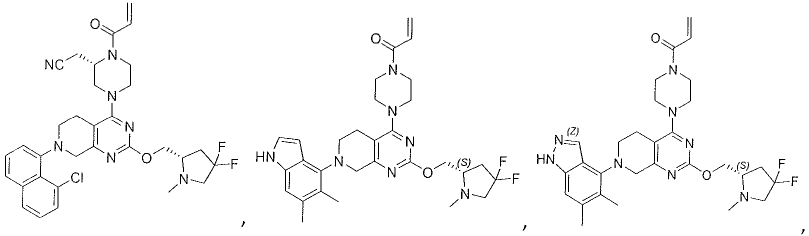 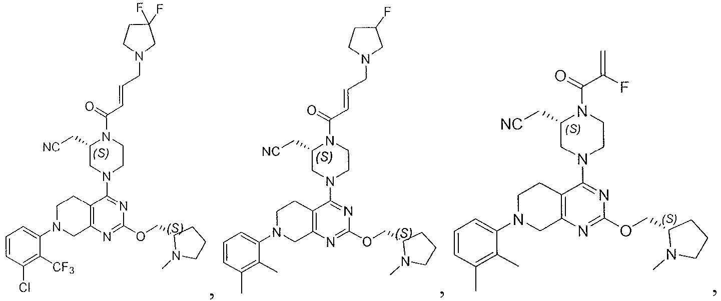 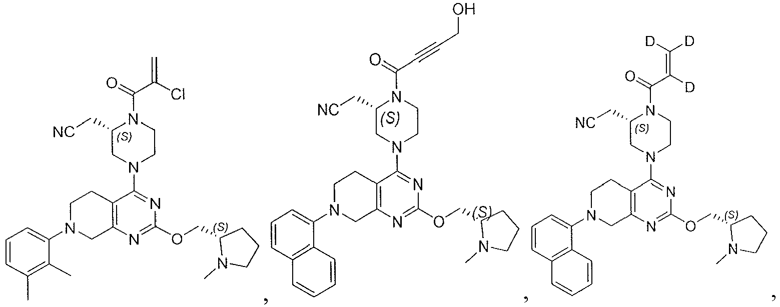 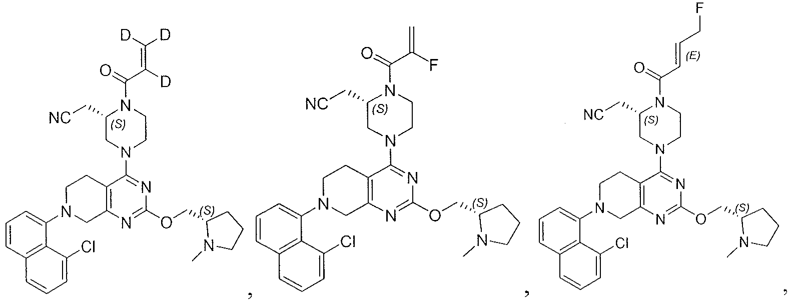 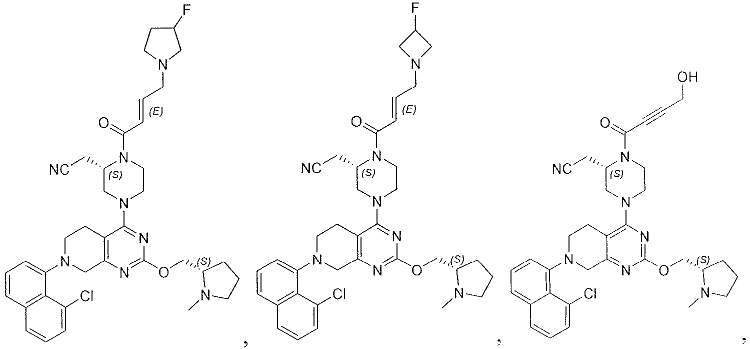 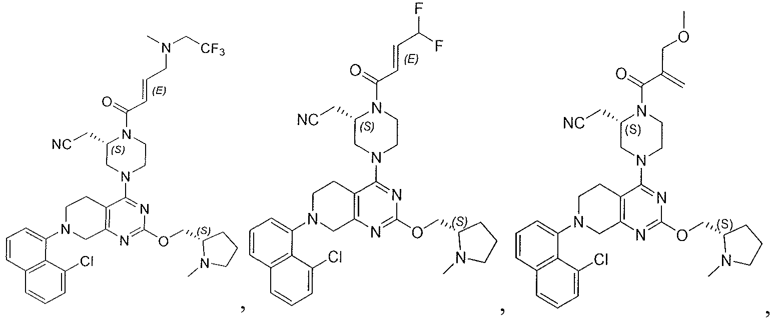 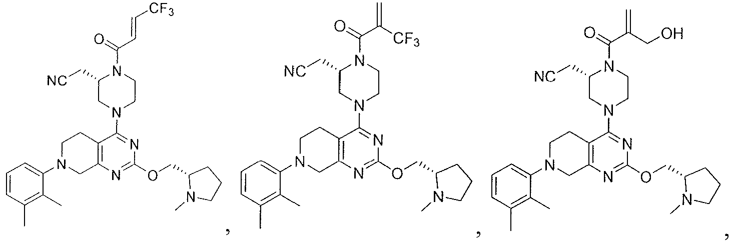 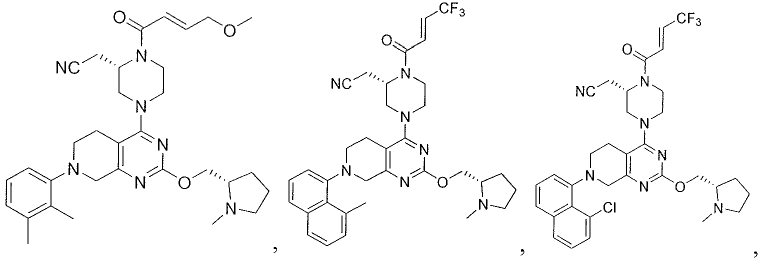 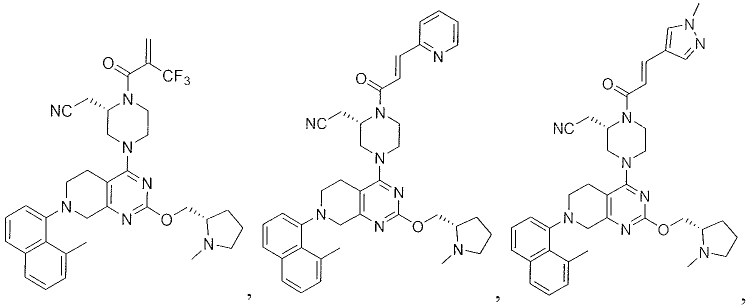 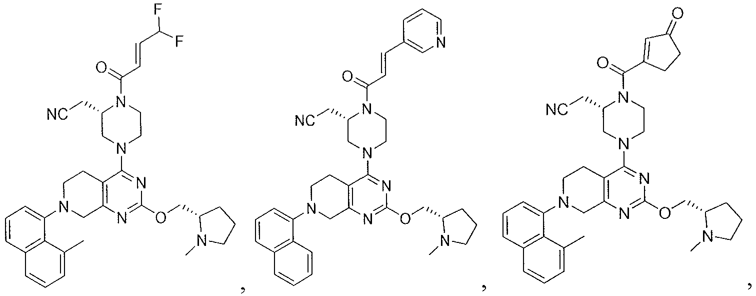 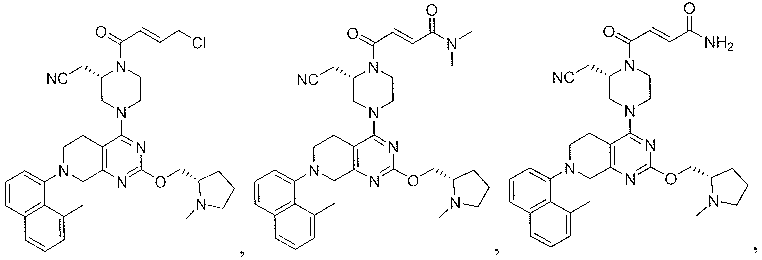 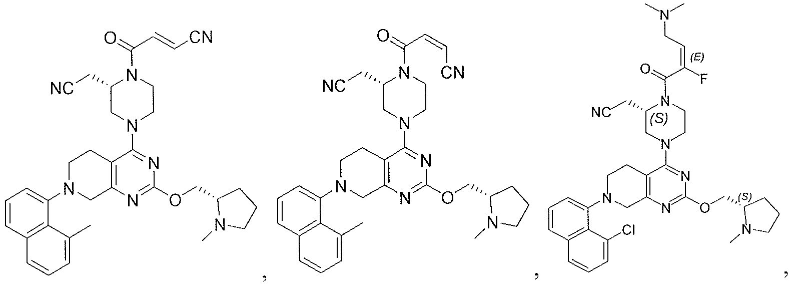 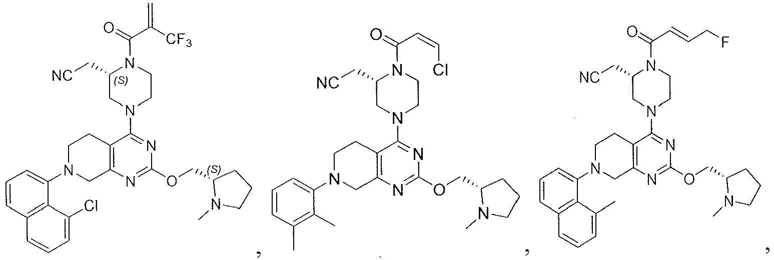 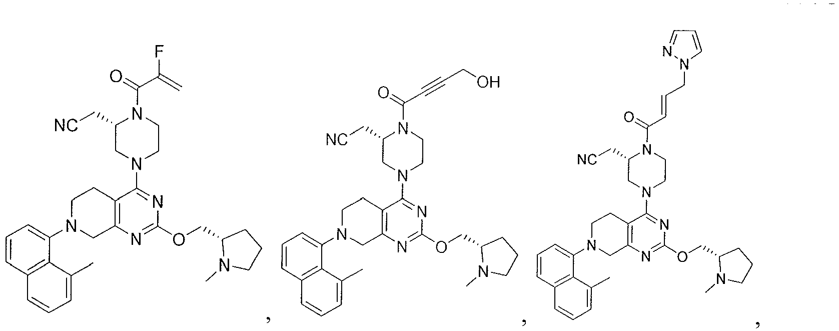 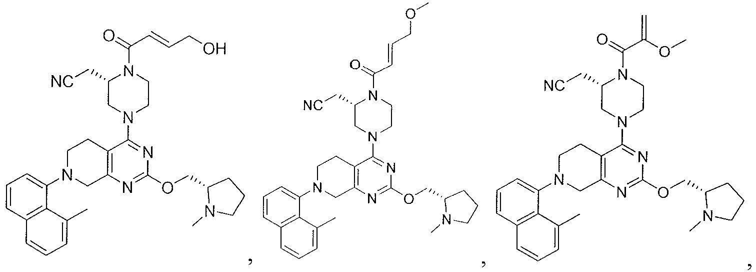 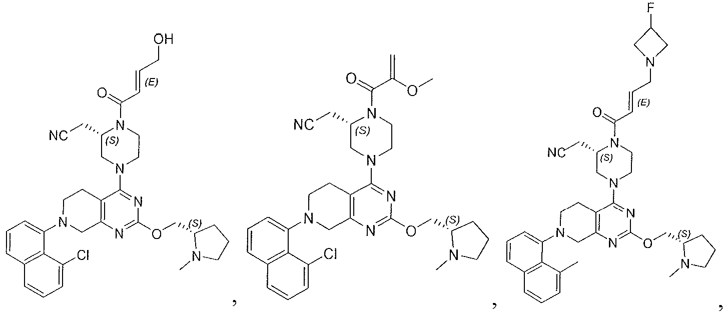 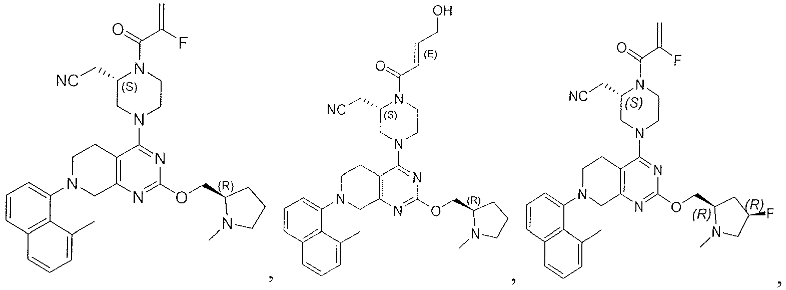 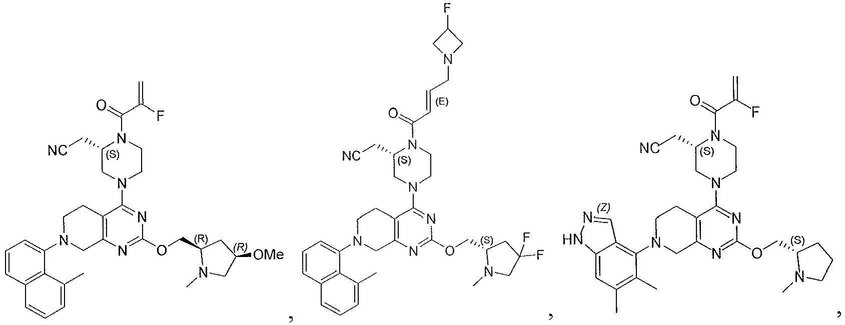 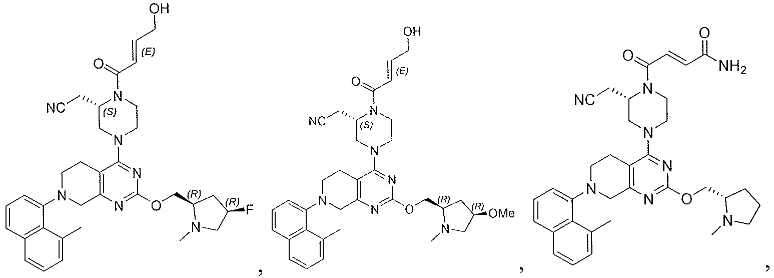 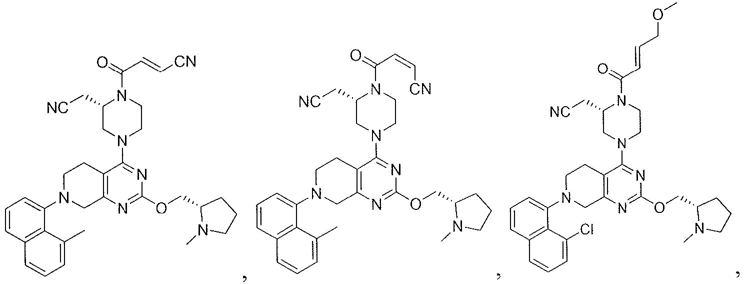 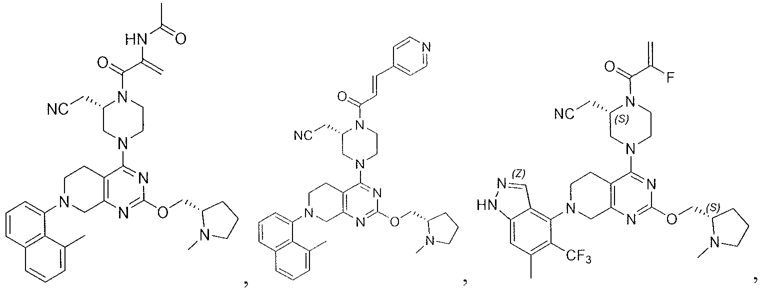 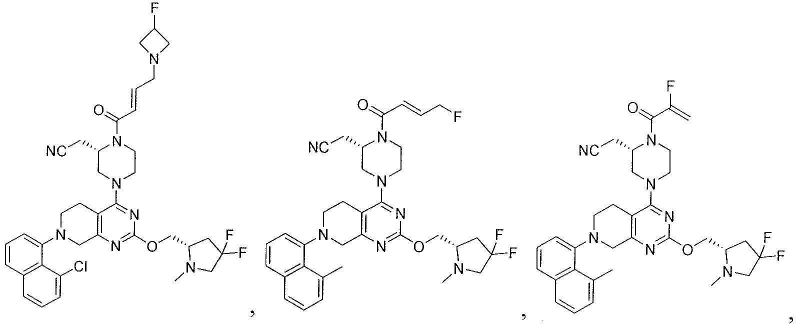 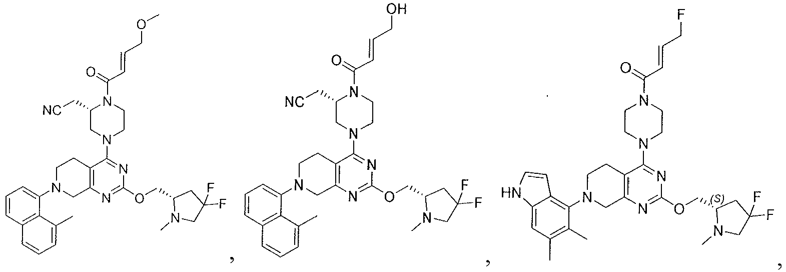 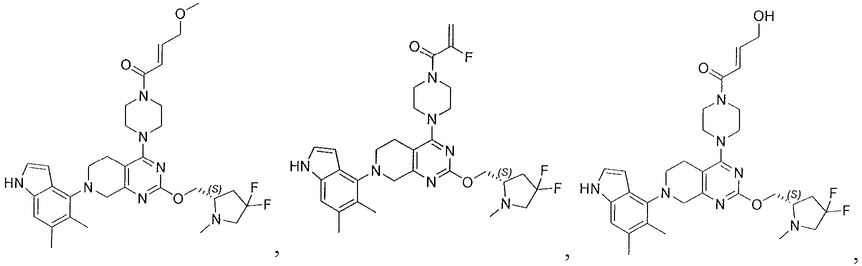 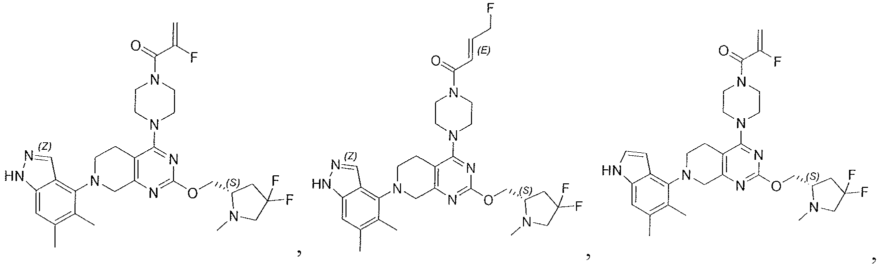 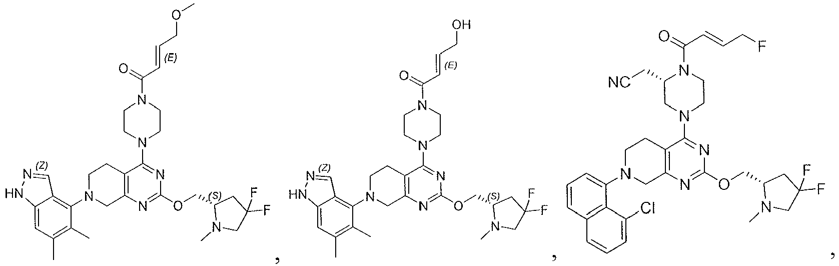 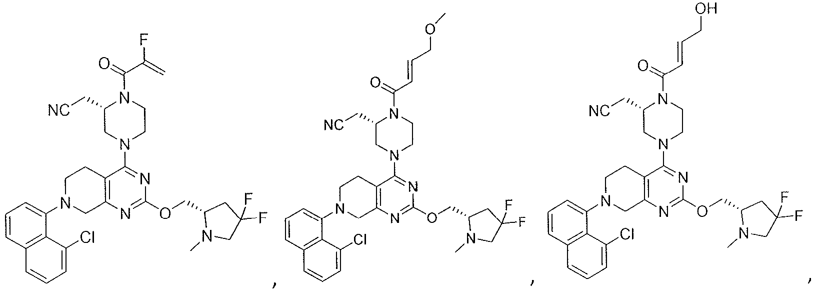 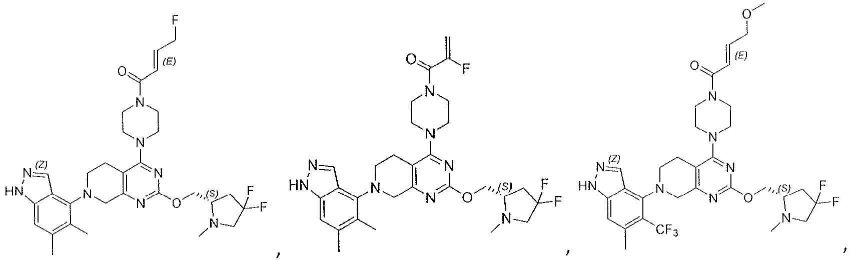 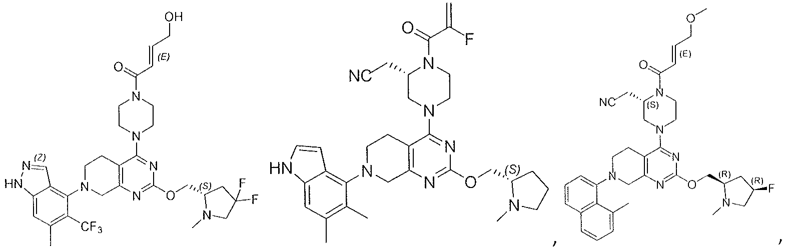 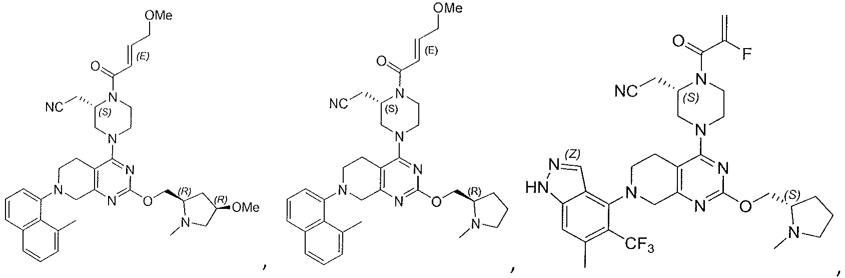 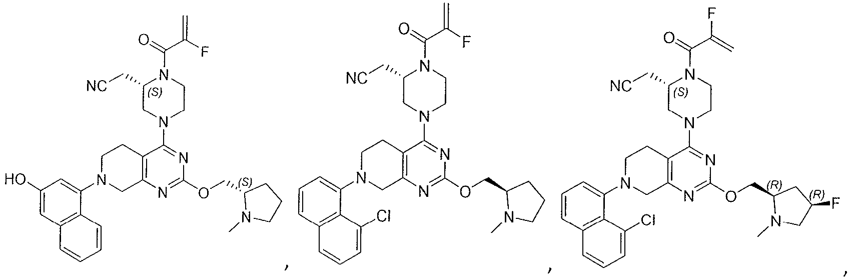 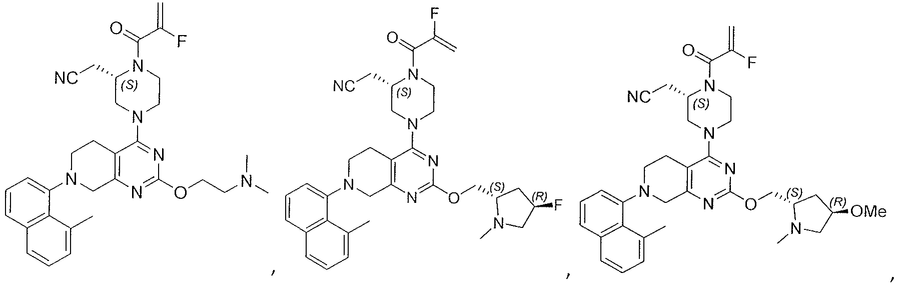 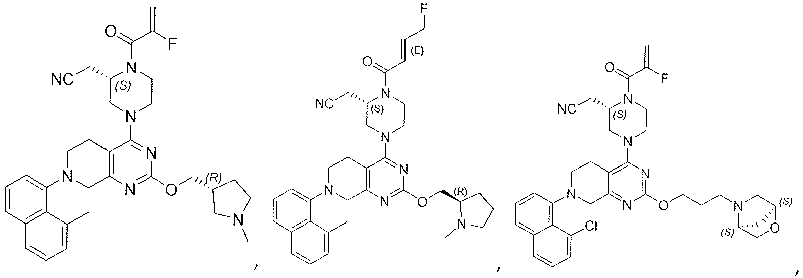 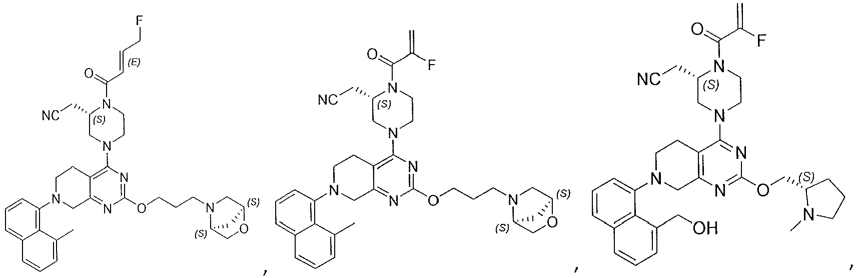 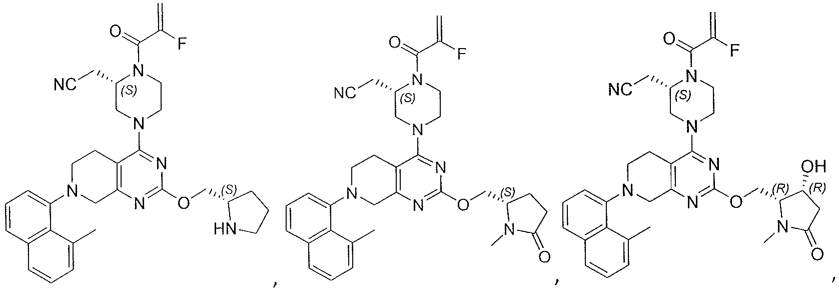 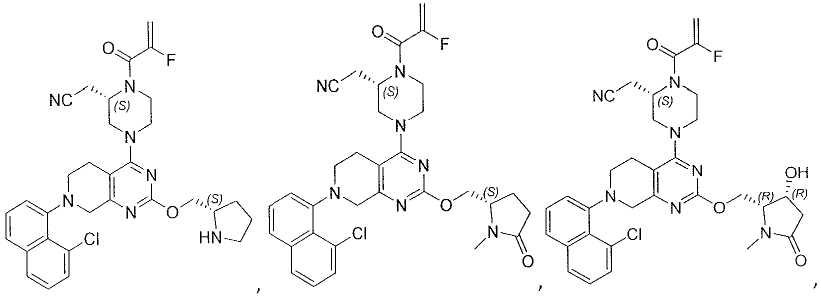 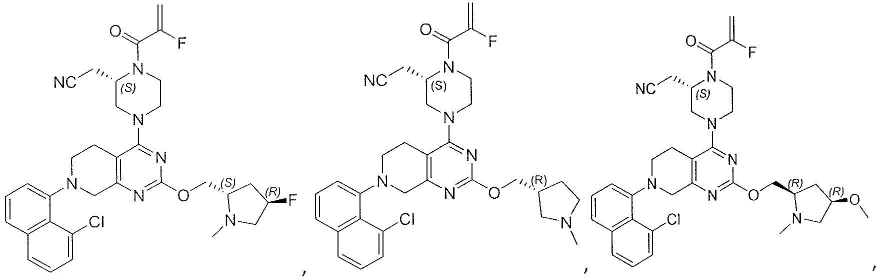 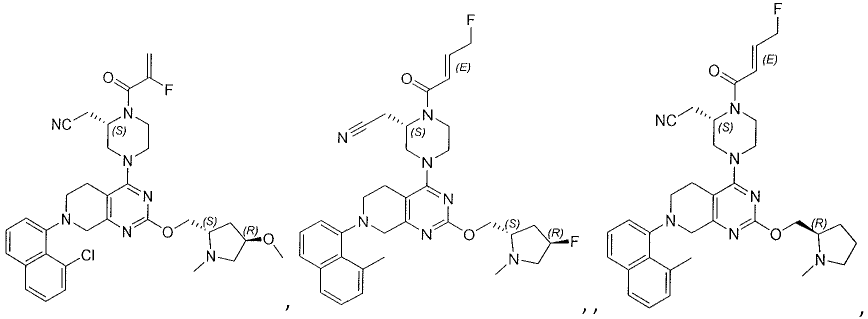 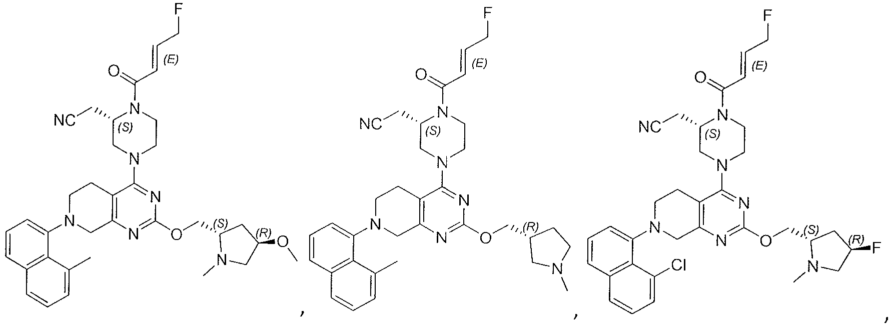 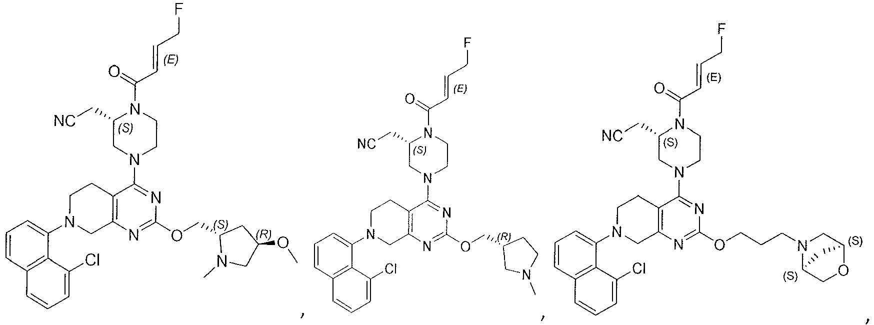 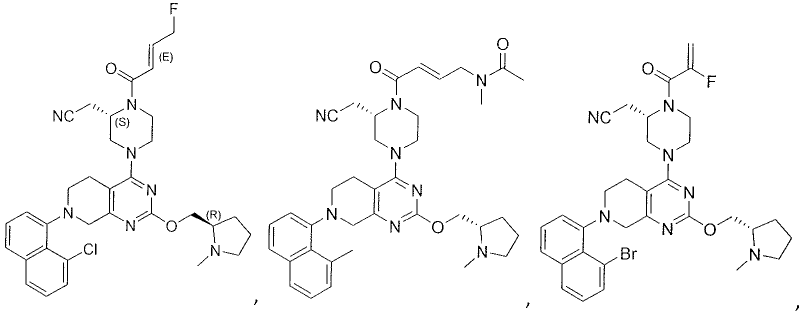 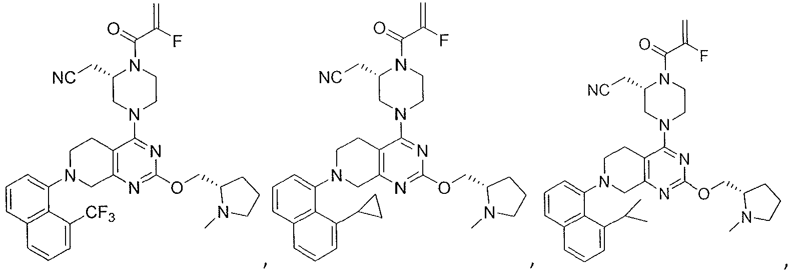 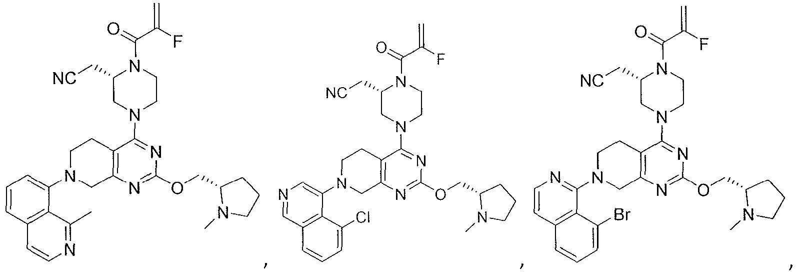 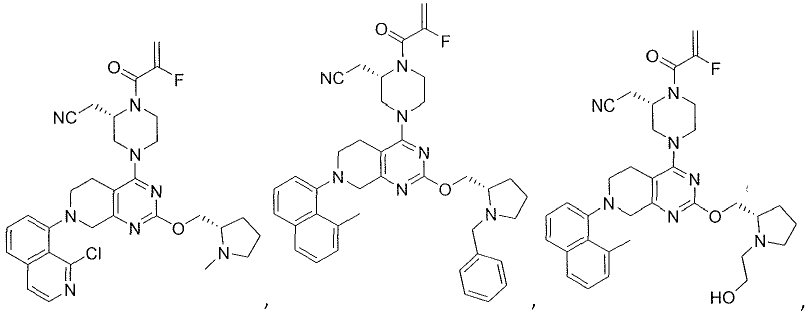 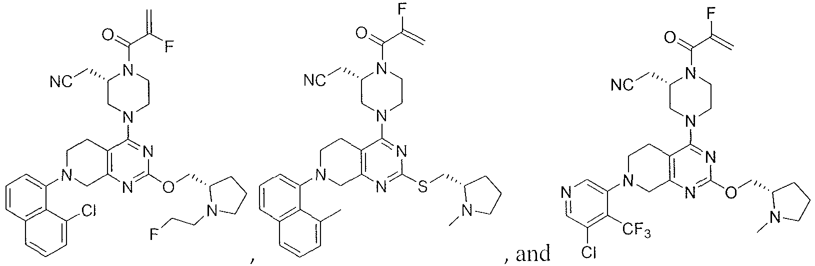 and pharmaceutically acceptable salts thereof.

In one embodiment, the KRas G12C inhibitor is selected from: 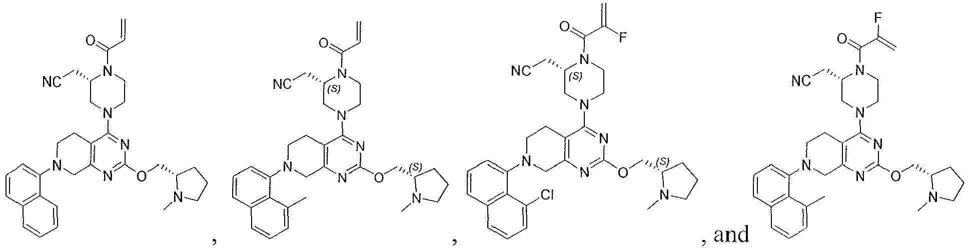 and pharmaceutically acceptable salts thereof.

In one embodiment, the KRas G12C inhibitor is: 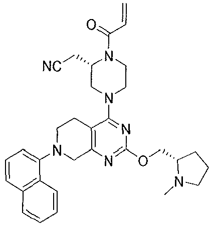 (also referred to herein as Example No. 234) or a pharmaceutically acceptable salt thereof.

In one embodiment, the KRas G12C inhibitor is: 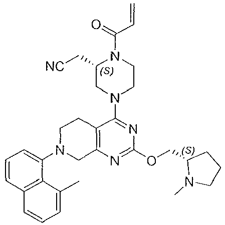 (also referred to herein as Example No. 359) or a pharmaceutically acceptable salt thereof.

In one embodiment, the KRas G12C inhibitor is: 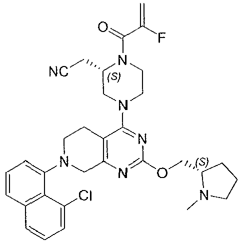 (also referred to herein as Example No. 478) or a pharmaceutically acceptable salt thereof.

In one embodiment, the KRas G12C inhibitor is: 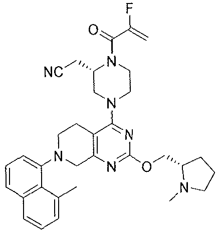 (also referred to herein as Example No. 507) or a pharmaceutically acceptable salt thereof.

The KRas G12C inhibitors used in the methods of the present invention may have one or more chiral center and may be synthesized as stereoisomeric mixtures, isomers of identical constitution that differ in the arrangement of their atoms in space. The compounds may be used as mixtures or the individual components/isomers may be separated using commercially available reagents and conventional methods for isolation of stereoisomers and enantiomers well-known to those skilled in the art, e.g., using CHIRALPAK^{®} (Sigma-Aldrich) or CHIRALCEL^{®} (Diacel Corp) chiral chromatographic HPLC columns according to the manufacturer's instructions. Alternatively, compounds of the present invention may be synthesized using optically pure, chiral reagents and intermediates to prepare individual isomers or enantiomers. Unless otherwise indicated, all chiral (enantiomeric and diastereomeric) and racemic forms are within the scope of the invention. Unless otherwise indicated, whenever the specification, including the claims, refers to compounds of the invention, the term "compound" is to be understood to encompass all chiral (enantiomeric and diastereomeric) and racemic forms.

In one embodiment, the KRas G12C inhibitor compounds of Formula I, Formula I-A, or Formula I-B used in the methods include trifluoroacetic acid salts of the above compounds.

Methods for manufacturing the KRas G12C inhibitors disclosed herein are known. For example, commonly owned published international PCT application numbers WO2017201161 and WO2019099524 describe general reaction schemes for preparing compounds of Formula I, Formula I-A, or Formula I-B and also provide detailed synthetic routes for the preparation of each KRas G12C inhibitor disclosed herein.

The Src-family kinase inhibitors and the KRas G12C compounds of Formula (I), Formula I-A, or Formula I-B or pharmaceutically acceptable salts thereof may be formulated into pharmaceutical compositions.

### PHARMACEUTICAL COMPOSITIONS

In another aspect, the invention provides pharmaceutical compositions comprising a Src-family kinase inhibitor and KRas G12C inhibitor according to the invention and a pharmaceutically acceptable carrier, excipient, or diluent that may be used in the methods disclosed herein. The Src-family kinase inhibitor and KRas G12C inhibitor may be independently formulated by any method well known in the art and may be prepared for administration by any route, including, without limitation, parenteral, oral, sublingual, transdermal, topical, intranasal, intratracheal, or intrarectal. In certain embodiments, Src-family kinase inhibitor and/or KRas G12C inhibitor are administered intravenously in a hospital setting. In one embodiment, administration may be by the oral route.

The characteristics of the carrier will depend on the route of administration. As used herein, the term "pharmaceutically acceptable" means a non-toxic material that is compatible with a biological system such as a cell, cell culture, tissue, or organism, and that does not interfere with the effectiveness of the biological activity of the active ingredient(s). Thus, compositions may contain, in addition to the inhibitor, diluents, fillers, salts, buffers, stabilizers, solubilizers, and other materials well known in the art. The preparation of pharmaceutically acceptable formulations is described in, e.g., Remington's Pharmaceutical Sciences, 18th Edition, ed. A. Gennaro, Mack Publishing Co., Easton, Pa., 1990.

As used herein, the term pharmaceutically acceptable salt refers to salts that retain the desired biological activity of the above-identified compounds and exhibit minimal or no undesired toxicological effects. Examples of such salts include, but are not limited to acid addition salts formed with inorganic acids (for example, hydrochloric acid, hydrobromic acid, sulfuric acid, phosphoric acid, nitric acid, and the like), and salts formed with organic acids such as acetic acid, oxalic acid, tartaric acid, succinic acid, malic acid, ascorbic acid, benzoic acid, tannic acid, pamoic acid, alginic acid, polyglutamic acid, naphthalenesulfonic acid, naphthalenedisulfonic acid, and polygalacturonic acid. The compounds can also be administered as pharmaceutically acceptable quaternary salts known by those skilled in the art, which specifically include the quaternary ammonium salt of the formula --NR+Z-, wherein R is hydrogen, alkyl, or benzyl, and Z is a counterion, including chloride, bromide, iodide, -O-alkyl, toluenesulfonate, methylsulfonate, sulfonate, phosphate, or carboxylate (such as benzoate, succinate, acetate, glycolate, maleate, malate, citrate, tartrate, ascorbate, benzoate, cinnamoate, mandeloate, benzyloate, and diphenylacetate).

The active compound is included in the pharmaceutically acceptable carrier or diluent in an amount sufficient to deliver to a patient a therapeutically effective amount without causing serious toxic effects in the patient treated. In one embodiment, a dose of the active compound for all of the above-mentioned conditions is in the range from about 0.01 to 300 mg/kg, for example 0.1 to 100 mg/kg per day, and as a further example 0.5 to about 25 mg per kilogram body weight of the recipient per day. A typical topical dosage will range from 0.01-3% wt/wt in a suitable carrier. The effective dosage range of the pharmaceutically acceptable derivatives can be calculated based on the weight of the parent compound to be delivered. If the derivative exhibits activity in itself, the effective dosage can be estimated as above using the weight of the derivative, or by other means known to those skilled in the art.

The pharmaceutical compositions comprising a Src-family kinase inhibitor and a KRas G12C inhibitor may be used in the methods of use described herein.

### CO-ADMINSTRATION

The Src-family kinase inhibitor and the KRas G12C inhibitor can be formulated into separate or individual dosage forms which can be co-administered one after the other. Another option is that if the route of administration is the same (e.g. oral) two active compounds can be formulated into a single form for co-administration, both methods of co-administration, however, being part of the same therapeutic treatment or regimen.

The pharmaceutical compositions comprising a Src-family kinase inhibitor and/or a KRas G12C inhibitor for use in the methods may be for simultaneous, separate or sequential use. In one embodiment, the Src-family kinase inhibitor is administered prior to administration of the KRas G12C inhibitor compound of Formula (I), Formula I-A or Formula I-B. In another embodiment, the Src-family kinase inhibitor is administered after administration of the KRas G12C inhibitor compound of Formula (I), Formula I-A or Formula I-B. In another embodiment, the Src-family kinase inhibitor is administered at about the same time as administration of the KRas G12C inhibitor compound of Formula (I), Formula I-A or Formula I-B.

Separate administration of each inhibitor, at different times and by different routes, in some cases would be advantageous. Thus, the components in the combination i.e. the KRas G12C inhibitor compound of Formula (I), Formula I-A or Formula I-B or a pharmaceutically acceptable salt thereof and the Src-family kinase inhibitor, need not be necessarily administered at essentially the same time or in any order.

Oncology drugs are typically administered at the maximum tolerated dose ("MTD"), which is the highest dose of drug that does not cause unacceptable side effects. In one embodiment, the KRas G12C inhibitor and the Src-family kinase inhibitor are each dosed at their respective MTDs. In one embodiment, the KRas G12C inhibitor is dosed at its MTD and the Src-family kinase inhibitor is dosed in an amount less than its MTD. In one embodiment, the KRas G12C inhibitor is dosed at an amount less than its MTD and the Src-family kinase inhibitor is dosed at its MTD. In one embodiment, the KRas G12C inhibitor and the Src-family kinase inhibitor are each dosed at less than their respective MTDs. The administration can be so timed that the peak pharmacokinetic effect of one compound coincides with the peak pharmacokinetic effect of the other.

In one embodiment, a single dose of KRas G12C inhibitor compound of Formula (I), Formula I-A or Formula I-B or a pharmaceutically acceptable salt thereof is administered per day (i.e., in about 24 hour intervals) (i.e., QD). In another embodiment, two doses of the KRas G12C inhibitor compound of Formula (I), Formula I-A or Formula I-B or a pharmaceutically acceptable salt thereof are administered per day (i.e., BID). In another embodiment, three doses of the KRas G12C inhibitor compound of Formula (I), Formula I-A or Formula I-B are administered per day (i.e., TID).

In one embodiment, the Src-family kinase inhibitor is administered QD. In another embodiment, the Src-family kinase inhibitor is administered BID. In another embodiment, the Src-family kinase inhibitor of the invention is administered TID.

In one embodiment, a single dose of KRas G12C inhibitor compound of Formula (I), Formula I-A or Formula I-B or a pharmaceutically acceptable salt thereof and Src-family kinase inhibitor are each administered once daily.

Exemplary inhibitors of Src-family kinases useful in the methods disclosed herein include, but are not limited to: dasatinib (N-(2-chloro-6-methylphenyl)-2-((6-(4-(2-hydroxyethyl)piperazin-1-yl)-2-methylpyrimidin-4-yl)amino)thiazole-5-carboxamide): ponatinib (3-(imidazo[1,2-b]pyridazin-3-ylethynyl)-4-methyl-N-(4-((4-methylpiperazin-1-yl)methyl)-3-(trifluoromethyl)phenyl)benzamide); vandetanib (N-(4-bromo-2-fluorophenyl)-6-methoxy-7-((1-methylpiperidin-4-yl)methoxy)quinazolin-4-amine); bosutinib (4-((2,4-dichloro-5-methoxyphenyl)amino)-6-methoxy-7-(3-(4-methylpiperazin-1-yl)propoxy)quinoline-3-carbonitrile); saracatinib (N-(5-chlorobenzo[d][1,3]dioxol-4-yl)-7-(2-(4-methylpiperazin-1-yl)ethoxy)-5-((tetrahydro-2H-pyran-4-yl)oxy)quinazolin-4-amine); KX2-391 (N-benzyl-2-(5-(4-(2-morpholinoethoxy)phenyl)pyridin-2-yl)acetamide); bafetinib (N-[3-(4,5'-bipyrimidin-2-ylamino)-4-methylphenyl]-4-[3(S)-(dimethylamino)pyrrolidin-1-ylmethyl]-3-(trifluoromethyl)benzamide; SU6656 ((Z)-N,N-dimethyl-2-oxo-3-((4,5,6,7-tetrahydro-1H-indol-2-yl)methylene)indoline-5-sulfonamide); PP1 (1-(tert-butyl)-3-(p-tolyl)-1H-pyrazolo[3,4-d]pyrimidin-4-amine); and WH-4-023 (2,6-dimethylphenyl (2,4-dimethoxyphenyl)(2-((4-(4-methylpiperazin-1-yl)phenyl)amino)pyrimidin-4-yl)carbamate); and KX-01(N-benzyl-2-[5-[4-[2-(4-morpholinyl)ethoxy]phenyl]pyridin-2-yl]acetamide). In one embodiment, the Src inhibitor is dasatinib. In one embodiment, the Src inhibitor is vandetanib. In one embodiment, the Src inhibitor is KX-01. In one embodiment, the Src inhibitor is saracatinib. In one embodiment, the Src inhibitor is ponatinib.

### COMBINATION THERAPIES

In one aspect of the invention, provided herein are methods of treating cancer in a subject in need thereof, comprising administering to the subject a therapeutically effective amount of a combination of a Src-family kinase inhibitor and a KRAS G12C inhibitor of Formula (I), Formula I-A or Formula I-B or a pharmaceutically acceptable salt thereof. In one embodiment, the cancer is a KRas G12C-associated cancer. In one embodiment, the KRas G12C-associated cancer is lung cancer.

In yet another aspect, the invention provides for methods for increasing the sensitivity of a cancer cell to a KRas G12C inhibitor, comprising contacting the cancer cell with an effective amount of a combination of a KRas G12C inhibitor compound of Formula (I), Formula I-A, or Formula I-B and a Src-family kinase inhibitor, or pharmaceutically acceptable salts or pharmaceutical compositions thereof, wherein the Src-family kinase inhibitor synergistically increases the sensitivity of the cancer cell to the KRas G12C inhibitor. In one embodiment, the contacting is in vitro. In one embodiment, the contacting is in vivo.

In one embodiment, the combination therapy comprises a combination of a compound having the formula 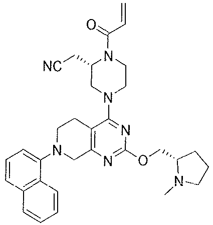 or a pharmaceutically acceptable salt thereof, and a Src inhibitor. In one embodiment, the Src inhibitor is dasatinib. In one embodiment, the Src inhibitor is vandetanib. In one embodiment, the Src inhibitor is KX-01. In one embodiment, the Src inhibitor is saracatinib. In one embodiment, the Src inhibitor is ponatinib.

In one embodiment, the combination therapy comprises a combination of a compound having the formula 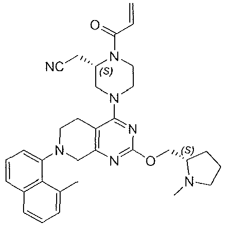 or a pharmaceutically acceptable salt thereof, and a Src inhibitor. In one embodiment, the Src inhibitor is dasatinib. In one embodiment, the Src inhibitor is vandetanib. In one embodiment, the Src inhibitor is KX-01. In one embodiment, the Src inhibitor is saracatinib. In one embodiment, the Src inhibitor is ponatinib.

In one embodiment, the combination therapy comprises a combination of a compound having the formula 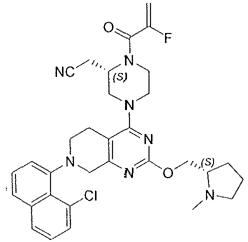 or a pharmaceutically acceptable salt thereof, and a Src inhibitor. In one embodiment, the Src inhibitor is dasatinib. In one embodiment, the Src inhibitor is vandetanib. In one embodiment, the Src inhibitor is KX-01. In one embodiment, the Src inhibitor is saracatinib. In one embodiment, the Src inhibitor is ponatinib.

In one embodiment, the combination therapy comprises a combination of a compound having the formula 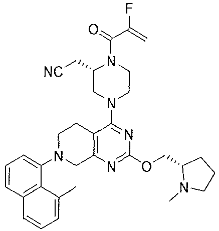 or a pharmaceutically acceptable salt thereof, and a Src inhibitor. In one embodiment, the Src inhibitor is dasatinib. In one embodiment, the Src inhibitor is vandetanib. In one embodiment, the Src inhibitor is KX-01. In one embodiment, the Src inhibitor is saracatinib. In one embodiment, the Src inhibitor is ponatinib.

As used herein, the term "contacting" refers to the bringing together of indicated moieties in an in vitro system or an in vivo system. For example, "contacting" a cancer cell includes the administration of a combination provided herein to an individual or subject, such as a human, having KRas G12C, as well as, for example, introducing a combination provided herein into a sample containing a cellular or purified preparation containing KRas G12C.

By negatively modulating the activity of KRas G12C, the methods described herein are designed to inhibit undesired cellular proliferation resulting from enhanced KRas G12C activity within the cell. The degree of covalent modification of KRas G12C may be monitored in vitro using well known methods, including those described herein and in published international PCT application numbers WO2017201161 and WO2019099524. In addition, the inhibitory activity of combination in cells may be monitored, for example, by measuring the inhibition of KRas G12C activity of the amount of phosphorylated ERK to assess the effectiveness of treatment and dosages may be adjusted accordingly by the attending medical practitioner.

The compositions and methods provided herein may be used for the treatment of a KRas G12C-associated cancer in a subject in need thereof, comprising administering to said subject a therapeutically effective amount of a combination of a Src-family kinase inhibitor and a KRas G12C inhibitor compound of Formula (I), Formula I-A, or Formula I-B, or pharmaceutically acceptable salts or pharmaceutical compositions thereof, wherein the Src-family kinase inhibitor synergistically increases the sensitivity of the KRas G12C-associated cancer to the KRas G12C inhibitor. In one embodiment, the KRas G12C-associated cancer is lung cancer.

In one embodiment, the therapeutically effective amount of the combination of a Src-family kinase inhibitor and a KRas G12C inhibitor compound of Formula (I), Formula I-A, or Formula I-B, or pharmaceutically acceptable salts or pharmaceutical compositions thereof, results in an increased duration of overall survival ("OS") in subjects relative to treatment with only the KRas G12C inhibitor. In one embodiment, the therapeutically effective amount of the combination of a Src-family kinase inhibitor and a KRas G12C inhibitor compound of Formula (I), Formula I-A, or Formula I-B, or pharmaceutically acceptable salts or pharmaceutical compositions thereof, results in an increased duration of progression-free survival ("PFS") in subjects relative to treatment with only the KRas G12C inhibitor. In one embodiment, the therapeutically effective amount of the combination of a Src-family kinase inhibitor and a KRas G12C inhibitor compound of Formula (I), Formula I-A, or Formula I-B, or pharmaceutically acceptable salts or pharmaceutical compositions thereof, results in increased tumor regression in subjects relative to treatment with only the KRas G12C inhibitor. In one embodiment, the therapeutically effective amount of the combination of a Src-family kinase inhibitor and a KRas G12C inhibitor compound of Formula (I), Formula I-A, or Formula I-B, or pharmaceutically acceptable salts or pharmaceutical compositions thereof, results in increased tumor growth inhibition in subjects relative to treatment with only the KRas G12C inhibitor. In one embodiment, the therapeutically effective amount of the combination of a Src-family kinase inhibitor and a KRas G12C inhibitor compound of Formula (I), Formula I-A, or Formula I-B, or pharmaceutically acceptable salts or pharmaceutical compositions thereof, results in an improvement in the duration of stable disease in subjects compared to treatment with only the KRas G12C inhibitor. In one embodiment, the KRas G12C inhibitor is a compound selected from compound Nos. 1-527, or a pharmaceutically acceptable salt thereof (e.g., Example No. 234, 359, 478 or 507 or a pharmaceutically acceptable salt thereof). In one embodiment, the Src inhibitor is selected from dasatinib, vandetanib, KX-01, saracatinib and ponatinib. In one embodiment, the therapeutic combination comprises therapeutically effective amounts of Example No. 234 and dasatinib. In one embodiment, the therapeutic combination comprises therapeutically effective amounts of Example No. 234 and vandetanib. In one embodiment, the therapeutic combination comprises therapeutically effective amounts of Example No. 234 and KX-01. In one embodiment, the therapeutic combination comprises therapeutically effective amounts of Example No. 234 and saracatinib. In one embodiment, the therapeutic combination comprises therapeutically effective amounts of Example No. 234 and ponatinib. In one embodiment, the therapeutic combination comprises a therapeutically effective amount of Example No. 359 and dasatinib. In one embodiment, the therapeutic combination comprises therapeutically effective amounts of Example No. 359 and vandetanib. In one embodiment, the therapeutic combination comprises therapeutically effective amounts of Example No. 359 and KX-01. In one embodiment, the therapeutic combination comprises therapeutically effective amounts of Example No. 359 and saracatinib. In one embodiment, the therapeutic combination comprises therapeutically effective amounts of Example No. 359 and ponatinib. In one embodiment, the therapeutic combination comprises a therapeutically effective amount of Example No. 478 and dasatinib. In one embodiment, the therapeutic combination comprises therapeutically effective amounts of Example No. 478 and vandetanib. In one embodiment, the therapeutic combination comprises therapeutically effective amounts of Example No. 478 and KX-01. In one embodiment, the therapeutic combination comprises therapeutically effective amounts of Example No. 478 and saracatinib. In one embodiment, the therapeutic combination comprises therapeutically effective amounts of Example No. 478 and ponatinib. In one embodiment, the therapeutic combination comprises a therapeutically effective amount of Example No. 507 and dasatinib. In one embodiment, the therapeutic combination comprises therapeutically effective amounts of Example No. 507 and vandetanib. In one embodiment, the therapeutic combination comprises therapeutically effective amounts of Example No. 507 and KX-01. In one embodiment, the therapeutic combination comprises therapeutically effective amounts of Example No. 507 and saracatinib. In one embodiment, the therapeutic combination comprises therapeutically effective amounts of Example No. 507 and ponatinib. In one embodiment of any of said combination therapies, the combination is useful for treating a KRas G12C-associated cancer. In one embodiment, the KRas G12C-associated cancer is lung cancer.

In another embodiment, the Src-family kinase inhibitor is administered in combination with the KRas G12C inhibitor once disease progression has been observed for KRas G12C monotherapy, in which the combination therapy results in enhanced clinical benefit for the patient by increasing OS, PFS, tumor regression, tumor growth inhibition or the duration of stable disease in the patient. In one embodiment, the KRas G12C inhibitor is a compound selected from compound Nos. 1-527, or a pharmaceutically acceptable salt thereof (e.g., Example No. 234, 359, 478 or 507 or a pharmaceutically acceptable salt thereof). In one embodiment, the Src inhibitor is selected from dasatinib, vandetanib, KX-01, saracatinib and ponatinib. In one embodiment, the therapeutic combination comprises therapeutically effective amounts of Example No. 234 and dasatinib. In one embodiment, the therapeutic combination comprises therapeutically effective amounts of Example No. 234 and vandetanib. In one embodiment, the therapeutic combination comprises therapeutically effective amounts of Example No. 234 and KX-01. In one embodiment, the therapeutic combination comprises therapeutically effective amounts of Example No. 234 and saracatinib. In one embodiment, the therapeutic combination comprises therapeutically effective amounts of Example No. 234 and ponatinib. In one embodiment, the therapeutic combination comprises a therapeutically effective amount of Example No. 359 and dasatinib. In one embodiment, the therapeutic combination comprises therapeutically effective amounts of Example No. 359 and vandetanib. In one embodiment, the therapeutic combination comprises therapeutically effective amounts of Example No. 359 and KX-01. In one embodiment, the therapeutic combination comprises therapeutically effective amounts of Example No. 359 and saracatinib. In one embodiment, the therapeutic combination comprises therapeutically effective amounts of Example No. 359 and ponatinib. In one embodiment, the therapeutic combination comprises a therapeutically effective amount of Example No. 478 and dasatinib. In one embodiment, the therapeutic combination comprises therapeutically effective amounts of Example No. 478 and vandetanib. In one embodiment, the therapeutic combination comprises therapeutically effective amounts of Example No. 478 and KX-01. In one embodiment, the therapeutic combination comprises therapeutically effective amounts of Example No. 478 and saracatinib. In one embodiment, the therapeutic combination comprises therapeutically effective amounts of Example No. 478 and ponatinib. In one embodiment, the therapeutic combination comprises a therapeutically effective amount of Example No. 507 and dasatinib. In one embodiment, the therapeutic combination comprises therapeutically effective amounts of Example No. 507 and vandetanib. In one embodiment, the therapeutic combination comprises therapeutically effective amounts of Example No. 507 and KX-01. In one embodiment, the therapeutic combination comprises therapeutically effective amounts of Example No. 507 and saracatinib. In one embodiment, the therapeutic combination comprises therapeutically effective amounts of Example No. 507 and ponatinib. In one embodiment of any of said combination therapies, the combination is useful for treating a KRas G12C-associated cancer. In one embodiment, the KRas G12C-associated cancer is lung cancer.

The compositions and methods provided herein may be used for the treatment of a wide variety of cancers including tumors such as lung, colorectal, pancreas, prostate, breast, brain, skin, cervical carcinomas, testicular carcinomas, etc. More particularly, cancers that may be treated by the compositions and methods of the invention include, but are not limited to, tumor types such as astrocytic, breast, cervical, colorectal, endometrial, esophageal, gastric, head and neck, hepatocellular, laryngeal, lung, oral, ovarian, prostate and thyroid carcinomas and sarcomas. More specifically, these compounds can be used to treat: Cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma; Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Kaposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor (nephroblastoma), lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Biliary tract: gall bladder carcinoma, ampullary carcinoma, cholangiocarcinoma; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma (pinealoma), glioblastoma multiform, oligodendroglioma, schwannoma, retinoblastoma, congenital tumors), spinal cord neurofibroma, meningioma, glioma, sarcoma); Gynecological: uterus (endometrial carcinoma), cervix (cervical carcinoma, pre-tumor cervical dysplasia), ovaries (ovarian carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma); Hematologic: blood (myeloid leukemia (acute and chronic), acute lymphoblastic leukemia, chronic lymphocytic leukemia, myeloproliferative diseases, multiple myeloma, myelodysplastic syndrome), Hodgkin's disease, non-Hodgkin's lymphoma (malignant lymphoma); Skin: malignant melanoma, basal cell carcinoma, squamous cell carcinoma, Kaposi's sarcoma, moles dysplastic nevi, lipoma, angioma, dermatofibroma, keloids, psoriasis; and Adrenal glands: neuroblastoma. In certain embodiments, the cancer is non-small cell lung cancer.

Also provided herein is a method for treating cancer in a subject in need thereof, the method comprising (a) determining that cancer is associated with a KRas G12C mutation (e.g., a KRas G12C-associated cancer) (e.g., as determined using a regulatory agency-approved, e.g., FDA-approved, assay or kit); and (b) administering to the patient a therapeutically effective amount of a combination of a Src-family inhibitor and a KRas G12C inhibitor compound of Formula I, Formula I-A, Formula 1-B, or pharmaceutically acceptable salts or pharmaceutical compositions thereof, wherein the Src-family kinase inhibitor synergistically increases the sensitivity of the KRas G12C-associated cancer to the KRas G12C inhibitor. In one embodiment, the KRas G12C inhibitor is a compound selected from compound Nos. 1-527, or a pharmaceutically acceptable salt thereof (e.g., Example No. 234, 359, 478 or 507 or a pharmaceutically acceptable salt thereof). In one embodiment, the Src inhibitor is selected from dasatinib, vandetanib, KX-01, saracatinib and ponatinib. In one embodiment, the therapeutic combination comprises therapeutically effective amounts of Example No. 234 and dasatinib. In one embodiment, the therapeutic combination comprises therapeutically effective amounts of Example No. 234 and vandetanib. In one embodiment, the therapeutic combination comprises therapeutically effective amounts of Example No. 234 and KX-01. In one embodiment, the therapeutic combination comprises therapeutically effective amounts of Example No. 234 and saracatinib. In one embodiment, the therapeutic combination comprises therapeutically effective amounts of Example No. 234 and ponatinib. In one embodiment, the therapeutic combination comprises a therapeutically effective amount of Example No. 359 and dasatinib. In one embodiment, the therapeutic combination comprises therapeutically effective amounts of Example No. 359 and vandetanib. In one embodiment, the therapeutic combination comprises therapeutically effective amounts of Example No. 359 and KX-01. In one embodiment, the therapeutic combination comprises therapeutically effective amounts of Example No. 359 and saracatinib. In one embodiment, the therapeutic combination comprises therapeutically effective amounts of Example No. 359 and ponatinib. In one embodiment, the therapeutic combination comprises a therapeutically effective amount of Example No. 478 and dasatinib. In one embodiment, the therapeutic combination comprises therapeutically effective amounts of Example No. 478 and vandetanib. In one embodiment, the therapeutic combination comprises therapeutically effective amounts of Example No. 478 and KX-01. In one embodiment, the therapeutic combination comprises therapeutically effective amounts of Example No. 478 and saracatinib. In one embodiment, the therapeutic combination comprises therapeutically effective amounts of Example No. 478 and ponatinib. In one embodiment, the therapeutic combination comprises a therapeutically effective amount of Example No. 507 and dasatinib. In one embodiment, the therapeutic combination comprises therapeutically effective amounts of Example No. 507 and vandetanib. In one embodiment, the therapeutic combination comprises therapeutically effective amounts of Example No. 507 and KX-01. In one embodiment, the therapeutic combination comprises therapeutically effective amounts of Example No. 507 and saracatinib. In one embodiment, the therapeutic combination comprises therapeutically effective amounts of Example No. 507 and ponatinib. In one embodiment of any of said combination therapies, the combination is useful for treating a KRas G12C-associated cancer. In one embodiment, the KRas G12C-associated cancer is lung cancer.

In one embodiment, a compound of Formula I is administered as a capsule during the period of time. In one embodiment, a tablet or capsule formulation of a compound of Formula I comprises about 10 mg to about 100 mg (e.g., about 10 mg to about 95 mg, about 10 mg to about 90 mg, about 10 mg to about 85 mg, about 10 mg to about 80 mg, about 10 mg to about 75 mg, about 10 mg to about 70 mg, about 10 mg to about 65 mg, about 10 mg to about 60 mg, about 10 mg to about 55 mg, about 10 mg to about 50 mg, about 10 mg to about 45 mg, about 10 mg to about 40 mg, about 10 mg to about 35 mg, about 10 mg to about 30 mg, about 10 mg to about 25 mg, about 10 mg to about 20 mg, about 10 mg to about 15 mg, about 15 mg to about 100 mg, about 15 mg to about 95 mg, about 15 mg to about 90 mg, about 15 mg to about 85 mg, about 15 mg to about 80 mg, about 15 mg to about 75 mg, about 15 mg to about 70 mg, about 15 mg to about 65 mg, about 15 mg to about 60 mg, about 15 mg to about 55 mg, about 15 mg to about 50 mg, about 15 mg to about 45 mg, about 15 mg to about 40 mg, about 15 mg to about 35 mg, about 15 mg to about 30 mg, about 15 mg to about 25 mg, about 15 mg to about 20 mg, about 20 mg to about 100 mg, about 20 mg to about 95 mg, about 20 mg to about 90 mg, about 20 mg to about 85 mg, about 20 mg to about 80 mg, about 20 mg to about 75 mg, about 20 mg to about 70 mg, about 20 mg to about 65 mg, about 20 mg to about 60 mg, about 20 mg to about 55 mg, about 20 mg to about 50 mg, about 20 mg to about 45 mg, about 20 mg to about 40 mg, about 20 mg to about 35 mg, about 20 mg to about 30 mg, about 20 mg to about 25 mg, about 25 mg to about 100 mg, about 25 mg to about 95 mg, about 25 mg to about 90 mg, about 25 mg to about 85 mg, about 25 mg to about 80 mg, about 25 mg to about 75 mg, about 25 mg to about 70 mg, about 25 mg to about 65 mg, about 25 mg to about 60 mg, about 25 mg to about 55 mg, about 25 mg to about 50 mg, about 25 mg to about 45 mg, about 25 mg to about 40 mg, about 25 mg to about 35 mg, about 25 mg to about 30 mg, about 30 mg to about 100 mg, about 30 mg to about 95 mg, about 30 mg to about 90 mg, about 30 mg to about 85 mg, about 30 mg to about 80 mg, about 30 mg to about 75 mg, about 30 mg to about 70 mg, about 30 mg to about 65 mg, about 30 mg to about 60 mg, about 30 mg to about 55 mg, about 30 mg to about 50 mg, about 30 mg to about 45 mg, about 30 mg to about 40 mg, about 30 mg to about 35 mg, about 35 mg to about 100 mg, about 35 mg to about 95 mg, about 35 mg to about 90 mg, about 35 mg to about 85 mg, about 35 mg to about 80 mg, about 35 mg to about 75 mg, about 35 mg to about 70 mg, about 35 mg to about 65 mg, about 35 mg to about 60 mg, about 35 mg to about 55 mg, about 35 mg to about 50 mg, about 35 mg to about 45 mg, about 35 mg to about 40 mg, about 40 mg to about 100 mg, about 40 mg to about 95 mg, about 40 mg to about 90 mg, about 40 mg to about 85 mg, about 40 mg to about 80 mg, about 40 mg to about 75 mg, about 40 mg to about 70 mg, about 40 mg to about 65 mg, about 40 mg to about 60 mg, about 40 mg to about 55 mg, about 40 mg to about 50 mg, about 40 mg to about 45 mg, about 45 mg to about 100 mg, about 45 mg to about 95 mg, about 45 mg to about 90 mg, about 45 mg to about 85 mg, about 45 mg to about 80 mg, about 45 mg to about 75 mg, about 45 mg to about 70 mg, about 45 mg to about 65 mg, about 45 mg to about 60 mg, about 45 mg to about 55 mg, about 45 mg to about 50 mg, about 50 mg to about 100 mg, about 50 mg to about 95 mg, about 50 mg to about 90 mg, about 50 mg to about 85 mg, about 50 mg to about 80 mg, about 50 mg to about 75 mg, about 50 mg to about 70 mg, about 50 mg to about 65 mg, about 50 mg to about 60 mg, about 50 mg to about 55 mg, about 55 mg to about 100 mg, about 55 mg to about 95 mg, about 55 mg to about 90 mg, about 55 mg to about 85 mg, about 55 mg to about 80 mg, about 55 mg to about 75 mg, about 55 mg to about 70 mg, about 55 mg to about 65 mg, about 55 mg to about 60 mg, about 60 mg to about 100 mg, about 60 mg to about 95 mg, about 60 mg to about 90 mg, about 60 mg to about 85 mg, about 60 mg to about 80 mg, about 60 mg to about 75 mg, about 60 mg to about 70 mg, about 60 mg to about 65 mg, about 65 mg to about 100 mg, about 65 mg to about 95 mg, about 65 mg to about 90 mg, about 65 mg to about 85 mg, about 65 mg to about 80 mg, about 65 mg to about 75 mg, about 65 mg to about 70 mg, about 70 mg to about 100 mg, about 70 mg to about 95 mg, about 70 mg to about 90 mg, about 70 mg to about 85 mg, about 70 mg to about 80 mg, about 70 mg to about 75 mg, about 75 mg to about 100 mg, about 75 mg to about 95 mg, about 75 mg to about 90 mg, about 75 mg to about 85 mg, about 75 mg to about 80 mg, about 80 mg to about 100 mg, about 80 mg to about 95 mg, about 80 mg to about 90 mg, about 80 mg to about 85 mg, about 85 mg to about 100 mg, about 85 mg to about 95 mg, about 85 mg to about 90 mg, about 90 mg to about 100 mg, about 90 mg to about 95 mg, about 95 mg to about 100 mg, about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, or about 100 mg) of a compound of Formula I (e.g., a compound selected from compound Nos. 1-553, e.g., compound No. 234, 359, 478 or 507). In one embodiment, a compound of Formula I is orally administered once a day (QD) on a daily basis during a period of time. In one embodiment, a compound of Formula I is orally administered twice a day (BID) on a daily basis during a period of time. In one embodiment, a compound of Formula I is orally administered in the amount of about 20 mg to about 500 mg (e.g., about 20 mg to about 480 mg, about 20 mg to about 460 mg, about 20 mg to about 440 mg, about 20 mg to about 420 mg, about 20 mg to about 400 mg, about 20 mg to about 380 mg, about 20 mg to about 360 mg, about 20 mg to about 340 mg, about 20 mg to about 320 mg, about 20 mg to about 300 mg, about 20 mg to about 280 mg, about 20 mg to about 260 mg, about 20 mg to about 240 mg, about 20 mg to about 220 mg, about 20 mg to about 200 mg, about 20 mg to about 180 mg, about 20 mg to about 160 mg, about 20 mg to about 140 mg, about 20 mg to about 120 mg, about 20 mg to about 100 mg, about 20 mg to about 80 mg, about 20 mg to about 60 mg, about 20 mg to about 40 mg, about 40 mg to about 500 mg, about 40 mg to about 480 mg, about 40 mg to about 460 mg, about 40 mg to about 440 mg, about 40 mg to about 420 mg, about 40 mg to about 400 mg, about 40 mg to about 380 mg, about 40 mg to about 360 mg, about 40 mg to about 340 mg, about 40 mg to about 320 mg, about 40 mg to about 300 mg, about 40 mg to about 280 mg, about 40 mg to about 260 mg, about 40 mg to about 240 mg, about 40 mg to about 220 mg, about 40 mg to about 200 mg, about 40 mg to about 180 mg, about 40 mg to about 160 mg, about 40 mg to about 140 mg, about 40 mg to about 120 mg, about 40 mg to about 100 mg, about 40 mg to about 80 mg, about 40 mg to about 60 mg, about 60 mg to about 500 mg, about 60 mg to about 480 mg, about 60 mg to about 460 mg, about 60 mg to about 440 mg, about 60 mg to about 420 mg, about 60 mg to about 400 mg, about 60 mg to about 380 mg, about 60 mg to about 360 mg, about 60 mg to about 340 mg, about 60 mg to about 320 mg, about 60 mg to about 300 mg, about 60 mg to about 280 mg, about 60 mg to about 260 mg, about 60 mg to about 240 mg, about 60 mg to about 220 mg, about 60 mg to about 200 mg, about 60 mg to about 180 mg, about 60 mg to about 160 mg, about 60 mg to about 140 mg, about 60 mg to about 120 mg, about 60 mg to about 100 mg, about 60 mg to about 80 mg, about 80 mg to about 500 mg, about 80 mg to about 480 mg, about 80 mg to about 460 mg, about 80 mg to about 440 mg, about 80 mg to about 420 mg, about 80 mg to about 400 mg, about 80 mg to about 380 mg, about 80 mg to about 360 mg, about 80 mg to about 340 mg, about 80 mg to about 320 mg, about 80 mg to about 300 mg, about 80 mg to about 280 mg, about 80 mg to about 260 mg, about 80 mg to about 240 mg, about 80 mg to about 220 mg, about 80 mg to about 200 mg, about 80 mg to about 180 mg, about 80 mg to about 160 mg, about 80 mg to about 140 mg, about 80 mg to about 120 mg, about 80 mg to about 100 mg, about 100 mg to about 500 mg, about 100 mg to about 480 mg, about 100 mg to about 460 mg, about 100 mg to about 440 mg, about 100 mg to about 420 mg, about 100 mg to about 400 mg, about 100 mg to about 380 mg, about 100 mg to about 360 mg, about 100 mg to about 340 mg, about 100 mg to about 320 mg, about 100 mg to about 300 mg, about 100 mg to about 280 mg, about 100 mg to about 260 mg, about 100 mg to about 240 mg, about 100 mg to about 220 mg, about 100 mg to about 200 mg, about 100 mg to about 180 mg, about 100 mg to about 160 mg, about 100 mg to about 140 mg, about 100 mg to about 120 mg, about 120 mg to about 500 mg, about 120 mg to about 480 mg, about 120 mg to about 460 mg, about 120 mg to about 440 mg, about 120 mg to about 420 mg, about 120 mg to about 400 mg, about 120 mg to about 380 mg, about 120 mg to about 360 mg, about 120 mg to about 340 mg, about 120 mg to about 320 mg, about 120 mg to about 300 mg, about 120 mg to about 280 mg, about 120 mg to about 260 mg, about 120 mg to about 240 mg, about 120 mg to about 220 mg, about 120 mg to about 200 mg, about 120 mg to about 180 mg, about 120 mg to about 160 mg, about 120 mg to about 140 mg, about 140 mg to about 500 mg, about 140 mg to about 480 mg, about 140 mg to about 460 mg, about 140 mg to about 440 mg, about 140 mg to about 420 mg, about 140 mg to about 400 mg, about 140 mg to about 380 mg, about 140 mg to about 360 mg, about 140 mg to about 340 mg, about 140 mg to about 320 mg, about 140 mg to about 300 mg, about 140 mg to about 280 mg, about 140 mg to about 260 mg, about 140 mg to about 240 mg, about 140 mg to about 220 mg, about 140 mg to about 200 mg, about 140 mg to about 180 mg, about 140 mg to about 160 mg, about 160 mg to about 500 mg, about 160 mg to about 480 mg, about 160 mg to about 460 mg, about 160 mg to about 440 mg, about 160 mg to about 420 mg, about 160 mg to about 400 mg, about 160 mg to about 380 mg, about 160 mg to about 360 mg, about 160 mg to about 340 mg, about 160 mg to about 320 mg, about 160 mg to about 300 mg, about 160 mg to about 280 mg, about 160 mg to about 260 mg, about 160 mg to about 240 mg, about 160 mg to about 220 mg, about 160 mg to about 200 mg, about 160 mg to about 180 mg, about 180 mg to about 500 mg, about 180 mg to about 480 mg, about 180 mg to about 460 mg, about 180 mg to about 440 mg, about 180 mg to about 420 mg, about 180 mg to about 400 mg, about 180 mg to about 380 mg, about 180 mg to about 360 mg, about 180 mg to about 340 mg, about 180 mg to about 320 mg, about 180 mg to about 300 mg, about 180 mg to about 280 mg, about 180 mg to about 260 mg, about 180 mg to about 240 mg, about 180 mg to about 220 mg, about 180 mg to about 200 mg, about 200 mg to about 500 mg, about 200 mg to about 480 mg, about 200 mg to about 460 mg, about 200 mg to about 440 mg, about 200 mg to about 420 mg, about 200 mg to about 400 mg, about 200 mg to about 380 mg, about 200 mg to about 360 mg, about 200 mg to about 340 mg, about 200 mg to about 320 mg, about 200 mg to about 300 mg, about 200 mg to about 280 mg, about 200 mg to about 260 mg, about 200 mg to about 240 mg, about 200 mg to about 220 mg, about 220 mg to about 500 mg, about 220 mg to about 480 mg, about 220 mg to about 460 mg, about 220 mg to about 440 mg, about 220 mg to about 420 mg, about 220 mg to about 400 mg, about 220 mg to about 380 mg, about 220 mg to about 360 mg, about 220 mg to about 340 mg, about 220 mg to about 320 mg, about 220 mg to about 300 mg, about 220 mg to about 280 mg, about 220 mg to about 260 mg, about 220 mg to about 240 mg, about 240 mg to about 500 mg, about 240 mg to about 480 mg, about 240 mg to about 460 mg, about 240 mg to about 440 mg, about 240 mg to about 420 mg, about 240 mg to about 400 mg, about 240 mg to about 380 mg, about 240 mg to about 360 mg, about 240 mg to about 340 mg, about 240 mg to about 320 mg, about 240 mg to about 300 mg, about 240 mg to about 280 mg, about 240 mg to about 260 mg, about 260 mg to about 500 mg, about 260 mg to about 480 mg, about 260 mg to about 460 mg, about 260 mg to about 440 mg, about 260 mg to about 420 mg, about 260 mg to about 400 mg, about 260 mg to about 380 mg, about 260 mg to about 360 mg, about 260 mg to about 340 mg, about 260 mg to about 320 mg, about 260 mg to about 300 mg, about 260 mg to about 280 mg, about 280 mg to about 500 mg, about 280 mg to about 480 mg, about 280 mg to about 460 mg, about 280 mg to about 440 mg, about 280 mg to about 420 mg, about 280 mg to about 400 mg, about 280 mg to about 380 mg, about 280 mg to about 360 mg, about 280 mg to about 340 mg, about 280 mg to about 320 mg, about 280 mg to about 300 mg, about 300 mg to about 500 mg, about 300 mg to about 480 mg, about 300 mg to about 460 mg, about 300 mg to about 440 mg, about 300 mg to about 420 mg, about 300 mg to about 400 mg, about 300 mg to about 380 mg, about 300 mg to about 360 mg, about 300 mg to about 340 mg, about 300 mg to about 320 mg, about 320 mg to about 500 mg, about 320 mg to about 480 mg, about 320 mg to about 460 mg, about 320 mg to about 440 mg, about 320 mg to about 420 mg, about 320 mg to about 400 mg, about 320 mg to about 380 mg, about 320 mg to about 360 mg, about 320 mg to about 340 mg, about 340 mg to about 500 mg, about 340 mg to about 480 mg, about 340 mg to about 460 mg, about 340 mg to about 440 mg, about 340 mg to about 420 mg, about 340 mg to about 400 mg, about 340 mg to about 380 mg, about 340 mg to about 360 mg, about 360 mg to about 500 mg, about 360 mg to about 480 mg, about 360 mg to about 460 mg, about 360 mg to about 440 mg, about 360 mg to about 420 mg, about 360 mg to about 400 mg, about 360 mg to about 380 mg, about 380 mg to about 500 mg, about 380 mg to about 480 mg, about 380 mg to about 460 mg, about 380 mg to about 440 mg, about 380 mg to about 420 mg, about 380 mg to about 400 mg, about 400 mg to about 500 mg, about 400 mg to about 480 mg, about 400 mg to about 460 mg, about 400 mg to about 440 mg, about 400 mg to about 420 mg, about 420 mg to about 500 mg, about 420 mg to about 480 mg, about 420 mg to about 460 mg, about 420 mg to about 440 mg, about 440 mg to about 500 mg, about 440 mg to about 480 mg, about 440 mg to about 460 mg, about 460 mg to about 500 mg, about 460 mg to about 480 mg, about 480 mg to about 500 mg, about 25, about 50, about 75, about 100, about 150, about 200, about 250, about 300, about 350, about 400, about 450, or about 500 mg), during a period of time.

In one embodiment, the combination therapy comprises oral administration of a compound of Formula I once or twice a day on a daily basis (during a period of time), e.g., in an amount of about 10 mg to about 400 mg (e.g., about 10 mg to about 380 mg, about 10 mg to about 360 mg, about 10 mg to about 340 mg, about 10 mg to about 320 mg, about 10 mg to about 300 mg, about 10 mg to about 280 mg, about 10 mg to about 260 mg, about 10 mg to about 240 mg, about 10 mg to about 220 mg, about 10 mg to about 200 mg, about 10 mg to about 180 mg, about 10 mg to about 160 mg, about 10 mg to about 140 mg, about 10 mg to about 120 mg, about 10 mg to about 100 mg, about 10 mg to about 80 mg, about 10 mg to about 60 mg, about 10 mg to about 40 mg, about 10 mg to about 20 mg, about 20 mg to about 400 mg, about 20 mg to about 380 mg, about 20 mg to about 360 mg, about 20 mg to about 340 mg, about 20 mg to about 320 mg, about 20 mg to about 300 mg, about 20 mg to about 280 mg, about 20 mg to about 260 mg, about 20 mg to about 240 mg, about 20 mg to about 220 mg, about 20 mg to about 200 mg, about 20 mg to about 180 mg, about 20 mg to about 160 mg, about 20 mg to about 140 mg, about 20 mg to about 120 mg, about 20 mg to about 100 mg, about 20 mg to about 80 mg, about 20 mg to about 60 mg, about 20 mg to about 40 mg, about 40 mg to about 400 mg, about 40 mg to about 380 mg, about 40 mg to about 360 mg, about 40 mg to about 340 mg, about 40 mg to about 320 mg, about 40 mg to about 300 mg, about 40 mg to about 280 mg, about 40 mg to about 260 mg, about 40 mg to about 240 mg, about 40 mg to about 220 mg, about 40 mg to about 200 mg, about 40 mg to about 180 mg, about 40 mg to about 160 mg, about 40 mg to about 140 mg, about 40 mg to about 120 mg, about 40 mg to about 100 mg, about 40 mg to about 80 mg, about 40 mg to about 60 mg, about 60 mg to about 400 mg, about 60 mg to about 380 mg, about 60 mg to about 360 mg, about 60 mg to about 340 mg, about 60 mg to about 320 mg, about 60 mg to about 300 mg, about 60 mg to about 280 mg, about 60 mg to about 260 mg, about 60 mg to about 240 mg, about 60 mg to about 220 mg, about 60 mg to about 200 mg, about 60 mg to about 180 mg, about 60 mg to about 160 mg, about 60 mg to about 140 mg, about 60 mg to about 120 mg, about 60 mg to about 100 mg, about 60 mg to about 80 mg, about 80 mg to about 400 mg, about 80 mg to about 380 mg, about 80 mg to about 360 mg, about 80 mg to about 340 mg, about 80 mg to about 320 mg, about 80 mg to about 300 mg, about 80 mg to about 280 mg, about 80 mg to about 260 mg, about 80 mg to about 240 mg, about 80 mg to about 220 mg, about 80 mg to about 200 mg, about 80 mg to about 180 mg, about 80 mg to about 160 mg, about 80 mg to about 140 mg, about 80 mg to about 120 mg, about 80 mg to about 100 mg, about 100 mg to about 400 mg, about 100 mg to about 380 mg, about 100 mg to about 360 mg, about 100 mg to about 340 mg, about 100 mg to about 320 mg, about 100 mg to about 300 mg, about 100 mg to about 280 mg, about 100 mg to about 260 mg, about 100 mg to about 240 mg, about 100 mg to about 220 mg, about 100 mg to about 200 mg, about 100 mg to about 180 mg, about 100 mg to about 160 mg, about 100 mg to about 140 mg, about 100 mg to about 120 mg, about 120 mg to about 400 mg, about 120 mg to about 380 mg, about 120 mg to about 360 mg, about 120 mg to about 340 mg, about 120 mg to about 320 mg, about 120 mg to about 300 mg, about 120 mg to about 280 mg, about 120 mg to about 260 mg, about 120 mg to about 240 mg, about 120 mg to about 220 mg, about 120 mg to about 200 mg, about 120 mg to about 180 mg, about 120 mg to about 160 mg, about 120 mg to about 140 mg, about 140 mg to about 400 mg, about 140 mg to about 380 mg, about 140 mg to about 360 mg, about 140 mg to about 340 mg, about 140 mg to about 320 mg, about 140 mg to about 300 mg, about 140 mg to about 280 mg, about 140 mg to about 260 mg, about 140 mg to about 240 mg, about 140 mg to about 220 mg, about 140 mg to about 200 mg, about 140 mg to about 180 mg, about 140 mg to about 160 mg, about 160 mg to about 400 mg, about 160 mg to about 380 mg, about 160 mg to about 360 mg, about 160 mg to about 360 mg, about 160 mg to about 340 mg, about 160 mg to about 320 mg, about 160 mg to about 300 mg, about 160 mg to about 280 mg, about 160 mg to about 260 mg, about 160 mg to about 240 mg, about 160 mg to about 220 mg, about 160 mg to about 200 mg, about 160 mg to about 180 mg, about 180 mg to about 400 mg, about 180 mg to about 380 mg, about 180 mg to about 360 mg, about 180 mg to about 340 mg, about 180 mg to about 320 mg, about 180 mg to about 300 mg, about 180 mg to about 280 mg, about 180 mg to about 260 mg, about 180 mg to about 240 mg, about 180 mg to about 220 mg, about 180 mg to about 200 mg, about 200 mg to about 400 mg, about 200 mg to about 380 mg, about 200 mg to about 360 mg, about 200 mg to about 340 mg, about 200 mg to about 320 mg, about 200 mg to about 300 mg, about 200 mg to about 280 mg, about 200 mg to about 260 mg, about 200 mg to about 240 mg, about 200 mg to about 220 mg, about 220 mg to about 400 mg, about 220 mg to about 380 mg, about 220 mg to about 360 mg, about 220 mg to about 340 mg, about 220 mg to about 320 mg, about 220 mg to about 300 mg, about 220 mg to about 280 mg, about 220 mg to about 260 mg, about 220 mg to about 240 mg, about 240 mg to about 400 mg, about 240 mg to about 380 mg, about 240 mg to about 360 mg, about 240 mg to about 340 mg, about 240 mg to about 320 mg, about 240 mg to about 300 mg, about 240 mg to about 280 mg, about 240 mg to about 260 mg, about 260 mg to about 400 mg, about 260 mg to about 380 mg, about 260 mg to about 360 mg, about 260 mg to about 340 mg, about 260 mg to about 320 mg, about 260 mg to about 300 mg, about 260 mg to about 280 mg, about 280 mg to about 400 mg, about 280 mg to about 380 mg, about 280 mg to about 360 mg, about 280 mg to about 340 mg, about 280 mg to about 320 mg, about 280 mg to about 300 mg, about 300 mg to about 400 mg, about 300 mg to about 380 mg, about 300 mg to about 360 mg, about 300 mg to about 340 mg, about 300 mg to about 320 mg, about 320 mg to about 400 mg, about 320 mg to about 380 mg, about 320 mg to about 360 mg, about 340 mg to about 360 mg, about 340 mg to about 400 mg, about 340 mg to about 380 mg, about 340 mg to about 360 mg, about 360 mg to about 400 mg, about 360 mg to about 380 mg, about 380 mg to about 400 mg, about 100 mg, about 200 mg, about 300 mg, or about 400 mg), and oral administration of a Src-family kinase inhibitor which is administered, for example once a day on a daily basis (during a period of time). In one embodiment, the KRAS inhibitor is orally administered once daily. In one embodiment, the KRAS inhibitor is orally administered twice daily.

One skilled in the art will recognize that, both in vivo and in vitro trials using suitable, known and generally accepted cell and/or animal models are predictive of the ability of a test compound of the combination or the combination to treat or prevent a given disorder.

One skilled in the art will further recognize that human clinical trials including first-inhuman, dose ranging and efficacy trials, in healthy patients and/or those suffering from a given disorder, may be completed according to methods well known in the clinical and medical arts.

### SYNERGY

In one embodiment, the addition of a Src-family kinase inhibitor synergistically increases the activity of KRas G12C inhibitor compound of Formula (I), Formula I-A or Formula I-B or a pharmaceutically acceptable salt thereof against cancer or cancer cell lines expressing KRas G12C. Any method for determining whether two compounds exhibit synergy may be used for determining the synergistic effect of the combination.

Several mathematical models have been developed to determine whether two compounds act synergistically, i.e., beyond a mere additive effect. For instance, Loewe Additivity (Loewe (1928) Physiol. 27: 47-187), Bliss Independence (Bliss (1939) Ann. Appl. Biol. 26: 585-615), Highest Single Agent, ZIP (Yadav et al (2015) Comput Struct Biotech J 13: 504-513) and other models (Chou & Talalay (1984) Adv Enzyme Regul 22: 27-55. #6382953; and Greco et al. (1995) Pharmacol Rev 47(2): 331-85. #7568331) are well known models in the pharmaceutical industry and may be used to calculate a "synergy score" that indicates whether synergy was detected and the magnitude of such synergy. Combining these synergy scores produces a composite synergy score which may be used to evaluate and characterize the KRas G12C inhibitor compounds of Formula (I), Formula I-A or Formula I-B or a pharmaceutically acceptable salt thereof in combination with a Src-family kinase inhibitor.

In general, the mathematical models use data obtained from single agent values to determine the predicted additive effect of the combination which is compared to the observed effect for the combination. If the observed effect is greater than the predicted effect, the combination is deemed to be synergistic. For example, the Bliss independence model compares the observed combination response (*Y_{O}*) with the predicted combination response (*Y_{P}*), which was obtained based on the assumption that there is no effect from drug-drug interactions. Typically, the combination effect is declared synergistic if *Y_{O}* is greater than *Y_{P}.*

In some embodiments, "synergistic effect" as used herein refers to combination of a KRAS inhibitor or a pharmaceutically acceptable salt thereof, and a Src-family kinase inhibitor or a pharmaceutically acceptable salt thereof producing an effect, for example, any of the beneficial or desired results including clinical results or endpoints as described herein, which is greater than the sum of the effect observed when a compound of Formula I or a pharmaceutically acceptable salt thereof (e.g., a compound selected from compound Example Nos. 1-527, or a pharmaceutically acceptable salt thereof, e.g., Example No. 234, 359, 478 or 507 or a pharmaceutically acceptable salt thereof) and a Src-family kinase inhibitor or a pharmaceutically acceptable salt thereof are administered alone. In one embodiment, the KRas G12C inhibitor is a compound selected from compound Nos. 1-527, or a pharmaceutically acceptable salt thereof (e.g., Example No. 234, 359, 478 or 507 or a pharmaceutically acceptable salt thereof). In one embodiment, the Src inhibitor is selected from dasatinib, vandetanib, KX-01, saracatinib and ponatinib. In one embodiment, the therapeutic combination comprises therapeutically effective amounts of Example No. 234 and dasatinib. In one embodiment, the therapeutic combination comprises therapeutically effective amounts of Example No. 234 and vandetanib. In one embodiment, the therapeutic combination comprises therapeutically effective amounts of Example No. 234 and KX-01. In one embodiment, the therapeutic combination comprises therapeutically effective amounts of Example No. 234 and saracatinib. In one embodiment, the therapeutic combination comprises therapeutically effective amounts of Example No. 234 and ponatinib. In one embodiment, the therapeutic combination comprises a therapeutically effective amount of Example No. 359 and dasatinib. In one embodiment, the therapeutic combination comprises therapeutically effective amounts of Example No. 359 and vandetanib. In one embodiment, the therapeutic combination comprises therapeutically effective amounts of Example No. 359 and KX-01. In one embodiment, the therapeutic combination comprises therapeutically effective amounts of Example No. 359 and saracatinib. In one embodiment, the therapeutic combination comprises therapeutically effective amounts of Example No. 359 and ponatinib. In one embodiment, the therapeutic combination comprises a therapeutically effective amount of Example No. 478 and dasatinib. In one embodiment, the therapeutic combination comprises therapeutically effective amounts of Example No. 478 and vandetanib. In one embodiment, the therapeutic combination comprises therapeutically effective amounts of Example No. 478 and KX-01. In one embodiment, the therapeutic combination comprises therapeutically effective amounts of Example No. 478 and saracatinib. In one embodiment, the therapeutic combination comprises therapeutically effective amounts of Example No. 478 and ponatinib. In one embodiment, the therapeutic combination comprises a therapeutically effective amount of Example No. 507 and dasatinib. In one embodiment, the therapeutic combination comprises therapeutically effective amounts of Example No. 507 and vandetanib. In one embodiment, the therapeutic combination comprises therapeutically effective amounts of Example No. 507 and KX-01. In one embodiment, the therapeutic combination comprises therapeutically effective amounts of Example No. 507 and saracatinib. In one embodiment, the therapeutic combination comprises therapeutically effective amounts of Example No. 507 and ponatinib.

In some embodiments, the methods provided herein can result in a 1% to 99% (e.g., 1% to 98%, 1% to 95%, 1% to 90%, 1 to 85%, 1 to 80%, 1% to 75%, 1% to 70%, 1% to 65%, 1% to 60%, 1% to 55%, 1% to 50%, 1% to 45%, 1% to 40%, 1% to 35%, 1% to 30%, 1% to 25%, 1% to 20%, 1% to 15%, 1% to 10%, 1% to 5%, 2% to 99%, 2% to 90%, 2% to 85%, 2% to 80%, 2% to 75%, 2% to 70%, 2% to 65%, 2% to 60%, 2% to 55%, 2% to 50%, 2% to 45%, 2% to 40%, 2% to 35%, 2% to 30%, 2% to 25%, 2% to 20%, 2% to 15%, 2% to 10%, 2% to 5%, 4% to 99%, 4% to 95%, 4% to 90%, 4% to 85%, 4% to 80%, 4% to 75%, 4% to 70%, 4% to 65%, 4% to 60%, 4% to 55%, 4% to 50%, 4% to 45%, 4% to 40%, 4% to 35%, 4% to 30%, 4% to 25%, 4% to 20%, 4% to 15%, 4% to 10%, 6% to 99%, 6% to 95%, 6% to 90%, 6% to 85%, 6% to 80%, 6% to 75%, 6% to 70%, 6% to 65%, 6% to 60%, 6% to 55%, 6% to 50%, 6% to 45%, 6% to 40%, 6% to 35%, 6% to 30%, 6% to 25%, 6% to 20%, 6% to 15%, 6% to 10%, 8% to 99%, 8% to 95%, 8% to 90%, 8% to 85%, 8% to 80%, 8% to 75%, 8% to 70%, 8% to 65%, 8% to 60%, 8% to 55%, 8% to 50%, 8% to 45%, 8% to 40%, 8% to 35%, 8% to 30%, 8% to 25%, 8% to 20%, 8% to 15%, 10% to 99%, 10% to 95%, 10% to 90%, 10% to 85%, 10% to 80%, 10% to 75%, 10% to 70%, 10% to 65%, 10% to 60%, 10% to 55%, 10% to 50%, 10% to 45%, 10% to 40%, 10% to 35%, 10% to 30%, 10% to 25%, 10% to 20%, 10% to 15%, 15% to 99%, 15% to 95%, 15% to 90%, 15% to 85%, 15% to 80%, 15% to 75%, 15% to 70%, 15% to 65%, 15% to 60%, 15% to 55%, 15% to 50%, 15% to 55%, 15% to 50%, 15% to 45%, 15% to 40%, 15% to 35%, 15% to 30%, 15% to 25%, 15% to 20%, 20% to 99%, 20% to 95%, 20% to 90%, 20% to 85%, 20% to 80%, 20% to 75%, 20% to 70%, 20% to 65%, 20% to 60%, 20% to 55%, 20% to 50%, 20% to 45%, 20% to 40%, 20% to 35%, 20% to 30%, 20% to 25%, 25% to 99%, 25% to 95%, 25% to 90%, 25% to 85%, 25% to 80%, 25% to 75%, 25% to 70%, 25% to 65%, 25% to 60%, 25% to 55%, 25% to 50%, 25% to 45%, 25% to 40%, 25% to 35%, 25% to 30%, 30% to 99%, 30% to 95%, 30% to 90%, 30% to 85%, 30% to 80%, 30% to 75%, 30% to 70%, 30% to 65%, 30% to 60%, 30% to 55%, 30% to 50%, 30% to 45%, 30% to 40%, 30% to 35%, 35% to 99%, 35% to 95%, 35% to 90%, 35% to 85%, 35% to 80%, 35% to 75%, 35% to 70%, 35% to 65%, 35% to 60%, 35% to 55%, 35% to 50%, 35% to 45%, 35% to 40%, 40% to 99%, 40% to 95%, 40% to 90%, 40% to 85%, 40% to 80%, 40% to 75%, 40% to 70%, 40% to 65%, 40% to 60%, 40% to 55%, 40% to 60%, 40% to 55%, 40% to 50%, 40% to 45%, 45% to 99%, 45% to 95%, 45% to 95%, 45% to 90%, 45% to 85%, 45% to 80%, 45% to 75%, 45% to 70%, 45% to 65%, 45% to 60%, 45% to 55%, 45% to 50%, 50% to 99%, 50% to 95%, 50% to 90%, 50% to 85%, 50% to 80%, 50% to 75%, 50% to 70%, 50% to 65%, 50% to 60%, 50% to 55%, 55% to 99%, 55% to 95%, 55% to 90%, 55% to 85%, 55% to 80%, 55% to 75%, 55% to 70%, 55% to 65%, 55% to 60%, 60% to 99%, 60% to 95%, 60% to 90%, 60% to 85%, 60% to 80%, 60% to 75%, 60% to 70%, 60% to 65%, 65% to 99%, 60% to 95%, 60% to 90%, 60% to 85%, 60% to 80%, 60% to 75%, 60% to 70%, 60% to 65%, 70% to 99%, 70% to 95%, 70% to 90%, 70% to 85%, 70% to 80%, 70% to 75%, 75% to 99%, 75% to 95%, 75% to 90%, 75% to 85%, 75% to 80%, 80% to 99%, 80% to 95%, 80% to 90%, 80% to 85%, 85% to 99%, 85% to 95%, 85% to 90%, 90% to 99%, 90% to 95%, or 95% to 100%) reduction in the volume of one or more solid tumors in a patient following treatment with the combination therapy for a period of time between 1 day and 2 years (e.g., between 1 day and 22 months, between 1 day and 20 months, between 1 day and 18 months, between 1 day and 16 months, between 1 day and 14 months, between 1 day and 12 months, between 1 day and 10 months, between 1 day and 9 months, between 1 day and 8 months, between 1 day and 7 months, between 1 day and 6 months, between 1 day and 5 months, between 1 day and 4 months, between 1 day and 3 months, between 1 day and 2 months, between 1 day and 1 month, between one week and 2 years, between 1 week and 22 months, between 1 week and 20 months, between 1 week and 18 months, between 1 week and 16 months, between 1 week and 14 months, between 1 week and 12 months, between 1 week and 10 months, between 1 week and 9 months, between 1 week and 8 months, between 1 week and 7 months, between 1 week and 6 months, between 1 week and 5 months, between 1 week and 4 months, between 1 week and 3 months, between 1 week and 2 months, between 1 week and 1 month, between 2 weeks and 2 years, between 2 weeks and 22 months, between 2 weeks and 20 months, between 2 weeks and 18 months, between 2 weeks and 16 months, between 2 weeks and 14 months, between 2 weeks and 12 months, between 2 weeks and 10 months, between 2 weeks and 9 months, between 2 weeks and 8 months, between 2 weeks and 7 months, between 2 weeks and 6 months, between 2 weeks and 5 months, between 2 weeks and 4 months, between 2 weeks and 3 months, between 2 weeks and 2 months, between 2 weeks and 1 month, between 1 month and 2 years, between 1 month and 22 months, between 1 month and 20 months, between 1 month and 18 months, between 1 month and 16 months, between 1 month and 14 months, between 1 month and 12 months, between 1 month and 10 months, between 1 month and 9 months, between 1 month and 8 months, between 1 month and 7 months, between 1 month and 6 months, between 1 month and 6 months, between 1 month and 5 months, between 1 month and 4 months, between 1 month and 3 months, between 1 month and 2 months, between 2 months and 2 years, between 2 months and 22 months, between 2 months and 20 months, between 2 months and 18 months, between 2 months and 16 months, between 2 months and 14 months, between 2 months and 12 months, between 2 months and 10 months, between 2 months and 9 months, between 2 months and 8 months, between 2 months and 7 months, between 2 months and 6 months, or between 2 months and 5 months, between 2 months and 4 months, between 3 months and 2 years, between 3 months and 22 months, between 3 months and 20 months, between 3 months and 18 months, between 3 months and 16 months, between 3 months and 14 months, between 3 months and 12 months, between 3 months and 10 months, between 3 months and 8 months, between 3 months and 6 months, between 4 months and 2 years, between 4 months and 22 months, between 4 months and 20 months, between 4 months and 18 months, between 4 months and 16 months, between 4 months and 14 months, between 4 months and 12 months, between 4 months and 10 months, between 4 months and 8 months, between 4 months and 6 months, between 6 months and 2 years, between 6 months and 22 months, between 6 months and 20 months, between 6 months and 18 months, between 6 months and 16 months, between 6 months and 14 months, between 6 months and 12 months, between 6 months and 10 months, or between 6 months and 8 months) (e.g., as compared to the size of the one or more solid tumors in the patient prior to treatment).

The phrase "time of survival" means the length of time between the identification or diagnosis of cancer (e.g., any of the cancers described herein) in a mammal by a medical professional and the time of death of the mammal (caused by the cancer). Methods of increasing the time of survival in a mammal having a cancer are described herein.

In some embodiments, any of the methods described herein can result in an increase (e.g., a 1% to 400%, 1% to 380%, 1% to 360%, 1% to 340%, 1% to 320%, 1% to 300%, 1% to 280%, 1% to 260%, 1% to 240%, 1% to 220%, 1% to 200%, 1% to 180%, 1% to 160%, 1% to 140%, 1% to 120%, 1% to 100%, 1% to 95%, 1% to 90%, 1% to 85%, 1% to 80%, 1% to 75%, 1% to 70%, 1% to 65%, 1% to 60%, 1% to 55%, 1% to 50%, 1% to 45%, 1% to 40%, 1% to 35%, 1% to 30%, 1% to 25%, 1% to 20%, 1% to 15%, 1% to 10%, 1% to 5%, 5% to 400%, 5% to 380%, 5% to 360%, 5% to 340%, 5% to 320%, 5% to 300%, 5% to 280%, 5% to 260%, 5% to 240%, 5% to 220%, 5% to 200%, 5% to 180%, 5% to 160%, 5% to 140%, 5% to 120%, 5% to 100%, 5% to 90%, 5% to 80%, 5% to 70%, 5% to 60%, 5% to 50%, 5% to 40%, 5% to 30%, 5% to 20%, 5% to 10%, 10% to 400%, 10% to 380%, 10% to 360%, 10% to 340%, 10% to 320%, 10% to 300%, 10% to 280%, 10% to 260%, 10% to 240%, 10% to 220%, 10% to 200%, 10% to 180%, 10% to 160%, 10% to 140%, 10% to 120%, 10% to 100%, 10% to 90%, 10% to 80%, 10% to 70%, 10% to 60%, 10% to 50%, 10% to 40%, 10% to 30%, 10% to 20%, 20% to 400%, 20% to 380%, 20% to 360%, 20% to 340%, 20% to 320%, 20% to 300%, 20% to 280%, 20% to 260%, 20% to 240%, 20% to 220%, 20% to 200%, 20% to 180%, 20% to 160%, 20% to 140%, 20% to 120%, 20% to 100%, 20% to 90%, 20% to 80%, 20% to 70%, 20% to 60%, 20% to 50%, 20% to 40%, 20% to 30%, 30% to 400%, 30% to 380%, 30% to 360%, 30% to 340%, 30% to 320%, 30% to 300%, 30% to 280%, 30% to 260%, 30% to 240%, 30% to 220%, 30% to 200%, 30% to 180%, 30% to 160%, 30% to 140%, 30% to 120%, 30% to 100%, 30% to 90%, 30% to 80%, 30% to 70%, 30% to 60%, 30% to 50%, 30% to 40%, 40% to 400%, 40% to 380%, 40% to 360%, 40% to 340%, 40% to 320%, 40% to 300%, 40% to 280%, 40% to 260%, 40% to 240%, 40% to 220%, 40% to 200%, 40% to 180%, 40% to 160%, 40% to 140%, 40% to 120%, 40% to 100%, 40% to 90%, 40% to 80%, 40% to 70%, 40% to 60%, 40% to 50%, 50% to 400%, 50% to 380%, 50% to 360%, 50% to 340%, 50% to 320%, 50% to 300%, 50% to 280%, 50% to 260%, 50% to 240%, 50% to 220%, 50% to 200%, 50% to 180%, 50% to 160%, 50% to 140%, 50% to 140%, 50% to 120%, 50% to 100%, 50% to 90%, 50% to 80%, 50% to 70%, 50% to 60%, 60% to 400%, 60% to 380%, 60% to 360%, 60% to 340%, 60% to 320%, 60% to 300%, 60% to 280%, 60% to 260%, 60% to 240%, 60% to 220%, 60% to 200%, 60% to 180%, 60% to 160%, 60% to 140%, 60% to 120%, 60% to 100%, 60% to 90%, 60% to 80%, 60% to 70%, 70% to 400%, 70% to 380%, 70% to 360%, 70% to 340%, 70% to 320%, 70% to 300%, 70% to 280%, 70% to 260%, 70% to 240%, 70% to 220%, 70% to 200%, 70% to 180%, 70% to 160%, 70% to 140%, 70% to 120%, to 100%, 70% to 90%, 70% to 80%, 80% to 400%, 80% to 380%, 80% to 360%, 80% to 340%, 80% to 320%, 80% to 300%, 80% to 280%, 80% to 260%, 80% to 240%, 80% to 220%, 80% to 200%, 80% to 180%, 80% to 160%, 80% to 140%, 80% to 120%, 80% to 100%, 80% to 90%, 90% to 400%, 90% to 380%, 90% to 360%, 90% to 340%, 90% to 320%, 90% to 300%, 90% to 280%, 90% to 260%, 90% to 240%, 90% to 220%, 90% to 200%, 90% to 180%, 90% to 160%, 90% to 140%, 90% to 120%, 90% to 100%, 100% to 400%, 100% to 380%, 100% to 360%, 100% to 340%, 100% to 320%, 100% to 300%, 100% to 280%, 100% to 260%, 100% to 240%, 100% to 220%, 100% to 200%, 100% to 180%, 100% to 160%, 100% to 140%, 100% to 120%, 120% to 400%, 120% to 380%, 120% to 360%, 120% to 340%, 120% to 320%, 120% to 300%, 120% to 280%, 120% to 260%, 120% to 240%, 120% to 220%, 120% to 200%, 120% to 180%, 120% to 160%, 120% to 140%, 140% to 400%, 140% to 380%, 140% to 360%, 140% to 340%, 140% to 320%, 140% to 300%, 140% to 280%, 140% to 260%, 140% to 240%, 140% to 220%, 140% to 200%, 140% to 180%, 140% to 160%, 160% to 400%, 160% to 380%, 160% to 360%, 160% to 340%, 160% to 320%, 160% to 300%, 160% to 280%, 160% to 260%, 160% to 240%, 160% to 220%, 160% to 200%, 160% to 180%, 180% to 400%, 180% to 380%, 180% to 360%, 180% to 340%, 180% to 320%, 180% to 300%, 180% to 280%, 180% to 260%, 180% to 240%, 180% to 220%, 180% to 200%, 200% to 400%, 200% to 380%, 200% to 360%, 200% to 340%, 200% to 320%, 200% to 300%, 200% to 280%, 200% to 260%, 200% to 240%, 200% to 220%, 220% to 400%, 220% to 380%, 220% to 360%, 220% to 340%, 220% to 320%, 220% to 300%, 220% to 280%, 220% to 260%, 220% to 240%, 240% to 400%, 240% to 380%, 240% to 360%, 240% to 340%, 240% to 320%, 240% to 300%, 240% to 280%, 240% to 260%, 260% to 400%, 260% to 380%, 260% to 360%, 260% to 340%, 260% to 320%, 260% to 300%, 260% to 280%, 280% to 400%, 280% to 380%, 280% to 360%, 280% to 340%, 280% to 320%, 280% to 300%, 300% to 400%, 300% to 380%, 300% to 360%, 300% to 340%, or 300% to 320%) in the time of survival of the patient (e.g., as compared to a patient having a similar cancer and administered a different treatment or not receiving a treatment).

In some embodiments of any of the methods described herein, before treatment with the compositions or methods of the invention, the patient was treated with one or more of a chemotherapy, a targeted anticancer agent, radiation therapy, and surgery, and optionally, the prior treatment was unsuccessful; and/or the patient has been administered surgery and optionally, the surgery was unsuccessful; and/or the patient has been treated with a platinum-based chemotherapeutic agent, and optionally, the patient has been previously determined to be non-responsive to treatment with the platinum-based chemotherapeutic agent; and/or the patient has been treated with a kinase inhibitor, and optionally, the prior treatment with the kinase inhibitor was unsuccessful; and/or the patient was treated with one or more other therapeutic agent(s).

### KITS

The present invention also relates to a kit comprising a Src-family kinase inhibitor and a KRas G12C inhibitor compound of Formula (I), Formula I-A or Formula I-B. Also provided is a kit comprising a Src-family kinase inhibitor or a pharmaceutically acceptable salt thereof, and a KRas G12C inhibitor compound of Formula (I), Formula I-A or Formula I-B, or a pharmaceutical salt thereof (e.g., a compound selected from compound Example Nos. 1-527, or a pharmaceutically acceptable salt thereof, e.g., Example No. 234, 359, 478 or 507 or a pharmaceutically acceptable salt thereof) for use in treating a hematological cancer.

In a related aspect, the invention provides a kit containing a dose of a Src-family kinase inhibitor and dose of a KRas G12C inhibitor compound of Formula (I), Formula I-A or Formula I-B or a pharmaceutical salt thereof (e.g., a compound selected from compound Example Nos. 1-527, or a pharmaceutically acceptable salt thereof, e.g., Example No. 234, 359, 478 or 507 or a pharmaceutically acceptable salt thereof) in an amount effective to inhibit proliferation of cancer cells, particularly KRas G12C-expressing cancer cells, in a subject. The kit in some cases includes an insert with instructions for administration of the a Src-family kinase inhibitor and a KRas G12C inhibitor compound of Formula (I), Formula I-A or Formula I-B. The insert may provide a user with one set of instructions for using the a Src-family kinase inhibitor in combination with a KRas G12C inhibitor compound of Formula (I), Formula I-A or Formula I-B.

### EXAMPLE A

### Src-family Kinase Inhibitors Synergistically Increase the Activity of KRas G12C Inhibitors Against Cell Lines Expressing KRas G12C

This Example illustrates that the combination of exemplary KRas G12C inhibitor compounds of Formula I, Formula I-A and Formula 1-B and a Src-family kinase inhibitor synergistically inhibits the growth of tumor cell lines that express KRas G12C.

A panel of 8 lung cancer and 1 colorectal cell lines harboring KRas G12C mutations was assembled to determine whether combining Src-family kinase inhibitors with exemplary KRas G12C inhibitors disclosed herein results in synergistic activity. The collection included NCI-H1373 (ATCC CRL-5866); NCI-H1792 (ATCC CRL-5895); NCI-H2030 (ATCC CRL-5985); NCI-H2122 (ATCC CRL-5985); HCC1171 (KCLB 71171); HCC44 (DSMZ ACC-534); LU99 (RCB1900); SW1573 (ATCC CRL-2170) and SW837 (ATCC CCL-235).

Assays for determining the synergy score for the pairwise combinations for each cell line were performed in triplicate. Three 96-well plates plus an additional 4 wells of a separate 96-well control plate for determining baseline luminescence were seeded with 2000 cells/well of a particular cell line in a total volume of 90µl of a suitable growth medium for that cell line, e.g., RPMI 1640 medium supplemented with 10% FBS and any cell line specific reagents need for growth. The plates were incubated overnight at 37°C in a 5% CO₂ atmosphere.

To each of the designated baseline wells, 30µl of Cell-Titer Glo reagent (CTG; Promega Corporation) was added to each well and the plates were incubated for 20 min with shaking at room temperature. Baseline luminescence was quantitated using a BMG ClarioStar multimode plate reader according to the manufacturer's instructions.

A series of working stock 1000X drug dilutions in 100% DMSO was prepared that includes an 8 point single agent dilution of the exemplary KRas G12C inhibitor of Formula (I) and a 5-point single agent dilution of the Src-family kinase inhibitor. The dilutions used for the KRas G12C inhibitor and the Src-family kinase inhibitor varied for each individual compound but were in the range of 3- to 6-fold/serial dilution.

Exemplary KRas G12C inhibitors tested in this Example included:

| **Example No.*** | **Structure** |
|---|---|
| 234 | 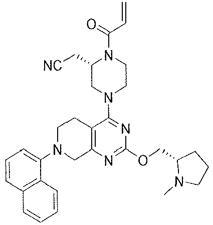 |
| 359 | 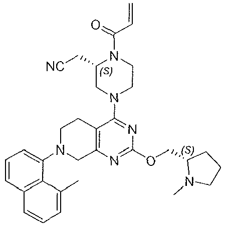 |
| 478 | 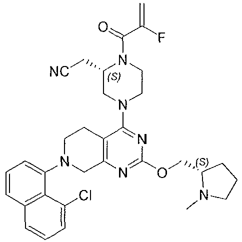 |
| 507 | 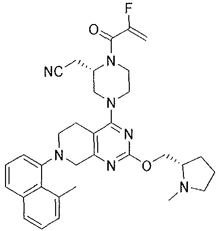 |

| | |
|---|---|
| *Example Number refers to the example number for each compound as disclosed in pending international PCT application WO 2019099524. | |

A 10X intermediate dosing plate was prepared in serum free RPMI medium that contains arrayed single agent dilutions of exemplary KRas G12C inhibitor of Formula (I) or the Sre-family kinase inhibitor. In addition, a matrix of 40 dilution combinations of exemplary KRas G12C inhibitor of Formula (I), Formula I-A or Formula I-B and the Src-family kinase inhibitor was prepared as test samples.

To each corresponding well of the three 96-well plates seeded with the appropriate cell line above, 10 µL of each 10X single agent and the 40 combinations of the dose matrix was added and the plates were incubated for 72 hours at 37 °C in 5% CO₂ atmosphere. A 30 µL aliquot of Cell-Titer Glo reagent (CTG) was added to each test well, the plates were incubated for 20 min with shaking at room temperature, and luminescence was quantitated using a BMG ClarioStar multimode plate reader according to the manufacturer's instructions.

The raw data and metadata files were used as input files to calculate percent effect for each treatment condition and analyzed using four independent mathematical reference models designed to determine whether the two test compounds demonstrate synergy: Loewe additivity, Bliss independence, Highest Single Agent and ZIP.

The output of the data from each mathematical model is the assignment of a relative synergy score. The data reported in Table 1 are the aggregate sum of the Loewe additivity, Bliss independence, Highest Single Agent and ZIP scores ("Composite Synergy Score").

**Table 1**

| Composite Synergy Scores for Exemplary Src-family Kinase Inhibitors Combined with Exemplary KRas G12C Inhibitors of Formula (I) Against KRas G12C Cell Lines | | | | | | | | | |
|---|---|---|---|---|---|---|---|---|---|
| **Src-family Inhibitor** | **Dasatinib** | **Dasatinib** | **Dasatinib** | **Dasatinib** | **Vandetanib** | **Vandetanib** | **KX-01** | **Saracatinib** | **Ponatinib** |
| **KRas G12C Example #** | **234** | **507** | **359** | **478** | **359** | **478** | **507** | **507** | **234** |
| **Cell Line** | | | | | | | | | |
| H1373 | 53.9 | 40.8 | 76.8 | 46.0 | 33.1 | 24.1 | 12.8 | 29.4 | 28.8 |
| H1792 | 42.9 | 10.1 | 34.2 | 25.9 | 15.3 | 23.5 | 7.6 | -6.5 | 32.4 |
| H2030 | 59.0 | 48.9 | 49.4 | 38.9 | 31.5 | 12.3 | 14.4 | 21.1 | 17.7 |
| H2122 | 73.4 | 28.9 | 58.3 | 44.4 | 33.9 | 26.9 | 14.4 | 20.1 | 24.5 |
| HCC1171 | 51.9 | 33.0 | 32.2 | 29.4 | 51.9 | 60.1 | 19.8 | 60.4 | 11.9 |
| HCC44 | 32.3 | 27.0 | 40.6 | 59.1 | 4.8 | -8.8 | 12.4 | -41.0 | 14.3 |
| LU99 | 59.0 | 51.4 | 43.8 | 43.2 | -1.9 | 15.4 | 11.0 | 11.7 | 21.7 |
| SW1573 | 31.4 | -10.3 | 12.7 | -21.1 | -16.0 | -6.9 | 4.9 | 6.1 | 16.3 |
| SW837 | 38.4 | 48.9 | 28.8 | 37.0 | 16.3 | 10.7 | 24.5 | 16.2 | 22.2 |

A composite score of greater than or equal to 27 was interpreted as a synergistic hit whereas a composite score between 17 and 26 indicates potential synergy. These results demonstrate that a synergistic effect was observed for the combination of a variety of Src-family inhibitors with exemplary KRas G12C inhibitor compounds of Formula (I) in a majority of cell lines harboring a KRas G12C mutation listed in Table 1 that are less sensitive to single agent KRas G12C inhibitor thereby increasing the sensitivity of the KRas G12C cell line to the KRas G12C inhibitor combination.

### EXAMPLE B

### In Vivo Models for Examining KRas G12C inhibitor plus Src-family Kinase Inhibitor Combinations

Immunocompromised nude/nude mice were inoculated in the right hind flank with cells or patient derived tumor samples harboring a KRas G12C mutation. When tumor volumes reached between 200 - 400 mm³ in size, the mice were divided into four groups of 5-12 mice each. The first group was administered vehicle only. The second group was administered a single agent dose of the KRas G12C inhibitor at a concentration that yielded a maximal biological effect or a less than maximal biological effect, depending on the cell line and the single agent activity, that does not result in complete tumor regression. The third group was administered a single agent dose of the Src-family kinase inhibitor at a concentration that yielded a maximal biological effect or a less than maximal biological effect, depending on the cell line and the single agent activity, that also does not result in complete tumor regression. The fourth group was administered the single agent dose of the KRas G12C inhibitor in combination with the single agent dose of the Src-family kinase inhibitor. The treatment period varied from cell line to cell line but typically was between 21-35 days. Tumor volumes were measured using a caliper every two - three days and tumor volumes were calculated by the formula: 0.5 x (Length x Width)². A greater degree of tumor growth inhibition for the combination in this model demonstrates that the combination therapy is likely to have a clinically meaningful benefit to treated subjects relative to treatment with only a KRas G12C inhibitor.

For example, on Day 1, 20 nude/nude mice were inoculated in the right hind limb with 5 x 10⁶ H2122 cells. When tumor volume reached ~300 mm³ (Day 11), 5 mice in each of the four groups were administered p.o. daily for 21 days: vehicle only (10% Captisol), 100 mg/kg of the KRas G12C inhibitor Compound 478 (10% Captisol in 50 mM citrate buffer, pH 5.0), 50 mg/kg of the Src-family kinase inhibitor Dasatinib (0.5% methylcellulose/0.4% Tween-80), or 100 mg/kg of the KRas G12C inhibitor Compound 478 and 50 mg/kg of Dasatinib. Tumor volumes were measured at pre-specified days set forth below. Tumor volumes for the five mice per group were averaged and are reported in Table 2.

**Table 2**

| Average Tumor Volumes (mm³) of Mice Treated with Single Agents and in Combination | | | | |
|---|---|---|---|---|
| Days from Inoculation | Vehicle | Compound 478 | Dasatinib | Compound 478 + Dasatinib Combination |
| 11 | 325 | 325 | 324 | 330 |
| 13 | 520 | 340 | 492 | 328 |
| 15 | 689 | 338 | 596 | 325 |
| 18 | 955 | 382 | 820 | 313 |
| 20 | 1127 | 462 | 938 | 327 |
| 22 | 1351 | 479 | 1086 | 299 |
| 25 | 1505 | 493 | 1216 | 336 |

As shown in Table 2, the administration of Compound 478 or Dasatinib as a single agent exhibited 82.8% and 23.5% tumor growth inhibition at Day 20 (Treatment Day 9), respectively. The combination of the Src-family kinase inhibitor Dasatinib and Compound 478 resulted in 99.7% tumor growth inhibition at Day 20, an increase of 16.9% compared to administration of Compound 478 as a single agent. These results demonstrate that the combination therapy resulted in greater amount of tumor growth inhibition compared to either single agent alone, demonstrating enhanced in vivo anti-tumor efficacy of the combination against a KRas G12C expressing cancer.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.
The following numbered clauses form part of the present disclosure.
1. A method of treating cancer in a subject in need thereof, comprising administering to the subject a therapeutically effective amount of a combination of a Src-family kinase inhibitor and a KRAS G12C inhibitor of formula (I): 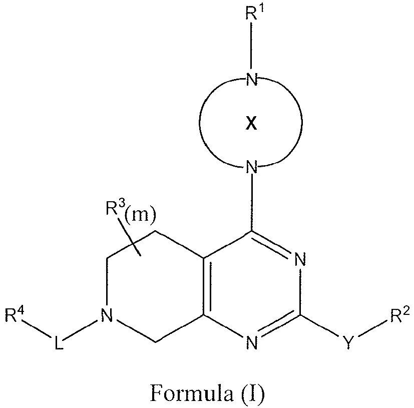 or a pharmaceutically acceptable salt thereof:
   wherein:
   X is a 4-12 membered saturated or partially saturated monocyclic, bridged or spirocyclic ring, wherein the saturated or partially saturated monocyclic ring is optionally substituted with one or more R⁸;
   Y is a bond, O, S or NR⁵;
   R¹ is -C(O)C(R^{A}) 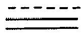 C(R^{B})ₚ or -SO₂C(R^{A}) 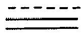 C(R^{B})ₚ;
   R² is hydrogen, alkyl, hydroxyalkyl, dihydroxyalkyl, alkylaminylalkyl, dialkylaminylalkyl, -Z-NR⁵R¹⁰, heterocyclyl, heterocyclylalkyl, aryl, heteroaryl, or heteroarylalkyl, wherein each of the Z, heterocyclyl, heterocyclylalkyl, aryl, heteroaryl, and heteroarylalkyl may be optionally substituted with one or more R⁹;
   each Z is C1 - C4 alkylene;
   each R³ is independently C1 - C3 alkyl, oxo, haloalkyl, hydroxyl or halogen;
   L is a bond, -C(O)-, or C1 - C3 alkylene;
   R⁴ is hydrogen, cycloalkyl, heterocyclyl, aryl, aralkyl or heteroaryl, wherein each of the cycloalkyl, heterocyclyl, aryl, aralkyl and heteroaryl may be optionally substituted with one or more R⁶, R⁷ or R⁸;
   each R⁵ is independently hydrogen or C1 - C3 alkyl;
   R⁶ is cycloalkyl, heterocyclyl, heterocyclylalkyl, aryl, or heteroaryl, wherein each of the cycloalkyl, heterocyclyl, aryl, or heteroaryl may be optionally substituted with one or more R⁷;
   each R⁷ is independently halogen, hydroxyl, C1 - C6 alkyl, cycloalkyl, alkoxy, haloalkyl, amino, cyano, heteroalkyl, hydroxyalkyl or Q-haloalkyl, wherein Q is O or S;
   R⁸ is oxo, C1 - C3 alkyl, C2 - C4 alkynyl, heteroalkyl, cyano, -C(O)OR⁵, -C(O)N(R⁵)₂, -N(R⁵)₂, wherein the C1 - C3 alkyl may be optionally substituted with cyano, halogen, -OR⁵, -N(R⁵)₂, or heteroaryl;
   each R⁹ is independently hydrogen, oxo, acyl, hydroxyl, hydroxyalkyl, cyano, halogen, C1 - C6 alkyl, aralkyl, haloalkyl, heteroalkyl, cycloalkyl, heterocyclylalkyl, alkoxy, dialkylaminyl, dialkylamidoalkyl, or dialkylaminylalkyl, wherein the C1 - C6 alkyl may be optionally substituted with cycloalkyl;
   each R¹⁰ is independently hydrogen, acyl, C1 - C3 alkyl, heteroalkyl or hydroxyalkyl;
   R¹¹ is haloalkyl;
   R^{A} is absent, hydrogen, deuterium, cyano, halogen, C1 - C-3 alkyl, haloalkyl, heteroalkyl, - C(O)N(R⁵)₂, or hydroxyalkyl;
   each R^{B} is independently hydrogen, deuterium, cyano, C1 - C3 alkyl, hydroxyalkyl, heteroalkyl, C1 - C3 alkoxy, halogen, haloalkyl, -ZNR⁵R¹¹, -C(O)N(R⁵)₂, -NHC(O)C1 - C3 alkyl, - CH₂NHC(O)C1 - C3 alkyl, heteroaryl, heteroarylalkyl, dialkylaminylalkyl, or heterocyclylalkyl wherein the heterocyclyl portion is substituted with one or more substituents independently selected from halogen, hydroxyl, alkoxy and C1 - C3 alkyl, wherein the heteroaryl or the heteroaryl portion of the heteroarylalkyl is optionally substituted with one or more R⁷;
   when is a triple bond then R^{A} is absent, R^{B} is present and p equals one,
   or when 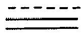 is a double bond then R^{A} is present, R^{B} is present and p equals two, or R^{A}, R^{B} and the carbon atoms to which they are attached form a 5-8 membered partially saturated cycloalkyl optionally substituted with one or more R⁷;
   m is zero or an integer between 1 and 2; and
   p is one or two.
2. The method of clause 1, wherein R¹-X is: 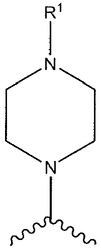 wherein the piperazinyl ring is optionally substituted with R⁸.
3. The method of clause 2, wherein R¹ is -C(O)C(R^{A}) 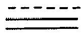 C(R^{B})ₚ
4. The method of clause 3, wherein is a triple bond and R^{A} is absent, p is one and R^{B} is C1 - C3 alkyl, hydroxyalkyl or C1 - C3 alkoxy.
5. The method of clause 3, wherein 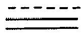 is a double bond and R^{A} is hydrogen and R^{B} is hydrogen.
6. The method of clause 3, wherein 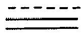 is a double bond and R^{A} is hydrogen, p is two and at least one R^{B} is independently deuterium, cyano, halogen, haloalkyl, hydroxyalkyl, heteroalkyl, heteroaryl, heteroarylalkyl, -ZNR⁵R¹¹, -C(O)N(R⁵)₂, -NHC(O)C1 - C3 alkyl or heterocyclylalkyl wherein the heterocyclyl portion is substituted with one or more substituents independently selected from halogen, hydroxyl, alkoxy or C1 - C3 alkyl.
7. The method of clause 6, wherein the at least one R^{B} is halogen.
8. The method of clause 6, wherein the at least one R^{B} is haloalkyl.
9. The method of clause 6, wherein the at least one R^{B} is -ZNR⁵R¹¹.
10. The method of clause 9, wherein Z is methylene, R⁵ is methyl and R¹¹ is trifluoromethyl.
11. The method of clause 6, wherein the at least one R^{B} is cyano.
12. The method of clause 6, wherein the at least one R^{B} is hydroxyalkyl.
13. The method of clause 6, wherein the at least one R^{B} is heteroalkyl.
14. The method of clause 13, wherein the heteroalkyl is methoxymethyl.
15. The method of clause 6, wherein the at least one R^{B} is -C(O)N(R⁵)₂, wherein each R⁵ is hydrogen.
16. The method of clause 6, wherein the at least one R^{B} is -C(O)N(R⁵)₂, wherein each R⁵ is C1 - C3 alkyl.
17. The method of clause 6, wherein the at least one R^{B} is heteroaryl optionally substituted with one or more R⁷.
18. The method of clause 17, wherein the heteroaryl is pyrrolyl, imidazolyl, pyrazolyl, triazolyl, pyridyl, pyridazinyl, pyrimidinyl, pyrazinyl or triazinyl, each substituted with one or more R⁷.
19. The method of clause 6, wherein the at least one R^{B} is heteroarylalkyl optionally substituted with one or more R⁷.
20. The method of clause 19, wherein the heteroaryl portion of the heteroarylalkyl is pyrrolyl, imidazolyl, pyrazolyl, triazolyl, pyridyl, pyridazinyl, pyrimidinyl, pyrazinyl or triazinyl, each optionally substituted with one or more R⁷.
21. The method of clause 6, wherein the at least one R^{B} is heterocyclylalkyl substituted with one or more R⁷.
22. The method of clause 21, wherein the heterocyclyl portion of the heterocyclylalkyl is azetindinyl, pyrrolidinyl, piperidinyl piperazinyl or morpholinyl, each substituted with one or more groups independently selected from halogen, hydroxyl, alkoxy or C1 - C3 alkyl.
23. The method according to any of clauses 6-22, wherein the double bond is in the E configuration.
24. The method according to any of clauses 6-22, wherein the double bond is in the Z configuration.
25. The method of clause 6, wherein 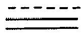 is a double bond and p is two, each R^{B} is hydrogen, and R^{A} is deuterium, cyano, halogen, haloalkyl, heteroalkyl, -C(O)N(R⁵)₂, or hydroxyalkyl.
26. The method of clause 25, wherein R^{A} is halogen.
27. The method of clause 25, wherein R^{A} is haloalkyl.
28. The method of clause 25, wherein R^{A} is cyano.
29. The method of clause 25, wherein R^{A} is heteroalkyl.
30. The method of clause 29, wherein the heteroalkyl is methoxymethyl.
31. The method of clause 29, wherein the heteroalkyl is alkoxy.
32. The method of clause 25, wherein R^{A} is -C(O)N(R⁵)₂, wherein each R⁵ is hydrogen.
33. The method of clause 25, wherein R^{A} is hydroxyalkyl.
34. The method of clause 2, wherein is a double bond and p is two, one R^{B} is hydrogen, the second R^{B} is dialkylaminylalkyl, and R^{A} is halogen.
35. The method of clause 2, wherein is a double bond and p is two, each R^{B} is deuterium, and R^{A} is deuterium.
36. The method of clause 2, wherein when is a double bond and p is two, one R^{B} is hydrogen and R^{A} and one R^{B} and the carbon atoms to which they are attached form a 5-8 membered partially saturated cycloalkyl substituted with oxo.
37. The method of according to any one of clauses 2-36, wherein Y is O.
38. The method according to any one of clauses 2-37, wherein R² is selected from the group consisting of hydroxyalkyl, alkylaminylalkyl, dialkylaminylalkyl, -ZNR⁵R¹⁰, heterocyclyl and heterocyclylalkyl, wherein each of the Z, heterocyclyl or heterocyclylalkyl are independently optionally substituted with R⁹.
39. The method of clause 38, wherein R² is heterocyclylalkyl optionally substituted with one or more R⁹.
40. The method of clause 39, wherein the heterocyclyl of the heterocyclylalkyl is independently azetidinyl, methylazetidinyl, difluoroazetidinyl, tetrahydropyran, pyrrolidinyl, methylpyrrolidinyl, diemethylpyrrolidinyl, isopropylpyrrolidinyl, cycloalkylalkylpyrrolidinyl, hydroxypyrrolindinyl, fluoropyrrolidinyl, difluoropyrrolidinyl, (N-methyl)fluoropyrrolidinyl, (N-methyl)difluoropyrrolidinyl, methoxyethylpyrrolidinyl, (N-methyl)methoxypyrrolidinyl, piperazinyl, dimethylaminylpyrrolidinyl, morpholinyl, methylmorpholinyl, 1,4-oxazepanyl, piperdinyl, methylpiperidinyl acylpiperdinyl, cyanopiperdinyl, cycloalkylpiperdinyl, halopiperdinyl, dihalopiperdinyl, fluoropiperdinyl, difluoropiperdinyl, alkoxypiperdinyl, pyrrolidonyl, piperidinonyl, thiomorpholinyl-1,1-dioxide, 3-azabicyclo[3.1.0]hexanyl, oxa-5-azabicyclo[2.2.1]heptan-5-yl, or azabicyclo[2.2.1]heptan-2-yl.
41. The method of clause 40, wherein the (N-methyl)difluoropyrrolidinyl is 3,3-difluoro-1-methylpyrrolidinyl.
42. The method of clause 40, wherein the heterocyclyl is N-methylpyrrolidinyl.
43. The method of clause 38, wherein R² is dialkylaminylalkyl optionally substituted with one or more R⁹.
44. The method according to any one of clauses 2-43, wherein R⁴ is aryl optionally substituted with one or more R⁷.
45. The method of clause 44, wherein the aryl is selected from the group consisting of phenyl and naphthyl optionally substituted with one or more R⁷.
46. The method of clause 45, wherein the phenyl and the naphthyl are each optionally substituted with one or more R⁷ selected from the group consisting of halogen, hydroxyl, C1- C6 alkyl, cyano, haloalkyl, Q-haloalkyl, and alkoxy.
47. The method of clause 46, wherein R⁷ is selected from the group consisting of halogen, haloalkyl, methyl, isopropyl, methoxy, Q-haloalkyl, hydroxyl and cyano.
48. The method according to any one of clauses 2-43, wherein R⁴ is heteroaryl.
49. The method according to any one of clauses 2-43, wherein R⁴ is aralkyl optionally substituted with one or more R⁷.
50. The method according to any one of clauses 2-49, wherein m is zero.
51. The method according to any one of clauses 2-50, wherein L is a bond.
52. The method according to any one of clauses 2-51, wherein R⁸ is heteroalkyl, C2-C4 alkynyl, or C1 - C3alkyl optionally substituted with -OR⁵, cyano or heteroaryl.
53. The method of clause 52, wherein R³ is C1 - C3 alkyl optionally substituted with cyano.
54. The method of clause 52, wherein R⁸ is cyanomethyl.
55. The method according to any one of clauses 52-54, wherein X is substituted with one R⁸.
56. The method of clause 1, wherein the KRas G12C inhibitor is: 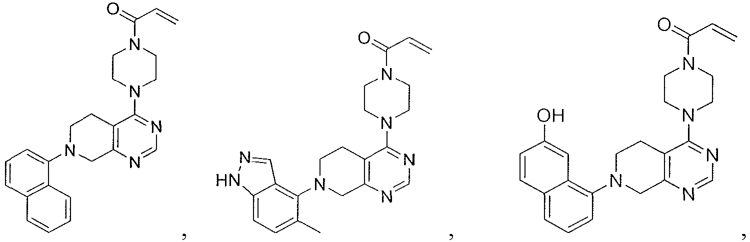 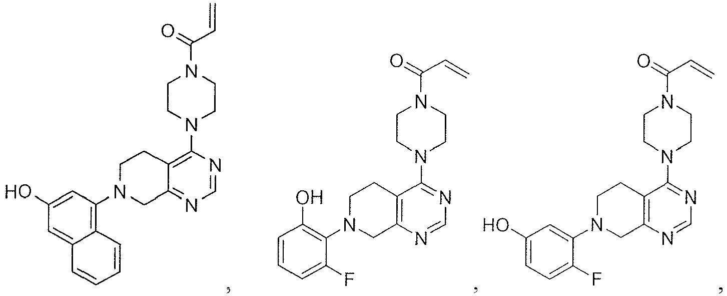 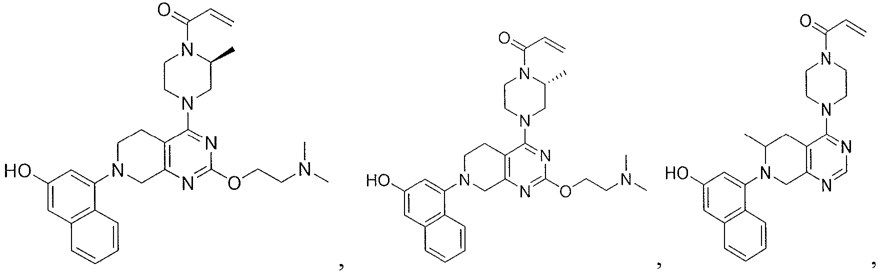 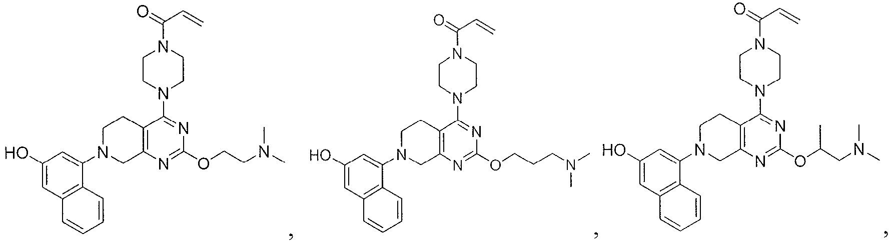 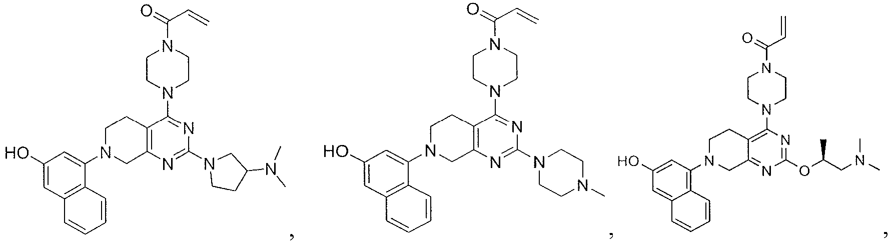 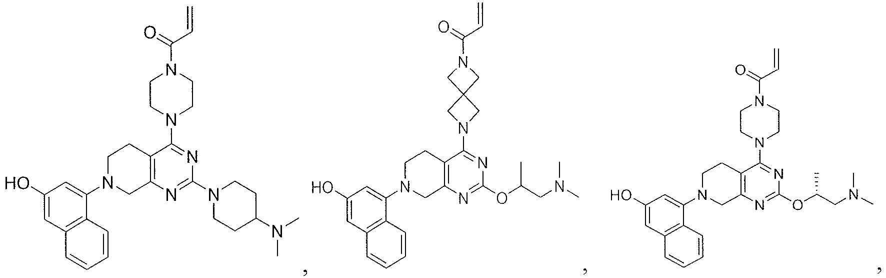 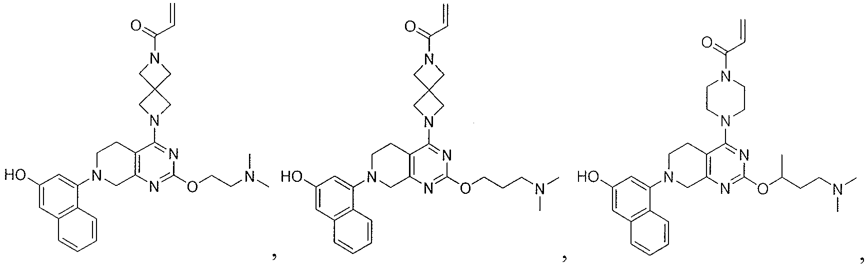 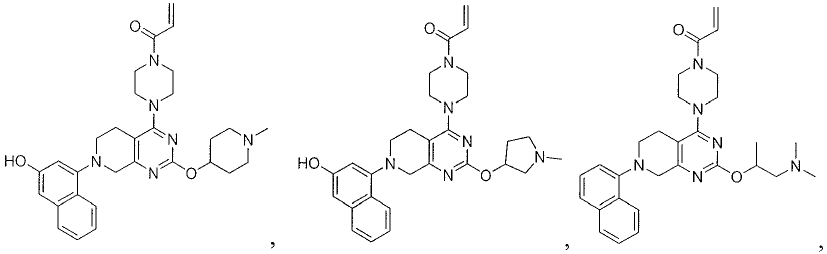 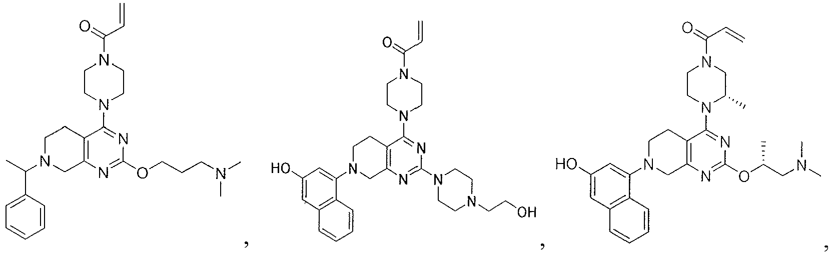 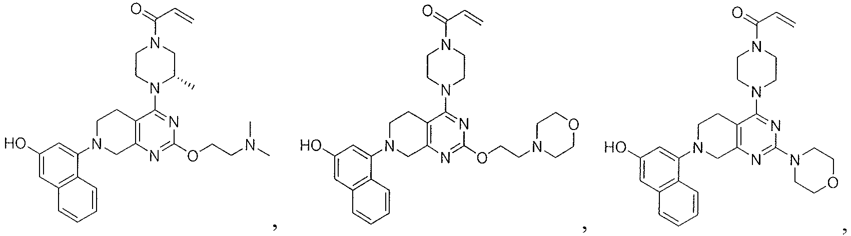 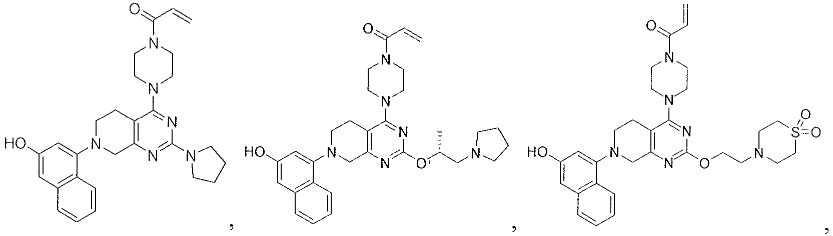 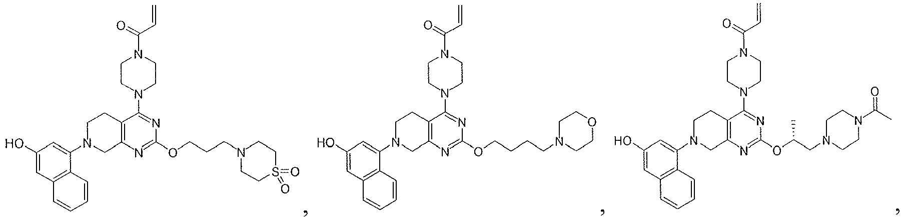 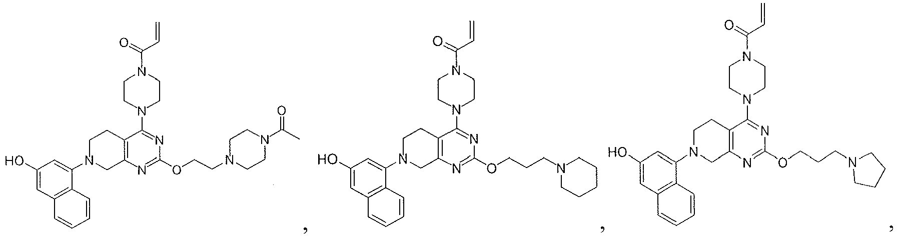 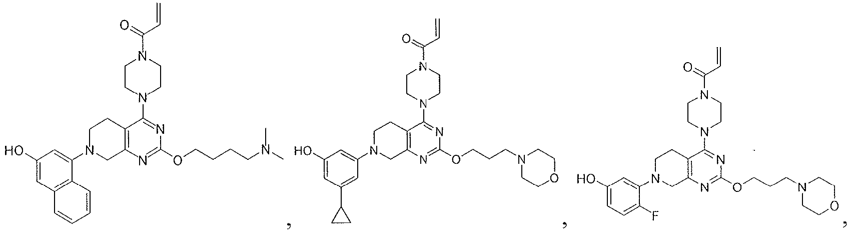 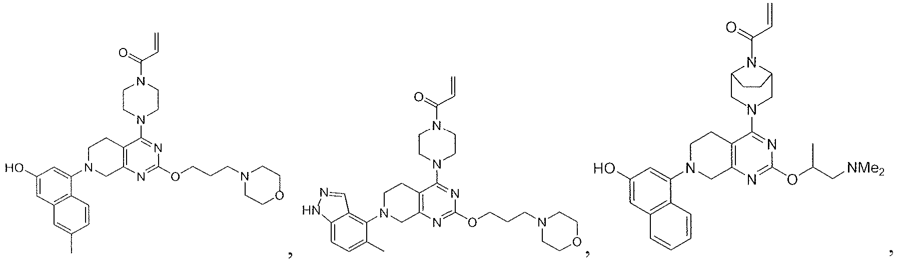 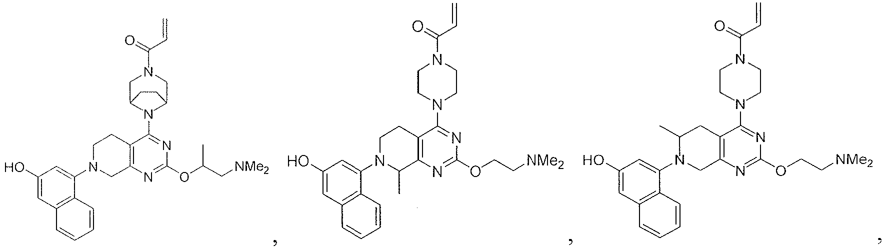 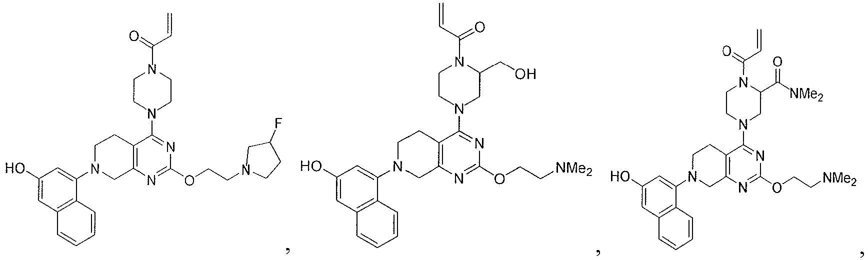 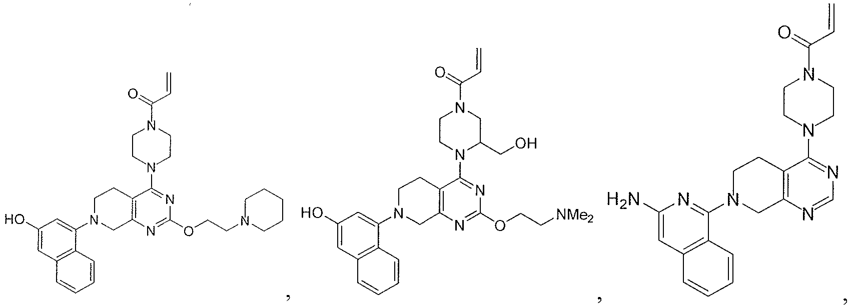 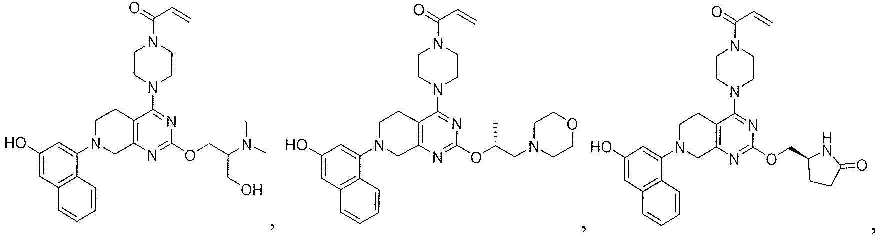 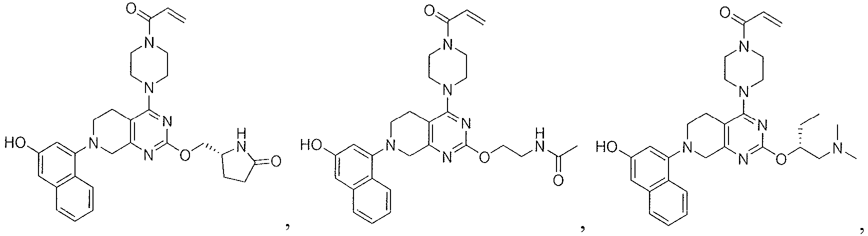 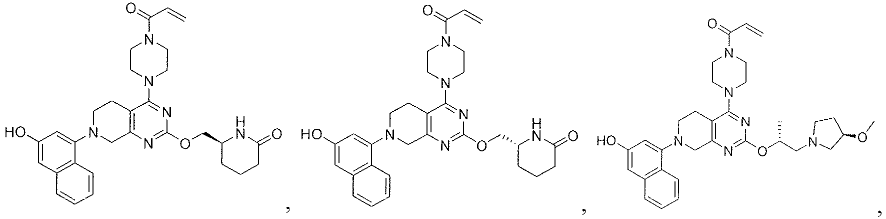 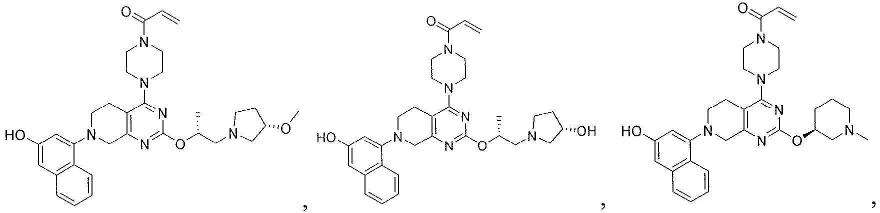 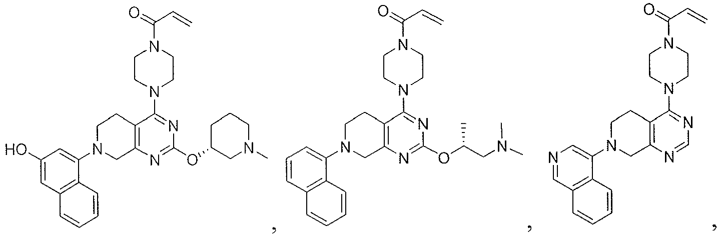 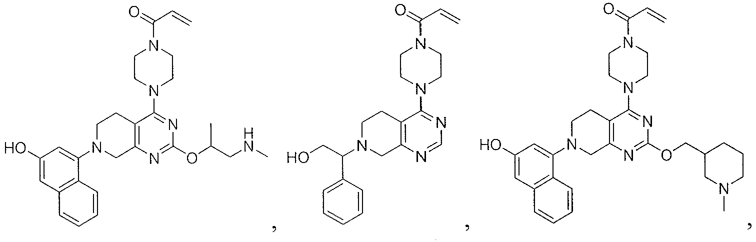 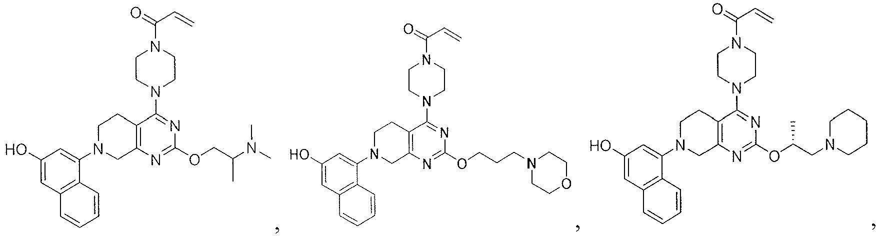 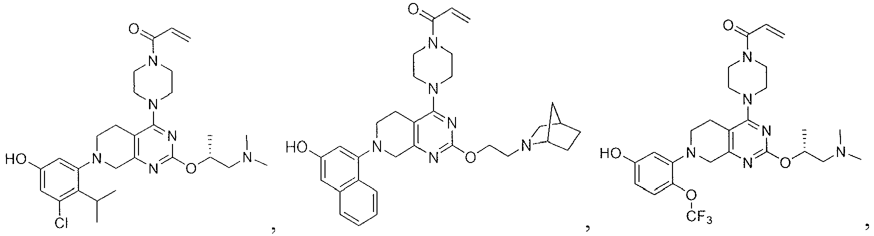 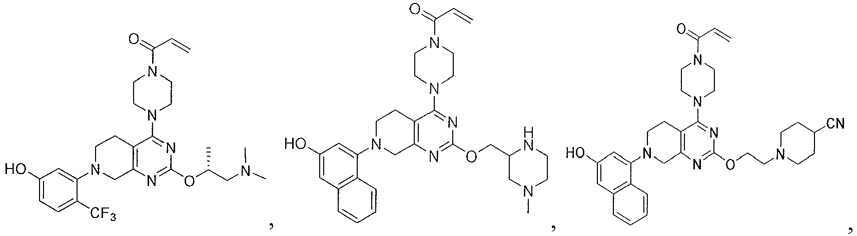 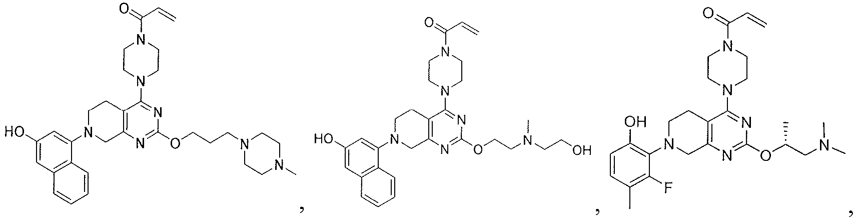 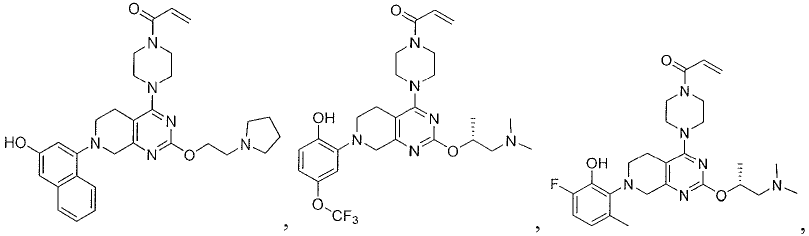 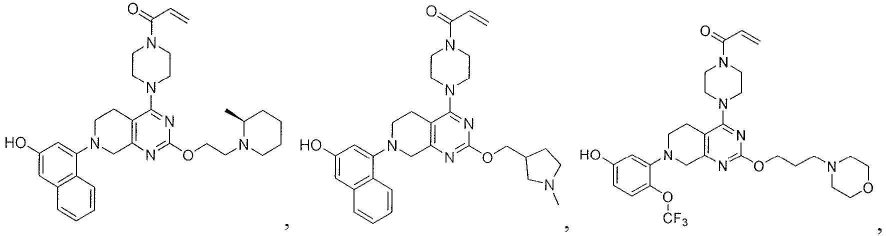 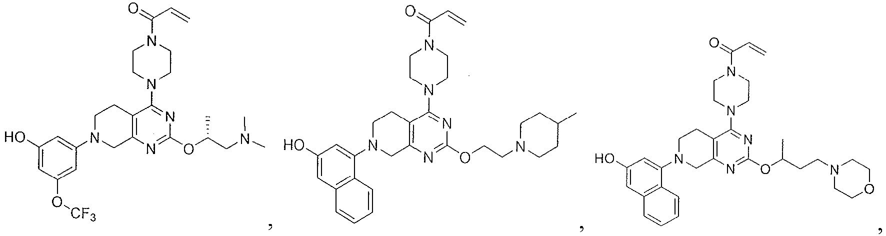 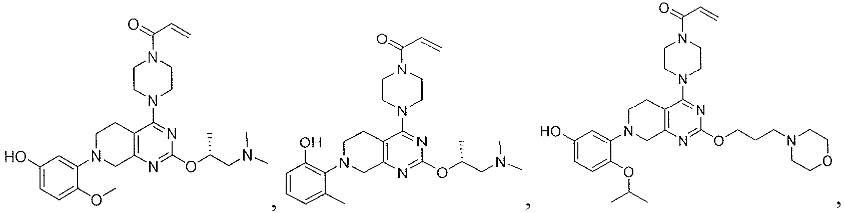 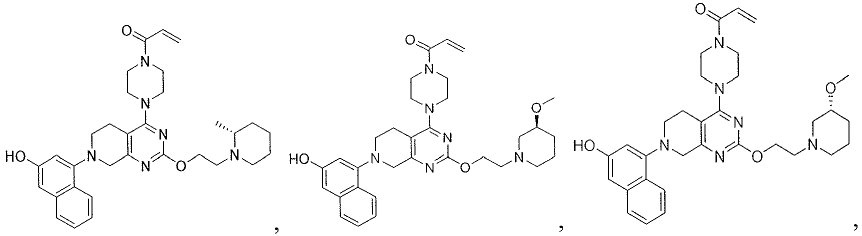 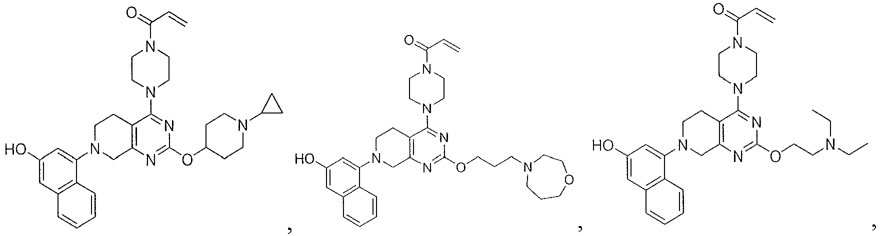 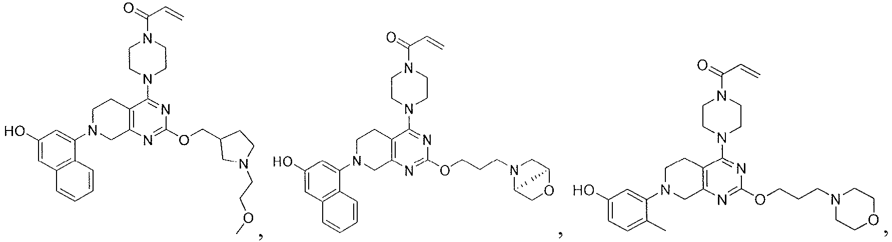 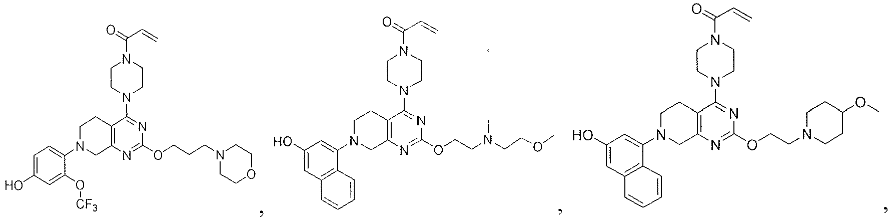 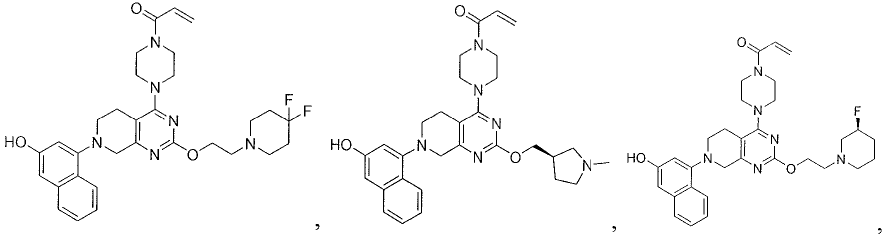 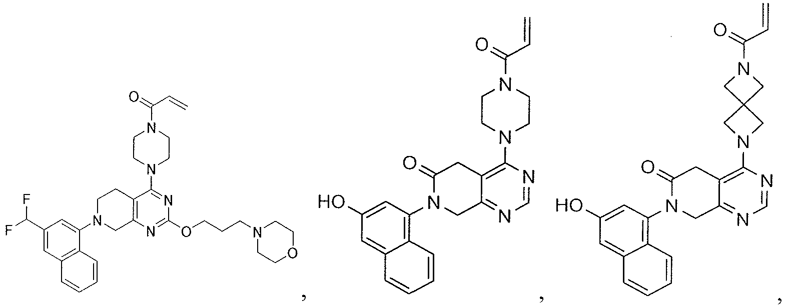 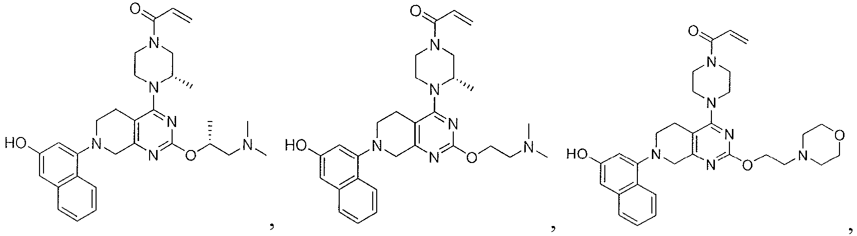 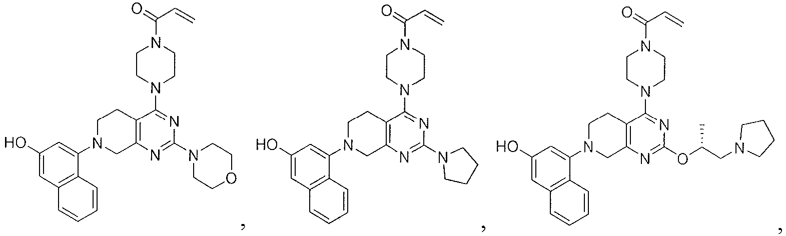 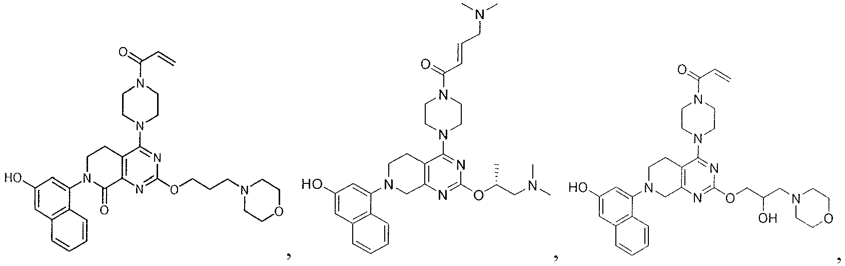 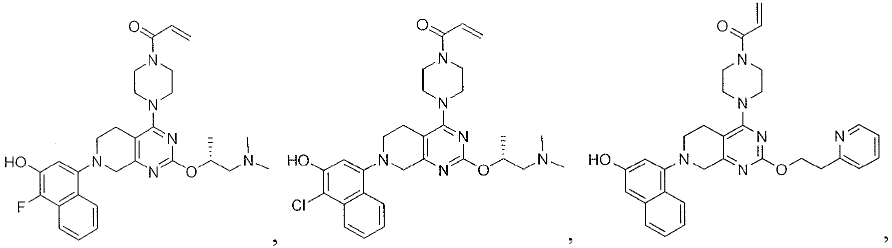 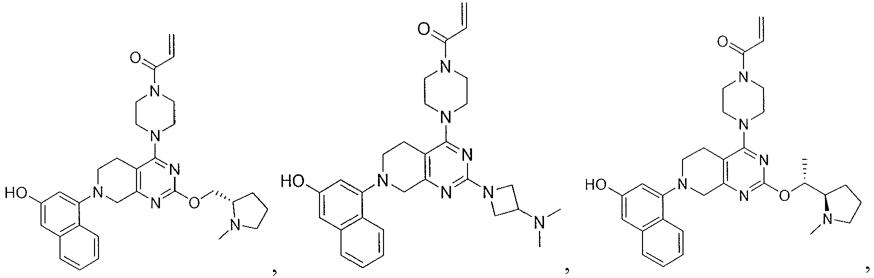 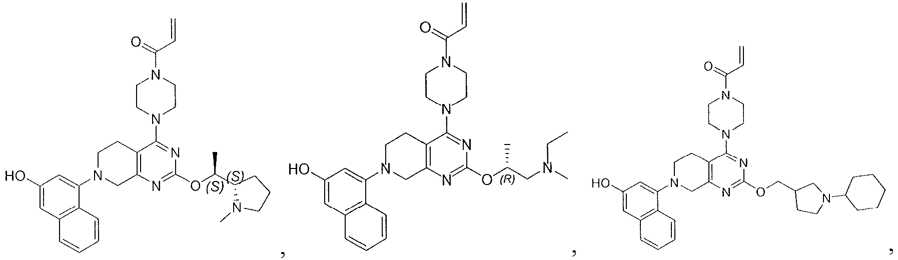 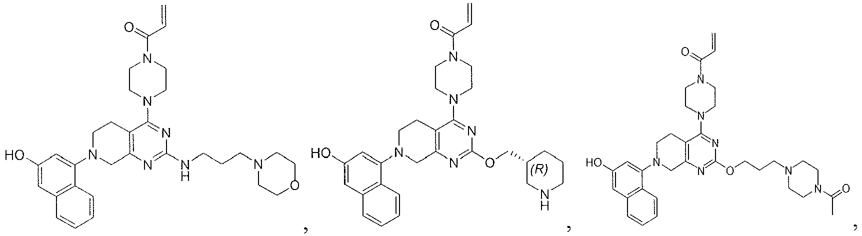 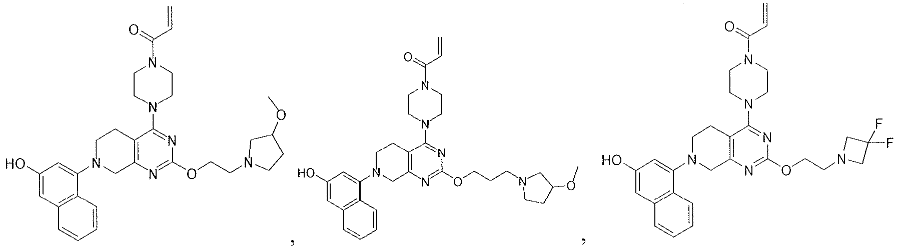 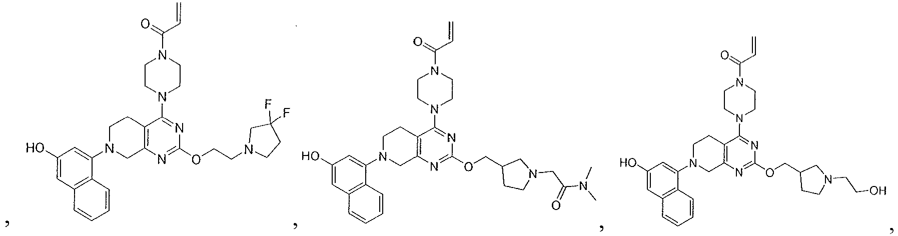 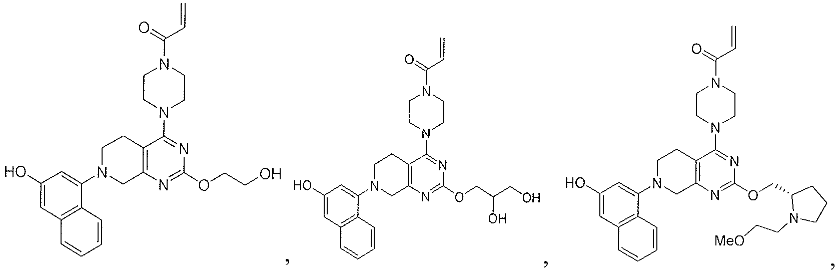 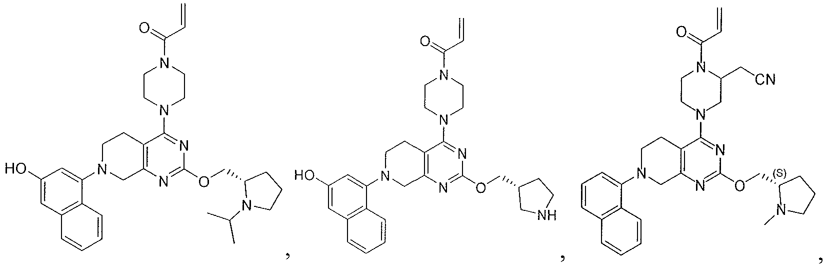 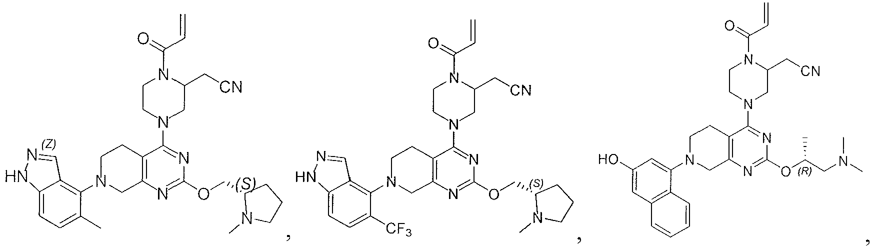 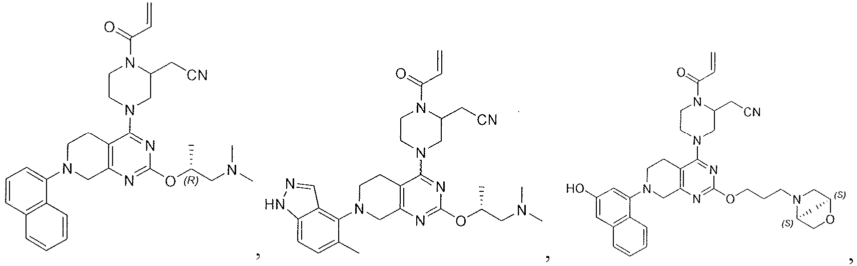 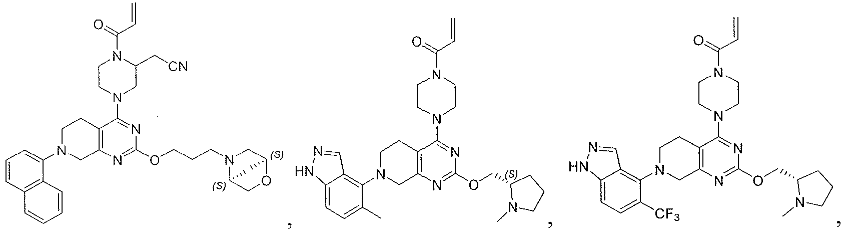 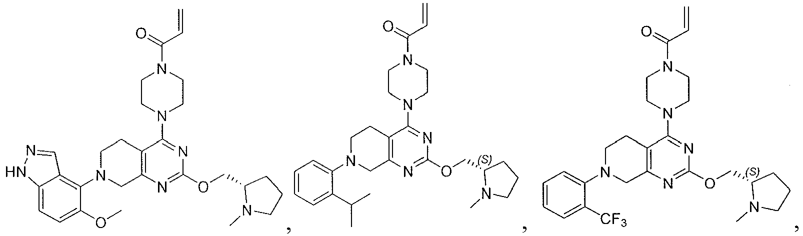 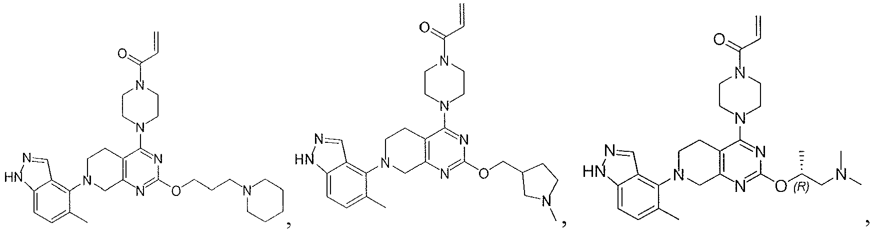 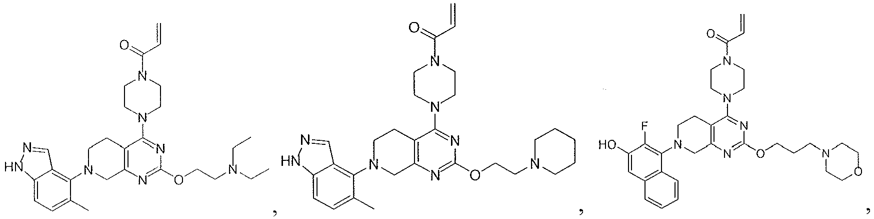 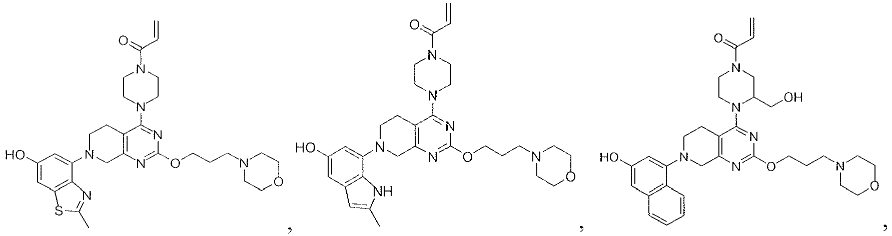 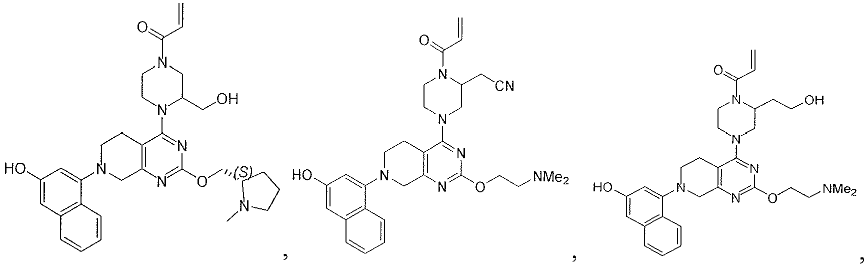 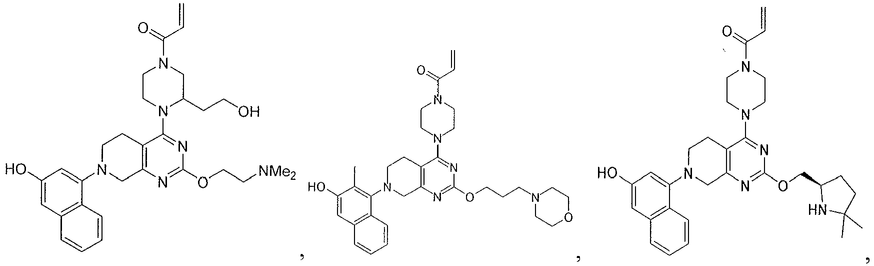 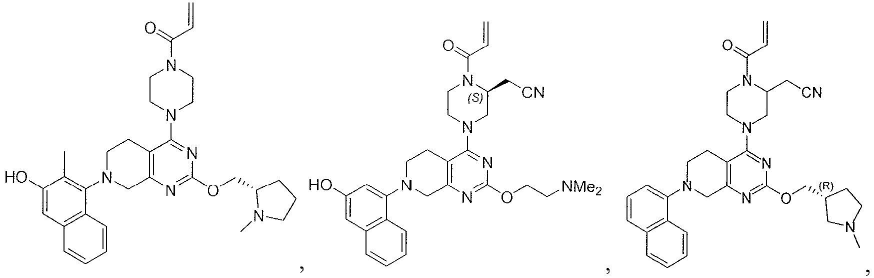 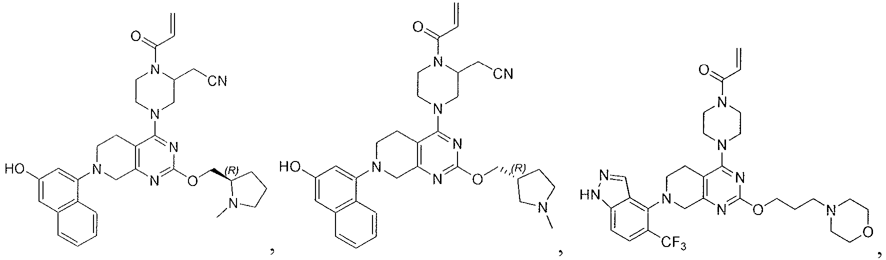 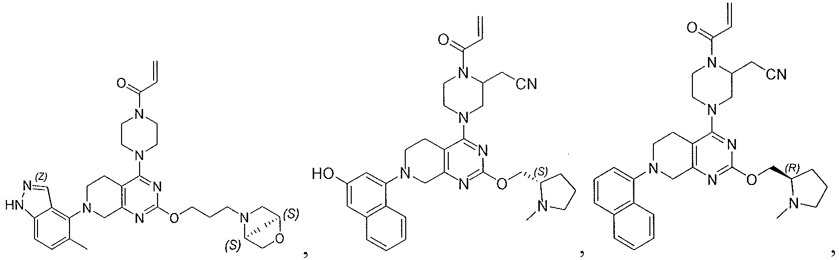 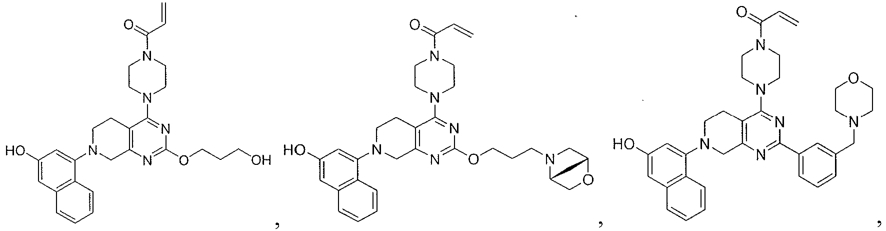 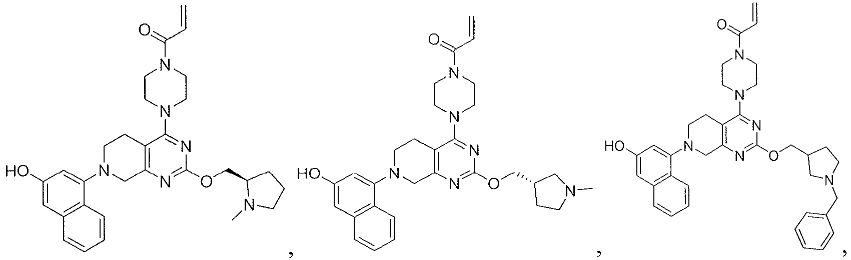 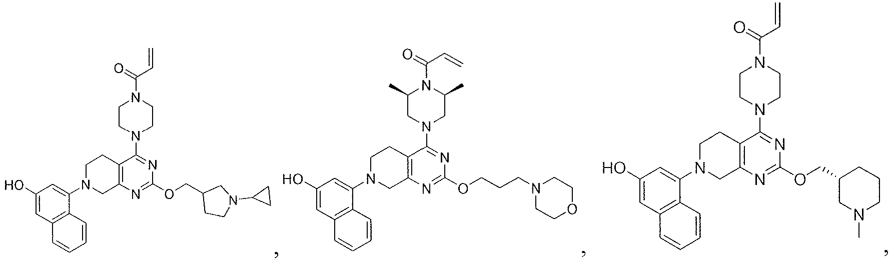 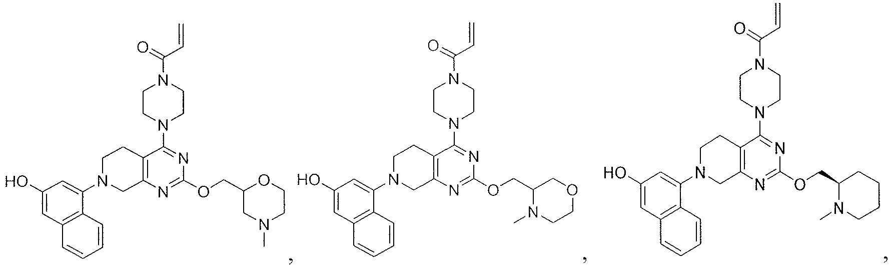 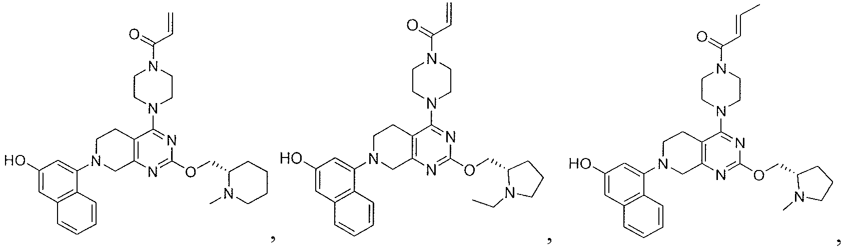 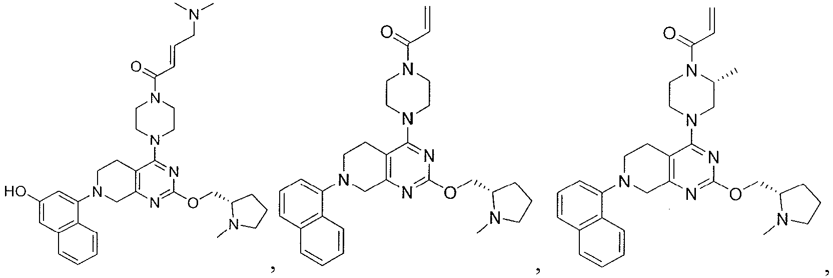 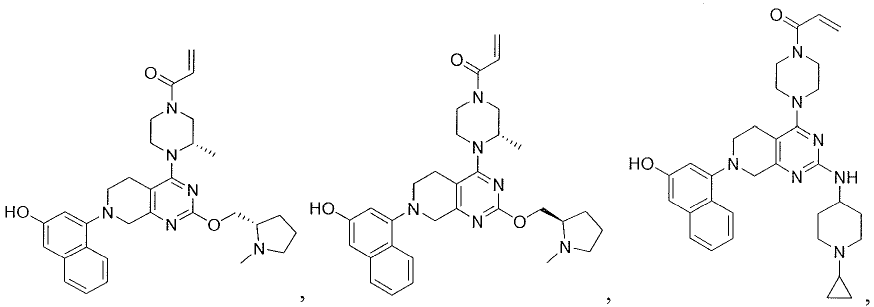 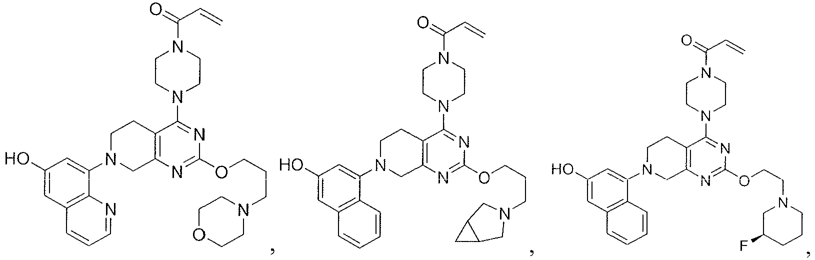 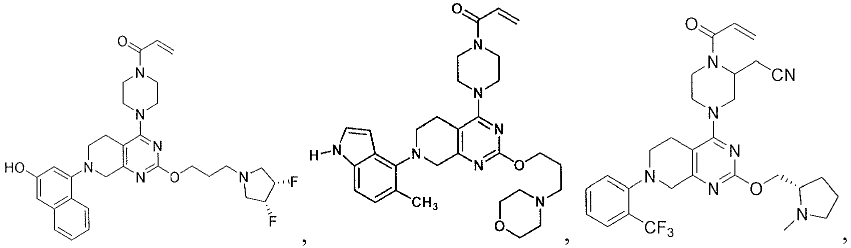 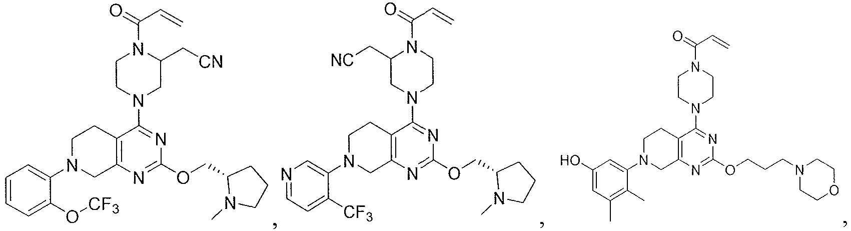 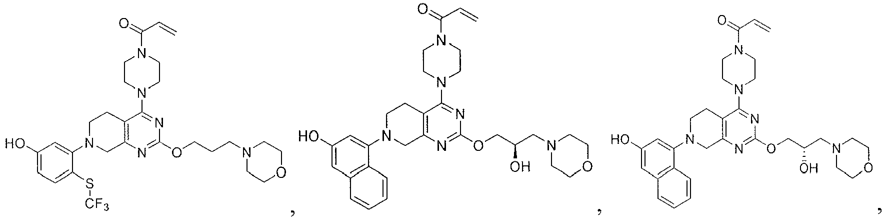 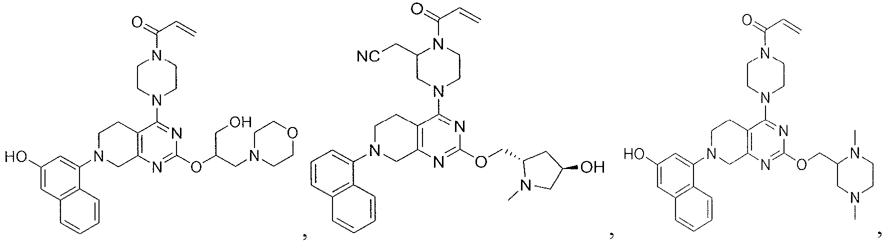 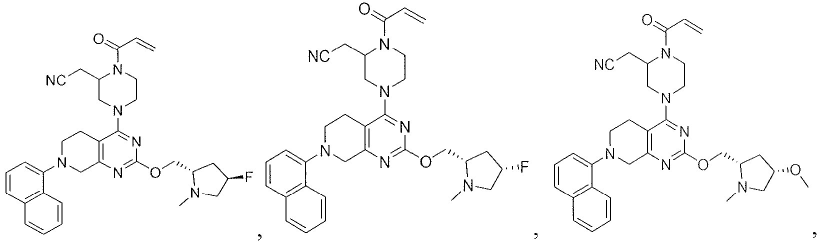 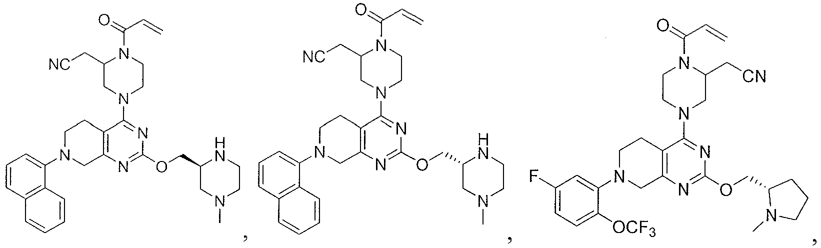 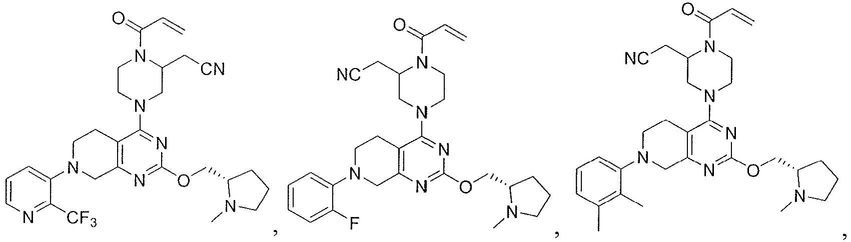 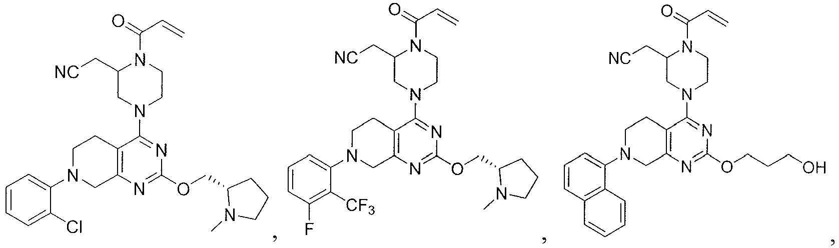 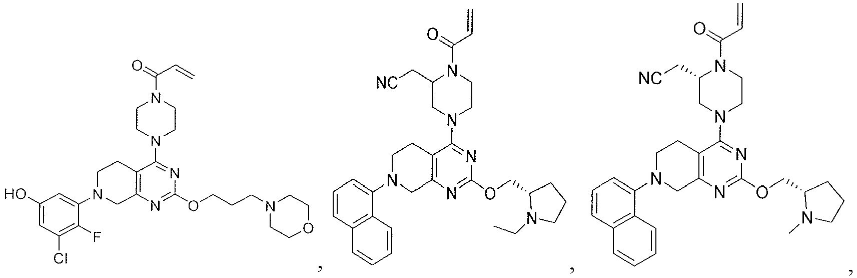 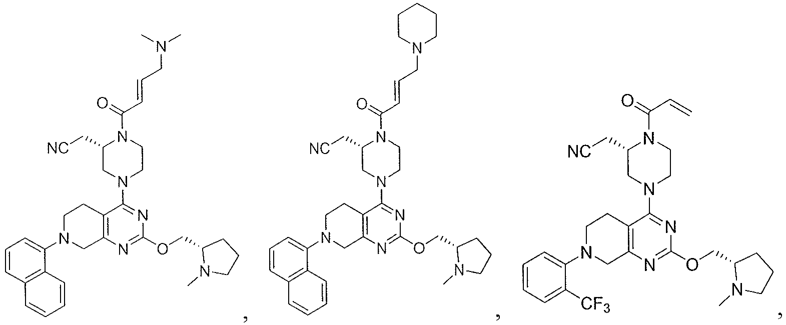 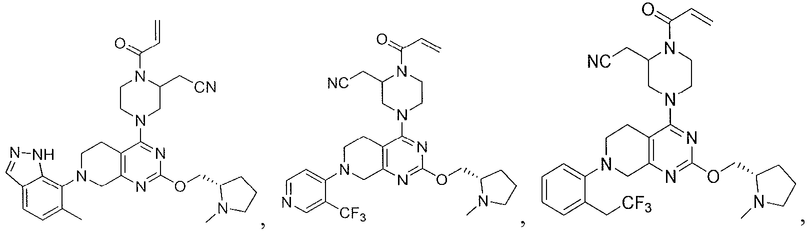 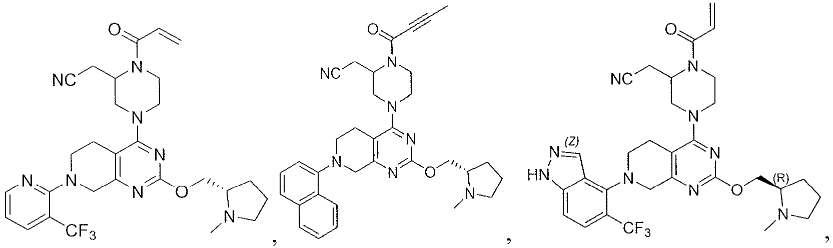 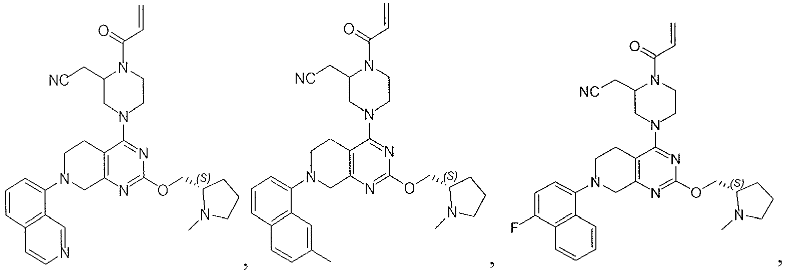 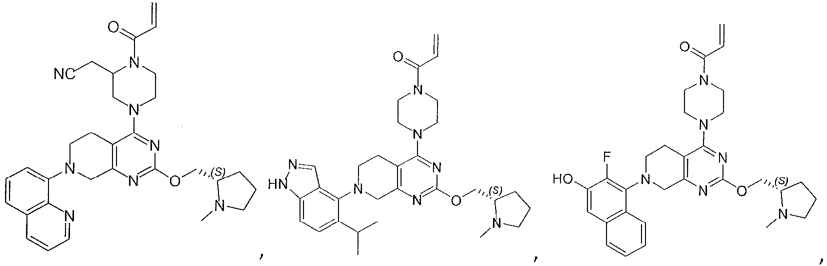 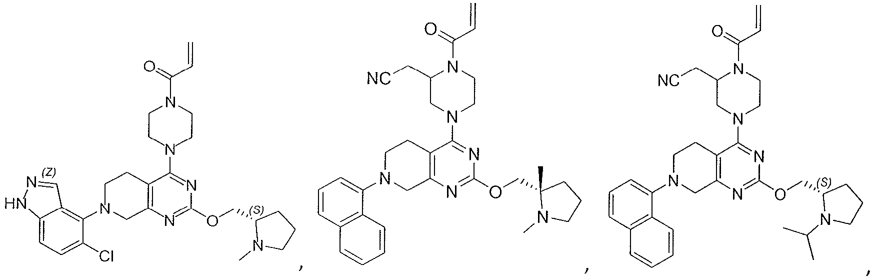 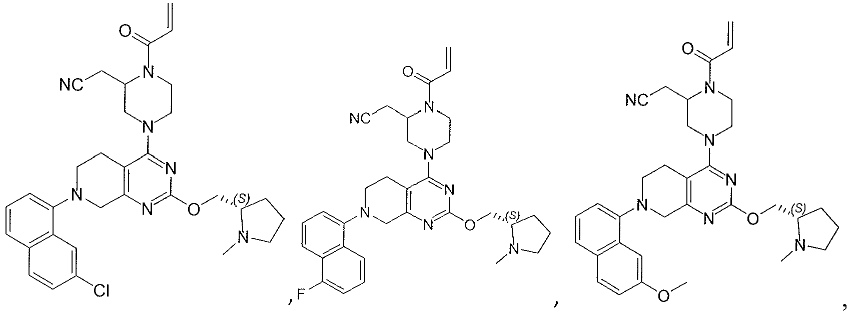 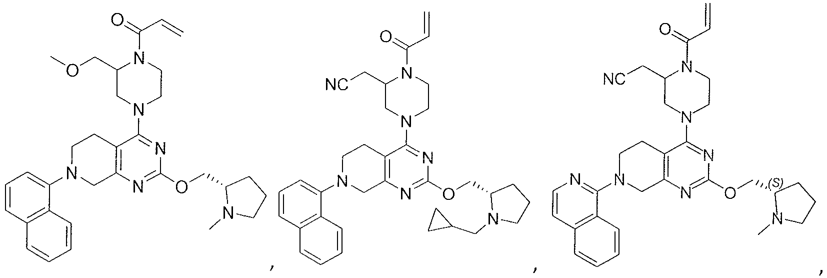 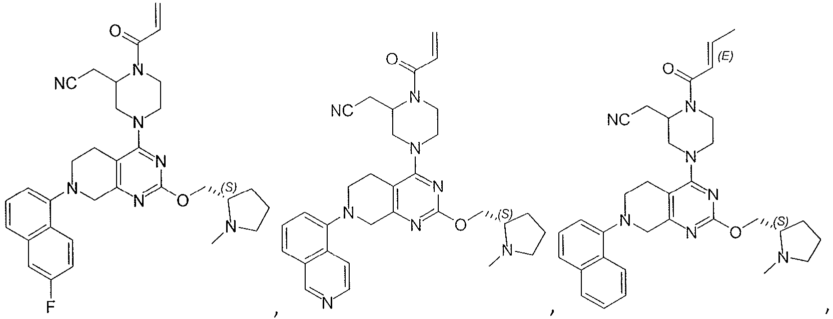 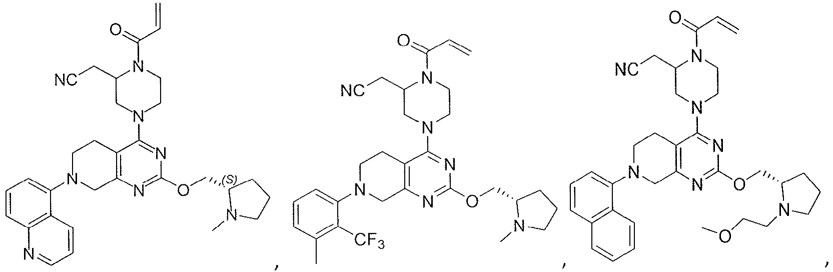 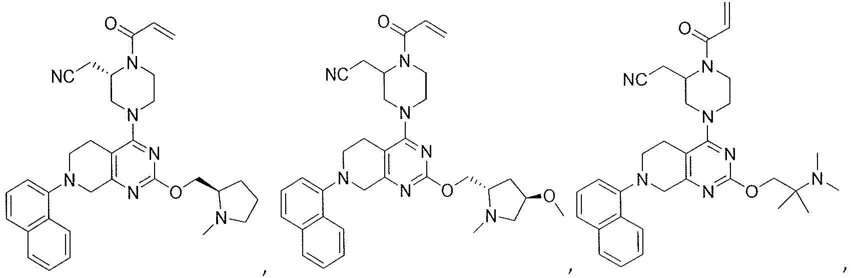 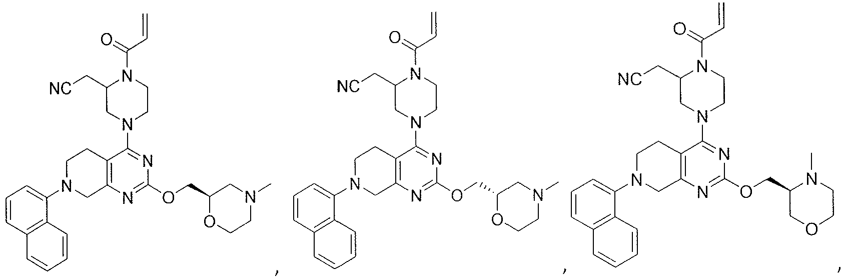 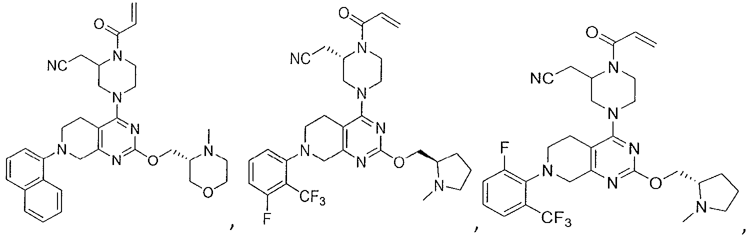 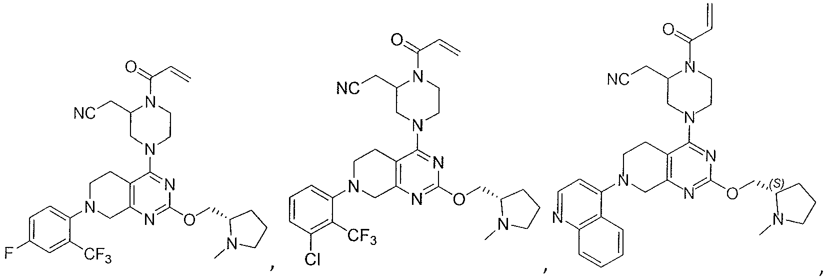 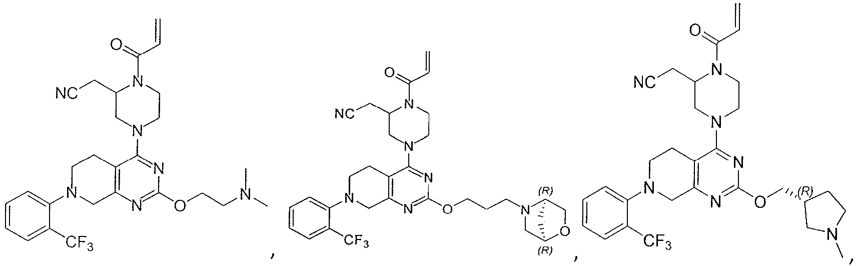 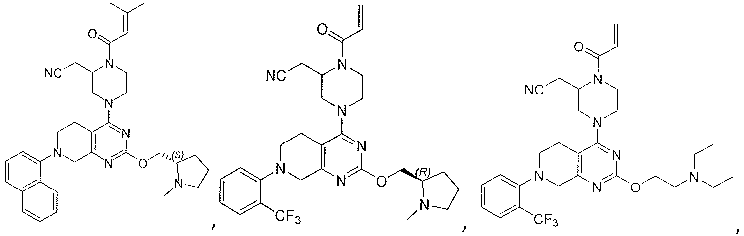 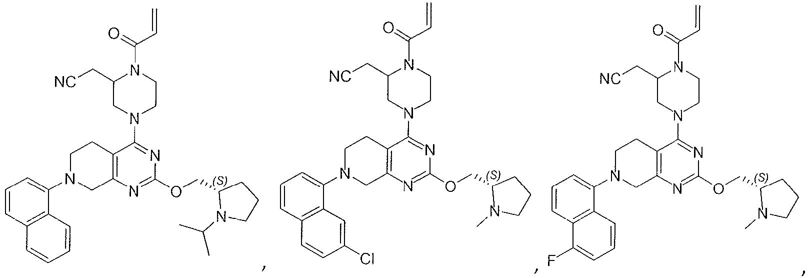 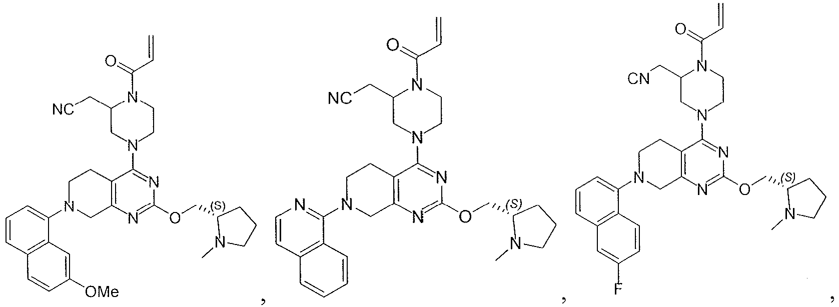 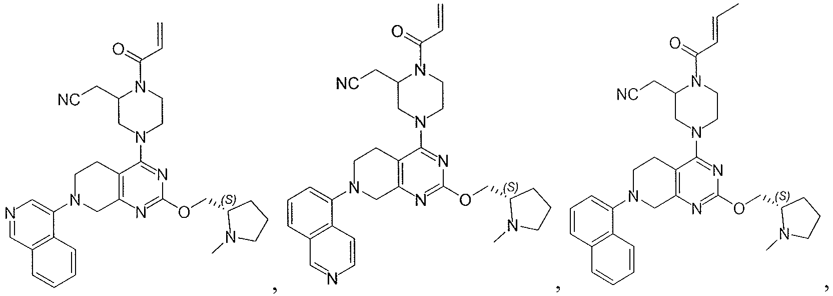 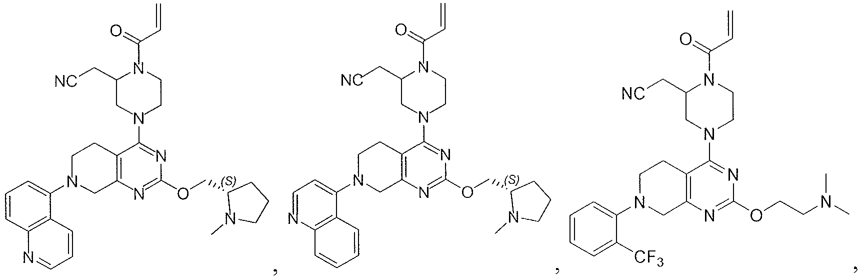 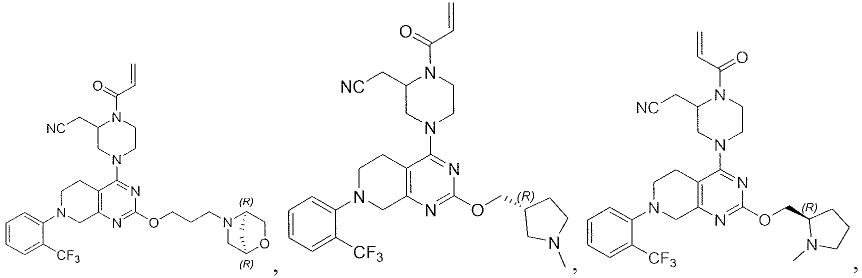 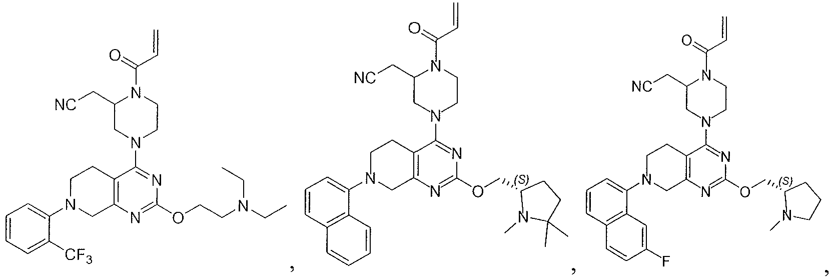 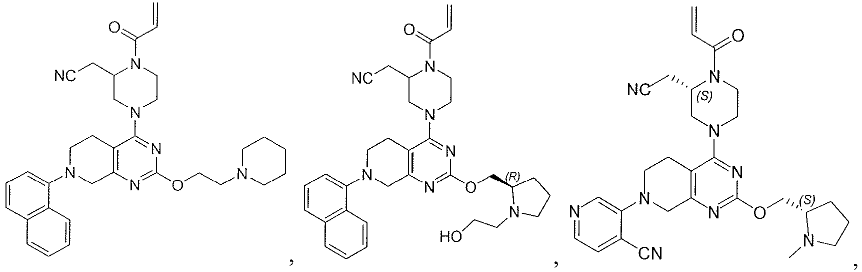 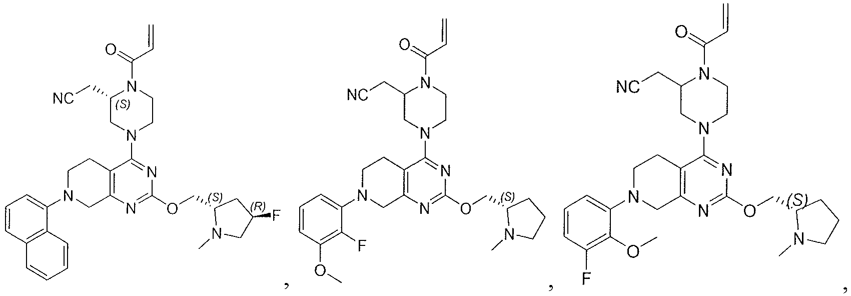 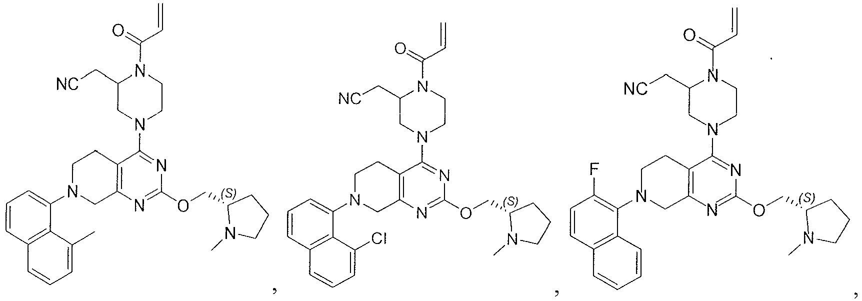 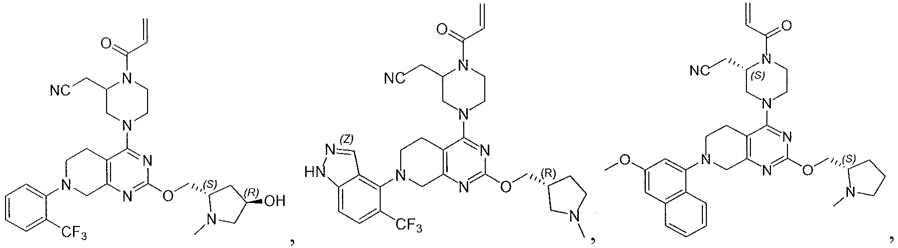 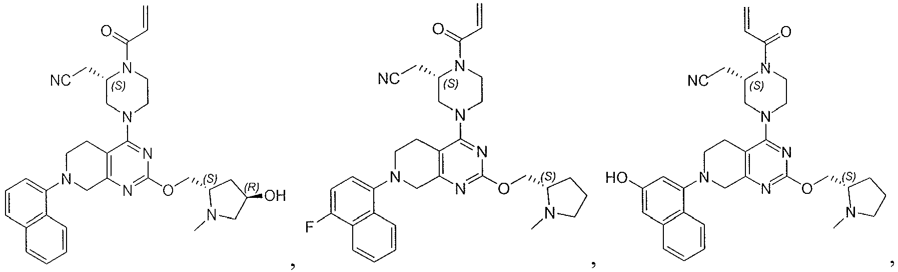 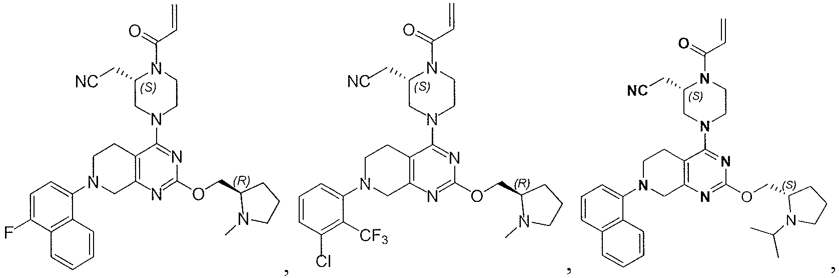 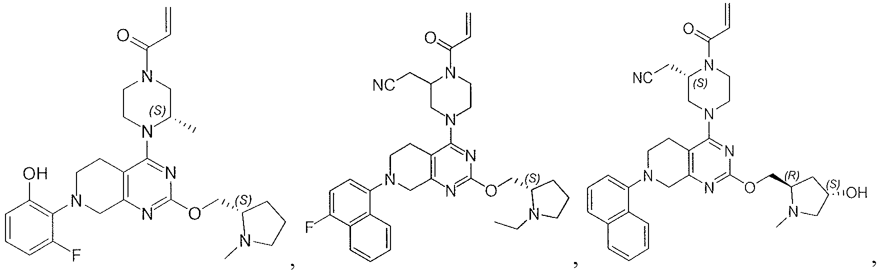 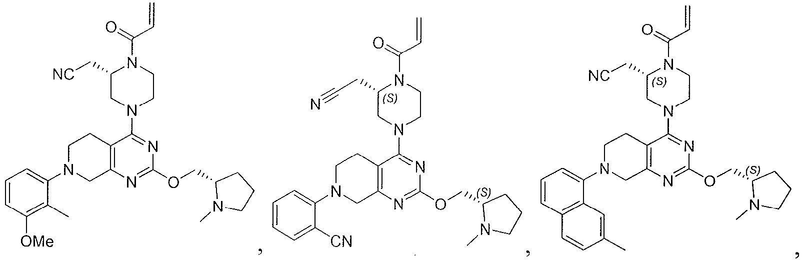 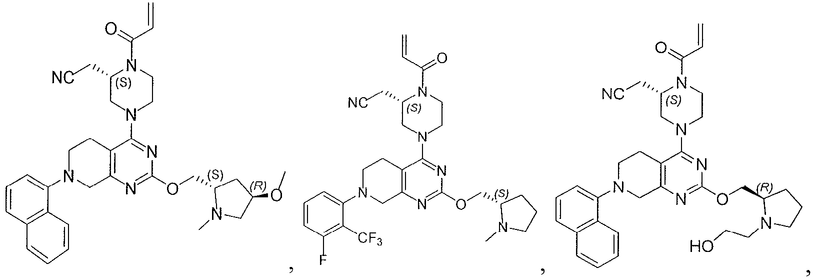 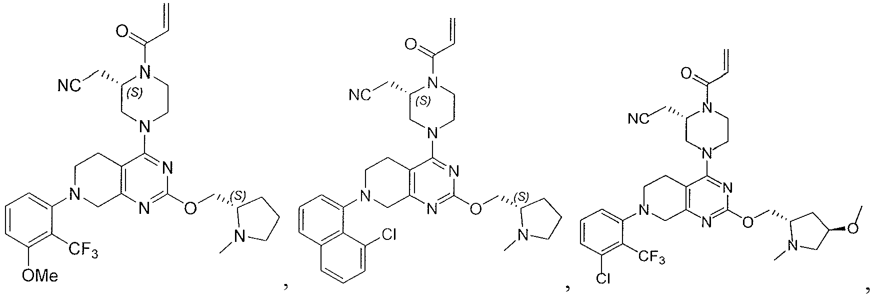 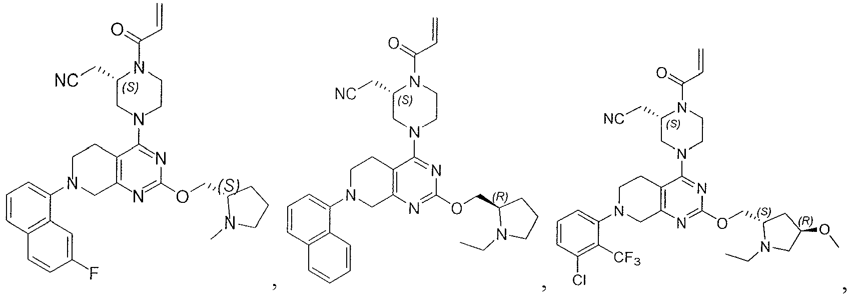 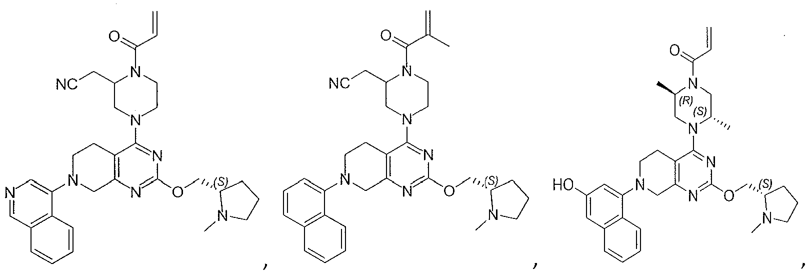 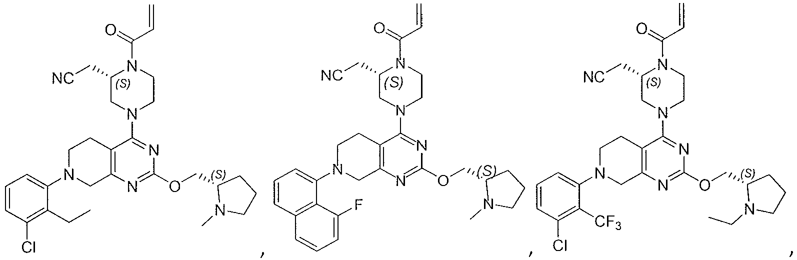 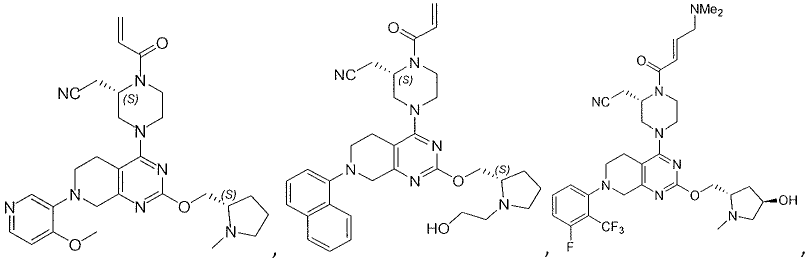 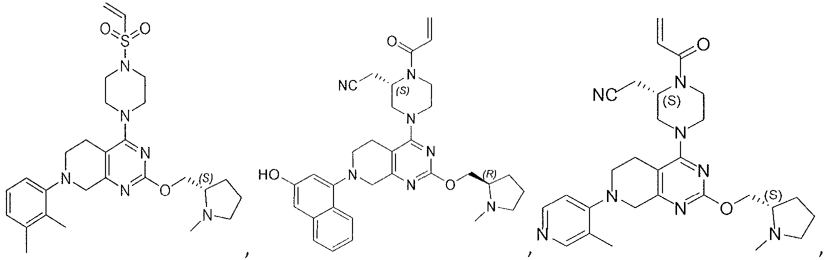 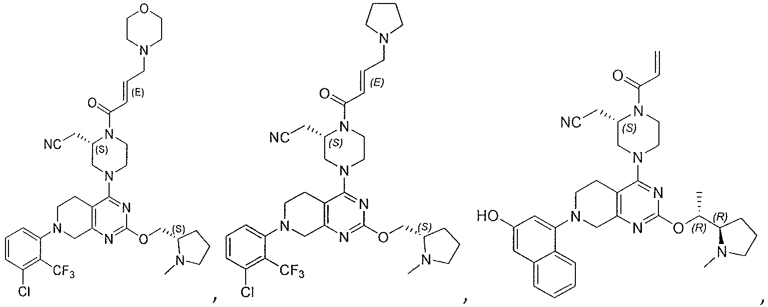 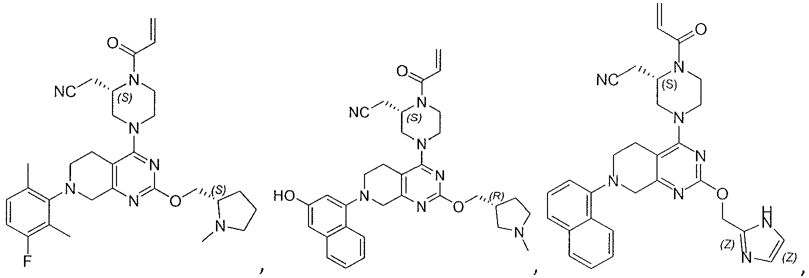 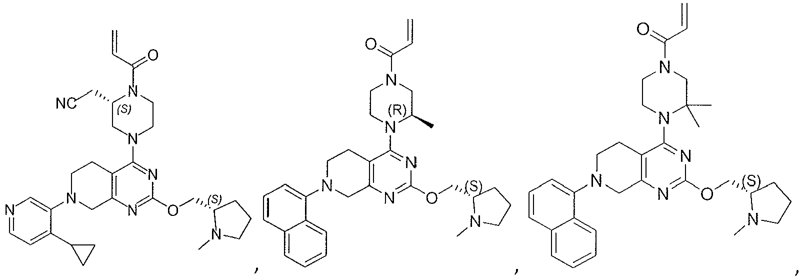 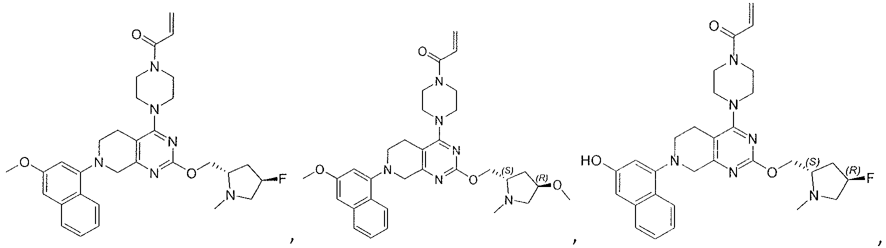 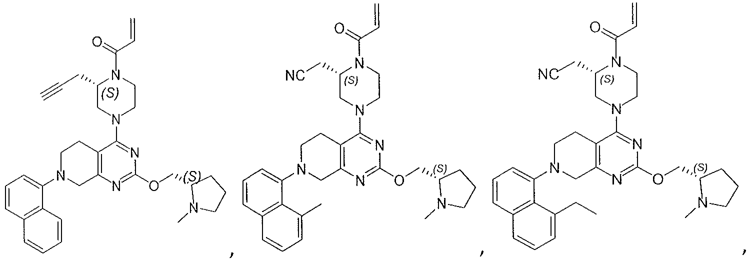 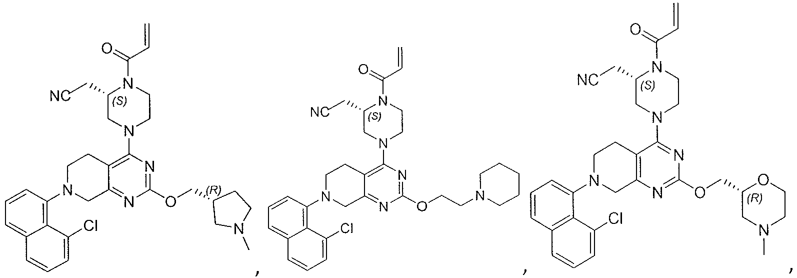 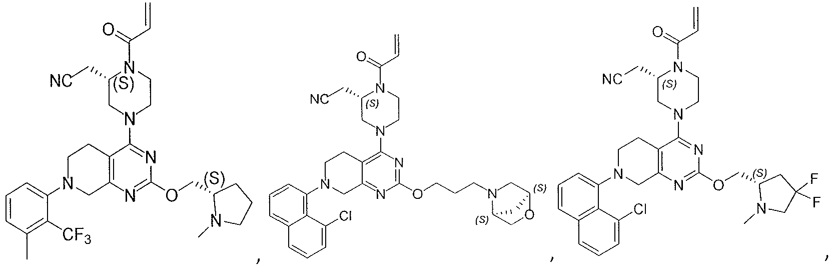 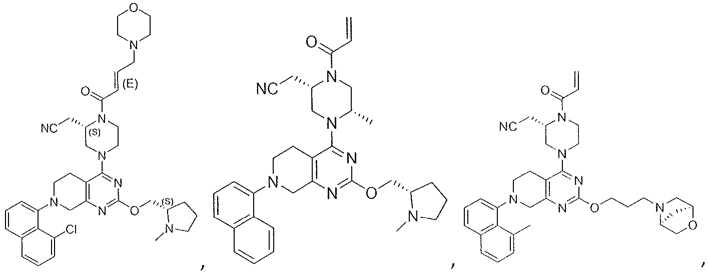 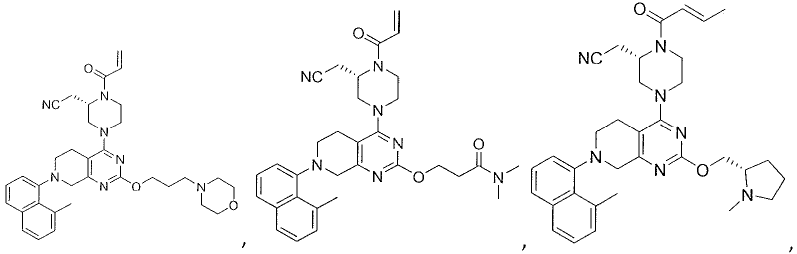 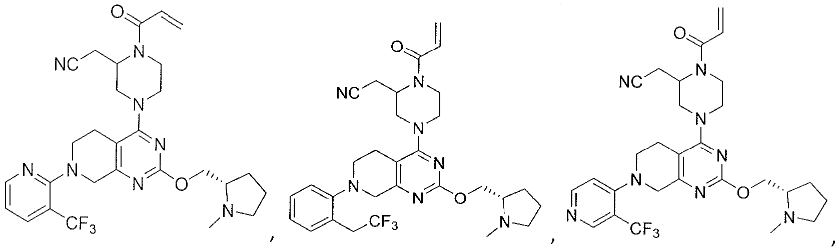 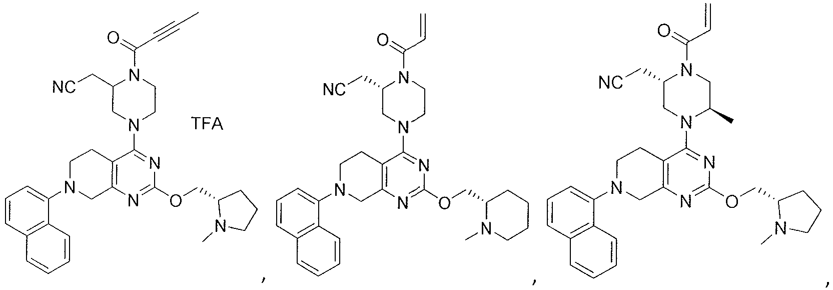 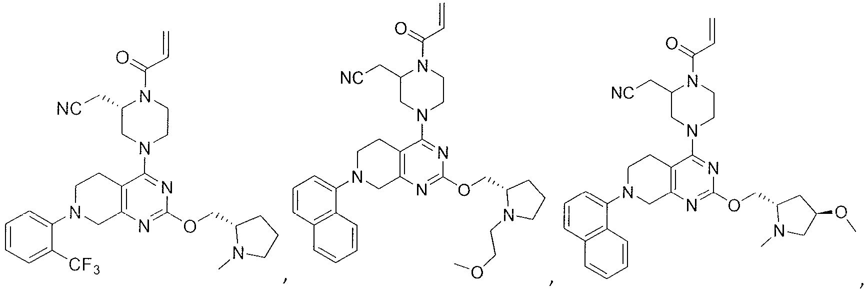 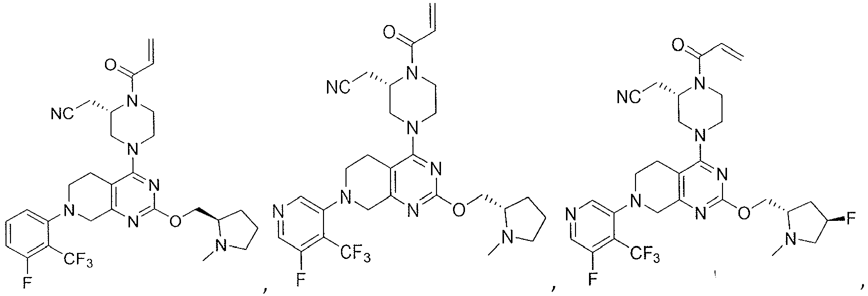 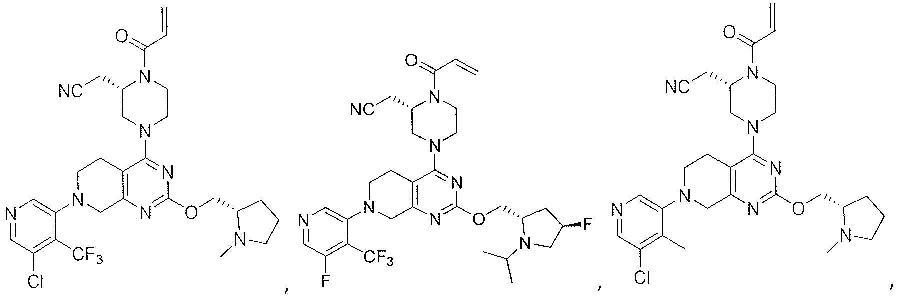 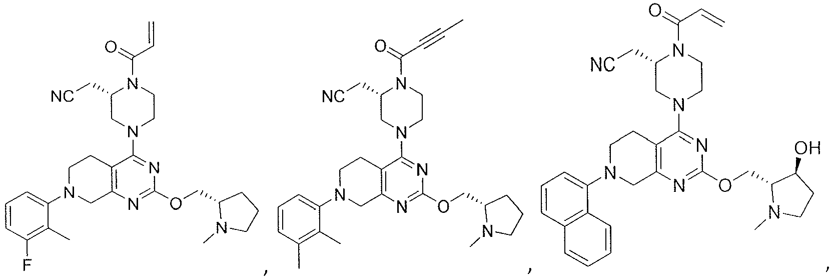 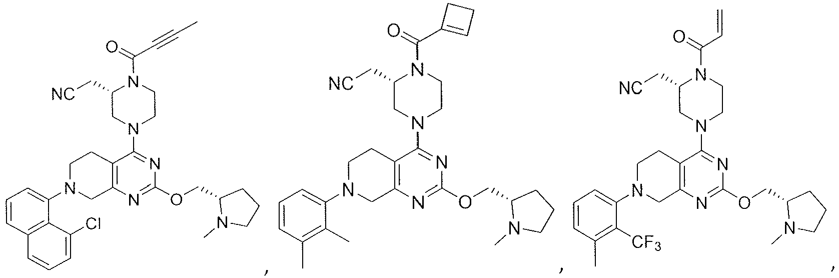 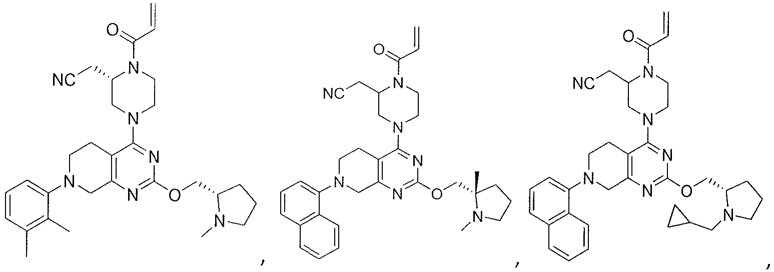 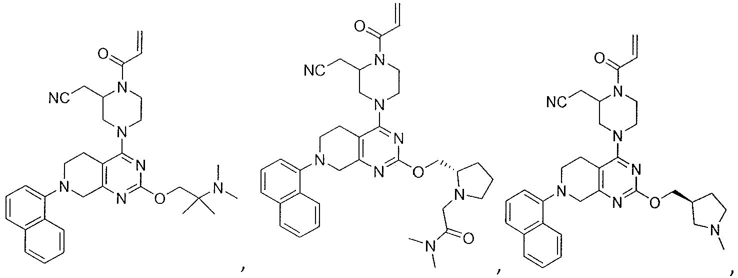 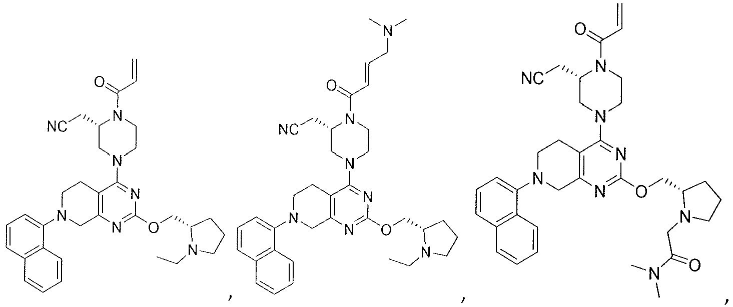 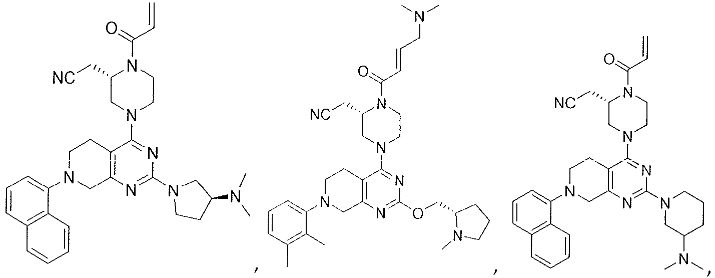 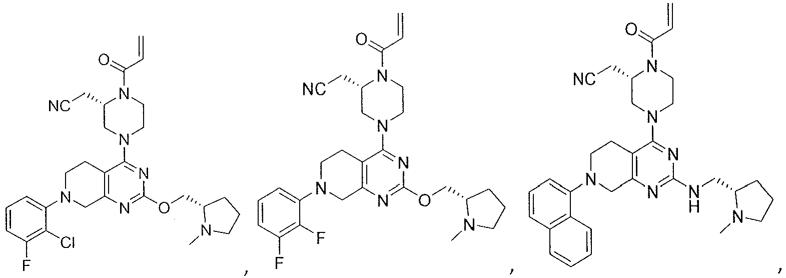 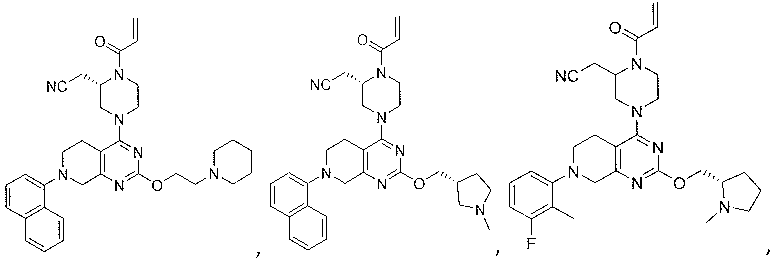 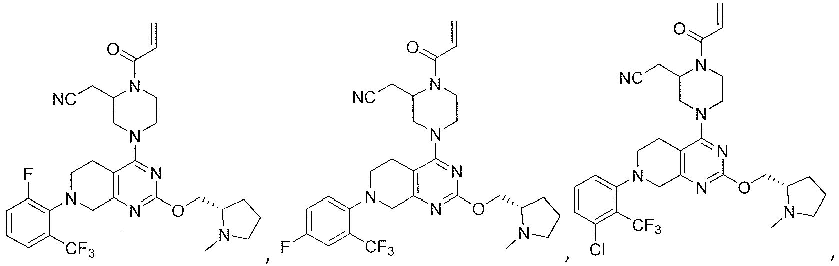 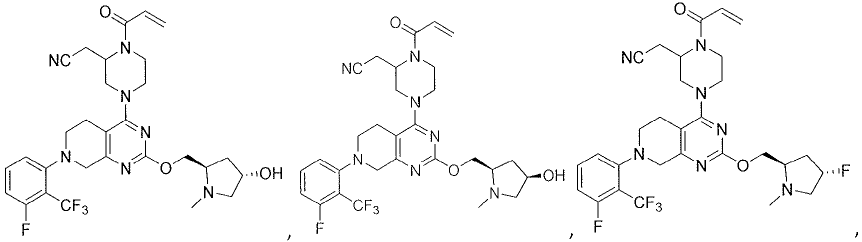 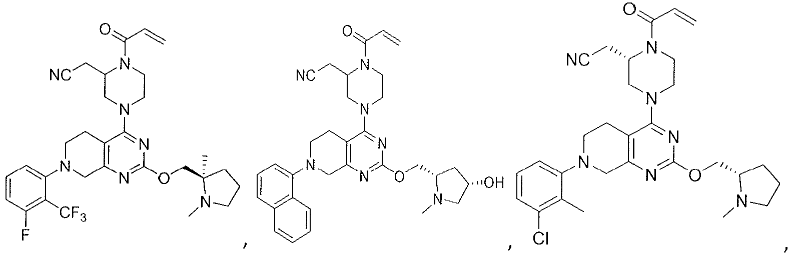 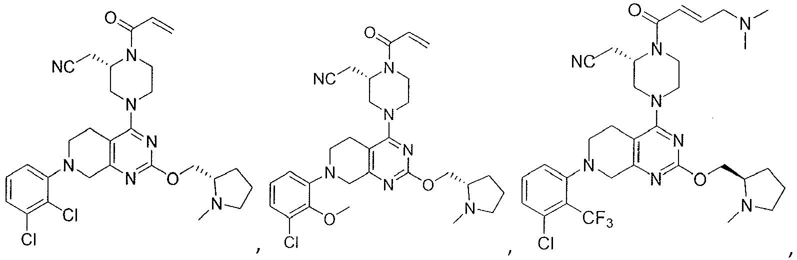 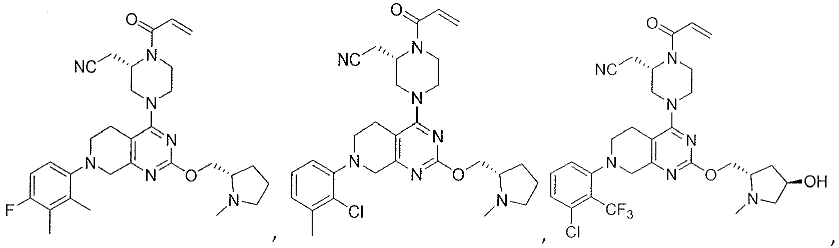 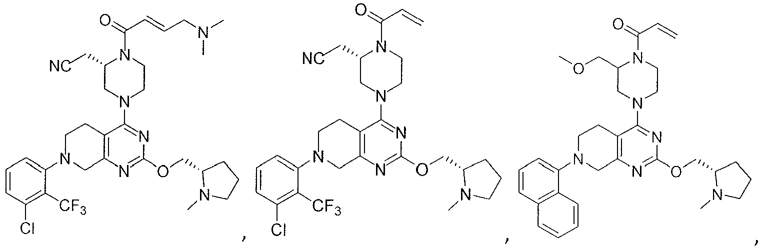 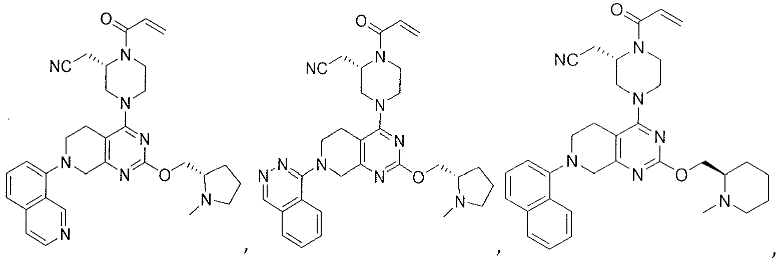 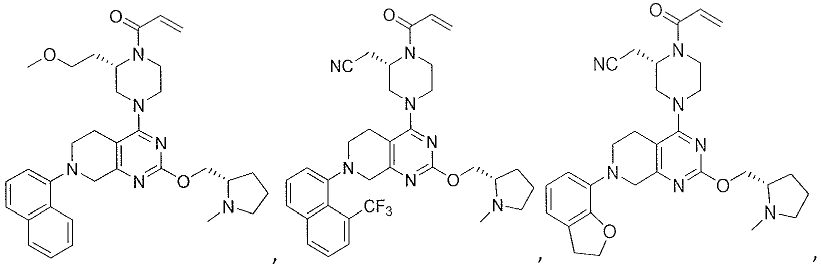 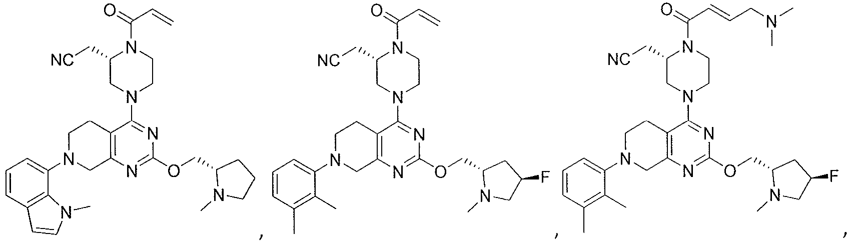 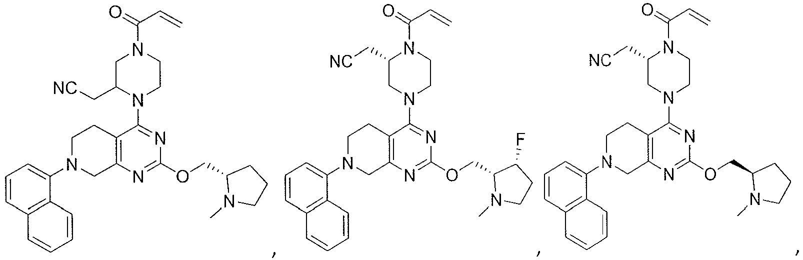 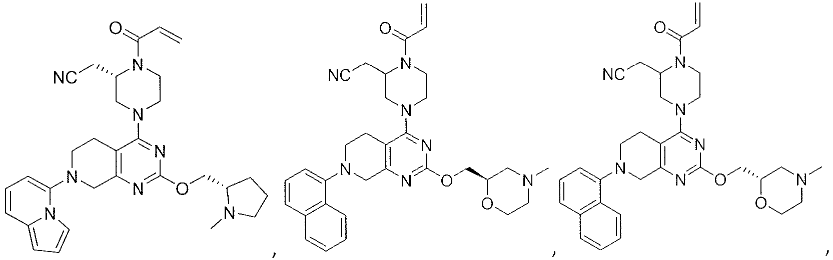 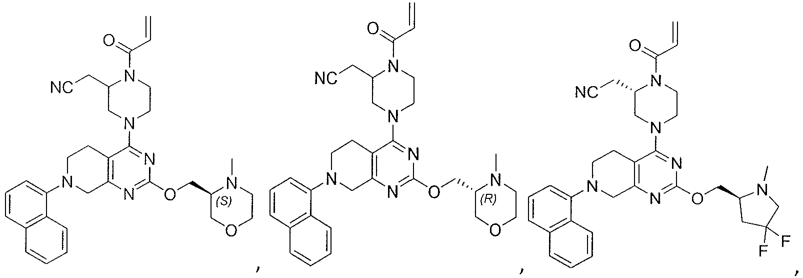 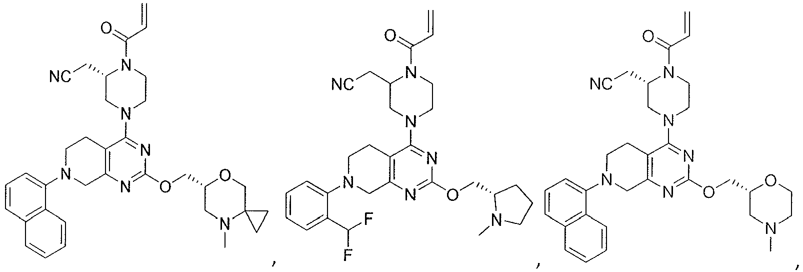 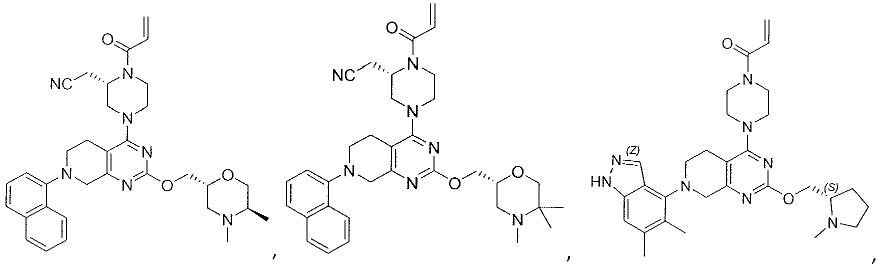 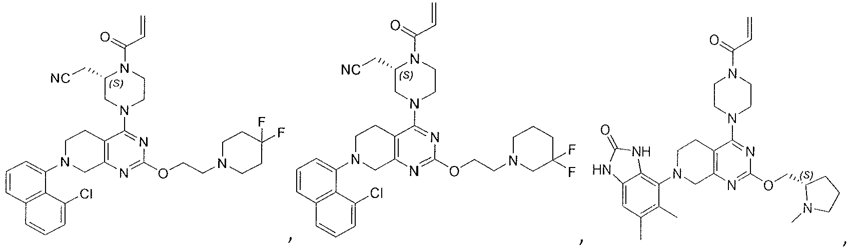 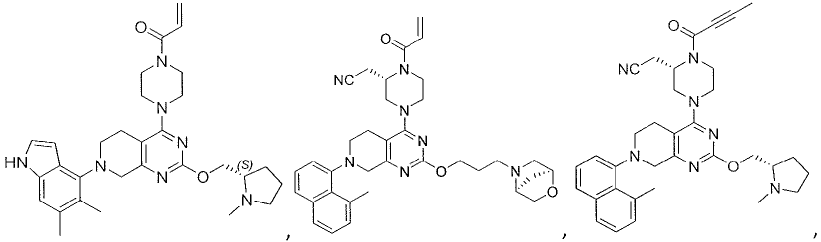 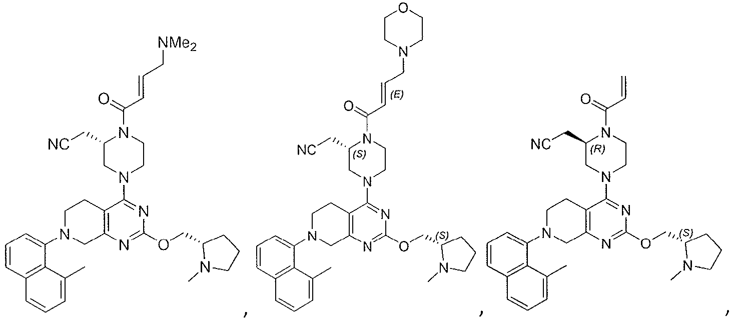 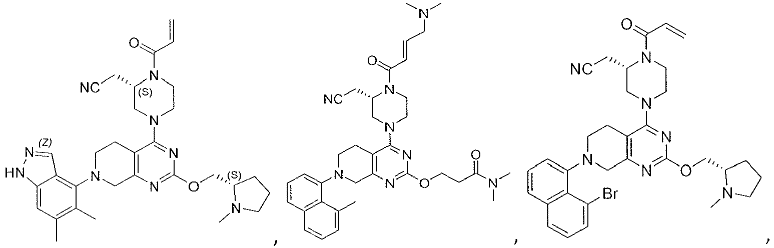 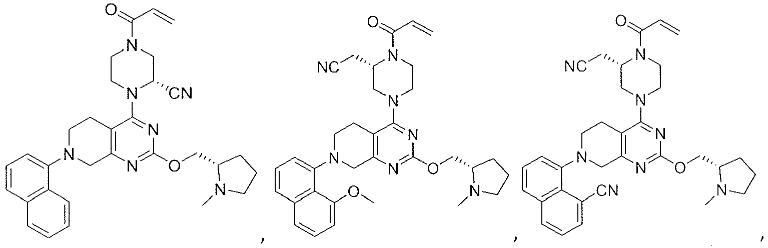 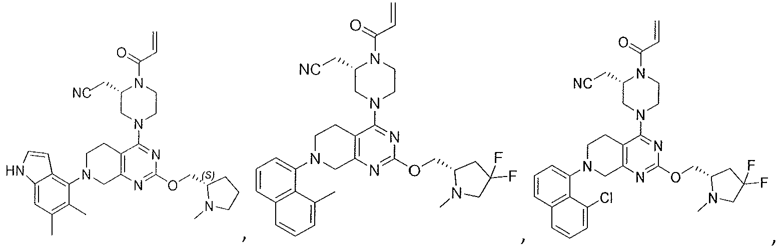 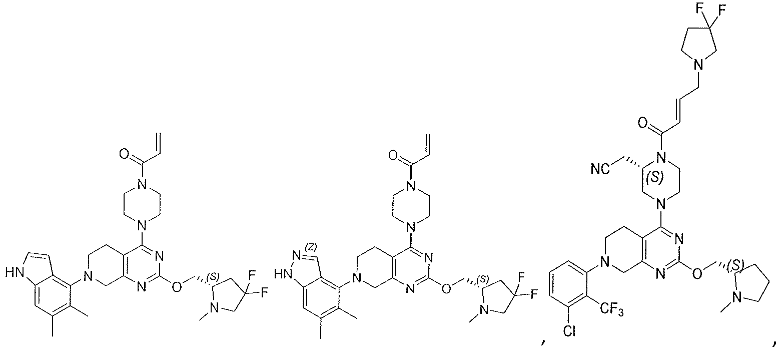 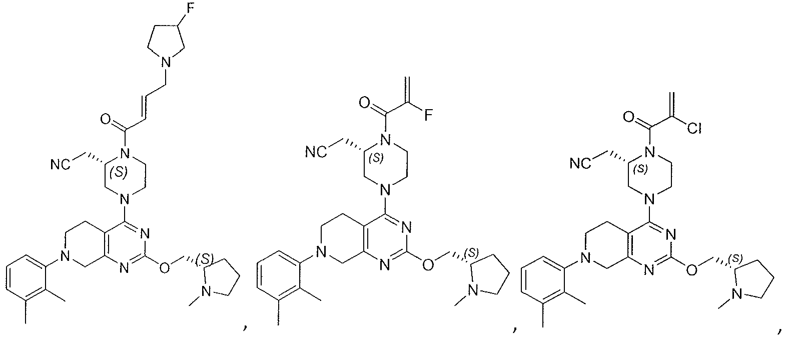 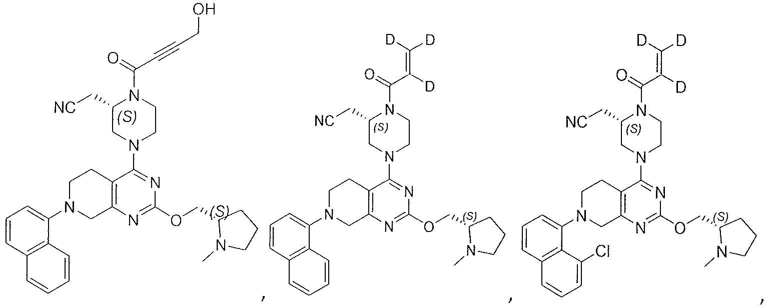 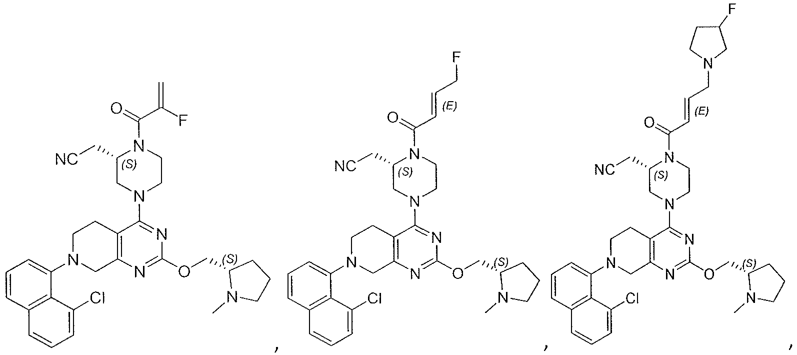 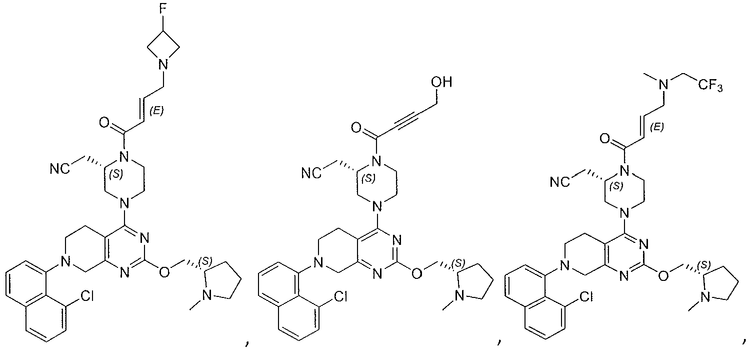 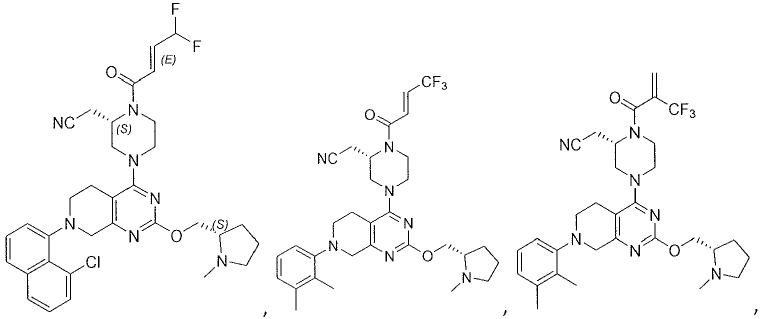 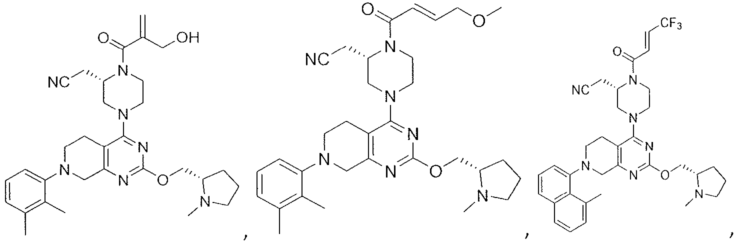 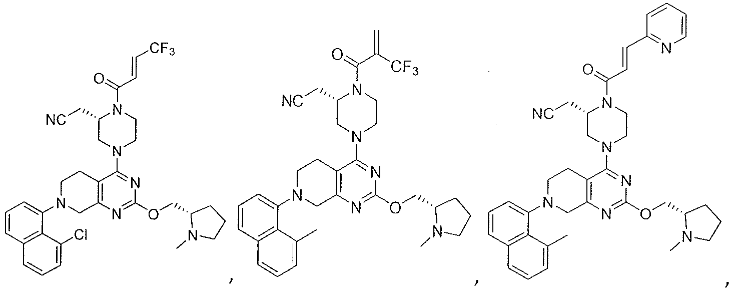 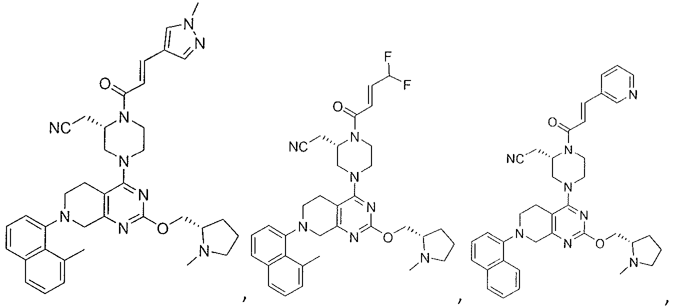 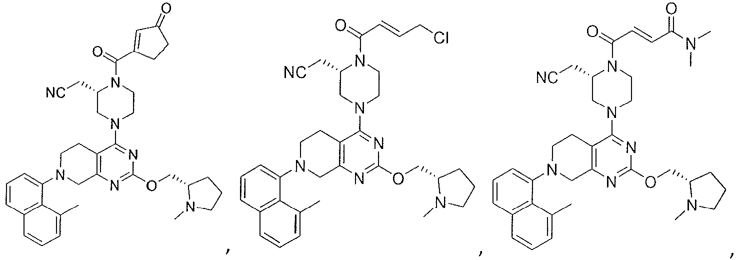 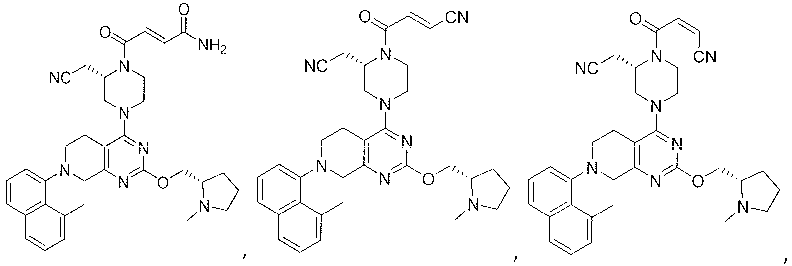 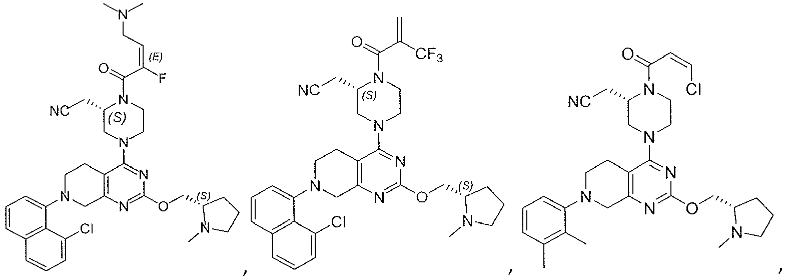 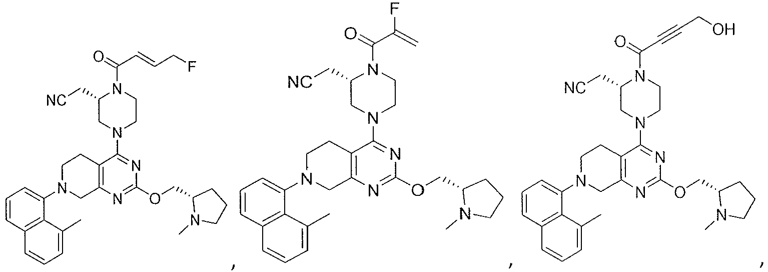 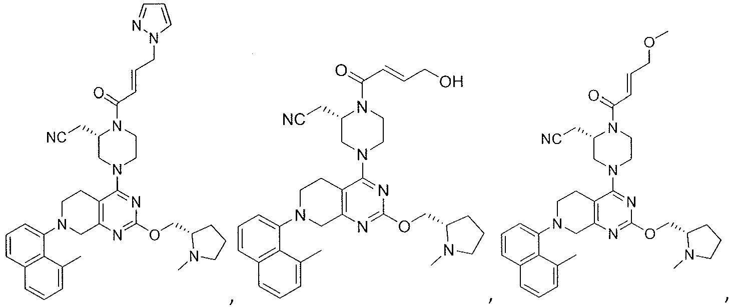 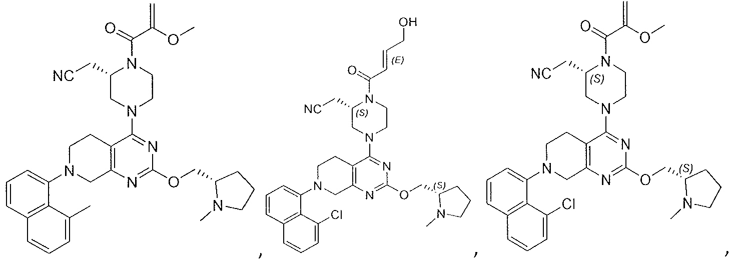 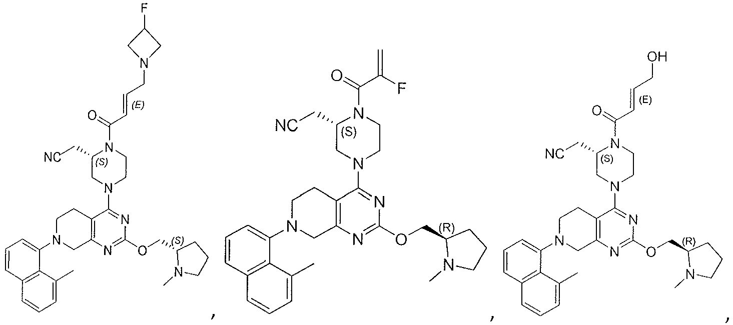 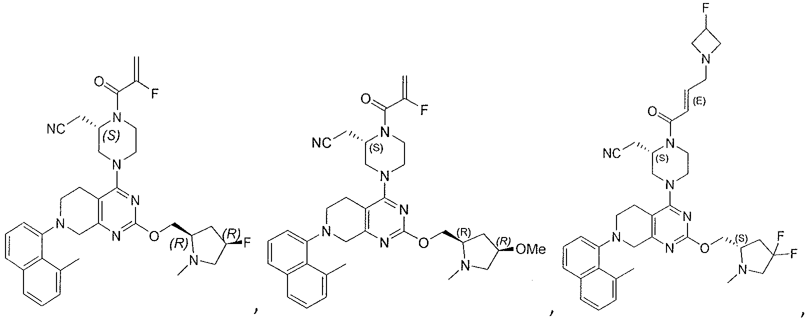 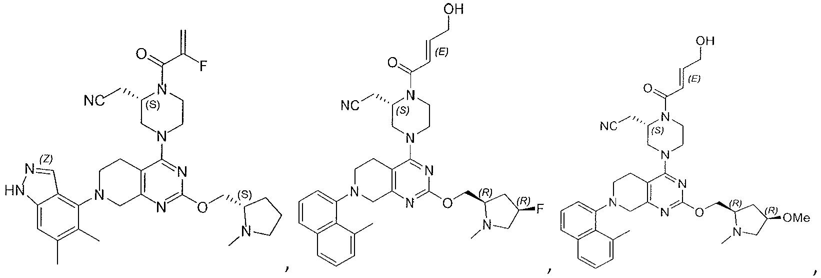 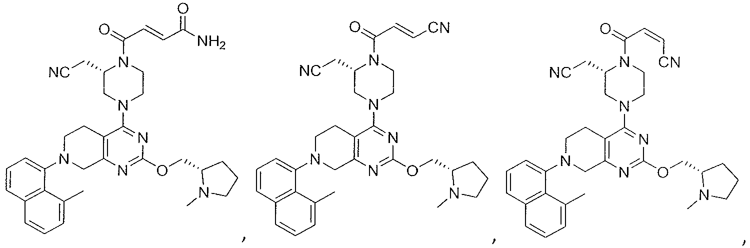 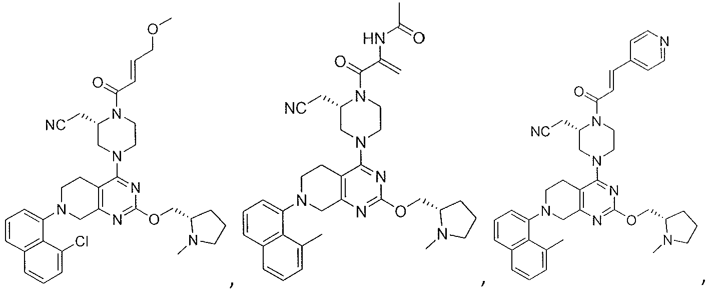 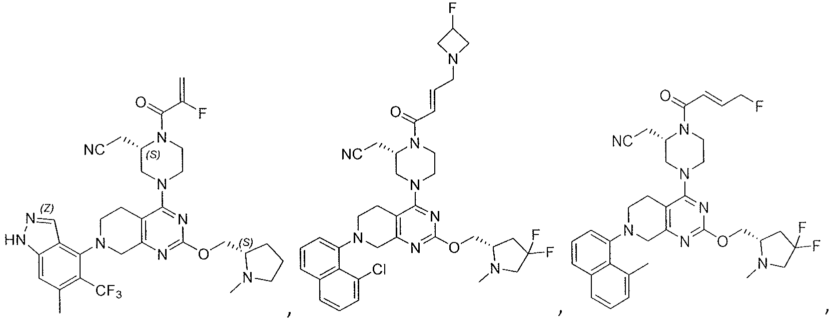 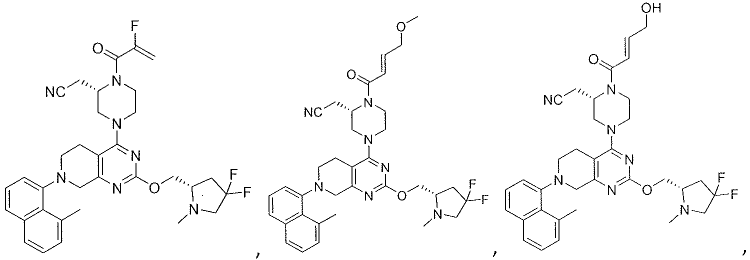 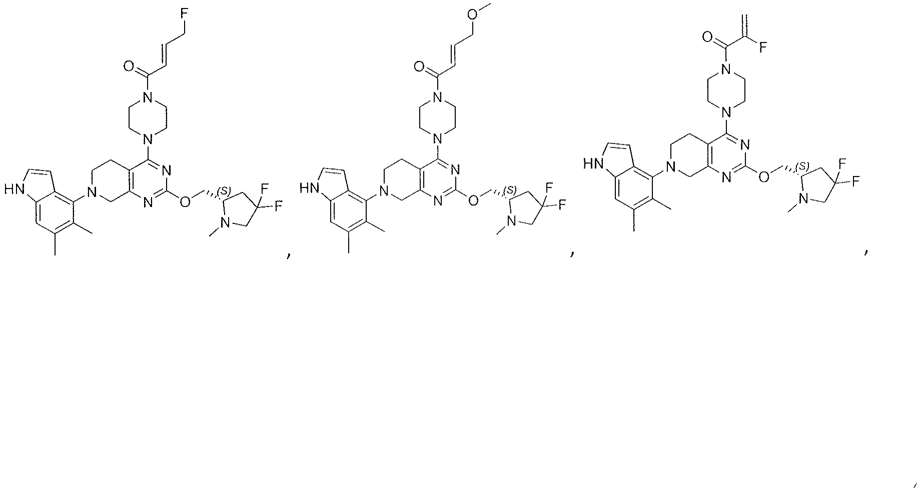 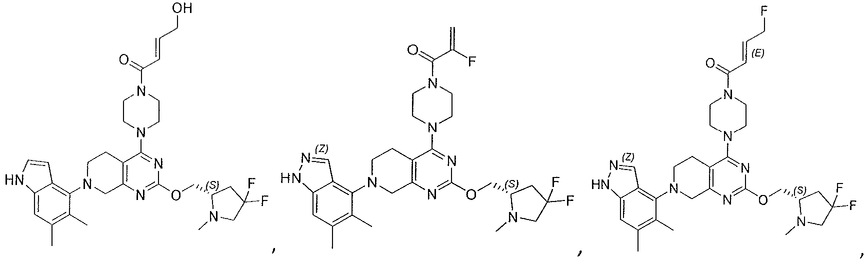 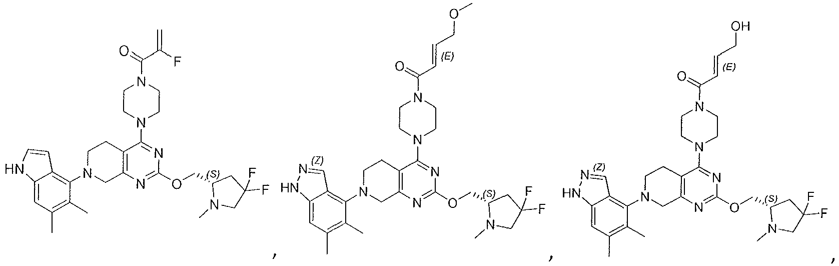 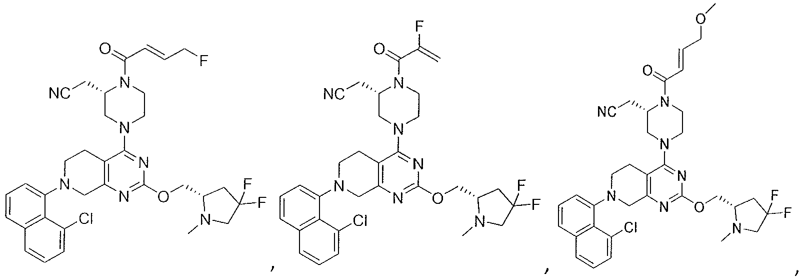 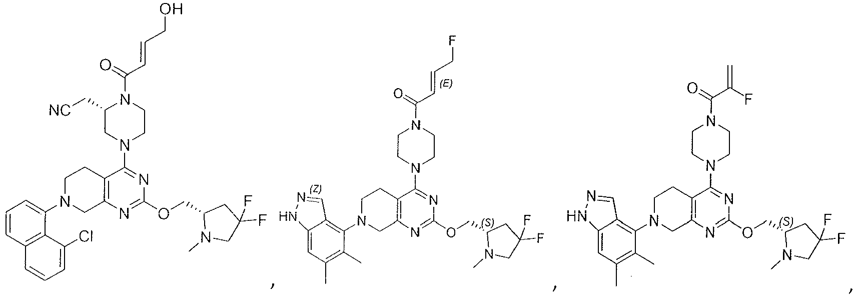 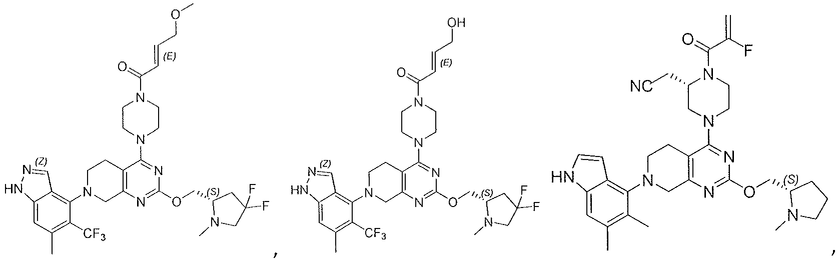 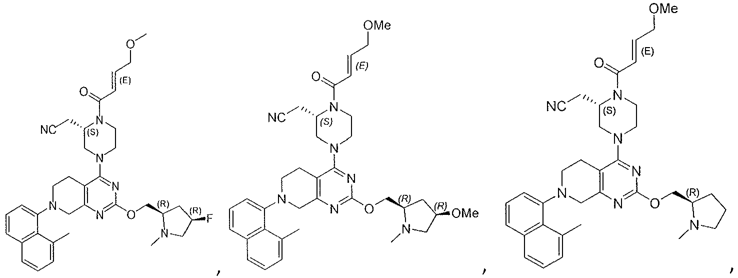 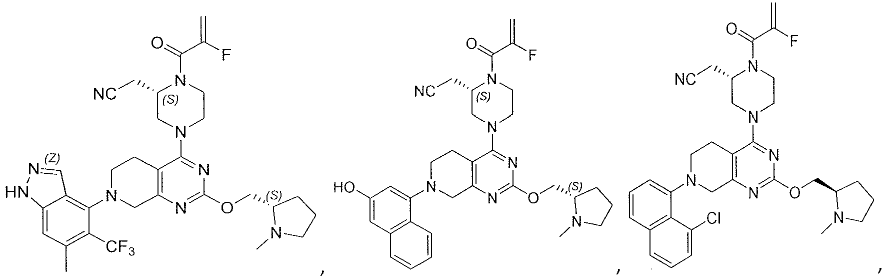 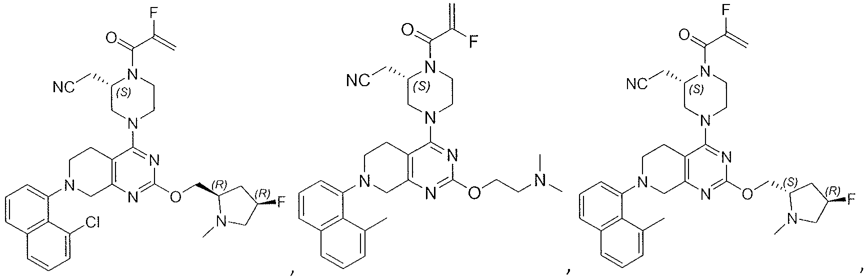 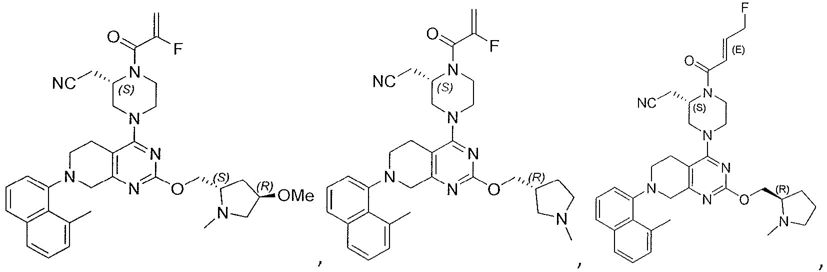 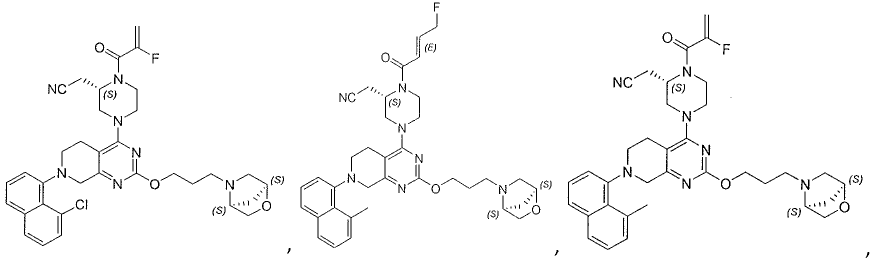 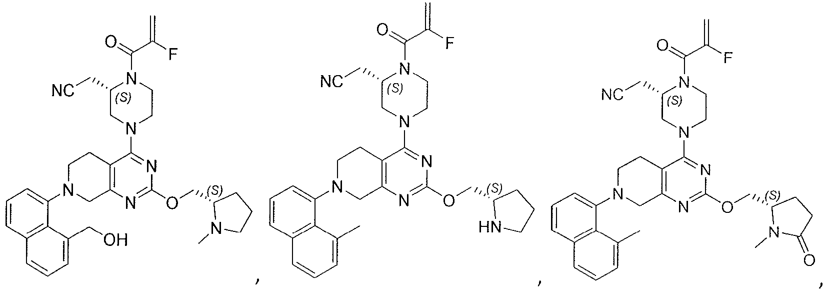 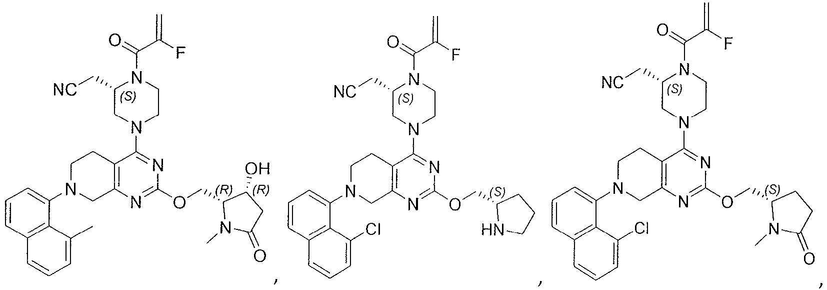 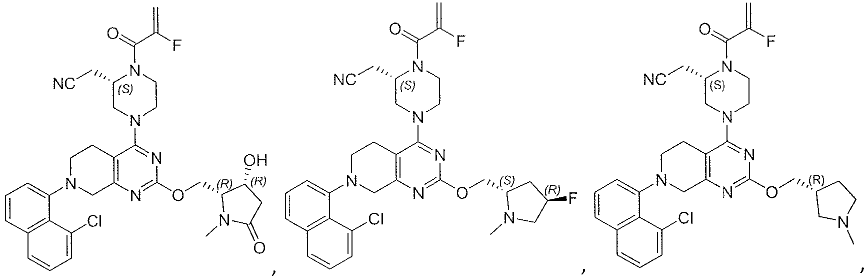 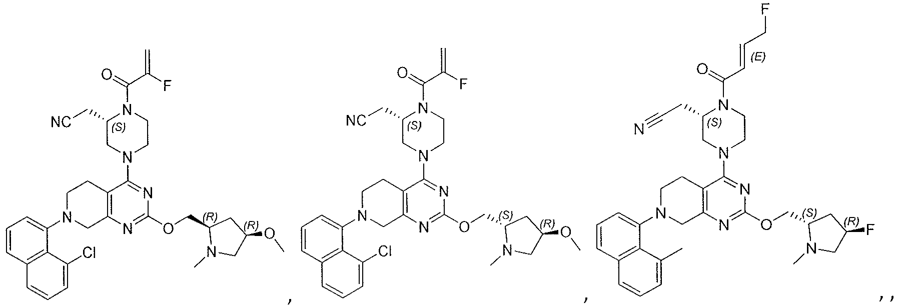 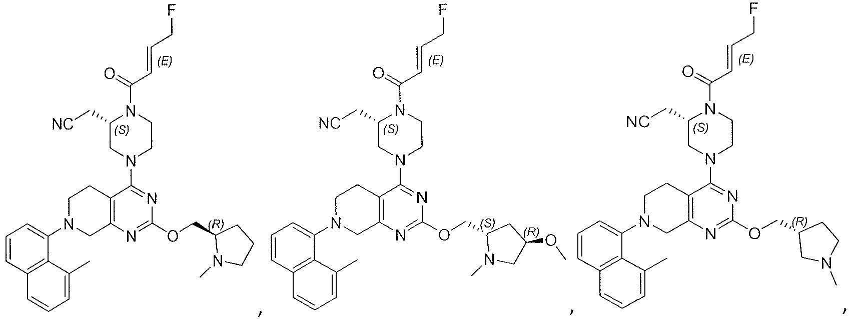 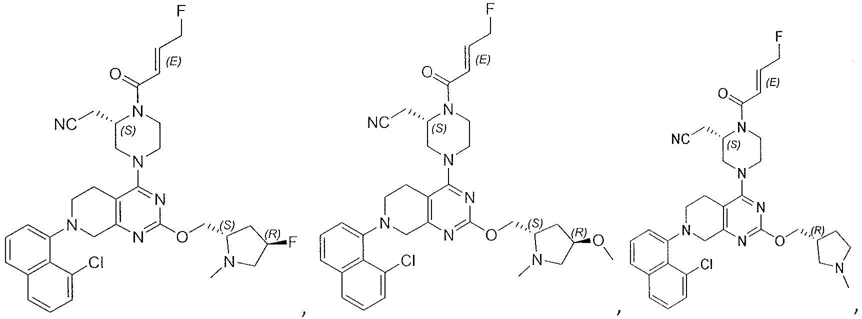 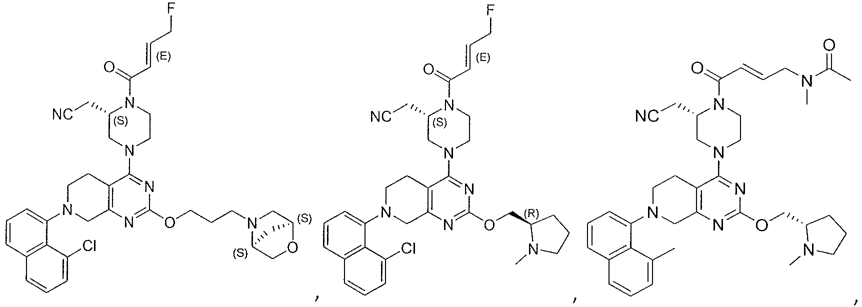 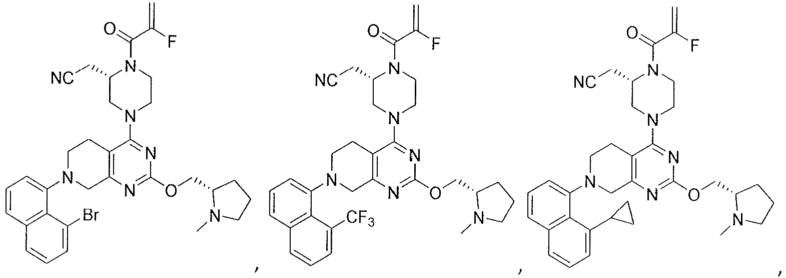 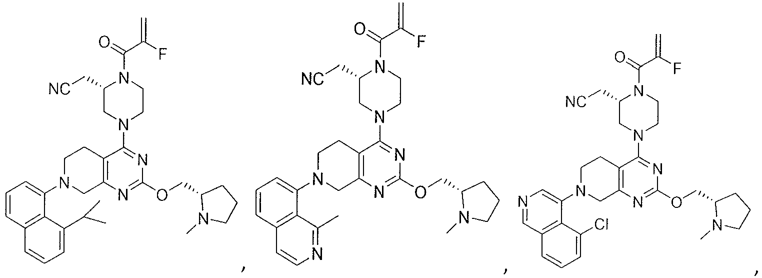 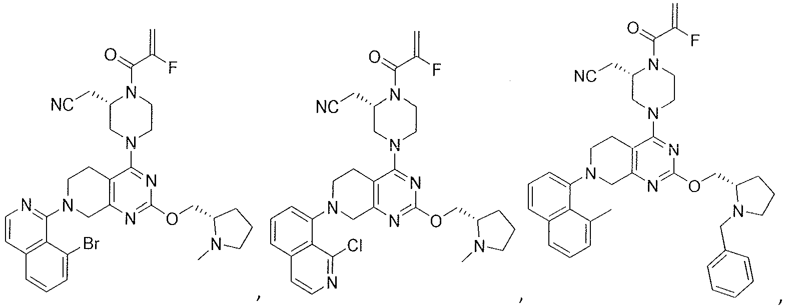 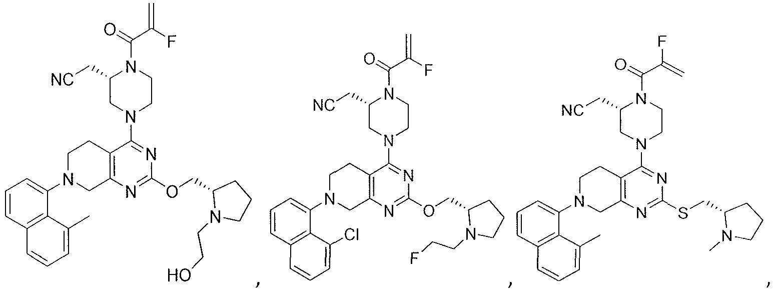 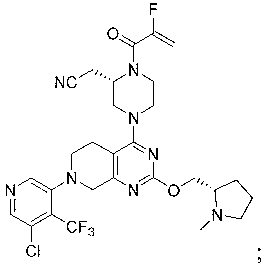 or a pharmaceutically acceptable salt thereof.
57. The method of clause 1, wherein the KRas G12C inhibitor is selected from the group consisting of: 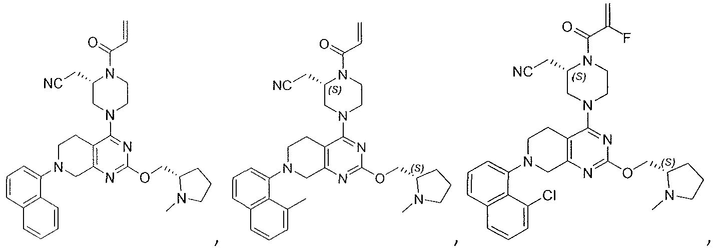 and 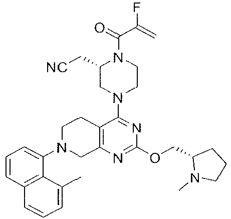 and pharmaceutically acceptable salts thereof.
58. The method of clause 1, wherein the KRas G12C inhibitor is: 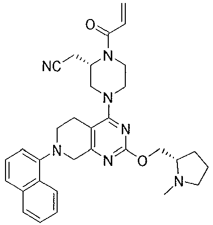 or a pharmaceutically acceptable salt thereof.
59. The method of clause 1, wherein the KRas G12C inhibitor is: 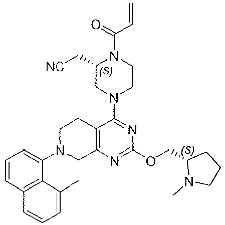 or a pharmaceutically acceptable salt thereof.
60. The method of clause 1, wherein the KRas G12C inhibitor is: 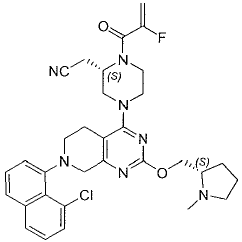 or a pharmaceutically acceptable salt thereof.
61. The method of clause 1, wherein the KRas G12C inhibitor is: 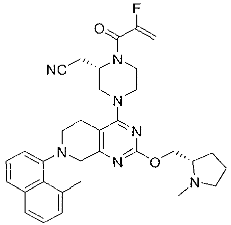 or a pharmaceutically acceptable salt thereof.
62. The method according to any one of clauses 1-61, wherein the Src-family kinase inhibitor is dasatinib, ponatinib, vandetanib, bosutinib, saracatinib, KX2-391, SU6656, PP1, WH-4-023 or KX-01.
63. The method of clause 62, wherein the Src-family kinase inhibitor is dasatinib, ponatinib, vandetanib, saracatinib or KX-01.
64. The method of clause 62, wherein the Src-family kinase inhibitor is dasatinib.
65. The method of clause 62, wherein the Src-family kinase inhibitor is ponatinib.
66. The method of clause 62, wherein the Src-family kinase inhibitor is vandetinib.
67. The method according to any one of clauses 1-66, wherein the Src-family kinase inhibitor and the KRAS G12C inhibitor are administered on the same day.
68. The method according to any one of clauses 1-66, wherein the Src-family kinase inhibitor and the KRAS G12C inhibitor are administered on different days.
69. The method according to any one of clauses 1-66, wherein the KRas G12C inhibitor is administered at a maximum tolerated dose.
70. The method according to any one of clauses 1-66, wherein the Src-family kinase inhibitor and the KRAS G12C inhibitor are each administered at a maximum tolerated dose.
71. The method according to any one of clauses 1-66, wherein the therapeutically effective amount of the combination of the Src-family kinase inhibitor and the KRAS G12C inhibitor results in an increased duration of overall survival, an increased duration of progression free survival, an increase in tumor growth regression, an increase in tumor growth inhibition or an increased duration of stable disease in the subjects relative to treatment with only the KRas G12C inhibitor.
72. A pharmaceutical composition, comprising a therapeutically effective amount of a combination of a Src-family kinase inhibitor and a KRas G12C inhibitor of Formula (I), Formula I-A or Formula I-B, and a pharmaceutically acceptable excipient.
73. A method for inhibiting KRas G12C activity in a cell, comprising contacting the cell in which inhibition of KRas G12C activity is desired with an effective amount of a Src-family kinase inhibitor and a KRas G12C inhibitor compound of a Formula (I), Formula I-A or Formula I-B, pharmaceutical compositions or pharmaceutically acceptable salts thereof, wherein the Src-family kinase inhibitor synergistically increases the sensitivity of the cancer cells to the KRas G12C inhibitor.
74. The method according to any one of clauses 1-66, wherein the Src-family kinase inhibitor synergistically increases the sensitivity of the cancer cells to the KRas G12C inhibitor.
75. A method for increasing the sensitivity of a cancer cell to a KRas G12C inhibitor compound of Formula (I), Formula I-A or Formula I-B comprising administering to a subject undergoing KRas G12C treatment with a compound of Formula (I), Formula I-A or Formula I-B or a pharmaceutically acceptable salt thereof, alone or combined with a pharmaceutically acceptable carrier, excipient or diluents, a therapeutically effective amount of a Src-family kinase inhibitor, wherein the Src-family kinase inhibitor synergistically increases the sensitivity of the cancer cell to the KRas G12C inhibitor.
76. The method according to any one of clauses 1-71 and 73-75, wherein the therapeutically effective amount of the KRas G12C inhibitor in the combination is between about 0.01 to 100 mg/kg per day.
77. The method of clause 76, wherein the therapeutically effective amount of the KRas G12C inhibitor in the combination is between about 0.1 to 50 mg/kg per day.
78. The method according to any one of clauses 1-71 and 73-77, wherein the therapeutically effective amount of the Src-family kinase inhibitor in the combination is between about 0.01 to 100 mg/kg per day.
79. The method of clause 78, wherein the therapeutically effective amount of the Src-family kinase inhibitor in the combination is between about 0.1 to 50 mg/kg per day.
80. The method according to any one of clauses 1-71 and 73-79, wherein the cancer is selected from the group consisting of Cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma; Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Kaposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor (nephroblastoma), lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Biliary tract: gall bladder carcinoma, ampullary carcinoma, cholangiocarcinoma; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma (pinealoma), glioblastoma multiform, oligodendroglioma, schwannoma, retinoblastoma, congenital tumors), spinal cord neurofibroma, meningioma, glioma, sarcoma); Gynecological: uterus (endometrial 'carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma); Hematologic: blood (myeloid leukemia (acute and chronic), acute lymphoblastic leukemia, chronic lymphocytic leukemia, myeloproliferative diseases, multiple myeloma, myelodysplastic syndrome), Hodgkin's disease, non-Hodgkin's lymphoma (malignant lymphoma); Skin: malignant melanoma, basal cell carcinoma, squamous cell carcinoma, Kaposi's sarcoma, moles dysplastic nevi, lipoma, angioma, dermatofibroma, keloids, psoriasis; and Adrenal glands: neuroblastoma.
81. The method according to any one of clauses 1-71 and 73-80, wherein the cancer wherein the cancer is a KRas G12C-associated cancer.
82. The method of clause 81, wherein the cancer is non-small cell lung cancer.
83. A kit comprising the pharmaceutical composition of clause 72 for treating KRas G12C cancer in a subject.
84. A kit comprising: a) a pharmaceutical composition comprising a Src-family kinase inhibitor and b) a pharmaceutical composition comprising a KRas G12C inhibitor of: 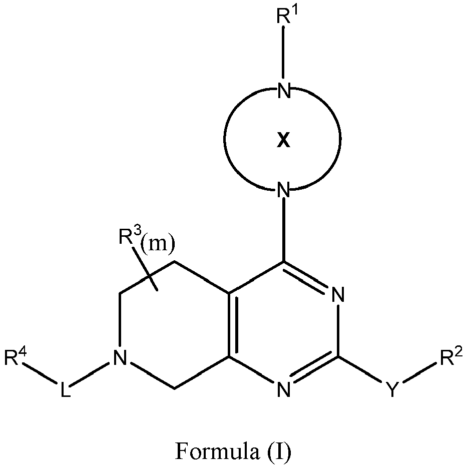 or a pharmaceutically acceptable salt thereof:
   wherein:
   X is a 4-12 membered saturated or partially saturated monocyclic, bridged or spirocyclic ring, wherein the saturated or partially saturated monocyclic ring is optionally substituted with one or more R⁸;
   Y is a bond, O, S or NR⁵;
   R¹ is -C(O)C(R^{A}) 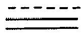 C(R^{B})ₚ or -SO₂C(R^{A}) 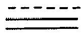 C(R^{B})ₚ;
   R² is hydrogen, alkyl, hydroxyalkyl, dihydroxyalkyl, alkylaminylalkyl, dialkylaminylalkyl, -Z-NR⁵R¹⁰, heterocyclyl, heterocyclylalkyl, aryl, heteroaryl, or heteroarylalkyl, wherein each of the Z, heterocyclyl, heterocyclylalkyl, aryl, heteroaryl, and heteroarylalkyl may be optionally substituted with one or more R⁹;
   each Z is C1 - C4 alkylene;
   each R³ is independently C1 - C3 alkyl, oxo, haloalkyl, hydroxyl or halogen;
   L is a bond, -C(O)-, or C1 - C3 alkylene;
   R⁴ is hydrogen, cycloalkyl, heterocyclyl, aryl, aralkyl or heteroaryl, wherein each of the cycloalkyl, heterocyclyl, aryl, aralkyl and heteroaryl may be optionally substituted with one or more R⁶, R⁷ or R⁸;
   each R⁵ is independently hydrogen or C1 - C3 alkyl;
   R⁶ is cycloalkyl, heterocyclyl, heterocyclylalkyl, aryl, or heteroaryl, wherein each of the cycloalkyl, heterocyclyl, aryl, or heteroaryl may be optionally substituted with one or more R⁷;
   each R⁷ is independently halogen, hydroxyl, C1 - C6 alkyl, cycloalkyl, alkoxy, haloalkyl, amino, cyano, heteroalkyl, hydroxyalkyl or Q-haloalkyl, wherein Q is O or S;
   R⁸ is oxo, C1 - C3 alkyl, C2 - C4 alkynyl, heteroalkyl, cyano, -C(O)OR⁵, -C(O)N(R⁵)₂, - N(R⁵)₂, wherein the C1 - C3 alkyl may be optionally substituted with cyano, halogen, - OR⁵, -N(R⁵)₂, or heteroaryl;
   each R⁹ is independently hydrogen, oxo, acyl, hydroxyl, hydroxyalkyl, cyano, halogen, C1 - C6 alkyl, aralkyl, haloalkyl, heteroalkyl, cycloalkyl, heterocyclylalkyl, alkoxy,
   dialkylaminyl, dialkylamidoalkyl, or dialkylaminylalkyl, wherein the C1 - C6 alkyl may be optionally substituted with cycloalkyl;
   each R¹⁰ is independently hydrogen, acyl, C1 - C3 alkyl, heteroalkyl or hydroxyalkyl;
   R¹¹ is haloalkyl;
   R^{A} is absent, hydrogen, deuterium, cyano, halogen, C1 - C-3 alkyl, haloalkyl, heteroalkyl, -C(O)N(R⁵)₂, or hydroxyalkyl;
   each R^{B} is independently hydrogen, deuterium, cyano, C1 - C3 alkyl, hydroxyalkyl, heteroalkyl, C1 - C3 alkoxy, halogen, haloalkyl, -ZNR⁵R¹¹, -C(O)N(R⁵)₂, -NHC(O)C1 - C3 alkyl, -CH₂NHC(O)C1 - C3 alkyl, heteroaryl, heteroarylalkyl, dialkylaminylalkyl, or heterocyclylalkyl wherein the heterocyclyl portion is substituted with one or more substituents independently selected from halogen, hydroxyl, alkoxy and C1 - C3 alkyl, wherein the heteroaryl or the heteroaryl portion of the heteroarylalkyl is optionally substituted with one or more R⁷;
   when 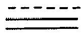 is a triple bond then R^{A} is absent, R^{B} is present and p equals one, or when 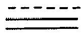 is a double bond then R^{A} is present, R^{B} is present and p equals two, or R^{A}, R^{B} and the carbon atoms to which they are attached form a 5-8 membered partially saturated cycloalkyl optionally substituted with one or more R⁷;
   m is zero or an integer between 1 and 2; and
   p is one or two.
      or 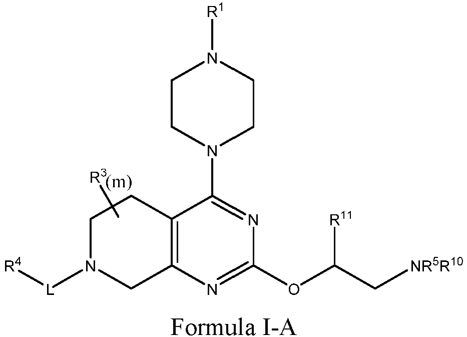
      and pharmaceutically acceptable salts thereof, wherein R¹, R³, R⁴, R⁵, R¹⁰, L and m are as defined for Formula I, R¹¹ is hydrogen, methyl or hydroxyalkyl, and the piperidinyl ring is optionally substituted with R⁸ wherein R⁸ is as defined for Formula I
         or 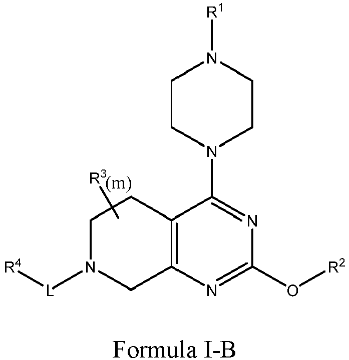
      and pharmaceutically acceptable salts thereof, wherein R¹, R³, R⁴, L and m are as defined for Formula I, R² is heterocyclylalkyl optionally substituted with one or more R⁹ where R⁹ is as defined for Formula I, and the piperazinyl ring is optionally substituted with R⁸, where R⁸ is as defined for Formula I,
      for treating a KRas G12C cancer in a subject.
85. The kit according to clause 83 or 84, further comprising an insert with instructions for administration of the pharmaceutical composition(s).

## Claims

1. A combination for use in a method of treating cancer, the combination comprising a Src-family kinase inhibitor and a KRAS G12C inhibitor of formula (I): 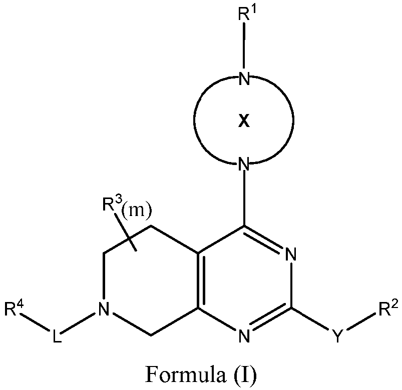 or a pharmaceutically acceptable salt thereof:
wherein:
X is a 4-12 membered saturated or partially saturated monocyclic, bridged or spirocyclic ring, wherein the saturated or partially saturated monocyclic ring is optionally substituted with one or more R⁸;
Y is a bond, O, S or NR⁵;
R¹ is -C(O)C(R^{A}) 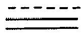 C(R^{B})ₚ or -SO₂C(R^{A}) 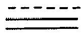 C(R^{B})ₚ;
R² is hydrogen, alkyl, hydroxyalkyl, dihydroxyalkyl, alkylaminylalkyl, dialkylaminylalkyl, -Z-NR⁵R¹⁰, heterocyclyl, heterocyclylalkyl, aryl, heteroaryl, or heteroarylalkyl, wherein each of the Z, heterocyclyl, heterocyclylalkyl, aryl, heteroaryl, and heteroarylalkyl may be optionally substituted with one or more R⁹;
each Z is C1 - C4 alkylene;
each R³ is independently C1 - C3 alkyl, oxo, haloalkyl, hydroxyl or halogen;
L is a bond, -C(O)-, or C1 - C3 alkylene;
R⁴ is hydrogen, cycloalkyl, heterocyclyl, aryl, aralkyl or heteroaryl, wherein each of the cycloalkyl, heterocyclyl, aryl, aralkyl and heteroaryl may be optionally substituted with one or more R⁶, R⁷ or R⁸;
each R⁵ is independently hydrogen or C1 - C3 alkyl;
R⁶ is cycloalkyl, heterocyclyl, heterocyclylalkyl, aryl, or heteroaryl, wherein each of the cycloalkyl, heterocyclyl, aryl, or heteroaryl may be optionally substituted with one or more R⁷;
each R⁷ is independently halogen, hydroxyl, C1 - C6 alkyl, cycloalkyl, alkoxy, haloalkyl, amino, cyano, heteroalkyl, hydroxyalkyl or Q-haloalkyl, wherein Q is O or S;
R⁸ is oxo, C1 - C3 alkyl, C2 - C4 alkynyl, heteroalkyl, cyano, -C(O)OR⁵, - C(O)N(R⁵)₂, -N(R⁵)₂, wherein the C1 - C3 alkyl may be optionally substituted with cyano, halogen, -OR⁵, -N(R⁵)₂, or heteroaryl;
each R⁹ is independently hydrogen, oxo, acyl, hydroxyl, hydroxyalkyl, cyano, halogen, C1 - C6 alkyl, aralkyl, haloalkyl, heteroalkyl, cycloalkyl, heterocyclylalkyl, alkoxy, dialkylaminyl, dialkylamidoalkyl, or dialkylaminylalkyl, wherein the C1 - C6 alkyl may be optionally substituted with cycloalkyl;
each R¹⁰ is independently hydrogen, acyl, C1 - C3 alkyl, heteroalkyl or hydroxyalkyl;
R¹¹ is haloalkyl;
R^{A} is absent, hydrogen, deuterium, cyano, halogen, C1 - C-3 alkyl, haloalkyl, heteroalkyl, -C(O)N(R⁵)₂, or hydroxyalkyl;
each R^{B} is independently hydrogen, deuterium, cyano, C1 - C3 alkyl, hydroxyalkyl, heteroalkyl, C1 - C3 alkoxy, halogen, haloalkyl, -ZNR⁵R¹¹, -C(O)N(R⁵)₂, - NHC(O)C1 - C3 alkyl, -CH₂NHC(O)C1 - C3 alkyl, heteroaryl, heteroarylalkyl, dialkylaminylalkyl, or heterocyclylalkyl wherein the heterocyclyl portion is substituted with one or more substituents independently selected from halogen, hydroxyl, alkoxy and C1 - C3 alkyl, wherein the heteroaryl or the heteroaryl portion of the heteroarylalkyl is optionally substituted with one or more R⁷;
when 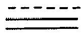 is a triple bond then R^{A} is absent, R^{B} is present and p equals one,
or when 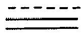 is a double bond then R^{A} is present, R^{B} is present and p equals two, or R^{A}, R^{B} and the carbon atoms to which they are attached form a 5-8 membered partially saturated cycloalkyl optionally substituted with one or more R⁷;
m is zero or an integer between 1 and 2; and
p is one or two.

2. The combination for use of claim 1, wherein the KRas G12C inhibitor is: 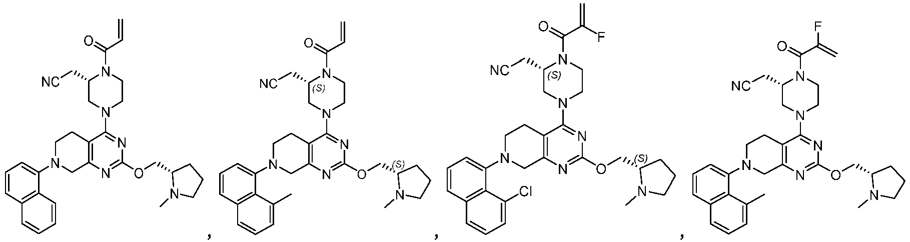 or a pharmaceutically acceptable salt thereof.

3. The combination for use according to claim 1 or claim 2, wherein the Src-family kinase inhibitor is dasatinib, ponatinib, vandetanib, bosutinib, saracatinib, KX2-391, SU6656, PP1, WH-4-023 or KX-01, optionally wherein the Src-family kinase inhibitor is dasatinib, ponatinib, vandetanib, saracatinib or KX-01, optionally wherein the Src-family kinase inhibitor is dasatinib, ponatinib or vandetinib.

4. The combination for use according to any one of the preceding claims, wherein the Src-family kinase inhibitor and the KRAS G12C inhibitor are administered on the same day or on different days.

5. The combination for use according to any one of the preceding claims, wherein the KRas G12C inhibitor is administered at a maximum tolerated dose, optionally wherein the Src-family kinase inhibitor and the KRAS G12C inhibitor are each administered at a maximum tolerated dose.

6. The combination for use according to any one of the preceding claims, wherein the combination of the Src-family kinase inhibitor and the KRAS G12C inhibitor results in an increased duration of overall survival, an increased duration of progression free survival, an increase in tumor growth regression, an increase in tumor growth inhibition or an increased duration of stable disease in the subjects relative to treatment with only the KRas G12C inhibitor.

7. The combination for use according to any one of the preceding claims, wherein the method of treatment includes increasing the sensitivity of a cancer cell to a KRas G12C inhibitor compound of Formula (I) comprising administering to a subject undergoing KRas G12C treatment with the compound of Formula (I) or a pharmaceutically acceptable salt thereof, alone or combined with a pharmaceutically acceptable carrier, excipient or diluents, the Src-family kinase inhibitor, wherein the Src-family kinase inhibitor synergistically increases the sensitivity of the cancer cell to the KRas G12C inhibitor.

8. The combination for use according to any one of the preceding claims, wherein the amount of the KRas G12C inhibitor in the combination is between about 0.01 to 100 mg/kg per day, optionally wherein the amount of the KRas G12C inhibitor in the combination is between about 0.1 to 50 mg/kg per day.

9. The combination for use according to any one of the preceding claims, wherein the amount of the Src-family kinase inhibitor in the combination is between about 0.01 to 100 mg/kg per day, optionally wherein the therapeutically effective amount of the Src-family kinase inhibitor in the combination is between about 0.1 to 50 mg/kg per day.

10. The combination for use according to any one of the preceding claims, wherein the cancer is selected from the group consisting of Cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma; Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Kaposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor (nephroblastoma), lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Biliary tract: gall bladder carcinoma, ampullary carcinoma, cholangiocarcinoma; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma (pinealoma), glioblastoma multiform, oligodendroglioma, schwannoma, retinoblastoma, congenital tumors), spinal cord neurofibroma, meningioma, glioma, sarcoma); Gynecological: uterus (endometrial carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma); Hematologic: blood (myeloid leukemia (acute and chronic), acute lymphoblastic leukemia, chronic lymphocytic leukemia, myeloproliferative diseases, multiple myeloma, myelodysplastic syndrome), Hodgkin's disease, non-Hodgkin's lymphoma (malignant lymphoma); Skin: malignant melanoma, basal cell carcinoma, squamous cell carcinoma, Kaposi's sarcoma, moles dysplastic nevi, lipoma, angioma, dermatofibroma, keloids, psoriasis; and Adrenal glands: neuroblastoma.

11. The combination for use according to any one of claims 1 to 9, wherein the cancer wherein the cancer is a KRas G12C-associated cancer.

12. The combination for use according to any one of claims 1 to 9, wherein the cancer is non-small cell lung cancer.

13. The combination for use according to any one of the preceding claims, provided in the form of a pharmaceutical composition comprising a pharmaceutically acceptable excipient.

14. A kit for use in a method of treating a KRas G12C cancer, the kit comprising:
a) a pharmaceutical composition comprising a Src-family kinase inhibitor; and
b) a pharmaceutical composition comprising a KRas G12C inhibitor of: 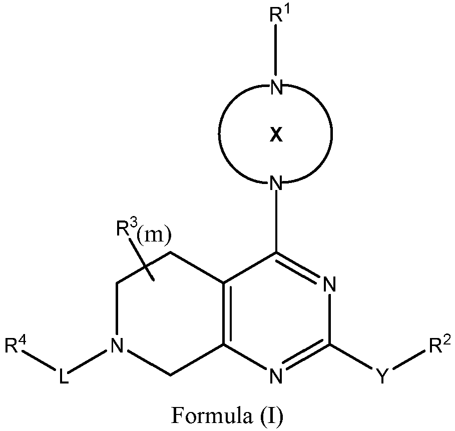 or a pharmaceutically acceptable salt thereof:
wherein:
X is a 4-12 membered saturated or partially saturated monocyclic, bridged or spirocyclic ring, wherein the saturated or partially saturated monocyclic ring is optionally substituted with one or more R⁸;
Y is a bond, O, S or NR⁵
R¹ is -C(O)C(R^{A}) 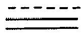 C(R^{B})ₚ or -SO₂C(R^{A}) 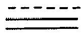 C(R^{B})ₚ;
R² is hydrogen, alkyl, hydroxyalkyl, dihydroxyalkyl, alkylaminylalkyl, dialkylaminylalkyl, -Z-NR⁵R¹⁰, heterocyclyl, heterocyclylalkyl, aryl, heteroaryl, or heteroarylalkyl, wherein each of the Z, heterocyclyl, heterocyclylalkyl, aryl, heteroaryl, and heteroarylalkyl may be optionally substituted with one or more R⁹;
each Z is C1 - C4 alkylene;
each R³ is independently C1 - C3 alkyl, oxo, haloalkyl, hydroxyl or halogen;
L is a bond, -C(O)-, or C1 - C3 alkylene;
R⁴ is hydrogen, cycloalkyl, heterocyclyl, aryl, aralkyl or heteroaryl, wherein each of the cycloalkyl, heterocyclyl, aryl, aralkyl and heteroaryl may be optionally substituted with one or more R⁶, R⁷ or R⁸;
each R⁵ is independently hydrogen or C1 - C3 alkyl;
R⁶ is cycloalkyl, heterocyclyl, heterocyclylalkyl, aryl, or heteroaryl, wherein each of the cycloalkyl, heterocyclyl, aryl, or heteroaryl may be optionally substituted with one or more R⁷;
each R⁷ is independently halogen, hydroxyl, C1 - C6 alkyl, cycloalkyl, alkoxy, haloalkyl, amino, cyano, heteroalkyl, hydroxyalkyl or Q-haloalkyl, wherein Q is O or S;
R⁸ is oxo, C1 - C3 alkyl, C2 - C4 alkynyl, heteroalkyl, cyano, -C(O)OR⁵, - C(O)N(R⁵)₂, -N(R⁵)₂, wherein the C1 - C3 alkyl may be optionally substituted with cyano, halogen, -OR⁵, -N(R⁵)₂, or heteroaryl;
each R⁹ is independently hydrogen, oxo, acyl, hydroxyl, hydroxyalkyl, cyano, halogen, C1 - C6 alkyl, aralkyl, haloalkyl, heteroalkyl, cycloalkyl, heterocyclylalkyl, alkoxy, dialkylaminyl, dialkylamidoalkyl, or dialkylaminylalkyl, wherein the C1 - C6 alkyl may be optionally substituted with cycloalkyl;
each R¹⁰ is independently hydrogen, acyl, C1 - C3 alkyl, heteroalkyl or hydroxyalkyl;
R¹¹ is haloalkyl;
R^{A} is absent, hydrogen, deuterium, cyano, halogen, C1 - C-3 alkyl, haloalkyl, heteroalkyl, -C(O)N(R⁵)₂, or hydroxyalkyl;
each R^{B} is independently hydrogen, deuterium, cyano, C1 - C3 alkyl, hydroxyalkyl, heteroalkyl, C1 - C3 alkoxy, halogen, haloalkyl, -ZNR⁵R¹¹, -C(O)N(R⁵)₂, - NHC(O)C1 - C3 alkyl, -CH₂NHC(O)C1 - C3 alkyl, heteroaryl, heteroarylalkyl, dialkylaminylalkyl, or heterocyclylalkyl wherein the heterocyclyl portion is substituted with one or more substituents independently selected from halogen, hydroxyl, alkoxy and C1 - C3 alkyl, wherein the heteroaryl or the heteroaryl portion of the heteroarylalkyl is optionally substituted with one or more R⁷;
when 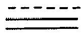 is a triple bond then R^{A} is absent, R^{B} is present and p equals one,
or when 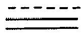 is a double bond then R^{A} is present, R^{B} is present and p equals two, or R^{A}, R^{B} and the carbon atoms to which they are attached form a 5-8 membered partially saturated cycloalkyl optionally substituted with one or more R⁷;
m is zero or an integer between 1 and 2; and
p is one or two.
or 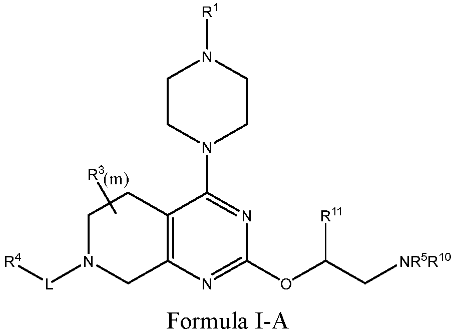
and pharmaceutically acceptable salts thereof, wherein R¹, R³, R⁴, R⁵, R¹⁰, L and m are as defined for Formula I, R¹¹ is hydrogen, methyl or hydroxyalkyl, and the piperidinyl ring is optionally substituted with R⁸ wherein R⁸ is as defined for Formula I
or 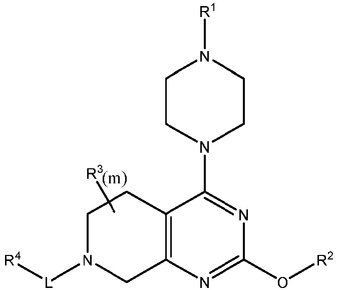 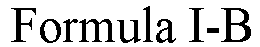
and pharmaceutically acceptable salts thereof, wherein R¹, R³, R⁴, L and m are as defined for Formula I, R² is heterocyclylalkyl optionally substituted with one or more R⁹ where R⁹ is as defined for Formula I, and the piperazinyl ring is optionally substituted with R⁸, where R⁸ is as defined for Formula I.

15. The kit for use according to claim 14, further comprising an insert with instructions for administration of the pharmaceutical composition(s).
